# EUROPEAN PATENT APPLICATION

(11) **EP 4 768 574 A2**
(43) Date of publication of application: **01.07.2026**
(21) Application number: 26161819.3
(22) Date of filing: 28.05.2020
(51) Int. Cl.: C12N 5/10

(54) **METHODS AND COMPOSITIONS FOR GENERATING DOMINANT ALLELES USING GENOME EDITING**

(30) Priority: 29.05.2019 US 201962854142 P; 14.08.2019 US 201962886726 P; 14.08.2019 US 201962886732 P
(62) Divisional of application: 20815356.9
(71) Applicant: Monsanto Technology LLC, St. Louis, MO 63167 (US)
(72) Inventor: CARGILL, Edward James, St. Louis, MO 63167 (US); EUDY, Douglas Michael, St. Louis, MO 63167 (US); KOURANOV, Andrei Y., St. Louis, MO 63167 (US); LAWRENCE, Richard Joseph, St. Louis, MO 63167 (US); SLEWINSKI, Thomas L., St. Louis, MO 63167 (US); SHULTZ, Randy, St. Louis, MO 63167 (US); TO, PokChun Jennifer, St. Louis, MO 63167 (US); YANG, Samuel Sukhwan, St. Louis, MO 63167 (US); ZHANG, Yuanji, St. Louis, MO 63167 (US)
(74) Representative: BIP Patents

(57) **Abstract**

The present disclosure provides methods and compositions for generating dominant alleles using targeted editing techniques. Also provided are modified chromosomes, cells, tissues, and plants comprising modified dominant allele.

## Description

### CROSS-REFERENCE TO RELATED APPLICATIONS

This application claims the benefit of U.S. Provisional Application No. 62/854,142, filed May 29, 2019, U.S. Provisional Application No. 62/886,726, filed August 14, 2019, and U.S. Provisional Application No. 62/886,732, filed August 14, 2019, all of which are incorporated by reference in their entirties herein.

### FIELD

The present disclosure relates to methods and compositions for generating dominant alleles via targeted editing of genomes.

### INCORPORATION OF SEQUENCE LISTING

A sequence listing contained in the file named "P34497WO00_SL.TXT" which is 172,842 bytes (measured in MS-Windows^{®}) and created on May 28, 2020, is filed electronically herewith and incorporated by reference in its entirety.

### BACKGROUND

Dominant alleles are alleles that mask the contribution of a second allele at the same locus. A dominant allele can be a dominant negative allele or a dominant positive allele. Dominant negative alleles, or antimorphs, are alleles that act in opposition to normal allelic function. For example, a dominant negative allele often abrogates the normal function of an allele in a heterozygous or homozygous state. Dominant positive alleles can increase normal gene function (*e.g.,* a hypermorph) and/or provide broadened or new functions for a gene (*e.g.,* a neomorph).

Naturally occurring and random mutagenesis techniques (*e.g*., ethyl methyl sulfonate and T-DNA insertions) have been used to generate mutations in a variety of cell types. However, dominant mutations occur at low frequencies and are difficult to obtain in a given gene of interest. Therefore, methods and compositions to selectively edit a genome to create a dominant negative allele or a dominant positive allele would be beneficial.

### SUMMARY

In one aspect, this disclosure provides a method of generating a dominant negative allele of a gene in a cell comprising inverting a portion of the gene using a targeted editing technique, generating an antisense RNA transcript capable of triggering suppression of an unmodified allele.

In one aspect, this disclosure provides a method of generating a dominant negative allele of a gene in a cell comprising deleting a portion of a chromosome between a first gene region and a second gene region using a targeted editing technique, where an antisense RNA transcript of the first gene region is generated following the deletion of the portion of the chromosome.

In one aspect, this disclosure provides a method of generating a dominant negative allele of a gene in one or more cells comprising: (a) inducing a first double-stranded break and a second double-stranded break flanking a targeted region of the gene; (b) identifying one or more cells comprising an inversion of the targeted region of the gene, where the inversion results in the production of an antisense RNA transcript from the targeted region; and (c) selecting one or more cells comprising the inversion of the targeted region of the gene.

In one aspect, this disclosure provides a method of reducing the expression of a protein in a cell comprising: (a) inducing a first double-stranded break and a second double-stranded break flanking a targeted region of a chromosome; and (b) identifying one or more cells comprising an inversion in the targeted region of the chromosome, where expression of the protein is reduced as compared to a control cell that does not comprise the inversion in the targeted region.

In one aspect, this disclosure provides a method comprising: (a) identifying a chromosomal region comprising a first gene region comprising a first promoter and a first coding region, and a second gene region comprising a second promoter and a second coding region, where the first coding region and the second coding region are separated by an intervening region, and where the first promoter and the second promoter are positioned in opposite orientations: (b) inducing a first double-stranded break and a second double-stranded break flanking a targeted region; (c) identifying one or more cells comprising a deletion of the targeted region of the chromosome: and (d) selecting one or more cells comprising the deletion of the targeted region of the chromosome.

In one aspect, this disclosure provides a method of reducing the expression of a gene in at least one cell comprising: (a) inducing a double-stranded break using a targeted editing technique at a target site of the gene; (b) inserting a donor sequence at the double-stranded break, where the donor sequence comprises a tissue-specific or tissue-preferred promoter, and where the donor sequence is inserted into the target site such that the tissue-specific or tissue-preferred promoter is in reverse orientation as compared to the gene; and (b) identifying at least one cell comprising the insertion of the donor sequence in reverse orientation, where expression of the gene is reduced as compared to a control cell that does not comprise the insertion of the donor sequence.

In one aspect, this disclosure provides a method of modifying gene expression comprising: (a) inducing a double-stranded break using a targeted editing technique at a target site; (b) inserting a donor sequence at the double-stranded break, where the donor sequence comprises an endogenous (e.g., promoter, enhancer or promoter/enhancer fragment) or designed element capable of inducing increased or ectopic expression of the gene; and (c) identifying at least one cell comprising the donor sequence, where expression of the target gene is increased in at least one tissue as compared to a control cell that does not comprise the donor sequence.

In one aspect, this disclosure provides a method of enhancing gene expression comprising: (a) inducing a double-stranded break using a targeted editing technique at a target site; (b) inserting a donor sequence at the double-stranded break, where the donor sequence comprises an endogenous (e.g., promoter, enhancer or promoter/enhancer fragment) or designed element capable of inducing increased or ectopic expression of the gene; and (c) identifying at least one cell comprising the donor sequence, where expression of the target gene is increased in at least one tissue as compared to a control cell that does not comprise the donor sequence.

In one aspect, this disclosure provides a method generating a dominant positive allele comprising: (a) inducing a double-stranded break using a targeted editing technique at a target site; (b) inserting a donor sequence at the double-stranded break, where the donor sequence comprises a sequence of an endogenous gene: and (c) identifying at least one cell comprising the donor sequence, where expression of the gene is increased in at least one tissue as compared to a control cell that does not comprise the donor sequence.

In one aspect, this disclosure provides a method of reducing expression of a gene in a cell comprising: (a) identifying a chromosomal region comprising a first gene region comprising a first promoter and a first coding region, and a second gene region comprising a second promoter and a second coding region, where the first coding region and the second coding region are separated by an intervening region, and where the first promoter and the second promoter are positioned in opposite orientations; (b) inducing a first double-stranded break and a second double-stranded break flanking a targeted region using a targeted editing technique, where the targeted region comprises the second coding region and the intervening region; and (c) identifying one or more cells comprising a deletion of the targeted region, where the second promoter generates at least one antisense RNA of the first coding region, and where expression of the first coding region is reduced as compared to a control cell that does not comprise the deletion of the targeted region.

In one aspect, this disclosure provides a method of reducing expression of a protein of interest in a cell comprising: (a) identifying a chromosomal region comprising a gene region encoding the protein of interest comprising a first promoter and a coding region for the protien, and a second chromosomal region comprising a second promoter and a intervening region, where the coding region for the protein of interest and the second promoter are separated by an the intervening region, and where the first promoter and the second promoter are positioned in opposite orientations; (b) inducing a first double-stranded break and a second double-stranded break flanking a the intervening region using a targeted editing technique; and (c) identifying one or more cells comprising a deletion of the intervening region, and where expression of the protein of interest is reduced as compared to a control cell that does not comprise the deletion of the intervening region.

In one aspect, this disclosure provides a method of generating an inversion in a targeted region of a gene comprising: (a) providing to one or more cells at least one RNA-guided nuclease or one or more vectors encoding at least one RNA-guided nuclease, where the at least one RNA-guided nuclease is capable of binding to at least 18, at least 19, at least 20, at least 21, at least 22, at least 23, at least 24, at least 25, or at least 26 consecutive nucleotides of a first target site and a second target site flanking the targeted region of the gene, where the first target site and the second target site are linked, where the at least one RNA-guided nuclease creates double-stranded breaks in the gene at the first target site and the second target site: (b) identifying one or more cells comprising an inversion in the targeted region of the gene, where the inversion results in the production of an antisense RNA transcript from the targeted region; and (c) selecting one or more cells comprising the inversion in the targeted region of the gene.

A method comprising: (a) identifying a chromosomal region comprising a first gene region comprising a first promoter and a first coding region, and a second gene region comprising a second promoter and a second coding region, where the first coding region and the second coding region are separated by an intervening region, and where the first promoter and the second promoter are positioned in opposite orientations; (b) providing to one or more cells at least one RNA-guided nuclease or one or more vectors encoding at least one RNA-guided nuclease, where the at least one RNA-guided nuclease is capable of binding to at least 18, at least 19, at least 20, at least 21, at least 22, at least 23, at least 24, at least 25, or at least 26 consecutive nucleotides of a first target site and a second target site flanking a targeted region of a chromosome, where the targeted region comprises the second encoding region and the intervening region, where the RNA-guided nuclease creates double-stranded breaks in the chromosome at the first target site and the second target site: (c) identifying one or more cells comprising a deletion of the targeted region; and (d) selecting one or more cells comprising the deletion of the targeted region.

In one aspect, this disclosure provides a method comprising: (a) providing to one or more cells at least one RNA-guided nuclease and at least one donor molecule, or one or more vectors encoding at least one RNA-guided nuclease and at least one donor molecule, where the at least one RNA-guided nuclease is capable of binding to at least 18, at least 19, at least 20, at least 21, at least 22, at least 23, at least 24, at least 25, or at least 26 consecutive nucleotides of a target site of at least one gene, where the donor molecule comprises a designed element, where the RNA-guided nuclease creates a double-stranded break at the target site, and where the designed element is inserted at the double-stranded break: (b) identifying one or more cells comprising an insertion of the designed element at the target site; and (c) selecting one or more cells comprising the insertion of the designed element at the target site.

In one aspect, this disclosure provides a method comprising: (a) providing to one or more cells at least one RNA-guided nuclease and at least one donor molecule, or one or more vectors encoding at least one RNA-guided nuclease and at least one donor molecule, where the at least one RNA-guided nuclease is capable of binding to at least 18, at least 19, at least 20, at least 21, at least 22, at least 23, at least 24, at least 25, or at least 26 consecutive nucleotides of a target site of at least one gene, where the donor molecule comprises a sequence encoding a tissue-specific or tissue-preferred promoter, where the RNA-guided nuclease creates a double-stranded break at the target site, and where the sequence encoding the tissue-specific or tissue-preferred promoter is inserted at the double-stranded break; (b) identifying one or more cells comprising the insertion of the sequence encoding the tissue-specific or tissue-preferred promoter at the target site such that the sequence encoding the tissue-specific or tissue-preferred promoter is in reverse orientation as compared to the gene; and (c) selecting one or more cells comprising the insertion of the sequence encoding the tissue-specific or tissue-preferred promoter at the target site.

In one aspect, this disclosure provides a method comprising: (a) providing to one or more cells one or more RNA-guided nucleases or one or more vectors encoding one or more RNA nucleases, where the one or more RNA-guided nucleases are capable of binding to at least 18, at least 19, at least 20, at least 21, at least 22, at least 23, at least 24, at least 25, or at least 26 consecutive nucleotides of a target site, where the one or more RNA-guided nucleases create a double-stranded break at the target site; (b) identifying at least one cell comprising an insertion or a deletion at the target site, where the insertion or deletion at the target site results in the generation of a dominant negative allele of the at least one gene; and (c) selecting one or more cells comprising the dominant negative allele of the at least one gene.

In one aspect, this disclosure provides a method comprising: (a) providing to one or more cells one or more RNA-guided nucleases or one or more vectors encoding one or more RNA nucleases, where the one or more RNA-guided nucleases are capable of binding to at least 18, at least 19, at least 20, at least 21, at least 22, at least 23, at least 24, at least 25, or at least 26 consecutive nucleotides of a target site, where the one or more RNA-guided nucleases create a double-stranded break at the target site; (b) identifying at least one cell comprising an insertion or a deletion at the target site, where the insertion or deletion at the target site results in the generation of a dominant positive allele of the at least one gene; and (c) selecting one or more cells comprising the dominant positive allele of the at least one gene.

In one aspect, this disclosure provides a method comprising: (a) generating a first double-stranded break (DSB) and a second DSB in a first allele of a gene in a cell using a targeted editing technique; (b) generating a third DSB in a second allele of the gene in the cell using a targeted editing technique; and (c) identifying a cell comprising an insertion of a region of the first allele in inverted orientation at the third DSB site in the second allele, thereby generating a modified second allele.

In one aspect, this disclosure provides a method of generating a dominant negative allele of a gene comprising using a targeted editing technique to introduce at least one non-coding RNA target site into the gene.

In one aspect, this disclosure provides a method of generating a dominant allele of a gene comprising using a targeted editing technique to introduce a nonsense mutation in the gene to create a truncated protein or protein with altered amino acid composition downstream of the mutation. This technique can also be paired with a second targeted editing mutation to restore the frame of normal amino acid sequence down stream of the initital mutation to create a nonsense region inside the poplypepetide.

In one aspect, this disclosure provides a method comprising: (a) providing to a cell an engineered pentatricopeptide repeat (PPR) protein or a vector encoding the engineered PPR protein operably linked to a promoter, where the engineered PPR protein is capable of binding to an RNA transcript of a target gene; (b) selecting one or more cells from step (a) expressing the engineered PPR protein; and (c) identifying one or more cells selected in step (b) comprising altered expression of the target gene.

In one aspect, this disclosure provides a method of generating a dominant negative allele of a gene in a cell comprising inserting an inverted copy of the gene, or a portion thereof, adjacent to a native copy of the gene using a targeted editing technique to generate an inverted repeat sequence capable of producing an antisense RNA transcript of the gene, or a portion thereof.

In one aspect, this disclosure provides a method of generating a dominant negative or dominant positive allele of a gene in a cell comprising deleting a portion of a gene using a targeted editing technique, where a microprotein is generated following the deletion of the portion of the gene.

In one aspect, this disclosure provides a method of generating a dominant negative or dominant positive allele of a gene in a cell comprising deleting a portion of an intergenic region using a targeted editing technique such that the deletion renders the gene under the control of an upstream promoter. In some embodiments, the upstream promoter drives increased expression of the gene. In some embodiments, the upstream promoter drives reduced expression of the gene. In some embodiments, the upstream promoter drives altered temporal expression of the gene. In some embodiments, the upstream promoter drives altered tissue specific expression of the gene.

In one aspect, this disclosure provides a method of generating a dominant negative allele of at least one gene in at least one cell comprising: (a) introducing to the at least one cell a genome editing system comprising: (i) a site-specific nuclease, or a molecule encoding a site-specific nuclease, (ii) a single guide RNA (sgRNA), or a molecule encoding a sgRNA and (iii) at least a first tether guided oligo (tgOligo) and a second tgOligo, or one or more molecules encoding a first and a second tgOligo, operably linked to at least one promoter; (b) generating a first double-stranded break (DSB) and a second DSB in the at least one gene, where the first tgOligo and the second tgOligo hybridize to the 3' free ends of opposing strands at the first DSB and second DSB, where at least 1, at least 2, at least 3, at least 4, at least 5, at least 10, at least 25, at least 50, at least 100, at least 250, at least 500, at least 750, at least 1000, at least 2500, or at least 5000 nucleotides of the at least one gene are deleted, thereby generating a dominant negative allele of the gene that encodes a truncated protein; and (c) identifying and selecting at least one cell comprising the truncated protein.

In one aspect, this disclosure provides a method of generating a dominant negative allele of at least one gene in at least one cell comprising: (a) introducing to the at least one cell one or more vectors encoding: (i) at least one site-specific nuclease, (ii) at least one single guide RNA (sgRNA), and (iii) at least a first tether guided oligo (tgOligo) and a second tgOligo operably linked to at least one promoter; (b) generating a first double-stranded break (DSB) and a second DSB in the gene, where the first tgOligo and the second tgOligo hybridize to the 3' free ends of opposing strands at the first DSB and second DSB, where the region of the at least one gene between the first DSB and second DSB is inverted in orientation, thereby generating a dominant negative allele of the at least one gene that encodes an antisense RNA transcript of the gene: and (c) identifying and selecting at least one cell comprising the antisense RNA transcript of the at least one gene.

In one aspect, this disclosure provides a modified plant cell comprising a non-transposon mediated genome deletion or inversion of a gene, or a portion thereof, at the endogenous locus of the gene, where the deletion or inversion results in the production of an RNA transcript comprising a sequence complementary to a native transcript sequence of the gene, or portion thereof.

In one aspect, this disclosure provides a modified chromosome comprising a non-transposon mediated deletion or inversion of a gene or, a portion thereof, at the endogenous locus of the gene, where the deletion or inversion results in the production of an RNA transcript comprising a sequence complementary to a native transcript sequence of the gene, or portion thereof.

In one aspect, this disclosure provides a modified plant, or part thereof, comprising a non-transposon mediated genome deletion or inversion of a gene, or a portion thereof, at the endogenous locus of the gene, where the deletion or inversion results in the production of an RNA transcript comprising a sequence complementary to a native transcript sequence of the gene, or portion thereof.

In one aspect, this disclosure provides a modified cell comprising (a) a non-transposon mediated genome deletion of at least one gene, or a portion thereof, at the endogenous locus of the at least one gene, or (b) a non-transposon mediated and non-T-DNA mediated insertion of a polynucleotide sequence into the at least one gene, where the deletion or insertion creates a dominant positive allele of the at least one gene.

In one aspect, this disclosure provides a modified cell comprising a non-transposon mediated genome deletion or inversion of a gene, or a portion thereof, at the endogenous locus of the at least one gene, where the deletion or inversion results in the production of an RNA transcript comprising a sequence complementary to a native transcript sequence of the gene.

In one aspect, this disclosure provides a modified cell comprising a targeted edit of at least one gene or a portion thereof, where the targeted edit generates an RNA transcript that is complementary to a native transcript sequence of the gene.

In one aspect, this disclosure provides a modified cell comprising at least one dominant negative allele of at least one gene generated by a targeted editing technique, where the allele generates an RNA transcript capable of forming a hairpin loop secondary structure when the at least one dominant negative allele is transcribed.

In one aspect, this disclosure provides a modified cell comprising a non-transgenic dominant negative allele of a gene, said dominant negative allele comprising a heterologous non-coding RNA target site in the endogenous locus of the gene.

In one aspect, this disclosure provides a modified cell comprising a non-transgenic dominant positive allele of a gene, said dominant positive allele comprising a heterologous non-coding RNA target site in the endogenous locus of the gene.

In one aspect, this disclosure provides a modified cell comprising at least one insertion or deletion at the endogenous locus of the at least one gene generated by a targeted editing technique, where the insertion or deletion results in expression of a truncated protein.

In one aspect, this disclosure provides a modified cell comprising a dominant negative allele of at least one gene comprising an inverted copy of the gene adjacent to a native copy of the gene at the endogenous locus of the gene.

### BRIEF DESCRIPTION OF THE DRAWINGS

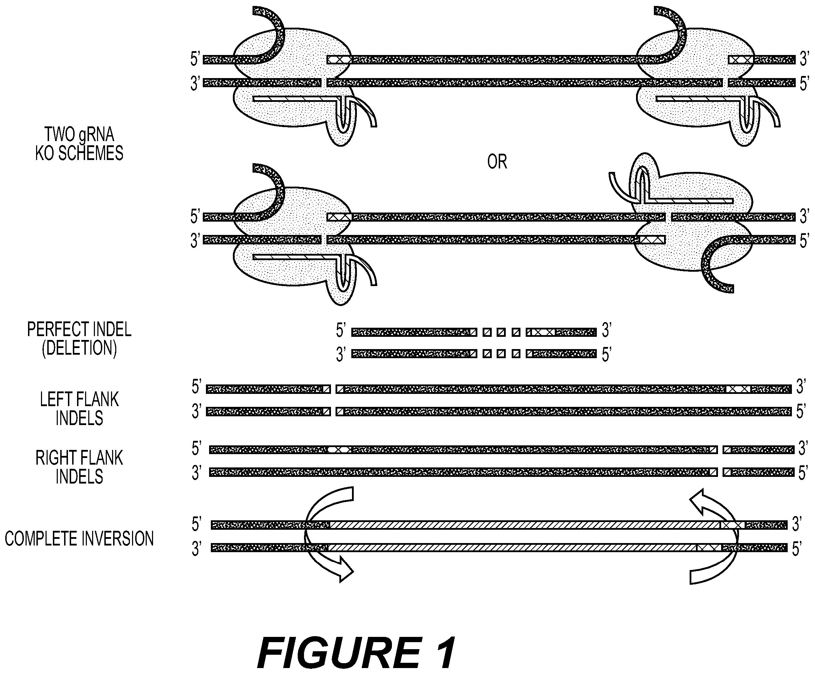

**Figure 1** comprises Panel A and Panel B. Panel A shows that two sRNAs coupled to two RNA-guided nucleases can hybridize target DNA on the same or different strands to isolate a region of DNA from the rest of the DNA strand(s) by generating two double-stranded breaks (DSBs). Panel B shows different outcomes that are possible after two RNA-guided nucleases generate two DSBs in DNA. Native cellular machinery can repair the two DSBs by deleting the entire region between the DSBs, by deleting a small portion of the region from the 5' or 3' end, or by inverting the region between the DSBs.

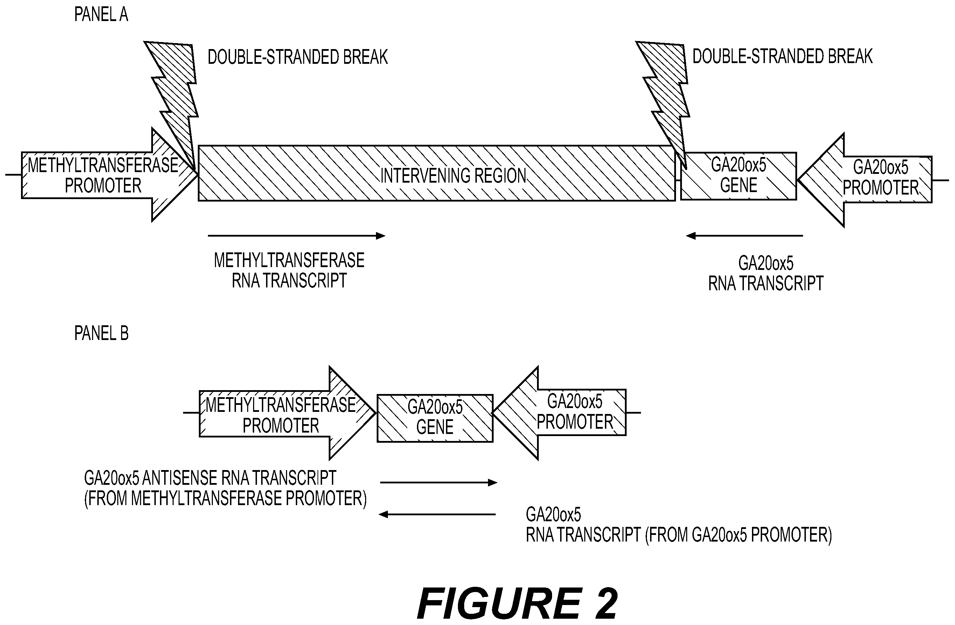

**Figure 2** comprises Panel A and Panel B. Panel A shows a representation of the maize genomic region near the GA20 oxidase_5 gene. As shown in Panel A, a methyltransferase/ SAMT gene is adjacent to the GA20 oxidase_5 gene, but it is in an opposite orientation. The intervening region between the SAMT promoter and the GA20 oxidase_5 coding gene is deleted after generating a double-stranded break on each end. Panel B shows the structure of the region following the deletion of the intervening region. By removing the intervening region the SAMT promoter can drive the expression of an antisense GA20 oxidase_5 RNA transcript, which can form a double-stranded RNA with sense GA20 oxidase_5 RNA transcripts generated by the native GA20 oxidase_5 promoter.

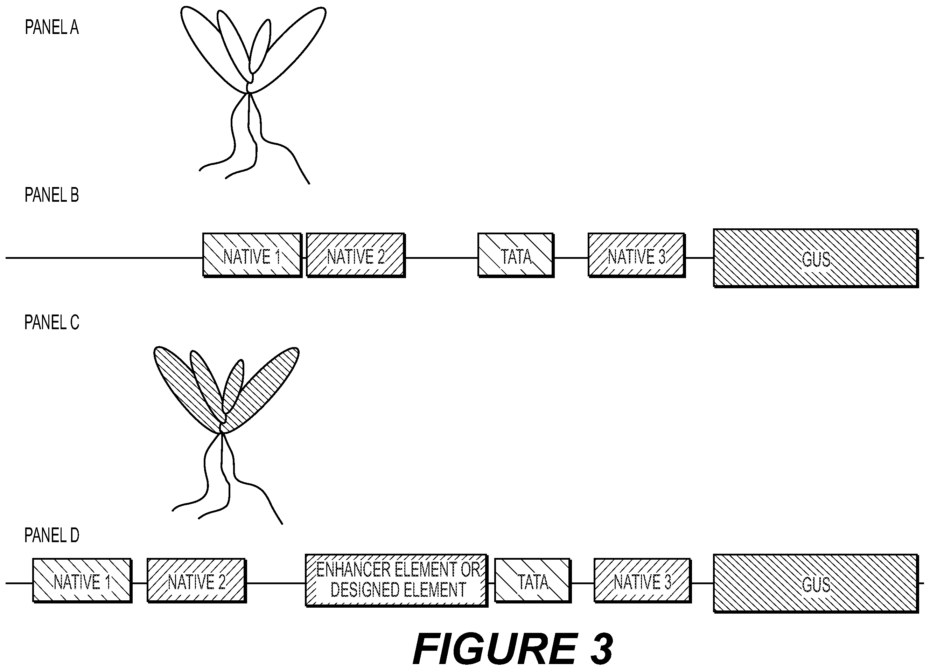

**Figure 3** comprises Panels A, B, C, and D. Panel A shows GUS staining of an *Arabidopsis thaliana* plant, which demonstrates that the Native 3 promoter expresses GUS only in root tissue. Panel B shows the genomic structure around the Native 3 promoter and GUS transgene. Panel C shows expanded GUS staining after insertion of a native genomic expression element such as an enhancer element or designed expression element upstream of the Native 3 TATA box. Panel D shows the genomic structure around the Native 3 promoter after targeted insertion of the native enhancer or designed element.

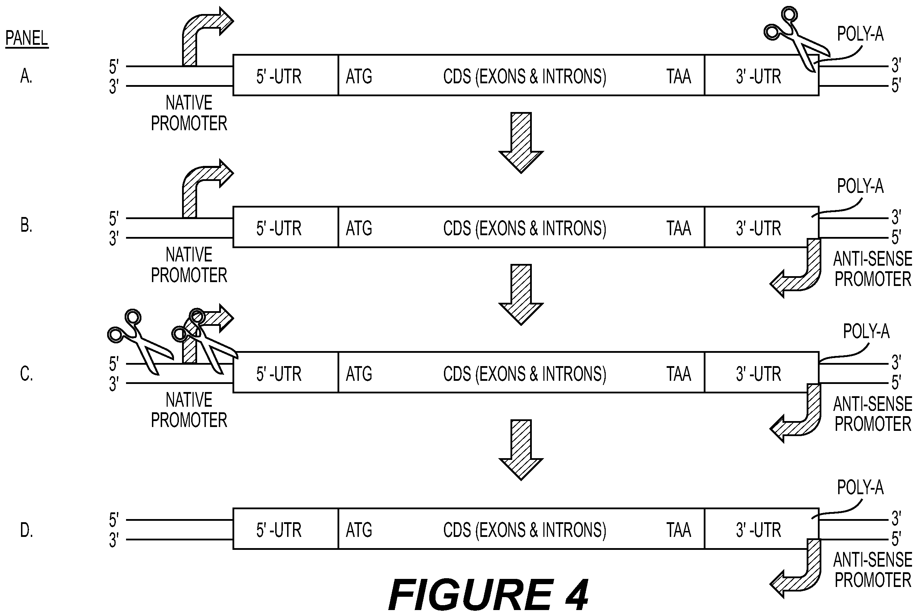

**Figure 4** comprises Panels A, B, C, and D. Panel A shows the structure of a gene of interest and indicates that a double-stranded break can be generated immediately upstream of the poly-adenylation site in the 3'-UTR of the gene of interest. Panel B shows the insertion of an anti-sense promoter at the double-stranded break site. The promoter can be the native promoter of the gene or any promoter of interest. Panel C shows the optional generation of two double-stranded breaks surrounding the native promoter of the gene of interest, leading to its deletion (Panel D).

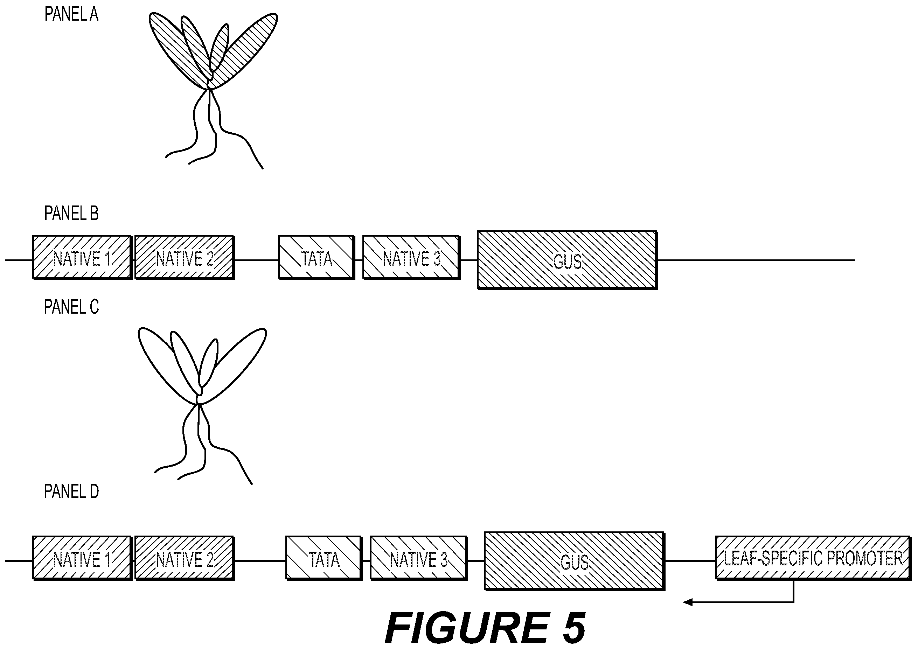

**Figure 5** comprises Panels A, B, C, and D. Panel A shows GUS staining of an *Arabidopsis thaliana* plant comprising a GUS transgene under the control of the Native 3 promoter. GUS is expressed throughout the plant. Panel B shows the genomic region around the Native 3 promoter and the GUS transgene. Panel C shows the reduction of GUS staining when a leaf-specific promoter is inserted downstream of the GUS transgene such that it transcribes antisense GUS RNA. Panel D shows the genomic region around the Native 3 promoter and GUS transgene following the insertion of the antisense leaf-specific promoter.

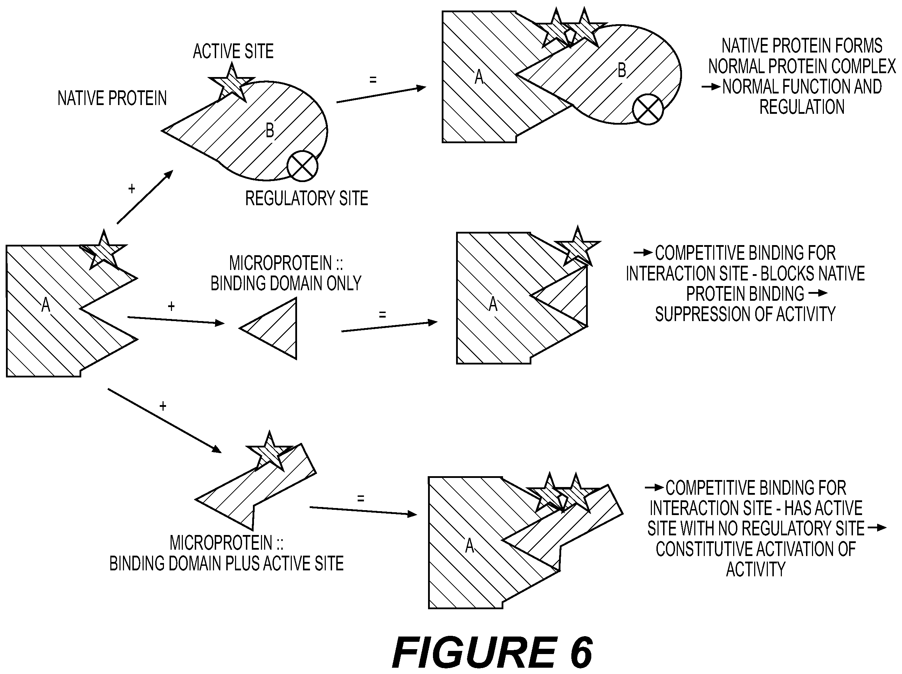

**Figure 6** shows potential outcomes of protein truncation. The upper flow shows normal protein interactions that brings all of the encoded components together in the correct conformation to allow function of a protein complex. The center flow shows a truncation of one of the protein components that removes a functional unit of the protein but retains the interaction domain that can still bind the interacting protein and block the interaction site from a complete fully functional version of the truncated protein. In the center case - this would act as a dominant negative allele. The lower flow shows the strategic deletion of part of a protein that encodes a regulatory element for activity and leaves the functional domain and the interaction domain intact - thus leading to a protein complex that is stuck in either a constitutively active or suppressed state of activity.

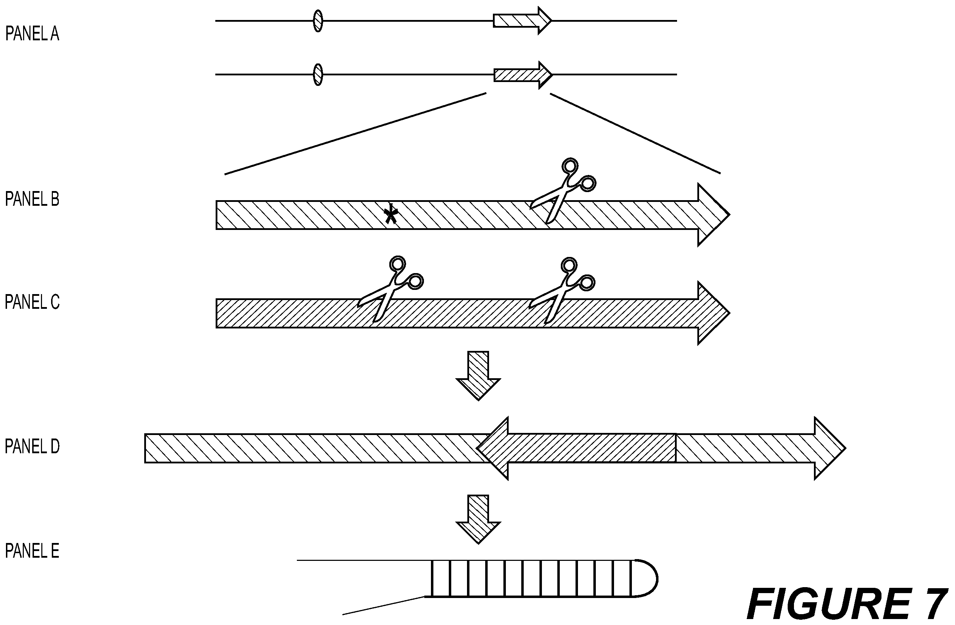

**Figure 7** comprises Panels A, B, C, D, and E. Panel A shows a heterozygous genomic locus. Panel B shows that a nuclease (represented by scissors) generates one DSB in the first allele. Panel C shows that a nuclease generates two DSBs in the second allele. In one possible outcome (Panel D) the region between the two DSBs on the second allele (Panel C) is inverted and inserted into the single DSB in the first allele. Panel E shows a hairpin RNA transcript produced form transcribing the allele structure in Panel D.

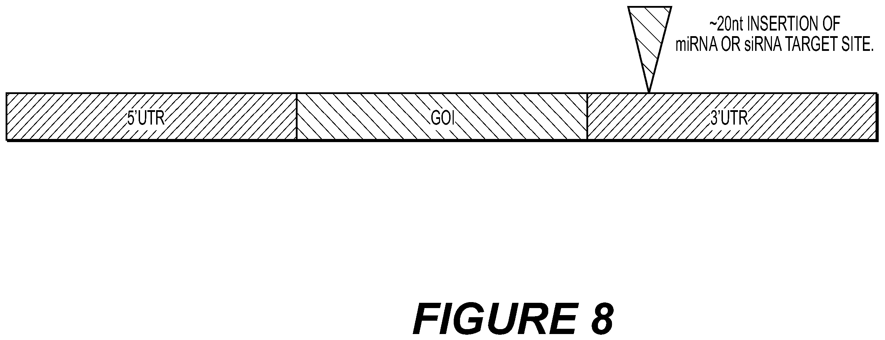

**Figure 8** provides a schematic of a non-coding RNA target site insertion in a gene of interest. Such an insertion can lead to the production of transient secondary siRNAs to down-regulate the gene of interest (GOI).

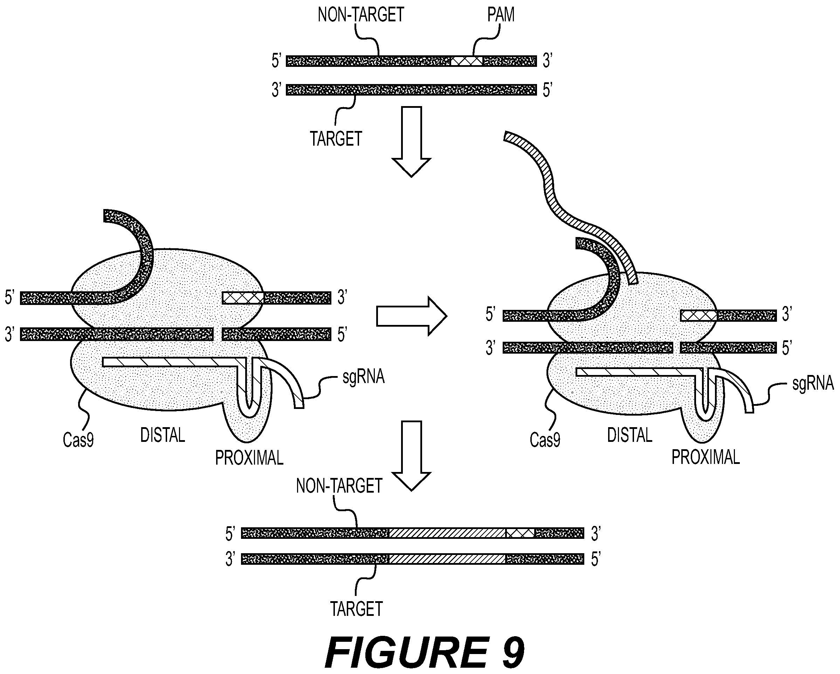

**Figure 9** provides a schematic of a Cas9-mediated double-stranded break (DSB) and a tether guide oligo (tgOligo) bound to a target DNA site. The Cas9-PAM interaction occurs on the non-target strand; sgRNA-DNA annealing occurs on the target strand. The blunt ends at the Cas9 cut site are held in place by Cas9 at the 5' end of the non-target strand (PAM location), and at both cut ends (3' and 5') of the target strand. The 3' cut end of the non-target strand is free and "flaps" around. The 3' free "flap" end of the non-target strand can be up to 35 nucleotides which can be sufficient for specific complementarity binding. A tgOligo (*e.g.,* a ssDNA template) can be included for integration of desired nucleotide modification. The drawing scheme used here is followed in the subsequent figures.

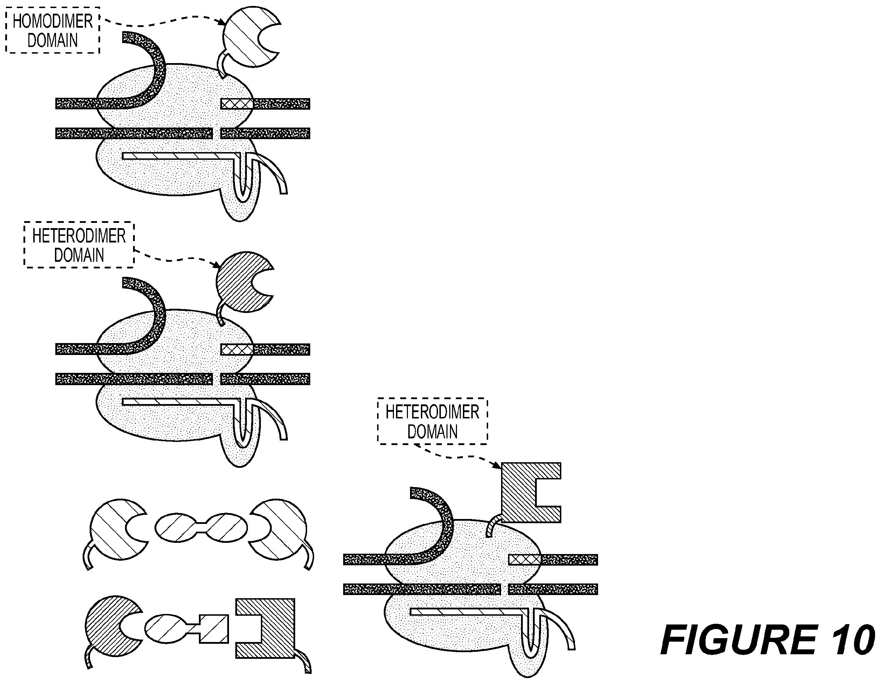

**Figure 10** depicts Cas9 conjugated with a homodimer domain (top) and heterodimer domains (middle and bottom right) to facilitate dimerization. Ligands for the homodimer and heterodimer domains are shown (bottom left). The drawing scheme used here is followed in the subsequent figures; *e.g.,* the ligands, the homodimer or heterodimer domains, ssDNA binding domains. Each component of the Cas9/sgRNA complex and target DNA are shown as illustrated in Figure 9. The drawing scheme used here for different dimerization domains is followed in the subsequent figures.

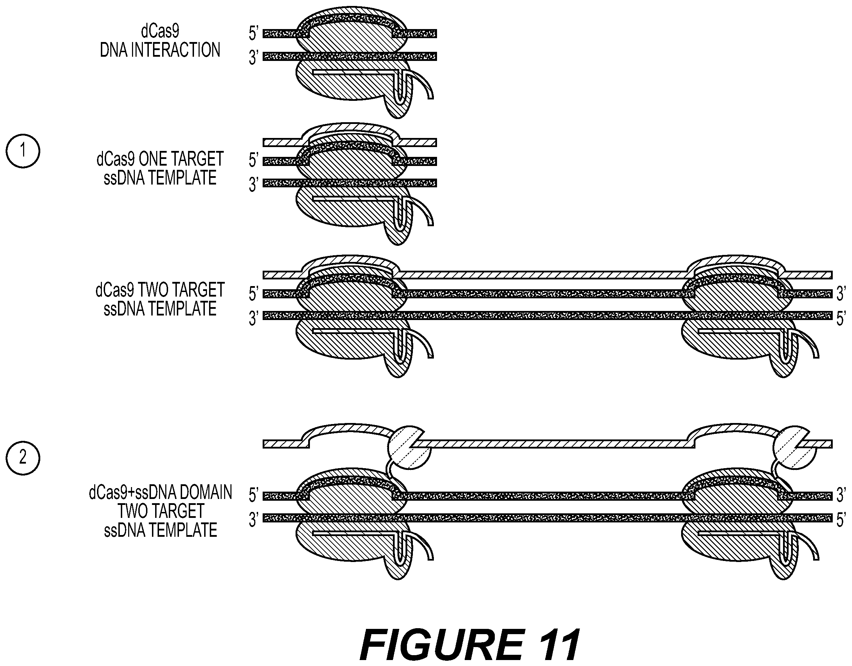

**Figure 11** depicts use of catalytically deactivated Cas9 (dCas9) to increase genome editing efficiency. Panel 1 illustrates that dCas9 binds to DNA at a target site specified by the gRNA and creates a loop structure accessible for template-based editing. Panel 2 illustrates a modified scheme for further facilitating template-based editing via a dCas9 conjugated with a ssDNA-binding domain. The editing efficiency with this modified scheme is expected to be higher compared to those in Panel 1. because a ssDNA template is bound to dCas9 complex and would be brought into proximity of the gRNA target.

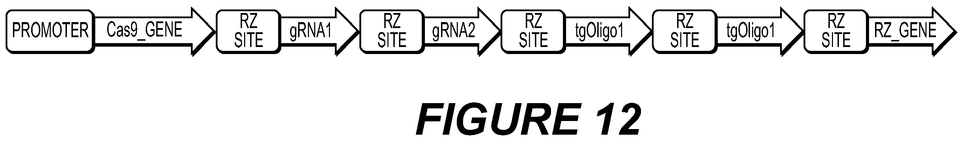

**Figure 12** provides an example construct containing Cas9, gRNAs, and tgOligos. RZ stands for Ribozyme, an enzyme that cleaves a 15bp recognition site in RNA (RZ site).

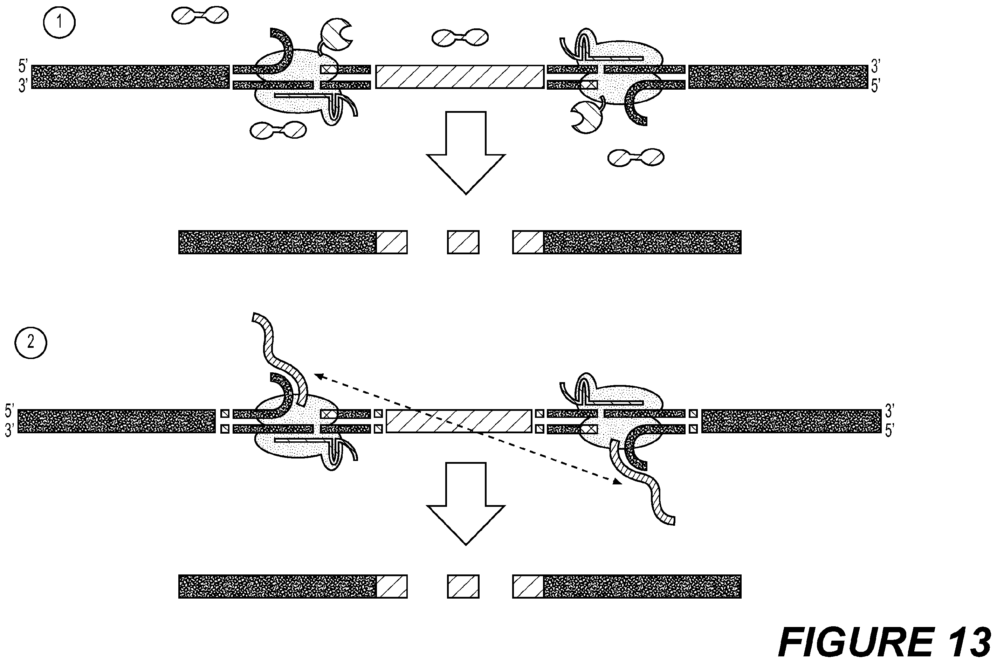

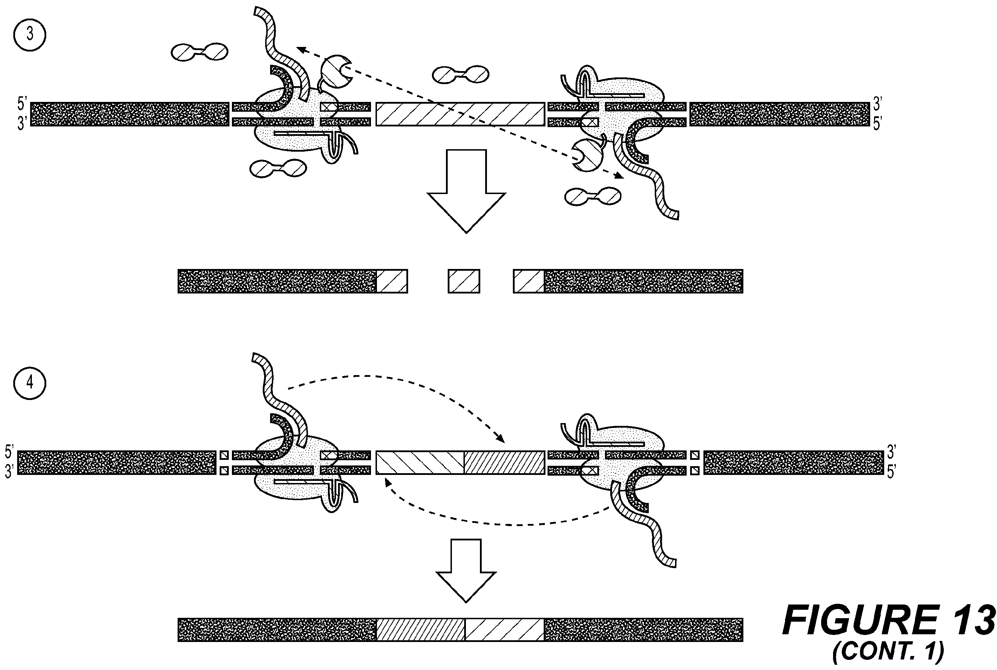

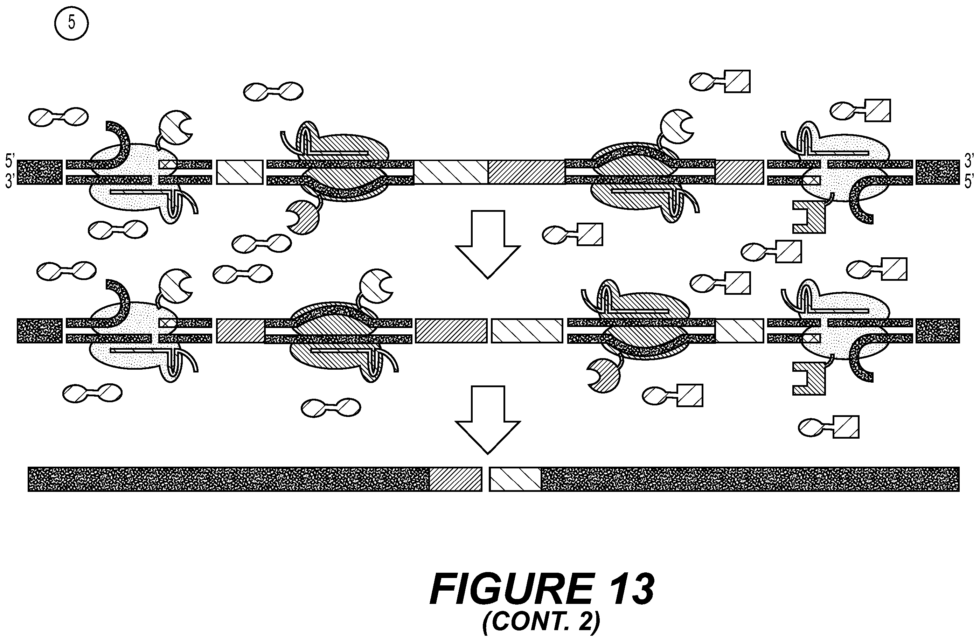

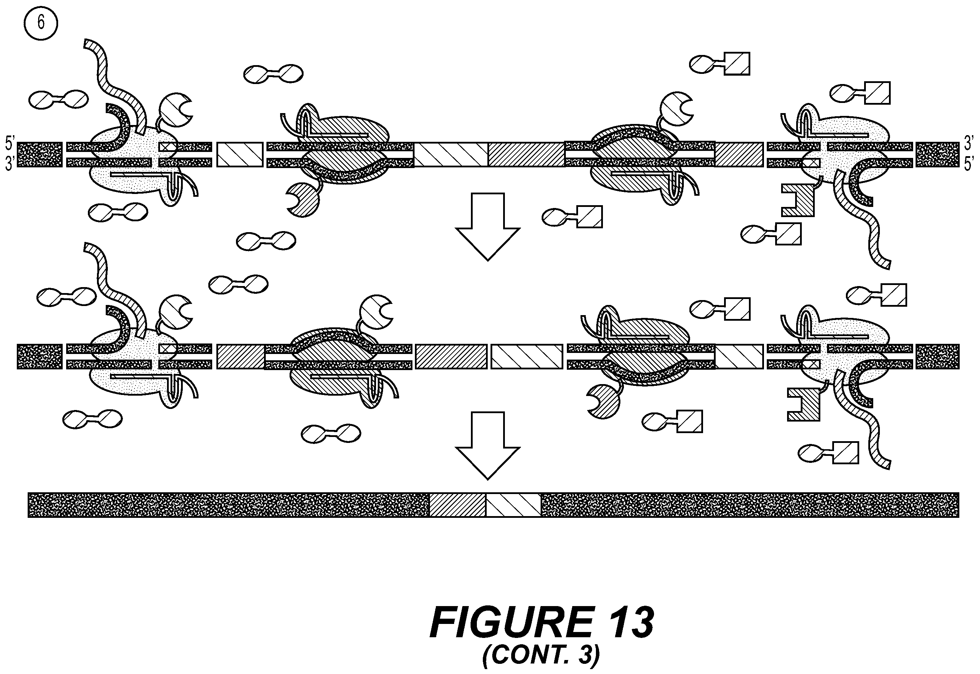

**Figure 13** provides anllustration of various approaches for improving genome editing efficiency. Using dimerization domains (*See* Figure 10), tgOligos (*See* Figure 9), or a combination of both can enhance recovery of complete knockout (deletion) of the genomic region flanked by the two gRNA target sites. Panel 1 shows a dimerization-enhanced knock out (KO) event. Panel 2 shows a tgOligo-enhanced KO event. Panel 3 shows an enhanced KO event via a combination of dimerization and tgOligos. Panel 4 shows a tgOligo-enhanced inversion event. Panel 5 shows a dimerization-enhanced inversion event. Panel 6 shows an inversion event assisted by a combination of Cas9 dimerization/deactivation and tgOligos. Only shown is the configuration where two gRNAs recognize different strands of a target dsDNA. The same concept is equally applicable to the other configuration where two gRNAs recognize the same strand of a target dsDNA.

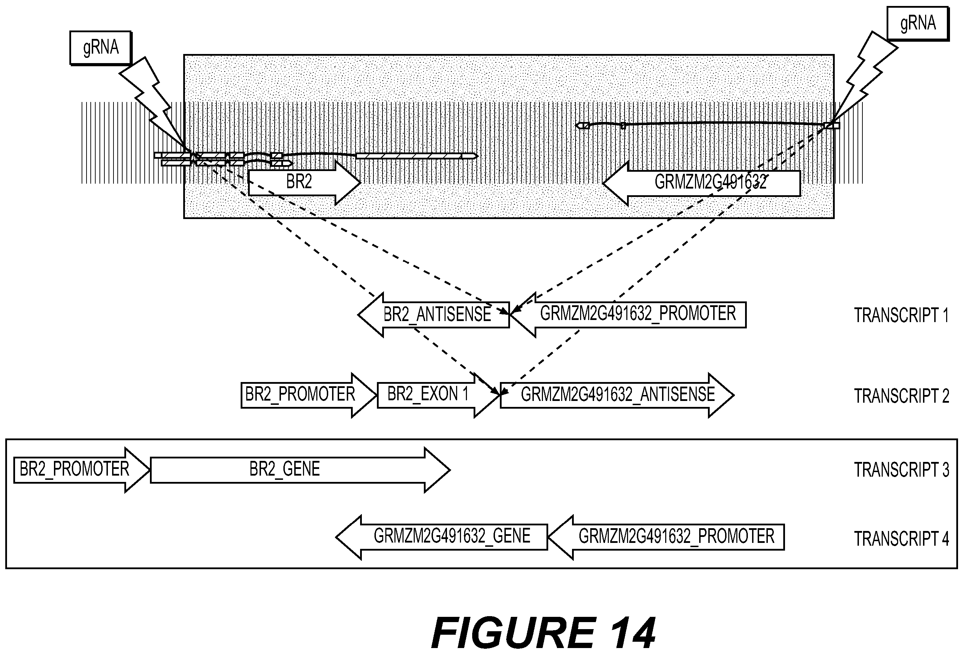

**Figure 14** provides an illustration of editing the corn BR2 gene to generate a dominant knockout allele via genome inversion. Two exemplary gRNAs are used. A first gRNA (shown on the left) targets the end of the first exon of BR2; a second gRNA (shown on the right) recognizes the start codon region of the adjacent GRMZM2G491632 gene. Inversion of the genomic segment flanked by these two gRNAs can lead to a BR2 antisense partial transcript (*See* Transcript 1). This BR2 antisense transcript is produced via the GRMZM2G491632 promoter activity. Adjusting the relative position of the two gRNAs can achieve a BR2 antisense complete transcript (*e.g*., moving the first gRNA on the left to target the start codon region of the BR2 gene) or a BR2 antisense transcript under the control of the native BR2 promoter (*e.g.,* moving the second gRNA on the right to target the stop codon region of the BR2 gene).

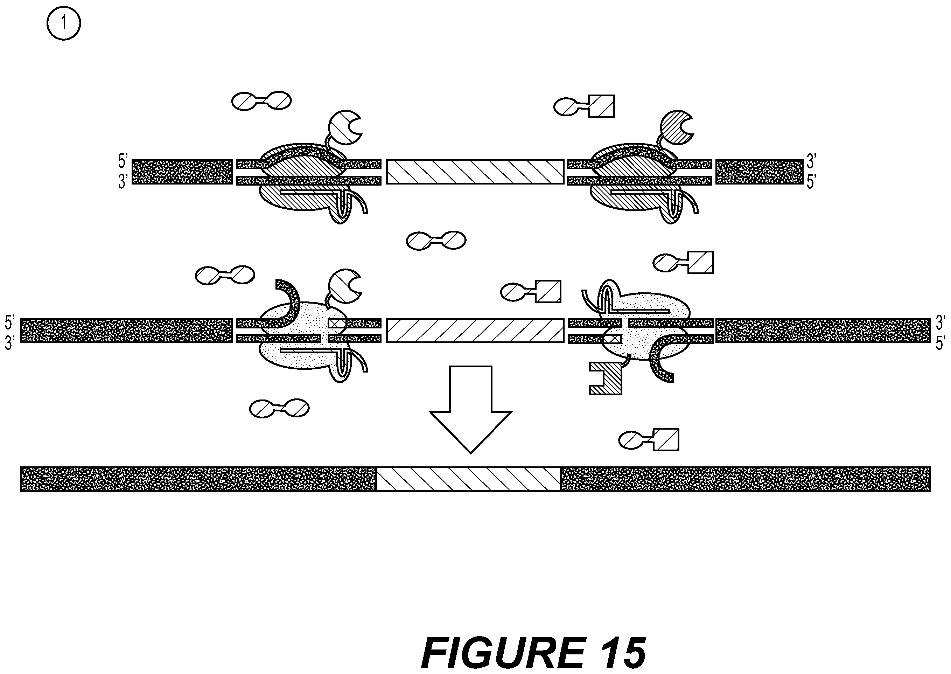

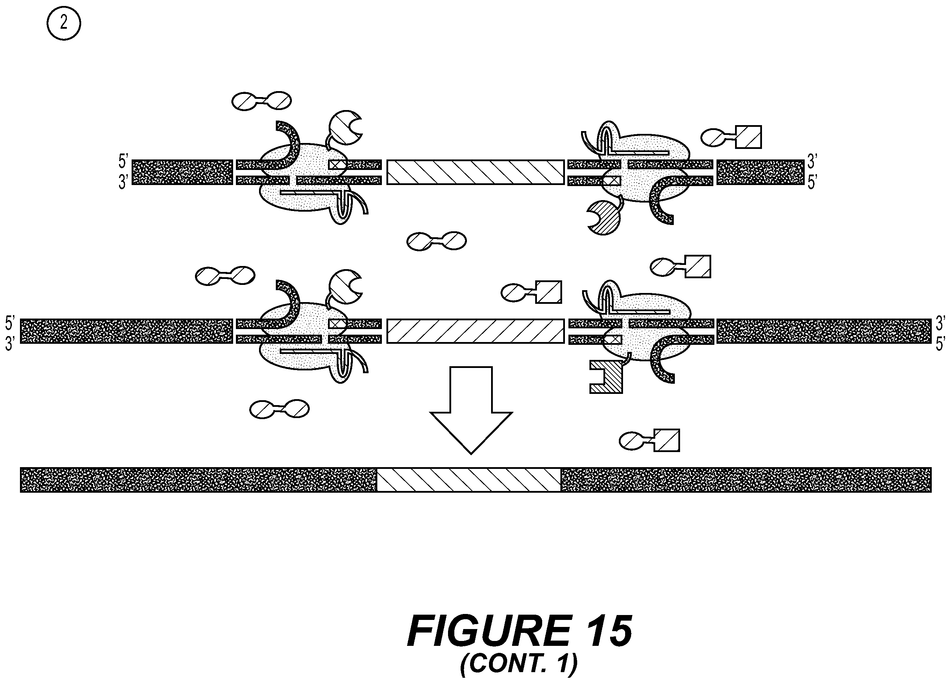

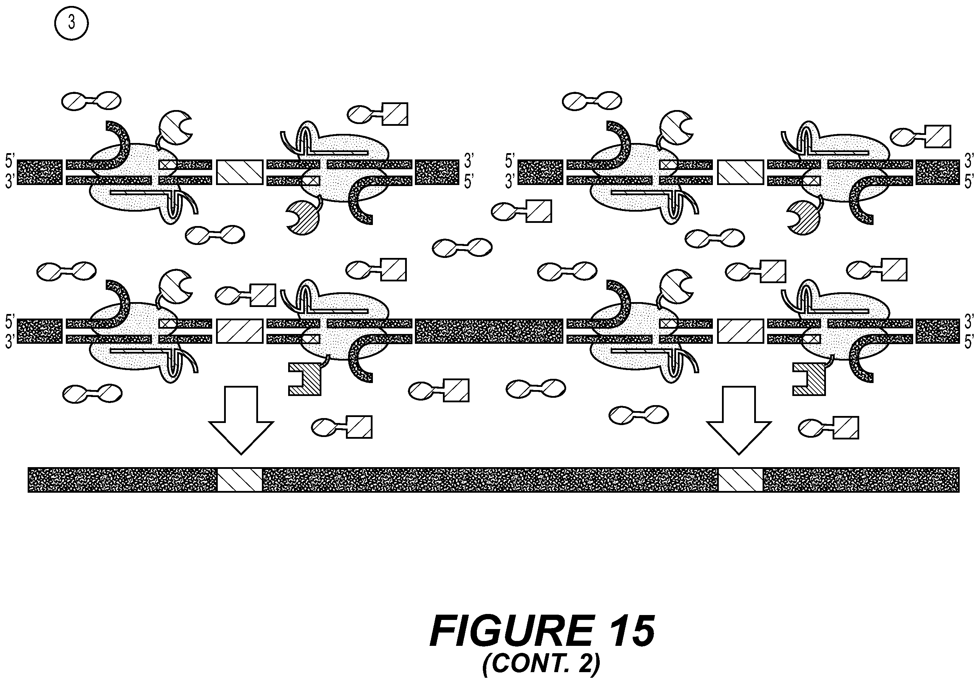

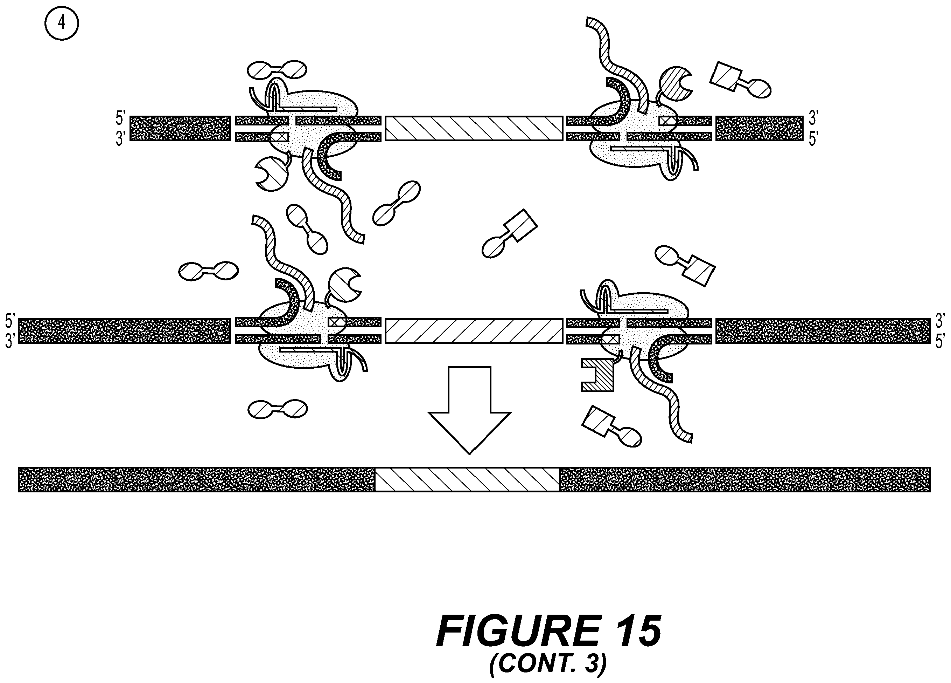

**Figure 15** provides an illustration of dimerization-enhanced template-based editing or site directed integration (SDI) at a single location (Panels 1 and 2) or multiple locations (Panel 3), and dimerization/tgOligo-enhanced template-based editing or SDI (Panel 4).

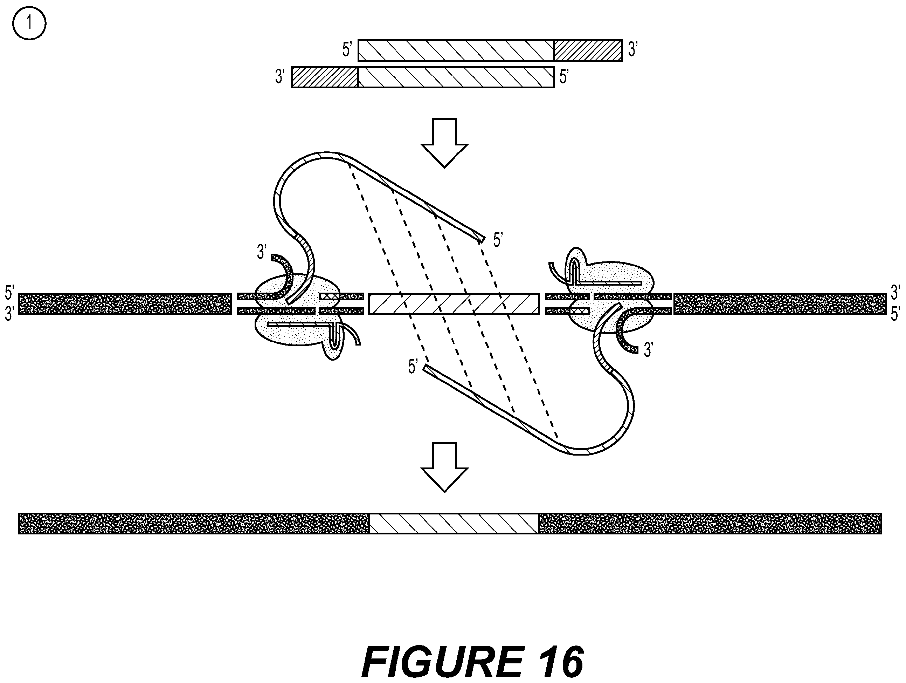

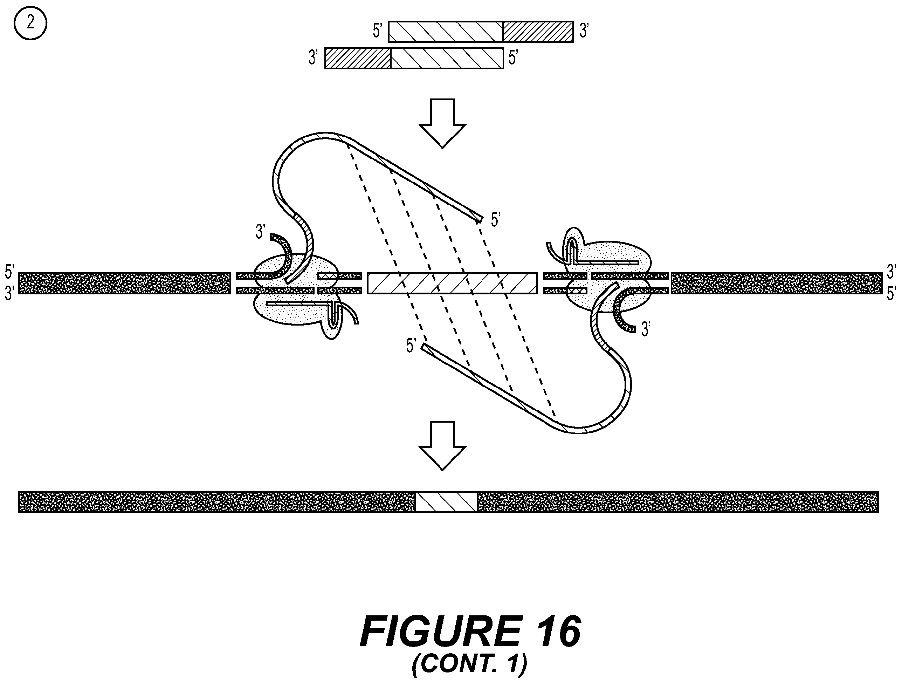

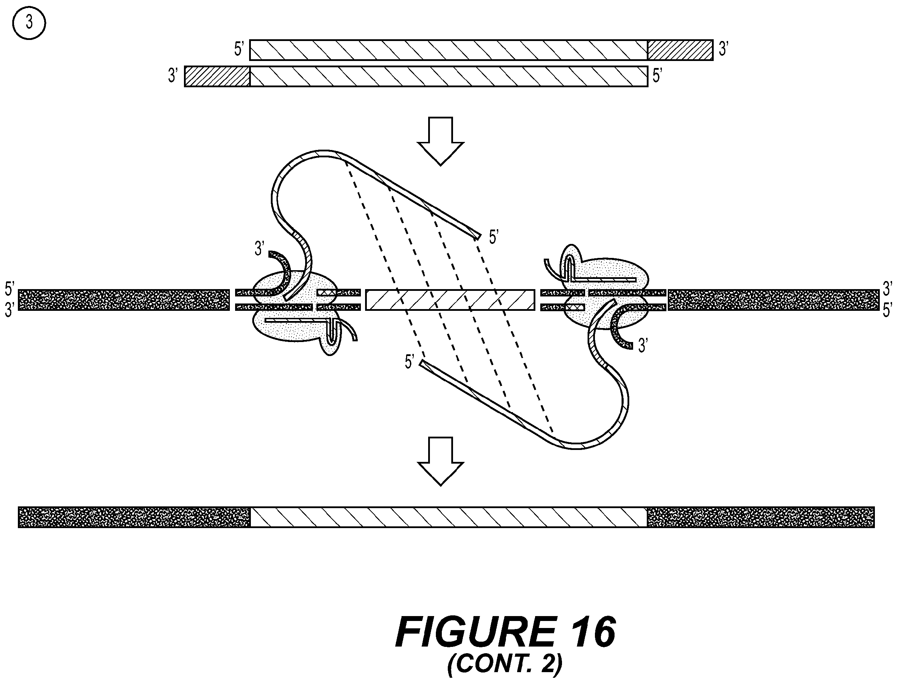

**Figure 16** provides an illustration of template editing (Panel 1), site directed integration (Panel 2), and/or recombination with tgOligos (Panel 3).

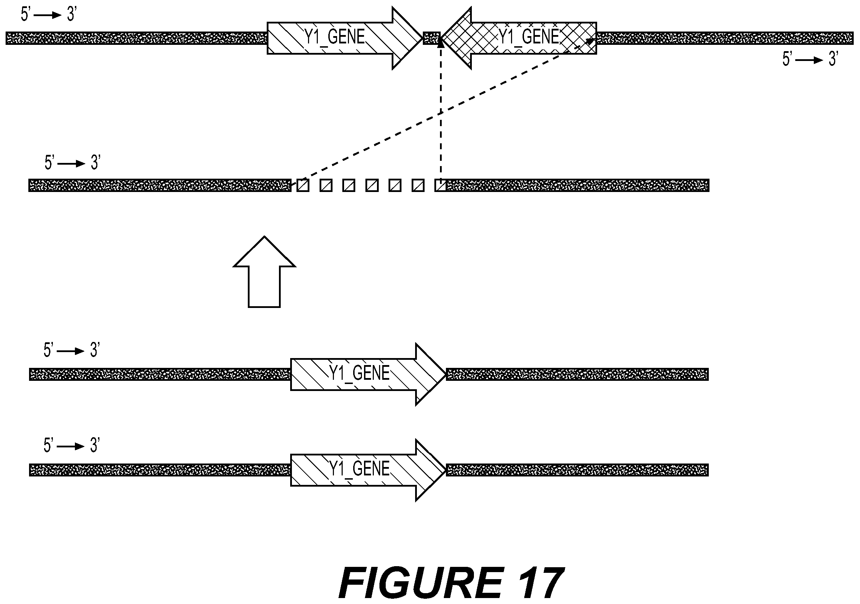

**Figure 17** provides an illustration of the stacking of an inverted Y1 gene head-to-tail to produce an antisense transcript to silence the gene expression. This approach can create a dominant mutant Y1 allele for a normally recessive trait. This dominant allele remains controlled by the native Y1 promoter.

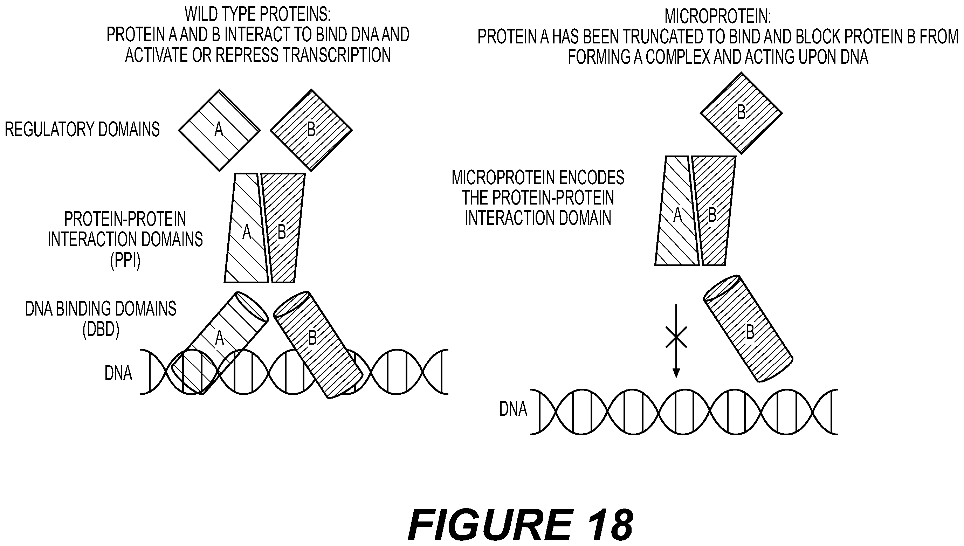

**Figure 18** provides an illustration of a microprotein. Targets of microproteins are often transcriptional regulators that bind to DNA as active homodimers. Microproteins interfere with their targets by forming non-functional heterodimeric complexes that cannot bind to DNA. DBD, DNA-binding domain; PPI, protein-protein interaction domain.

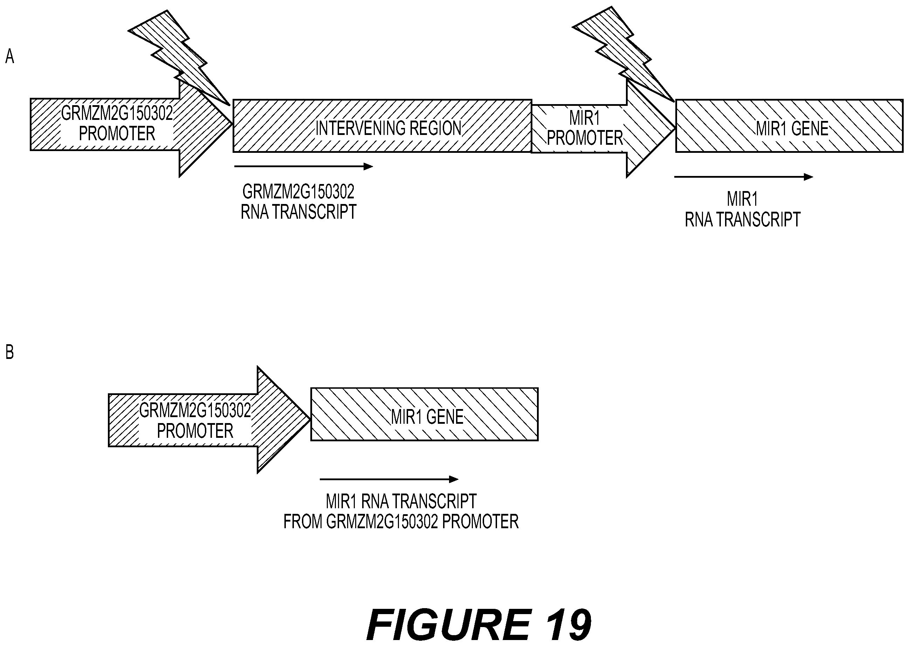

**Figure 19** comprises Panel A and Panel B. Panel A shows a representation of the maize genomic region near the MIR1 gene. As shown in Panel A, the GRMZM2G150302 gene is adjacent and upstream to the GA20 oxidase_5 gene. The intervening region between the GRMZM2G150302 promoter and the MIR1 coding gene is deleted after generating a double-stranded break on each end. Panel B shows the structure of the region following the deletion of the intervening region. By removing the intervening region the GRMZM2G150302 promoter can drive the expression of the MIRIgene.

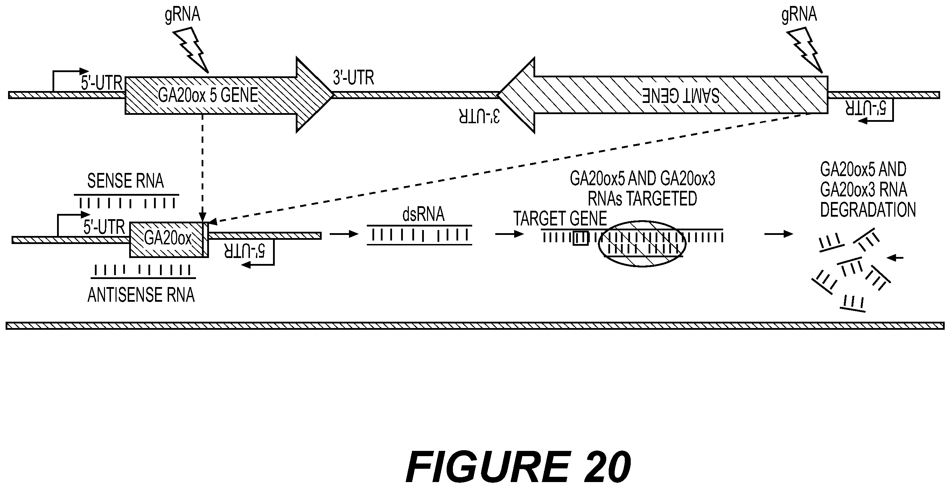

**Figure 20** provides illustrative examples for creating an antisense RNA molecule that targets the Zm.GA20ox5 gene and the Zm.GA20ox3 gene by deleting a genomic region between the Zm.GA20ox5 and its neighboring gene Zm.SAMT oriented in the opposite direction, through genome editing.

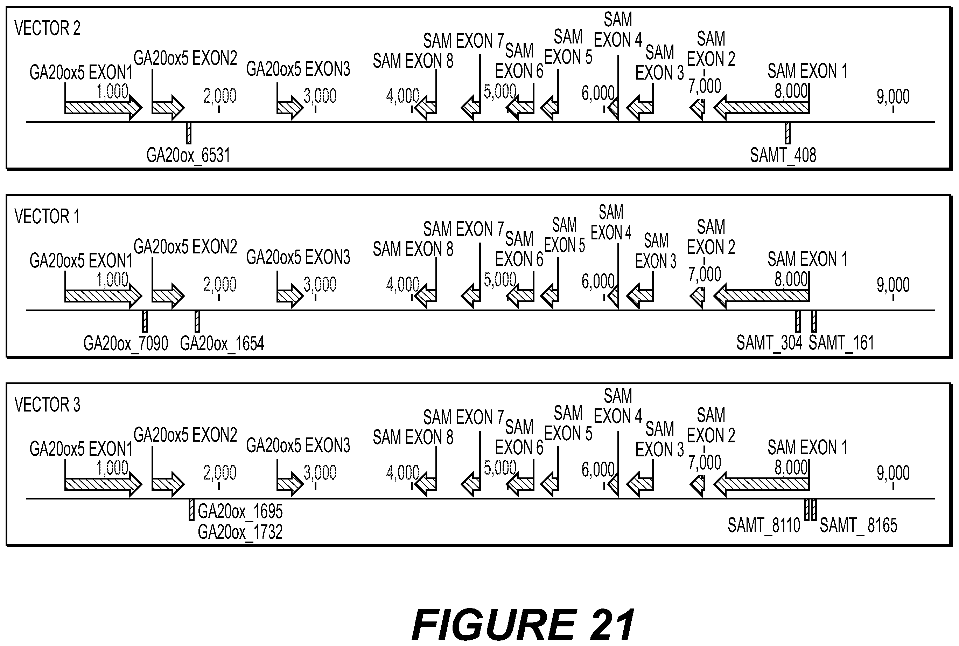

**Figure 21** illustrates the genomic position of various guide RNA target sites in three exemplified vectors for creating a genomic deletion between the Zm.GA20ox5 gene and its neighboring Zm.SAMT gene.

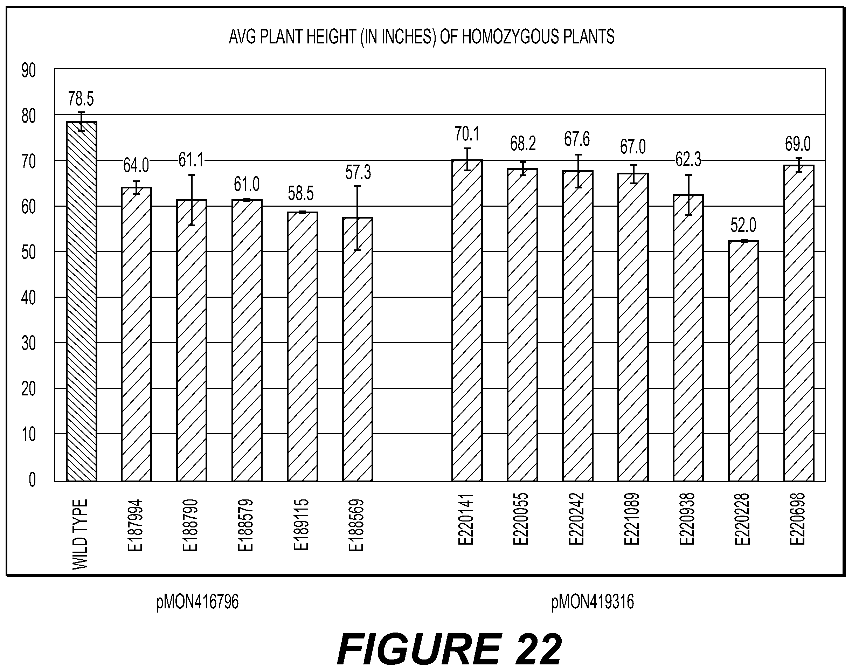

**Figure 22** depicts the average height of wild type plants and homozygous edited plants in inches (Y-axis).

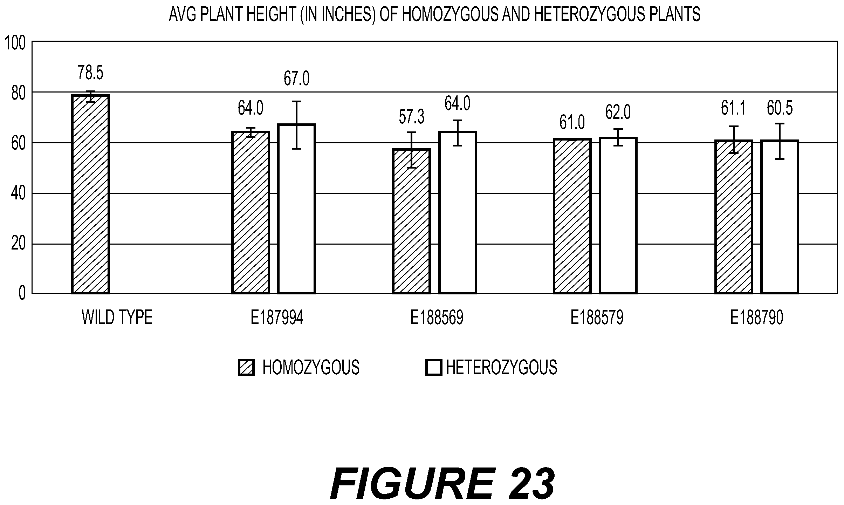

**Figure 23** depicts the average height of wild type plants and homozygous or heterozygous edited plants in inches (Y-axis).

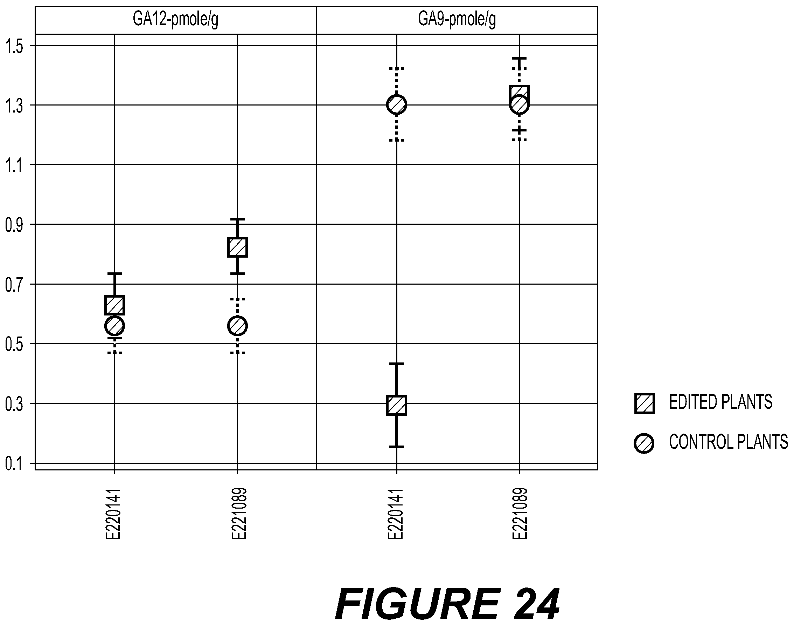

**Figure 24** depicts the concentration of GA12 and GA9 in pmol/g (Y-axis) in edited and control plants.

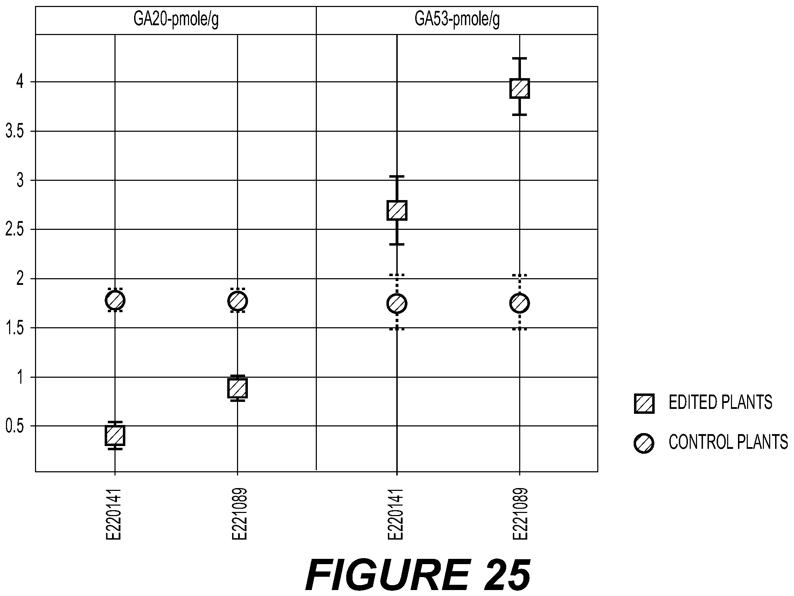

**Figure 25** depicts the concentration of GA20 and GA53 in pmol/g (Y-axis) in edited and control plants.

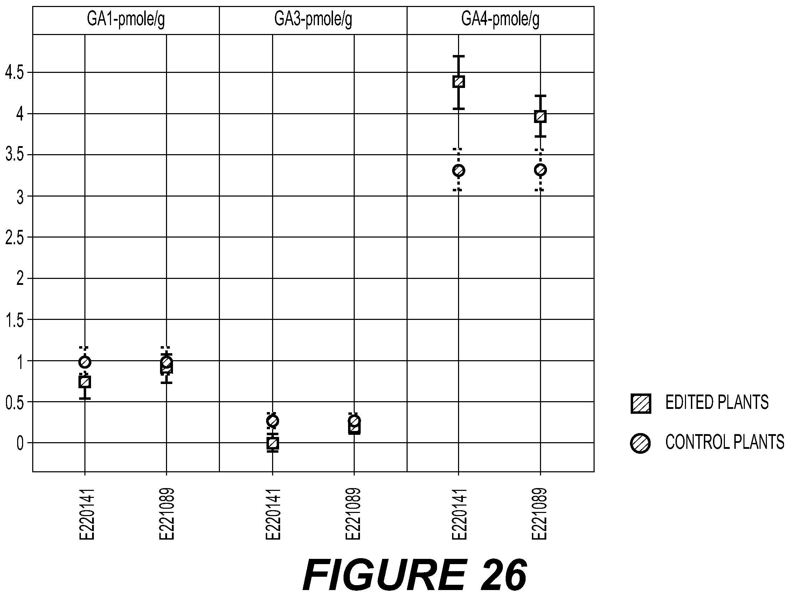

**Figure 26** depicts the concentration of the active gibberellic acids GA1, GA3, and GA4 in pmol/g (Y-axis) in edited and control plants.

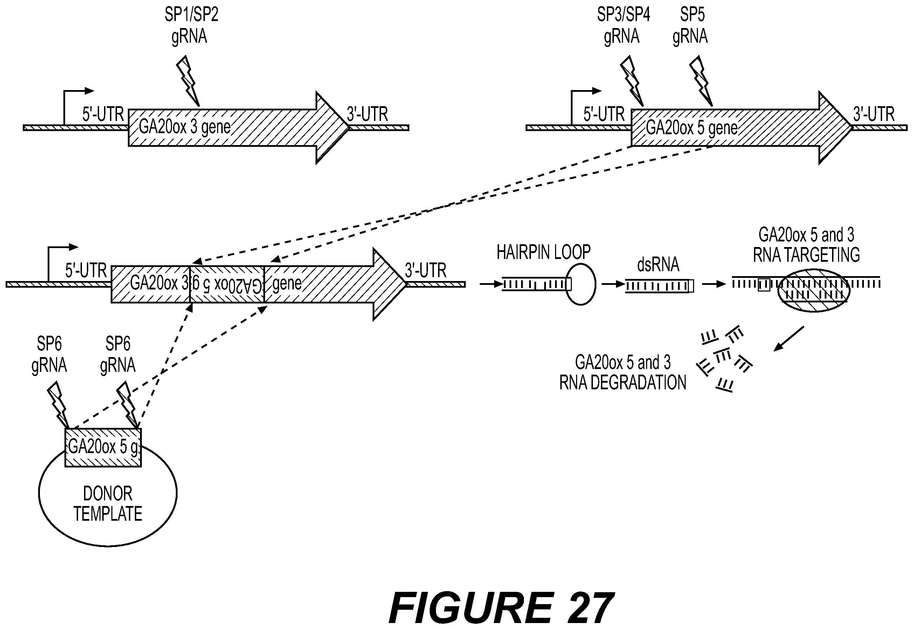

**Figure 27** provides illustrative examples for the production of a genomic modification of the Zm.GA20ox3 locus to encode a RNA transcript with an inverted sequence that can hybridize to a corresponding sequence of the RNA transcript to produce a stem-loop structure, to cause the suppression of one or both copies or alleles at the endogenous Zm. GA20ox3 and Zm. GA20ox5 loci.

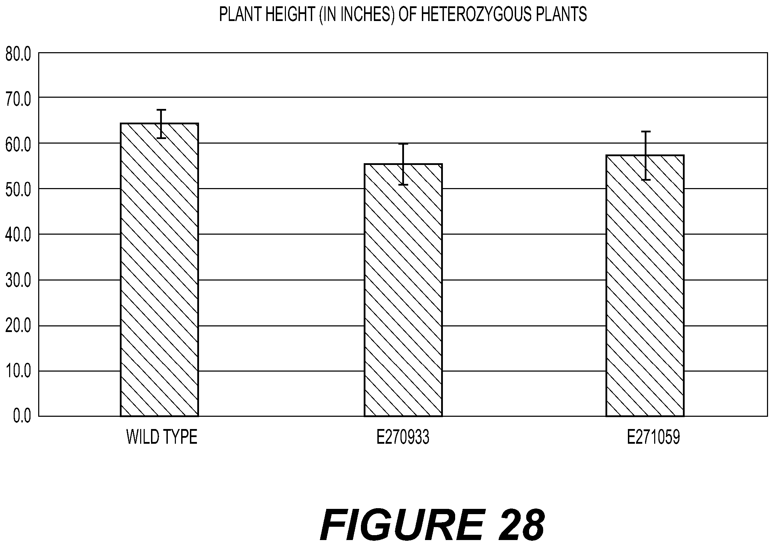

**Figure 28** depicts the average heights of wild type and heterozygous edited corn plants.

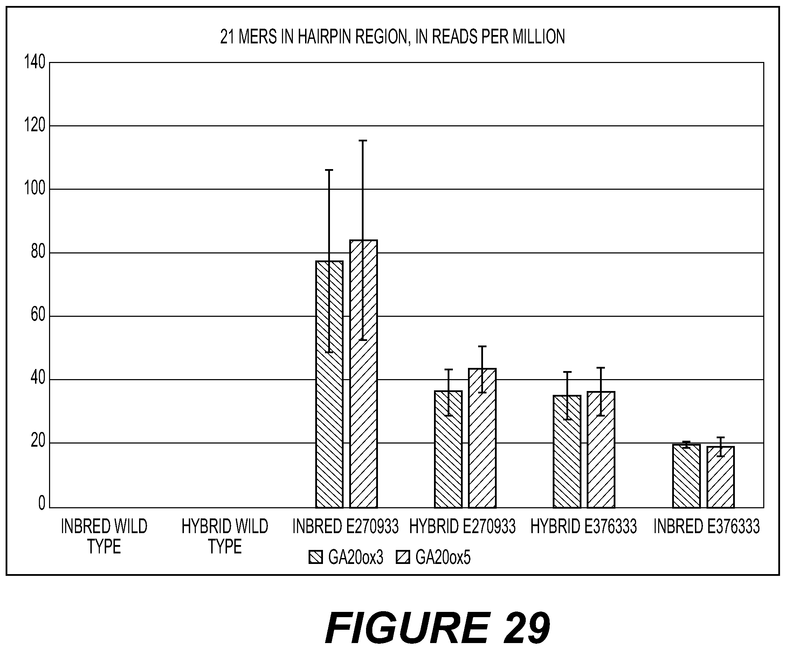

**Figure 29** depicts the number of 21-mer small RNAs (Y-axis) per million reads that mapped to the regions in the stem of the edited stem-loop comprising the inversion sequence from GA20ox5 and a corresponding sequence of the edited GA20ox3 gene that were detected in samples from plants containing edited GA20ox3 alleles.

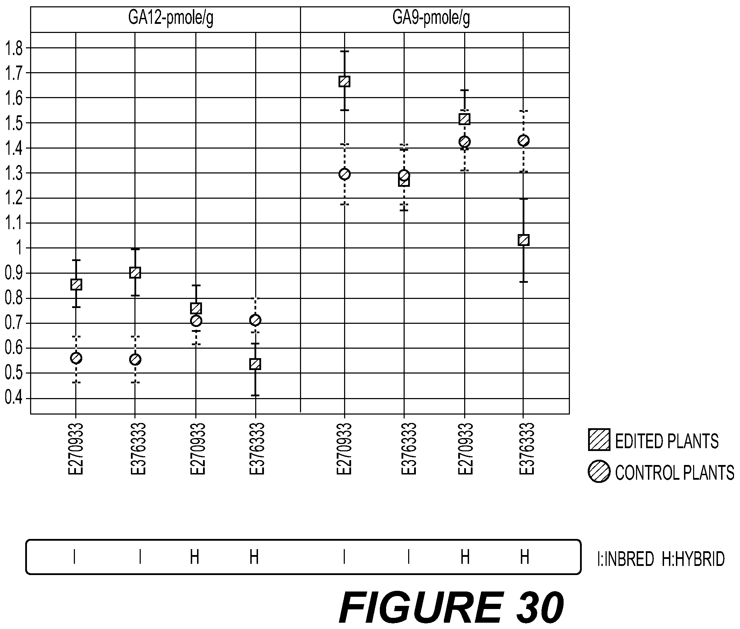

**Figure 30** depicts the concentrations of GA12 and GA9 in pmole/g (Y-axis) in edited and control com plants.

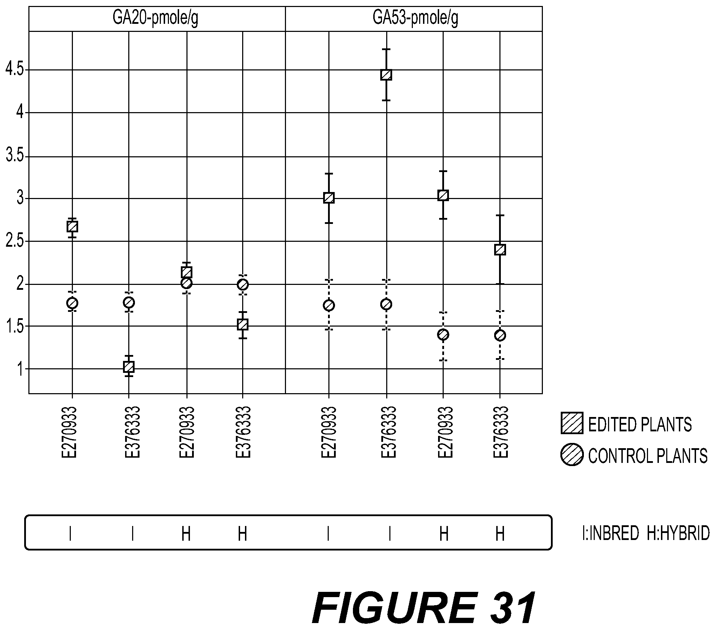

**Figure 31** depicts the concentrations of GA20 and GA53 in pmole/g (Y-axis) in edited and control com plants.

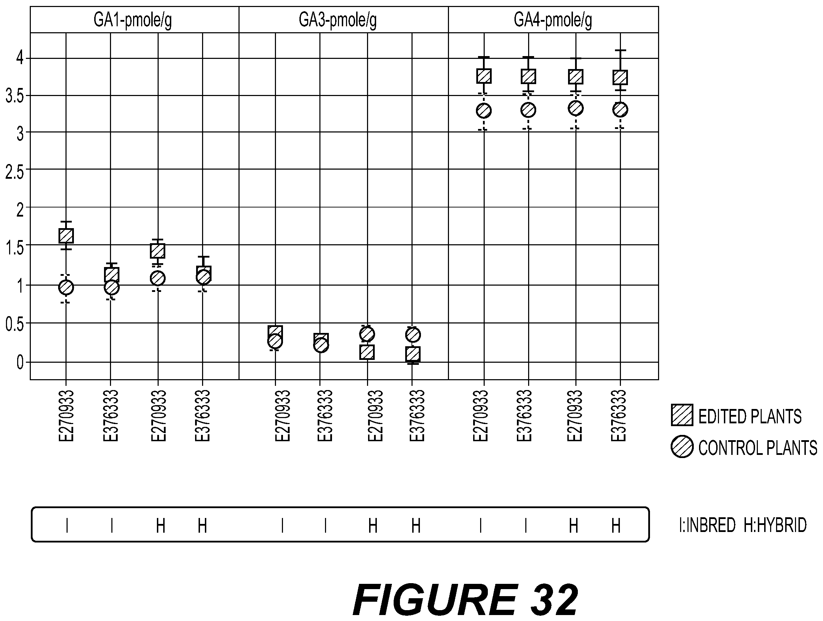

**Figure 32** depicts the concentrations of GA1 and GA3 and GA4 in pmole/g (Y-axis) in edited and control corn plants.

### DETAILED DESCRIPTION

Unless defined otherwise, technical and scientific terms used herein have the same meaning as commonly understood by one of ordinary skill in the art. One skilled in the art will recognize many methods can be used in the practice of the present disclosure. Indeed, the present disclosure is in no way limited to the methods and materials described. For purposes of the present disclosure, the following terms are defined below.

The present specification provides methods and compositions for generating dominant alleles using targeted editing techniques in a wide range of organisms including plants, animals, fungi, and protozoa. Dominant alleles are alleles that mask the contribution of a second allele at the same locus. A dominant allele can be a "dominant negative allele" or a "dominant positive allele." Dominant negative alleles, or antimorphs, are alleles that act in opposition to normal allelic function. A dominant negative allele typically does not function normally and either directly inhibits the activity of a wild-type protein (*e.g*., through dimerization) or inhibits the activity of a second protein that is required for the normal function of the wild-type protein (*e.g.,* an activator or a downstream component of a pathway). For example, a dominant negative allele abrogates or reduces the normal function of an allele in a heterozygous or homozygous state. Dominant positive alleles can increase or expand normal gene function (*e.g.,* a hypermorph) or provide new functions for a gene (*e.g.,* a neomorph). A semi-dominant allele occurs when penetrance of a linked phenotype in individuals heterozygous for the allele is less than that which is observed in individuals homozygous for the allele.

The practice of the present disclosure employs, unless otherwise indicated, conventional techniques of biochemistry, chemistry, molecular biology, microbiology, cell biology, genomics and biotechnology, which are within the skill of the art. See Green and Sambrook, Molecular Cloning: A Laboratory Manual, 4th Edition (2012); Current Protocols In Molecular Biology (F. M. Ausubel, et al. eds., (1987)); the series Methods In Enzymology (Academic Press, Inc.): PCR 2: A Practical Approach (M. J. MacPherson, B. D. Hames and G. R. Taylor eds. (1995)); Harlow and Lane, eds. (1988) Antibodies, A Laboratory Manual; Animal Cell Culture (R. I. Freshney, ed. (1987)); Recombinant Protein Purification: Principles And Methods, 18-1142-75, GE Healthcare Life Sciences; C. N. Stewart, A. Touraev, V. Citovsky, T. Tzfira eds. (2011) Plant Transformation Technologies (Wiley-Blackwell); and R. H. Smith (2013) Plant Tissue Culture. Techniques And Experiments (Academic Press, Inc.).

Any references cited herein, including, *e.g*., all patents, published patent applications, and non-patent publications, are incorporated by reference in their entirety.

As used herein, the singular form "a," "an," and "the" include plural references unless the context clearly dictates otherwise. For example, the term "a compound" or "at least one compound" can include a plurality of compounds, including mixtures thereof.

The term "and/or" when used in a list of two or more items, means that any one of the listed items can be employed by itself or in combination with any one or more of the listed items. For example, the expression "A and/or B" is intended to mean either or both of A and B *- i.e.,* A alone, B alone, or A and B in combination. The expression "A, B and/or C" is intended to mean A alone, B alone, C alone, A and B in combination, A and C in combination, B and C in combination, or A, B, and C in combination.

Nucleic acid molecules provided herein include deoxyribonucleic acids (DNA) and ribonucleic acids (RNA) and functional analogues thereof, such as complementary DNA (cDNA). Nucleic acid molecules provided herein can be single stranded or double stranded. Nucleic acid molecules comprise the nucleotide bases adenine (A), guanine (G), thymine (T), cytosine (C). Uracil (U) replaces thymine in RNA molecules. The symbol "N" can be used to represent any nucleotide base (*e.g.,* A, G, C, T, or U). As used herein, "encoding" refers to a polynucleotide encoding for the amino acids of a polypeptide. A series of three nucleotide bases encodes one amino acid. As used herein, "expressed," "expression," or "expressing" refers to transcription of RNA from a DNA molecule. As used herein, terms "polypeptide", "peptide" and "protein" are used interchangeably to refer to a polymer of amino acid residues. The term also applies to amino acid polymers in which one or more amino acids are chemical analogues or modified derivatives of a corresponding naturally-occurring amino acids. A "messenger RNA" or "mRNA" refers to an RNA transcript that is transcribed from a polynucleotide, where the RNA transcript is capable of being translated into a protein. Typically, DNA encodes an mRNA, which encodes a protein. When DNA is transcribed by an RNA polymerase to ultimately generate a protein, a sense mRNA strand is typically produced by the RNA polymerase from the antisense DNA strand. A sense strand of DNA or RNA runs from 5' to 3', while the antisense strand runs from 3' to 5'. Sense and antisense strands of the same polynucleotide are complementary to each other.

In one aspect, a nucleic acid molecule provided herein comprises a protein coding nucleic acid molecule that is codon optimized for a eukaryotic cell. In another aspect, a protein-coding nucleic acid molecule is codon optimized for a plant cell. In another aspect, a protein-coding nucleic acid molecule is codon optimized for a monocot species. In a further aspect, a protein-coding nucleic acid molecule is codon optimized for a corn or soybean cell.

The terms "percent identity" or "percent identical" as used herein in reference to two or more nucleotide or protein sequences is calculated by (i) comparing two optimally aligned sequences (nucleotide or protein) over a window of comparison, (ii) determining the number of positions at which the identical nucleic acid base (for nucleotide sequences) or amino acid residue (for proteins) occurs in both sequences to yield the number of matched positions, (iii) dividing the number of matched positions by the total number of positions in the window of comparison, and then (iv) multiplying this quotient by 100% to yield the percent identity. If the "percent identity" is being calculated in relation to a reference sequence without a particular comparison window being specified, then the percent identity is determined by dividing the number of matched positions over the region of alignment by the total length of the reference sequence. Accordingly, for purposes of the present application, when two sequences (query and subject) are optimally aligned (with allowance for gaps in their alignment), the "percent identity" for the query sequence is equal to the number of identical positions between the two sequences divided by the total number of positions in the query sequence over its length (or a comparison window), which is then multiplied by 100%. When percentage of sequence identity is used in reference to proteins it is recognized that residue positions which are not identical often differ by conservative amino acid substitutions, where amino acid residues are substituted for other amino acid residues with similar chemical properties (*e.g*., charge or hydrophobicity) and therefore do not change the functional properties of the molecule. When sequences differ in conservative substitutions, the percent sequence identity can be adjusted upwards to correct for the conservative nature of the substitution. Sequences that differ by such conservative substitutions are said to have "sequence similarity" or "similarity."

For optimal alignment of sequences to calculate their percent identity, various pair-wise or multiple sequence alignment algorithms and programs are known in the art, such as ClustalW or Basic Local Alignment Search Tool^{®} (BLAST), etc., that can be used to compare the sequence identity or similarity between two or more nucleotide or protein sequences. Although other alignment and comparison methods are known in the art, the alignment and percent identity between two sequences (including the percent identity ranges described above) can be as determined by the ClustalW algorithm, see, e.g., Chenna R. et al., "Multiple sequence alignment with the Clustal series of programs," Nucleic Acids Research 31: 3497-3500 (2003); Thompson JD et al., "Clustal W: Improving the sensitivity of progressive multiple sequence alignment through sequence weighting, position-specific gap penalties and weight matrix choice," Nucleic Acids Research 22: 4673-4680 (1994); Larkin MA et al., "Clustal W and Clustal X version 2.0," Bioinformatics 23: 2947-48 (2007); and Altschul, S.F., Gish, W., Miller, W., Myers, E.W. & Lipman, D.J. (1990) "Basic local alignment search tool." J. Mol. Biol. 215:403-410 (1990), the entire contents and disclosures of which are incorporated herein by reference.

The terms "percent complementarity" or "percent complementary" as used herein in reference to two nucleotide sequences is similar to the concept of percent identity but refers to the percentage of nucleotides of a query sequence that optimally base-pair or hybridize to nucleotides a subject sequence when the query and subject sequences are linearly arranged and optimally base paired without secondary folding structures, such as loops, stems or hairpins. Such a percent complementarity can be between two DNA strands, two RNA strands, or a DNA strand and a RNA strand. The "percent complementarity" can be calculated by (i) optimally base-pairing or hybridizing the two nucleotide sequences in a linear and fully extended arrangement (i.e., without folding or secondary structures) over a window of comparison, (ii) determining the number of positions that base-pair between the two sequences over the window of comparison to yield the number of complementary positions, (iii) dividing the number of complementary positions by the total number of positions in the window of comparison, and (iv) multiplying this quotient by 100% to yield the percent complementarity of the two sequences. Optimal base pairing of two sequences can be determined based on the known pairings of nucleotide bases, such as G-C, A-T, and A-U, through hydrogen binding. If the "percent complementarity" is being calculated in relation to a reference sequence without specifying a particular comparison window, then the percent identity is determined by dividing the number of complementary positions between the two linear sequences by the total length of the reference sequence. Thus, for purposes of the present application, when two sequences (query and subject) are optimally base-paired (with allowance for mismatches or non-base-paired nucleotides), the "percent complementarity" for the query sequence is equal to the number of base-paired positions between the two sequences divided by the total number of positions in the query sequence over its length, which is then multiplied by 100%.

The term "operably linked" refers to a functional linkage between a promoter or other regulatory element and an associated transcribable DNA sequence or coding sequence of a gene (or transgene), such that the promoter, etc., operates to initiate, assist, affect, cause, and/or promote the transcription and expression of the associated transcribable DNA sequence or coding sequence, at least in certain tissue(s), developmental stage(s) and/or condition(s). In addition to promoters, regulatory elements include, without being limiting, an enhancer, a leader, a transcription start site (TSS), a linker, 5' and 3' untranslated regions (UTRs), an intron, a polyadenylation signal, and a termination region or sequence, etc., that are suitable, necessary or preferred for regulating or allowing expression of the gene or transcribable DNA sequence in a cell. Such additional regulatory element(s) can be optional and used to enhance or optimize expression of the gene or transcribable DNA sequence. For purposes of the present application, an "enhancer" can be distinguished from a "promoter" in that an enhancer typically lacks a transcription start site, TATA box, or equivalent sequence and is thus insufficient alone to drive transcription. As used herein, a "leader" can be defined generally as the DNA sequence of the 5'-UTR of a gene (or transgene) between the transcription start site (TSS) and 5' end of the transcribable DNA sequence or protein coding sequence start site of the gene.

As commonly understood in the art, the term "promoter" refers to a DNA sequence that contains an RNA polymerase binding site, transcription start site, and/or TATA box and assists or promotes the transcription and expression of an associated transcribable polynucleotide sequence and/or gene (or transgene). A promoter can be synthetically produced, varied or derived from a known or naturally occurring promoter sequence or other promoter sequence. A promoter can also include a chimeric promoter comprising a combination of two or more heterologous sequences. A promoter of the present application can thus include variants of promoter sequences that are similar in composition, but not identical to, other promoter sequence(s) known or provided herein. A promoter can be classified according to a variety of criteria relating to the pattern of expression of an associated coding or transcribable sequence or gene (including a transgene) operably linked to the promoter, such as constitutive, developmental, tissue-specific, inducible, etc. Promoters that drive expression in all or most tissues of the plant are referred to as "constitutive" promoters. Promoters that drive expression during certain periods or stages of development are referred to as "developmental" promoters. Promoters that drive enhanced expression in certain tissues of the plant relative to other plant tissues are referred to as "tissue-enhanced" or "tissue-preferred" promoters. Thus, a "tissue-preferred" promoter causes relatively higher or preferential expression in a specific tissue(s) of the plant, but with lower levels of expression in other tissue(s) of the plant. Promoters that express within a specific tissue(s) of the plant, with little or no expression in other plant tissues, are referred to as "tissue-specific" promoters. An "inducible" promoter is a promoter that initiates transcription in response to an environmental stimulus such as cold, drought or light, or other stimuli, such as wounding or chemical application. A promoter can also be classified in terms of its origin, such as being heterologous, homologous, chimeric, synthetic, etc. A "heterologous" promoter is a promoter sequence having a different origin relative to its associated transcribable sequence, coding sequence, or gene (or transgene), and/or not naturally occurring in the plant species to be transformed.

Examples describing a promoter that can be used herein include without limitation U.S. Pat. No. 6,437,217 (maize RS81 promoter), U.S. Pat. No. 5,641,876 (rice actin promoter), U.S. Pat. No. 6,426,446 (maize RS324 promoter), U.S. Pat. No. 6,429,362 (maize PR-1 promoter), U.S. Pat. No. 6,232,526 (maize A3 promoter), U.S. Pat. No. 6,177,611 (constitutive maize promoters). U.S. Pat. Nos. 5,322,938, 5,352,605, 5,359,142 and 5,530,196 (35S promoter), U.S. Pat. No. 6,433,252 (maize L3 oleosin promoter), U.S. Pat. No. 6,429,357 (rice actin 2 promoter as well as a rice actin 2 intron), U.S. Pat. No. 5,837,848 (root specific promoter), U.S. Pat. No. 6,294,714 (light inducible promoters), U.S. Pat. No. 6,140,078 (salt inducible promoters), U.S. Pat. No. 6,252,138 (pathogen inducible promoters), U.S. Pat. No. 6,175,060 (phosphorus deficiency inducible promoters), U.S. Pat. No. 6,635,806 (gamma-coixin promoter), and U.S. patent application Ser. No. 09/757,089 (maize chloroplast aldolase promoter). Additional promoters that can find use are a nopaline synthase (NOS) promoter (Ebert et al., 1987), the octopine synthase (OCS) promoter (which is carried on tumor-inducing plasmids of *Agrobacterium tumefaciens*)*,* the caulimovirus promoters such as the cauliflower mosaic virus (CaMV) 19S promoter (Lawton et al., Plant Molecular Biology (1987) 9: 315-324), the CaMV 35S promoter (Odell et al., Nature (1985) 313: 810-812), the figwort mosaic virus 35S-promoter (U.S. Pat. Nos. 6,051,753; 5,378,619), the sucrose synthase promoter (Yang and Russell, Proceedings of the National Academy of Sciences, USA (1990) 87: 4144-4148), the R gene complex promoter (Chandler et al., Plant Cell (1989) 1: 1175-1183), and the chlorophyll a/b binding protein gene promoter. PC1SV (U.S. Pat. No. 5,850,019), and AGRtu.nos (GenBank Accession V00087; Depicker et al., Journal of Molecular and Applied Genetics (1982) 1: 561-573; Bevan et al., 1983) promoters.

Promoter hybrids can also be used and constructed to enhance transcriptional activity (*see* U.S. Pat. No. 5,106,739), or to combine desired transcriptional activity, inducibility and tissue specificity or developmental specificity. Promoters that function in plants include but are not limited to promoters that are inducible, viral, synthetic, constitutive, temporally regulated, spatially regulated, and spatio-temporally regulated. Other promoters that are tissue-enhanced, tissue-specific, or developmentally regulated are also known in the art and envisioned to have utility in the practice of this disclosure.

As used herein, the term "heterologous" in reference to a promoter is a promoter sequence having a different origin relative to its associated transcribable DNA sequence, coding sequence or gene (or transgene), and/or not naturally occurring in the plant species to be transformed. The term "heterologous" can refer more broadly to a combination of two or more DNA molecules or sequences, such as a promoter and an associated transcribable DNA sequence, coding sequence or gene, when such a combination is man-made and not normally found in nature.

In addition, the term "recombinant" in reference to a polynucleotide (DNA or RNA) molecule, protein, construct, vector, etc., refers to a polynucleotide or protein molecule or sequence that is man-made and not normally found in nature, and/or is present in a context in which it is not normally found in nature, including a polynucleotide (DNA or RNA) molecule, protein, construct, etc., comprising a combination of polynucleotide or protein sequences that would not naturally occur contiguously or in close proximity together without human intervention, and/or a polynucleotide molecule, protein, construct, etc., comprising at least two polynucleotide or protein sequences that are heterologous with respect to each other. A recombinant polynucleotide or protein molecule, construct, etc., can comprise polynucleotide or protein sequence(s) that is/are (i) separated from other polynucleotide or protein sequence(s) that exist in proximity to each other in nature, and/or (ii) adjacent to (or contiguous with) other polynucleotide or protein sequence(s) that are not naturally in proximity with each other. Such a recombinant polynucleotide molecule, protein, construct, etc., can also refer to a polynucleotide or protein molecule or sequence that has been genetically engineered and/or constructed outside of a cell. For example, a recombinant DNA molecule can comprise any suitable plasmid, vector, etc., and can include a linear or circular DNA molecule. Such plasmids, vectors, etc., can contain various maintenance elements including a prokaryotic origin of replication and selectable marker, as well as one or more transgenes or expression cassettes perhaps in addition to a plant selectable marker gene, etc.

As used herein, "adjacent" refers to a nucleic acid sequence that is in close proximity, or next to another nucleic acid sequence. In one aspect, adjacent nucleic acid sequences are physically linked. In another aspect, adjacent nucleic acid sequences or genes are immediately next to each other such that there are no intervening nucleotides between the end of a first nucleic acid sequence and the start of a second nucleic acid sequence. In an aspect, a first gene and a second gene are adjacent to each other if they are separated by less than 50,000, less than 25,000, less than 10,000, less than 9000, less than 8000, less than 7000, less than 6000, less than 5000, less than 4000, less than 3000, less than 2500, less than 2000, less than 1750, less than 1500, less than 1250, less than 1000, less than 900, less than 800, less than 700, less than 600, less than 500, less than 400, less than 300, less than 200, less than 100, less than 75, less than 50, less than 25, less than 20, less than 10, less than 5, less than 4, less than 3, less than 2, or less than 1 nucleotides.

In one aspect, methods and compositions provided herein comprise a vector. As used herein, the terms "vector" or "plasmid" are used interchangeably and refer to a circular, double-stranded DNA molecule that is physically separate from chromosomal DNA. In one aspect, a plasmid or vector used herein is capable of replication *in vivo.* A "transformation vector," as used herein, is a plasmid that is capable of transforming a plant cell. In an aspect, a plasmid provided herein is a bacterial plasmid. In another aspect, a plasmid provided herein is an *Agrobacterium* Ti plasmid or derived from an *Agrobacterium* Ti plasmid.

In one aspect, a plasmid or vector provided herein is a recombinant vector. As used herein, the term "recombinant vector" refers to a vector formed by laboratory methods of genetic recombination, such as molecular cloning. In another aspect, a plasmid provided herein is a synthetic plasmid. As used herein, a "synthetic plasmid" is an artificially created plasmid that is capable of the same functions (*e.g.,* replication) as a natural plasmid (*e.g.,* Ti plasmid). Without being limited, one skilled in the art can create a synthetic plasmid *de novo* via synthesizing a plasmid by individual nucleotides, or by splicing together nucleic acid molecules from different pre-existing plasmids.

As used herein, "modified", in the context of plants, seeds, plant components, plant cells, and plant genomes, refers to a state containing changes or variations from their natural or native state. For instance, a "native transcript" of a gene refers to an RNA transcript that is generated from an unmodified gene. Typically, a native transcript is a sense transcript. Modified plants or seeds contain molecular changes in their genetic materials, including either genetic or epigenetic modifications. Typically, modified plants or seeds, or a parental or progenitor line thereof, have been subjected to mutagenesis, genome editing (*e.g.,* without being limiting, via methods using site-specific nucleases), genetic transformation (*e.g.,* without being limiting, via methods of *Agrobacterium* transformation or microprojectile bombardment), or a combination thereof. In one aspect, a modified plant provided herein comprises no non-plant genetic material or sequences. In yet another aspect, a modified plant provided herein comprises no interspecies genetic material or sequences. In one aspect, this disclosure provides methods and compositions related to modified plants, seeds, plant components, plant cells, and products made from modified plants, seeds, plant parts, and plant cells. In one aspect, a modified seed provided herein gives rise to a modified plant provided herein. In one aspect, a modified plant, seed, plant component, plant cell, or plant genome provided herein comprises a recombinant DNA construct or vector provided herein. In another aspect, a product provided herein comprises modified a plant, plant component, plant cell, or plant chromosome or genome provided herein. The present disclosure provides modified plants with desirable or enhanced properties, *e.g*.. without being limiting, disease, insect, or pest tolerance (for example, virus tolerance, bacteria tolerance, fungus tolerance, nematode tolerance, arthropod tolerance, gastropod tolerance); herbicide tolerance; environmental stress resistance; quality improvements such as yield, nutritional enhancements, environmental or stress tolerances; any desirable changes in plant physiology, growth, development, morphology or plant product(s) including starch production, modified oils production, high oil production, modified fatty acid content, high protein production, fruit ripening, enhanced animal and human nutrition, biopolymer production, pharmaceutical peptides and secretable peptides production; improved processing traits; improved digestibility; low raffinose; industrial enzyme production; improved flavor; nitrogen fixation; hybrid seed production; and fiber production.

As used herein, "genome editing" or editing refers to targeted mutagenesis, insertion, deletion, inversion, substitution, or translocation of a nucleotide sequence of interest in a genome using a targeted editing technique. A nucleotide sequence of interest can be of any length, *e.g.,* at least 1, at least 2, at least 3, at least 4, at least 5, at least 6, at least 7, at least 8, at least 9, at least 10, at least 15, at least 20, at least 25, at least 30, at least 35, at least 40, at least 45, at least 50, at least 75, at least 100, at least 250, at least 500, at least 1000, at least 2500, at least 5000, at least 10,000, or at least 25,000 nucleotides. As used herein, a "targeted editing technique" refers to any method, protocol, or technique that allows the precise and/or targeted editing of a specific location in a genome (*e.g.,* the editing is not random). Without being limiting, use of a site-specific nuclease is one example of a targeted editing technique. Another non-limiting example of a targeted editing technique is the use of one or more tether guide Oligos (tgOligos). As used herein, a "targeted edit" refers to a targeted mutagenesis, insertion, deletion, inversion, or substitution caused by a targeted editing technique. A nucleotide sequence of interest can be an endogenous genomic sequence or a transgenic sequence.

In one aspect, a "targeted editing technique" refers to any method, protocol, or technique that allows the precise and/or targeted editing of a specific location in a genome (e.g., the editing is not random). Without being limiting, use of a site-specific nuclease is one example of a targeted editing technique.

In one aspect, a targeted editing technique is used to edit an endogenous locus or an endogenous gene. In another aspect, a targeted editing technique is used to edit a transgene. As used herein, an "endogenous gene" or a "native copy" of a gene refers to a gene that originates from within a given organism, cell, tissue, genome, or chromosome. An "endogenous gene" or a "native copy" of a gene is a gene that was not previously modified by human action.

As used herein, a "locus" refers to a specific position on a chromosome or other nucleic acid molecule. Without being limiting, a locus can comprise a polynucleotide that encodes a protein or an RNA. A locus can also comprise a non-coding RNA. A locus can comprise a gene. A locus can comprise a promoter, a 5'-untranslated region (UTR), an exon, an intron, a 3'-UTR, or any combination thereof. A locus can comprise a coding region.

As used herein, "physically linked" refers to two or more loci that are positioned on the same nucleic acid molecule.

As used herein, a "coding region," a "gene region," or a "gene" refers to a polynucleotide that can produce a functional unit (*e.g..* without being limiting, for example, a protein, or a non-coding RNA molecule). A "coding region," "gene," or "gene region" can comprise a promoter, an enhancer sequence, a leader sequence, a transcriptional start site, a transcriptional stop site, a polyadenylation site, one or more exons, one or more introns, a 5'-UTR, a 3'-UTR, or any combination thereof. A "coding region sequence," a "gene sequence," or a "gene region sequence" can comprise a polynucleotide sequence encoding a promoter, an enhancer sequence, a leader sequence, a transcriptional start site, a transcriptional stop site, a polyadenylation site, one or more exons, one or more introns, a 5'-UTR, a 3'-UTR, or any combination thereof. In one aspect, a "gene" encodes a non-coding RNA molecule or a precursor thereof. In another aspect, a "gene" encodes a protein.

Non-limiting examples of a non-coding RNA molecule include a microRNA (miRNA), a miRNA precursor (pre-miRNA), a small interfering RNA (siRNA), a small RNA (18-26 nt in length) and precursor encoding same, a heterochromatic siRNA (hc-siRNA), a Piwi-interacting RNA (piRNA), a hairpin double strand RNA (hairpin dsRNA), a *trans-*acting siRNA (ta-siRNA), a naturally occurring antisense siRNA (nat-siRNA), a CRISPR RNA (crRNA), a tracer RNA (tracrRNA), a guide RNA (gRNA), and a single-guide RNA (sgRNA). In one aspect, a non-coding RNA provided herein is selected from the group consisting of a microRNA, a small interfering RNA, a secondary small interfering RNA, a transfer RNA, a ribosomal RNA, a *trans-*acting small interfering RNA, a naturally occurring antisense small interfering RNA, a heterochromatic small interfering RNA, and precursors thereof. In another aspect, a non-coding RNA provided herein is selected from the group consisting of a miRNA, a pre-miRNA, a siRNA, a hc-siRNA, a piRNA, a hairpin dsRNA, a ta-siRNA, a nat-siRNA, a crRNA, a tracrRNA, a gRNA, and a sgRNA. Non-coding RNAs are often 100% complementary, or at least 80%, at least 81%, at least 82%, at least 83%, at least 84%, at least 85%, at least 90%, at least 91%, at least 92%, at least 93%, at least 94%, at least 95%, at least 96%, at least 97%, at least 98%, at least 99%, at least 99.5% complementary, to a non-coding RNA target site. A non-coding RNA target site can be present in a DNA molecule or an RNA molecule. Non-coding RNA target sites can be hybridized (or bound) by a non-coding RNA to effect various outcomes. For instance, some non-coding RNAs (without being limiting, *e.g*., miRNA, siRNA, ta-siRNA) assist in the cleavage of mRNA transcripts comprising a complementary non-coding RNA target site. Alternatively, a non-coding RNA (without being limiting, *e.g.,* miRNA, siRNA, ta-siRNA) can assist with the inhibition of protein translation of an mRNA transcript by binding to a non-coding RNA target site in the mRNA. Some non-coding RNAs (without being limiting, *e.g.*, hc-siRNA) assist with inducing epigenetic changes to DNA. In one aspect, a non-coding RNA target site is a miRNA target site or an siRNA target site. In another aspect, a gene provided herein comprises at least one, at least two, at least three, at least four, at least five, at least six, at least seven, at least eight, at least nine, or at least ten non-coding RNA target sites. In another aspect, a gene provided herein comprises at least one, at least two, at least three, at least four, at least five, at least six, at least seven, at least eight, at least nine, or at least ten heterologous non-coding RNA target sites. In another aspect, a dominant negative allele provided herein comprises at least one, at least two, at least three, at least four, at least five, at least six, at least seven, at least eight, at least nine, or at least ten non-coding RNA target sites. In another aspect, a dominant negative allele provided herein comprises at least one, at least two, at least three, at least four, at least five, at least six, at least seven, at least eight, at least nine, or at least ten heterologous non-coding RNA target sites. In an aspect, an endogenous gene provided herein is modified to comprise at least one, at least two, at least three, at least four, at least five, at least six, at least seven, at least eight, at least nine, or at least ten heterologous non-coding RNA target sites.

As used herein, an "allele" refers to a variant of a given locus or gene in a genome. If the same allele is present on both chromosomes of a chromosome pair in a cell the cell is considered homozygous at the given locus. If each member of the chromosome pair comprises a different allele for the given locus the cell is heterozygous for the locus. A minimum of one allele is possible for a given locus, although typically multiple alleles are possible for any given locus in a genome.

As used herein, the terms "suppress," "inhibit," "inhibition," "inhibiting", and "downregulation" are defined as any method known in the art or described herein that decreases the expression or function of a gene product (*e.g*., an mRNA, a protein, a non-coding RNA). "Inhibition" can be in the context of a comparison between two cells, for example, a modified cell versus a control cell. Inhibition of expression or function of a gene product can also be in the context of a comparison between plant cells, organelles, organs, tissues, or plant components within the same plant or between different plants, and includes comparisons between developmental or temporal stages within the same plant or plant component or between plants or plant components. "Inhibition" includes any relative decrement of function or production of a gene product of interest, up to and including complete elimination of function or production of that gene product. The term "inhibition" encompasses any method or composition that down-regulates translation and/or transcription of the target gene product or functional activity of the target gene product. "Inhibition" need not comprise complete elimination of expression of a gene product. In an aspect, a gene product in a modified cell provided herein comprises expression that is at least 1%, at least 2%, at least 3%, at least 4%, at least 5%, at least 10%, at least 20%, at least 25%, at least 30%, at least 40%, at least 50%, at least 60%, at least 70%, at least 80%, at least 90%, at least 95%, or 100% lower than the expression of the gene product in a control cell. In another aspect, a gene product in a modified cell provided herein comprises expression that is between 1% and 100%, between 1% and 95%, between 1% and 90%, between 1% and 80%, between 1% and 70%, between 1% and 60%, between 1% and 50%, between 1% and 40%, between 1% and 30%, between 1% and 25%, between 1% and 20%, between 1% and 15%, between 1% and 10%, between 1% and 5%, between 5% and 25%, between 5% and 50%, between 5% and 75%, between 5% and 100%, between 10% and 25%, between 10% and 50%, between 10% and 75%, between 10% and 100%, between 25% and 50%, between 25% and 75%, between 25% and 100%, or between 50% and 100% lower than the expression of the gene product in a control cell.

As used herein, a "target site" refers to a location of a polynucleotide sequence that is bound to and cleaved by a site-specific nuclease introducing a double stranded break into the nucleic acid backbone. In another aspect a target site comprises at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 29, or at least 30 consecutive nucleotides. In another aspect, a target site provided herein is at least 10, at least 20, at least 30, at least 40, at least 50, at least 75, at least 100, at least 125, at least 150, at least 200, at least 250, at least 300, at least 400, or at least 500 nucleotides. In one aspect a site-specific nuclease binds to a target site. In another aspect a site-specific nuclease binds to a target site via a guiding non-coding RNA (*i.e.,* such as, without being limiting, a CRISPR RNA or single-guide RNA (both described in detail below)). In one aspect, a non-coding RNA provided herein is complementary to a target site. It will be appreciated that perfect complementarity is not required for a non-coding RNA to bind to a target site; at least 1, at least 2, at least 3, at least 4, or at least 5, at least 6, at least 7 or at least 8 mismatches between a target site and a non-coding RNA can be tolerated. As used herein, a "target region" or a "targeted region" refers to a polynucleotide sequence that desired to be modified. In one aspect, a "target region," "targeted region", or a "target gene" is flanked by two or more, three or more, four or more, five or more, six or more, seven or more, eight or more, nine or more, or ten or more target sites. A "target gene" refers to a polynucleotide sequence encoding a gene that is desired to be modified. In one aspect, a polynucleotide sequence comprising a target gene further comprises one or more target sites. In another aspect, a target region comprises one or more, two or more, three or more, four or more, five or more, six or more, seven or more, eight or more, nine or more, or ten or more target genes. Without being limiting, in one aspect a target region can be subject to deletion or inversion. As used herein, "flanked" when used to describe a target region, refers to two or more target sites physically surrounding the target region, with one target site on each side of the target region.

A target site can be positioned in a polynucleotide sequence encoding a leader, an enhancer, a transcriptional start site, a promoter, a 5'-UTR, an exon, an intron, a 3'-UTR, a polyadenylation site, or a termination sequence. It will be appreciated that a target site can be also be positioned upstream or downstream of a sequence encoding a leader, an enhancer, a transcriptional start site, a promoter, a 5'-UTR, an exon, an intron, a 3'-UTR, a polyadenylation site, or a termination sequence. In one aspect, a target site is positioned within 10, within 20, within 30, within 40, within 50, within 75, within 100, within 125, within 150, within 200, within 250, within 300, within 400, within 500, within 600, within 700, within 800, within 900, within 1000, within 1250, within 1500, within 2000, within 2500, within 5000, within 10,000, or within 25,000 nucleotides of a polynucleotide encoding a leader, an enhancer, a transcriptional start site, a promoter, a 5'-UTR, an exon, an intron, a 3'-UTR, a polyadenylation site, a gene, or a termination sequence.

As used herein, "upstream" refers to a nucleic acid sequence that is positioned before the 5' end of a linked nucleic acid sequence. As used herein, "downstream" refers to a nucleic acid sequence is positioned after the 3' end of a linked nucleic acid sequence. As used herein, "5''' refers to the start of a coding DNA sequence or the beginning of an RNA molecule. As used herein, "3'" refers to the end of a coding DNA sequence or the end of an RNA molecule. It will be appreciated that an "inversion" refers to reversing the orientation of a given polynucleotide sequence. For example, if the sample sequence 5'-ATGATC-3' is inverted it will read 5'-CTAGTA-3' in reverse orientation. Additionally, the sample sequence 5'-ATGATC-3' is considered to be in "opposite orientation" to the sample sequence 5'-CTAGTA-3'.

As used herein a "donor molecule" is defined as a nucleic acid sequence that has been selected for site directed, targeted insertion into a genome. In an aspect, a donor molecule comprises a "donor sequence." In one aspect, a targeted editing technique provided herein comprises the use of one or more, two or more, three or more, four or more, or five or more donor molecules or donor sequences. A donor molecule or donor sequence provided herein can be of any length. For example, a donor molecule or donor sequence provided herein is between 2 and 50,000, between 2 and 10,000, between 2 and 5000, between 2 and 1000, between 2 and 500, between 2 and 250, between 2 and 100, between 2 and 50, between 2 and 30, between 15 and 50, between 15 and 100, between 15 and 500, between 15 and 1000, between 15 and 5000, between 18 and 30, between 18 and 26, between 20 and 26, between 20 and 50, between 20 and 100, between 20 and 250, between 20 and 500, between 20 and 1000, between 20 and 5000 or between 20 and 10,000 nucleotides in length. A donor molecule or donor sequence can comprise one or more genes that encode actively transcribed and/or translated gene sequences. Such transcribed sequences can encode a protein or a non-coding RNA. In one embodiment, the donor molecule or donor sequence can comprise a polynucleotide sequence which does not comprise a functional gene or an entire gene (*i.e.,* the donor molecule can simply comprise regulatory sequences such as a promoter), or does not contain any identifiable gene expression elements or any actively transcribed gene sequence. Further, the donor molecule or donor sequence can be can be linear or circular, and can be single-stranded or double-stranded. It can be delivered to the cell as naked nucleic acid, as a complex with one or more delivery agents (*e.g*., liposomes, poloxamers, T-strand encapsulated with proteins, etc.) or contained in a bacterial or viral delivery vehicle, such as, for example, *Agrobacterium tumefaciens* or a geminivirus, respectively. In another aspect, a donor molecule or donor sequence provided herein is operably linked to a promoter. In a still further aspect, a donor molecule or donor sequence provided herein is transcribed into RNA. In another aspect, a donor molecule or donor sequence provided herein is not operably linked to a promoter.

In an aspect, a donor molecule or donor sequence provided herein can comprise at least one, at least two, at least three, at least four, at least five, at least six, at least seven, at least eight, at least nine, or at least ten genes. In an aspect, a donor molecule or donor sequence provided herein comprises no genes. Without being limiting, a gene provided herein can include an insecticidal resistance gene, an herbicide tolerance gene, a nitrogen use efficiency gene, a water use efficiency gene, a nutritional quality gene, a DNA binding gene, a selectable marker gene, an RNAi construct, a site-specific genome modification enzyme gene, a single guide RNA of a CRISPR/Cas9 system, a geminivirus based expression cassette, or a plant viral expression vector system. In one aspect, a donor molecule or donor sequence comprises a polynucleotide that encodes a promoter. In another aspect, a donor molecule or donor sequence provided herein comprises a polynucleotide that encodes a tissue-specific or tissue-preferred promoter. In still another aspect, a donor molecule or donor sequence provided herein comprises a polynucleotide that encodes a constitutive promoter. In another aspect, a donor molecule or donor sequence provided herein comprises a polynucleotide that encodes an inducible promoter. In another aspect, a donor molecule or donor sequence comprises a polynucleotide that encodes a structure selected from the group consisting of a leader, an enhancer, a transcriptional start site, a 5'-UTR, an exon, an intron, a 3'-UTR, a polyadenylation site, a transcriptional termination site, a promoter, a full-length gene, a partial gene, a gene, or a non-coding RNA. In one aspect, a donor molecule or donor sequence provided herein comprises one or more, two or more, three or more, four or more, or five or more designed elements.

As used herein, a "donor template", which may be a recombinant DNA donor template, is defined as a nucleic acid molecule having a nucleic acid template or insertion sequence for site-directed, targeted insertion or recombination into the genome of a plant cell via repair of a nick or double-stranded DNA break in the genome of a plant cell. For example, a "donor template" may be used for site-directed integration of a DNA segment encoding an antisense sequence of interest, or as a template to introduce a mutation, such as an insertion, deletion, etc., into a target site within the genome of a plant. A targeted genome editing technique provided herein may comprise the use of one or more, two or more, three or more, four or more, or five or more donor templates. A "donor template" may be a single-stranded or double-stranded DNA or RNA molecule or plasmid. An "insertion sequence" of a donor template is a sequence designed for targeted insertion into the genome of a plant cell, which may be of any suitable length. For example, the insertion sequence of a donor template may be between 2 and 50,000, between 2 and 10,000, between 2 and 5000, between 2 and 1000, between 2 and 500, between 2 and 250, between 2 and 100, between 2 and 50, between 2 and 30, between 15 and 50, between 15 and 100, between 15 and 500, between 15 and 1000, between 15 and 5000, between 18 and 30, between 18 and 26, between 20 and 26, between 20 and 50, between 20 and 100, between 20 and 250, between 20 and 500, between 20 and 1000, between 20 and 5000, between 20 and 10,000, between 50 and 250, between 50 and 500, between 50 and 1000, between 50 and 5000, between 50 and 10,000, between 100 and 250, between 100 and 500, between 100 and 1000, between 100 and 5000, between 100 and 10,000, between 250 and 500, between 250 and 1000, between 250 and 5000, or between 250 and 10,000 nucleotides or base pairs in length. A donor template may also have at least one homology sequence or homology arm, such as two homology arms, to direct the integration of a mutation or insertion sequence into a target site within the genome of a plant via homologous recombination, wherein the homology sequence or homology arm(s) are identical or complementary, or have a percent identity or percent complementarity, to a sequence at or near the target site within the genome of the plant. When a donor template comprises homology arm(s) and an insertion sequence, the homology arm(s) will flank or surround the insertion sequence of the donor template.

A donor template may be linear or circular and may be single-stranded or double-stranded. A donor template may be delivered to the cell as a naked nucleic acid (*e.g.,* via particle bombardment), as a complex with one or more delivery agents (*e.g*., liposomes, proteins, poloxamers, T-strand encapsulated with proteins, etc.), or contained in a bacterial or viral delivery vehicle, such as, for example, *Agrobacterium tumefaciens* or a geminivirus, respectively. An insertion sequence of a donor template or insertion sequence provided herein may comprise a transcribable DNA sequence or segment that may be transcribed into all or a portion of an RNA molecule, such as an antisense sequence or portion of a RNA molecule.

As used herein, a "designed element" refers to a polynucleotide capable of inducing a desired expression pattern of an operably linked polynucleotide. In one aspect, a designed element comprises at least 10, at least 11, at least 12, at least 13, at least 14, at least 15, at least 16, at least 17, at least 18, at least 19, at least 20, at least 25, at least 30, at least 35, at least 40, at least 45, at least 50, at least 60, at least 70, at least 80, at least 90, at least 100, at least 200, at least 300, at least 400, at least 500, at least 600, at least 700, at least 800, at least 900, at least 1000, at least 1500, at least 2000, at least 2500, at least 3000, at least 4000, or at least 5000 nucleotides. In another aspect, a designed element comprises 20-50 nucleotides. In still another aspect, a designed element comprises between 10 and 5000, between 10 and 2500, between 10 and 1000, between 10 and 500, between 10 and 100, between 20 and 50, between 20 and 100, between 20 and 500, between 20 and 1000, between 20 and 2000, between 20 and 5000, between 50 and 100, between 50 and 500, between 50 and 1000, or between 50 and 5000 nucleotides. In one aspect, a designed element comprises a constitutive promoter. In another aspect, a designed element comprises an inducible promoter. In another aspect, a designed element comprises a tissue-specific or tissue-preferred promoter. In another aspect, a designed element comprises a native promoter. In another aspect, a designed element comprises a non-native promoter. In another aspect, a designed element comprises a tissue-specific or tissue-preferred promoter element. In another aspect, a designed element comprises a transcriptional enhancer element. In another aspect, a designed element comprises a transcriptional repressor element.

One aspect of the present application relates to methods of screening and selecting cells for targeted edits and methods of selecting cells comprising targeted edits. Nucleic acids can be isolated using various techniques. For example, nucleic acids can be isolated using any method including, without limitation, recombinant nucleic acid technology, and/or the polymerase chain reaction (PCR). General PCR techniques are described, for example in PCR Primer: A Laboratory Manual, Dieffenbach & Dveksler, Eds., Cold Spring Harbor Laboratory Press, 1995. Recombinant nucleic acid techniques include, for example, restriction enzyme digestion and ligation, which can be used to isolate a nucleic acid. Isolated nucleic acids also can be chemically synthesized, either as a single nucleic acid molecule or as a series of oligonucleotides. Polypeptides can be purified from natural sources (e.g., a biological sample) by known methods such as DEAE ion exchange, gel filtration, and hydroxyapatite chromatography. A polypeptide also can be purified, for example, by expressing a nucleic acid in an expression vector. In addition, a purified polypeptide can be obtained by chemical synthesis. The extent of purity of a polypeptide can be measured using any appropriate method, *e.g.,* column chromatography, polyacrylamide gel electrophoresis, or HPLC analysis.

In one aspect, this disclosure provides methods of detecting recombinant nucleic acids and polypeptides in modified and unmodified plant cells. Without being limiting, nucleic acids also can be detected using hybridization. Hybridization between nucleic acids is discussed in detail in Sambrook et al. (1989, Molecular Cloning: A Laboratory Manual, 2nd Ed., Cold Spring Harbor Laboratory Press, Cold Spring Harbor, NY).

Polypeptides can be detected using antibodies. Techniques for detecting polypeptides using antibodies include enzyme linked immunosorbent assays (ELISAs), Western blots, immunoprecipitations and immunofluorescence. An antibody provided herein can be a polyclonal antibody or a monoclonal antibody. An antibody having specific binding affinity for a polypeptide provided herein can be generated using methods well known in the art. An antibody provided herein can be attached to a solid support such as a microtiter plate using methods known in the art.

Detection (*e.g.,* of an amplification product, of a hybridization complex, of a polypeptide) can be accomplished using detectable labels. The term "label" is intended to encompass the use of direct labels as well as indirect labels. Detectable labels include enzymes, prosthetic groups, fluorescent materials, luminescent materials, bioluminescent materials, and radioactive materials.

The screening and selection of modified, engineered, or transgenic plants or plant cells can be through any methodologies known to those having ordinary skill in the art. Examples of screening and selection methodologies include, but are not limited to, Southern analysis, PCR amplification for detection of a polynucleotide, Northern blots, RNase protection, primer-extension, RT-PCR amplification for detecting RNA transcripts, Sanger sequencing, Next Generation sequencing technologies (*e.g*., Illumina, PacBio, Ion Torrent, 454) enzymatic assays for detecting enzyme or ribozyme activity of polypeptides and polynucleotides, and protein gel electrophoresis, Western blots, immunoprecipitation, and enzyme-linked immunoassays to detect polypeptides. Other techniques such as *in situ* hybridization, enzyme staining, and immunostaining also can be used to detect the presence or expression of polypeptides and/or polynucleotides. Methods for performing all of the referenced techniques are known.

Genome editing or targeted editing can be effected via the use of one or more site-specific nucleases. Site-specific nucleases can induce a double-stranded break (DSB) at a target site of a genome sequence that is then repaired by the natural processes of either homologous recombination (HR) or non-homologous end-joining (NHEJ). Sequence modifications, such as insertions, deletions, can occur at the DSB locations via NHEJ repair. If two DSBs flanking one target region are created, the breaks can be repaired via NHEJ by reversing the orientation of the targeted DNA (also referred to as an "inversion"). HR can be used to integrate a donor nucleic acid sequence into a target site. Without being limited by any theory, in order to integrate a donor nucleic acid sequence (or donor molecule) into a DSB, the donor molecule comprises a polynucleotide of interest flanked by a first and second homologous region, where the first and second homologous regions are homologous to each side of the DSB at the target site. Homologous recombination machinery in the cell then repairs the DSB by integrating the donor molecule into the target site. In one aspect, a double-stranded break provided herein is repaired by NHEJ. In another aspect, a double-stranded break provided herein is repaired by HR.

Although a double-stranded break only occurs between two nucleotides on each strand, a double-stranded break site can comprise at least 2, at least 3, at least 4, at least 5, at least 6, at least 7, at least 8, at least 9, at least 10, at least 15, at least 20, at least 25, at least 30, at least 40, at least 50, at least 60, at least 70, at least 75, at least 80, at least 90, or at least 100 nucleotides. As used herein, a "double-stranded break site" refers to a polynucleotide sequence that is recognized and bound by a site-specific nuclease or guide RNA.

In an aspect, a vector or construct provided herein comprises polynucleotides encoding at least 1, at least 2, at least 3, at least 4, at least 5, at least 6, at least 7, at least 8, at least 9, or at least 10 site-specific nuclease. In another aspect, a cell provided herein already comprises a site-specific nuclease. In an aspect, a polynucleotide encoding a site-specific nuclease provided herein is stably transformed into a cell. In another aspect, a polynucleotide encoding a site-specific nuclease provided herein is transiently transformed into a cell. In another aspect, a polynucleotide encoding a site-specific nuclease is under the control of a regulatable promoter, a constitutive promoter, a tissue specific promoter, or any promoter useful for expression of the site-specific nuclease.

In one aspect, a vector comprises *in cis* a cassette encoding a site-specific nuclease and a donor molecule such that when contacted with the genome of a cell, the site-specific nuclease enables site-specific integration of the donor molecule. In one aspect, a first vector comprises a cassette encoding a site-specific nuclease and a second vector comprises a donor molecule such that when contacted with the genome of a cell, the site-specific nuclease provided *in trans* enables site-specific integration of the donor molecule.

Site-specific nucleases provided herein can be used as part of a targeted editing technique. Non-limiting examples of site-specific nucleases used in methods and/or compositions provided herein include meganucleases, zinc finger nucleases (ZFNs), transcription activator-like effector nucleases (TALENs), RNA-guided nucleases (*e.g*., Cas9 and Cpf1), a recombinase (without being limiting, for example, a serine recombinase attached to a DNA recognition motif, a tyrosine recombinase attached to a DNA recognition motif), a transposase (without being limiting, for example, a DNA transposase attached to a DNA binding domain), or any combination thereof. In one aspect, a method provided herein comprises the use of one or more, two or more, three or more, four or more, or five or more site-specific nucleases to induce one, two, three, four, five, or more than five DSBs at one, two, three, four, five, or more than five target sites.

In one aspect, a site-specific nuclease protein is provided to a cell. In another aspect, a nucleic acid sequence (*e.g*., a vector) encoding a site-specific nuclease protein is provided to a cell. In another aspect, a site-specific nuclease protein and a guide RNA are provided to a cell separately. In another aspect, a site-specific nuclease protein and a guide RNA are provided to a cell as a complex. In an aspect, a site-specific nuclease protein and a guide RNA are assembled into a complex *in vitro, in vivo,* or *ex vivo.*

In one aspect, a genome editing system provided herein (*e.g.,* a meganuclease, a ZFN, a TALEN, a CRISPR/Cas9 system, a CRISPR/Cpf1 system, a recombinase, a transposase), or a combination of genome editing systems provided herein, is used in a method to introduce one or more insertions, deletions, substitutions, or inversions to a locus in a cell to generate a dominant negative allele or a dominant positive allele.

Site-specific nucleases, such as meganucleases, ZFNs, TALENs, Argonaute proteins (non-limiting examples of Argonaute proteins include *Thermus thermophilus* Argonaute (TtAgo), *Pyrococcus furiosus* Argonaute (PfAgo), *Natronobacterium gregoryi* Argonaute (NgAgo), homologs thereof, or modified versions thereof), Cas9 nucleases (non-limiting examples of RNA-guided nucleases include Cas1, Cas1B, Cas2, Cas3, Cas4, Cas5, Cas6, Cas7, Cas8, Cas9 (also known as Csn1 and Csx12), Cas10, Csy1, Csy2, Csy3, Csel, Cse2, Csc1, Csc2, Csa5, Csn2, Csm2, Csm3, Csm4, Csm5, Csm6, Cmrl, Cmr3, Cmr4, Cmr5, Cmr6, Csb1, Csb2, Csb3, Csx17, Csx14, Csx10, Csx16, CsaX, Csx3, Csx1, Csx15, Csfl, Csf2, Csf3, Csf4, Cpf1, CasX, CasY, homologs thereof, or modified versions thereof), induce a double-strand DNA break at the target site of a genomic sequence that is then repaired by the natural processes of HR or NHEJ. Sequence modifications then occur at the cleaved sites, which can include inversions, deletions, or insertions that result in gene disruption in the case of NHEJ, or integration of nucleic acid sequences by HR.

In an aspect, a site-specific nuclease provided herein is selected from the group consisting of a zinc-finger nuclease, a meganuclease, an RNA-guided nuclease, a TALE-nuclease, a recombinase, a transposase, or any combination thereof. In another aspect, a site-specific nuclease provided herein is selected from the group consisting of a Cas9 or a Cpfl. In another aspect a site-specific nuclease provided herein is selected from the group consisting of a Cas1, a Cas1B, a Cas2, a Cas3, a Cas4, a Cas5, a Cas6, a Cas7, a Cas8, a Cas9, a Cas10, a Csy1, a Csy2, a Csy3, a Csel, a Cse2, a Csc1, a Csc2, a Csa5, a Csn2, a Csm2, a Csm3, a Csm4, a Csm5, a Csm6, a Cmr1, a Cmr3, a Cmr4, a Cmr5, a Cmr6, a Csb1, a Csb2, a Csb3, a Csx17, a Csx14, a Csx10, a Csx16, a CsaX, a Csx3, a Csx1, a Csx15, a Csf1, a Csf2, a Csf3, a Csf4, a Cpfl, a CasX, a CasY, a homolog thereof, or a modified version thereof. In another aspect, an RNA-guided nuclease provided herein is selected from the group consisting of a Cas9 or a Cpf1. In another aspect an RNA guided nuclease provided herein is selected from the group consisting of a Cas1, a Cas1B, a Cas2, a Cas3, a Cas4, a Cas5, a Cas6, a Cas7, a Cas8, a Cas9, a Cas10, a Csy1, a Csy2, a Csy3, a Csel, a Cse2, a Csc1, a Csc2, a Csa5, a Csn2, a Csm2, a Csm3, a Csm4, a Csm5, a Csm6, a Cmr1, a Cmr3, a Cmr4, a Cmr5, a Cmr6, a Csb1, a Csb2, a Csb3, a Csx17, a Csx14, a Csxl 0, a Csx16, a CsaX, a Csx3, a Csx1, a Csx15, a Csf1, a Csf2, a Csf3, a Csf4, a Cpfl, a CasX, a CasY, a homolog thereof, or a modified version thereof. In another aspect, an RNA-guided nuclease is a Cas9 nuclease or a homolog or modified version thereof. In one aspect, an RNA-guided nuclease is a Cas9 protein, or a modified version thereof, from *Streptococcus pyogenes, Streptococcus thermophilius, Staphylococcus aureus, Neisseria meningitides,* or *Treponema denticola.* In another aspect, an RNA-guided nuclease is Cpfl or a homolog or modified version thereof.

In another aspect, a method and/or a composition provided herein comprises at least one, at least two, at least three, at least four, at least five, at least six, at least seven, at least eight, at least nine, or at least ten site-specific nucleases. In yet another aspect, a method and/or a composition provided herein comprises at least one, at least two, at least three, at least four, at least five, at least six, at least seven, at least eight, at least nine, or at least ten polynucleotides encoding at least one, at least two, at least three, at least four, at least five, at least six, at least seven, at least eight, at least nine, or at least ten site-specific nucleases.

In one aspect, a targeted editing technique described herein comprises the use of a recombinase. In an aspect, a tyrosine recombinase attached to a DNA recognition motif provided herein is selected from the group consisting of a Cre recombinase, a *Gin* recombinase a Flp recombinase, and a Tnp1 recombinase. In an aspect, a Cre recombinase or a *Gin* recombinase provided herein is tethered to a zinc-finger DNA binding domain. The Flp*-FRT* site-directed recombination system comes from the 2µ plasmid from the baker's yeast *Saccharomyces cerevisiae.* In this system, Flp recombinase (flippase) recombines sequences between flippase recognition target (*FRT*) sites. *FRT* sites comprise 34 nucleotides. Flp binds to the "arms" of the *FRT* sites (one arm is in reverse orientation) and cleaves the *FRT* site at either end of an intervening nucleic acid sequence. After cleavage, Flp recombines nucleic acid sequences between two *FRT* sites. Cre-lox is a site-directed recombination system derived from the bacteriophage P1 that is similar to the Flp*-FRT* recombination system. Cre-lox can be used to invert a nucleic acid sequence, delete a nucleic acid sequence, or translocate a nucleic acid sequence. In this system, Cre recombinase recombines a pair of lox nucleic acid sequences. Lox sites comprise 34 nucleotides, with the first and last 13 nucleotides (arms) being palindromic. During recombination, Cre recombinase protein binds to two lox sites on different nucleic acids and cleaves at the lox sites. The cleaved nucleic acids are spliced together (reciprocally translocated) and recombination is complete. In another aspect, a lox site provided herein is a loxP, lox 2272, loxN, lox 511, lox 5171, lox71, lox66, M2, M3, M7, or M11 site.

In another aspect, a serine recombinase attached to a DNA recognition motif provided herein is selected from the group consisting of a PhiC31 integrase, an R4 integrase, and a TP-901 integrase. In another aspect, a DNA transposase attached to a DNA binding domain provided herein is selected from the group consisting of a TALE-piggyBac and TALE-Mutator.

In one aspect, a targeted editing technique described herein comprises the use of a zinc-finger nuclease (ZFN). ZFNs are synthetic proteins consisting of an engineered zinc finger DNA-binding domain fused to the cleavage domain of the *FokI* restriction nuclease. ZFNs can be designed to cleave almost any long stretch of double-stranded DNA for modification of the zinc finger DNA-binding domain. ZFNs form dimers from monomers composed of a non-specific DNA cleavage domain of *FokI* nuclease fused to a zinc finger array engineered to bind a target DNA sequence.

The DNA-binding domain of a ZFN is typically composed of 3-4 zinc-finger arrays. The amino acids at positions -1, +2, +3, and +6 relative to the start of the zinc finger ∞-helix, which contribute to site-specific binding to the target DNA, can be changed and customized to fit specific target sequences. The other amino acids form the consensus backbone to generate ZFNs with different sequence specificities. Rules for selecting target sequences for ZFNs are known in the art.

The *FokI* nuclease domain requires dimerization to cleave DNA and therefore two ZFNs with their C-terminal regions are needed to bind opposite DNA strands of the cleavage site (separated by 5-7 nt). The ZFN monomer can cute the target site if the two-ZF-binding sites are palindromic. The term ZFN, as used herein, is broad and includes a monomeric ZFN that can cleave double stranded DNA without assistance from another ZFN. The term ZFN is also used to refer to one or both members of a pair of ZFNs that are engineered to work together to cleave DNA at the same site.

Without being limited by any scientific theory, because the DNA-binding specificities of zinc finger domains can in principle be re-engineered using one of various methods, customized ZFNs can theoretically be constructed to target nearly any gene sequence. Publicly available methods for engineering zinc finger domains include Context-dependent Assembly (CoDA), Oligomerized Pool Engineering (OPEN), and Modular Assembly.

In one aspect, a method and/or composition provided herein comprises one or more, two or more, three or more, four or more, or five or more ZFNs. In another aspect, a ZFN provided herein is capable of generating a targeted DSB. In one aspect, vectors comprising polynucleotides encoding one or more, two or more, three or more, four or more, or five or more ZFNs are provided to a cell by transformation methods known in the art (e.g., without being limiting, viral transfection, particle bombardment, PEG-mediated protoplast transfection or *Agrobacterium*-mediated transformation).

In one aspect, a targeted editing technique described herein comprises the use of a meganuclease. Meganucleases, which are commonly identified in microbes, are unique enzymes with high activity and long recognition sequences (> 14 nt) resulting in site-specific digestion of target DNA. Engineered versions of naturally occurring meganucleases typically have extended DNA recognition sequences (for example, 14 to 40 nt). The engineering of meganucleases can be more challenging than that of ZFNs and TALENs because the DNA recognition and cleavage functions of meganucleases are intertwined in a single domain. Specialized methods of mutagenesis and high-throughput screening have been used to create novel meganuclease variants that recognize unique sequences and possess improved nuclease activity.

In one aspect, a method and/or composition provided herein comprises one or more, two or more, three or more, four or more, or five or more meganucleases. In another aspect, a meganuclease provided herein is capable of generating a targeted DSB. In one aspect, vectors comprising polynucleotides encoding one or more, two or more, three or more, four or more, or five or more meganucleases are provided to a cell by transformation methods known in the art (*e.g.,* without being limiting, viral transfection, particle bombardment, PEG-mediated protoplast transfection or *Agrobacterium-*mediated transformation).

In one aspect, a targeted editing technique described herein comprises the use of a transcription activator-like effector nuclease (TALEN). TALENs are artificial restriction enzymes generated by fusing the transcription activator-like effector (TALE) DNA binding domain to a *FokI* nuclease domain. When each member of a TALEN pair binds to the DNA sites flanking a target site, the *FokI* monomers dimerize and cause a double-stranded DNA break at the target site. Besides the wild-type *FokI* cleavage domain, variants of the *FokI* cleavage domain with mutations have been designed to improve cleavage specificity and cleavage activity. The *FokI* domain functions as a dimer, requiring two constructs with unique DNA binding domains for sites in the target genome with proper orientation and spacing. Both the number of amino acid residues between the TALEN DNA binding domain and the *FokI* cleavage domain and the number of bases between the two individual TALEN binding sites are parameters for achieving high levels of activity.

TALENs are artificial restriction enzymes generated by fusing the transcription activator-like effector (TALE) DNA binding domain to a nuclease domain. In one aspect, the nuclease is selected from a group consisting of *PvuII, MutH, TevI* and *FokI, AlwI, MlyI, SbfI, SdaI, StsI, CleDORF, Clo051, Pept071.* When each member of a TALEN pair binds to the DNA sites flanking a target site, the *FokI* monomers dimerize and cause a double-stranded DNA break at the target site.

The term TALEN, as used herein, is broad and includes a monomeric TALEN that can cleave double stranded DNA without assistance from another TALEN. The term TALEN is also used to refer to one or both members of a pair of TALENs that work together to cleave DNA at the same site.

Transcription activator-like effectors (TALEs) can be engineered to bind practically any DNA sequence. TALE proteins are DNA-binding domains derived from various plant bacterial pathogens of the genus *Xanthomonas.* The X pathogens secrete TALEs into the host plant cell during infection. The TALE moves to the nucleus, where it recognizes and binds to a specific DNA sequence in the promoter region of a specific DNA sequence in the promoter region of a specific gene in the host genome. TALE has a central DNA-binding domain composed of 13-28 repeat monomers of 33-34 amino acids. The amino acids of each monomer are highly conserved, except for hypervariable amino acid residues at positions 12 and 13. The two variable amino acids are called repeat-variable diresidues (RVDs). The amino acid pairs NI, NG, HD, and NN of RVDs preferentially recognize adenine, thymine, cytosine, and guanine/adenine, respectively, and modulation of RVDs can recognize consecutive DNA bases. This simple relationship between amino acid sequence and DNA recognition has allowed for the engineering of specific DNA binding domains by selecting a combination of repeat segments containing the appropriate RVDs.

Besides the wild-type *FokI* cleavage domain, variants of the *FokI* cleavage domain with mutations have been designed to improve cleavage specificity and cleavage activity. The *FokI* domain functions as a dimer, requiring two constructs with unique DNA binding domains for sites in the target genome with proper orientation and spacing. Both the number of amino acid residues between the TALEN DNA binding domain and the *FokI* cleavage domain and the number of bases between the two individual TALEN binding sites are parameters for achieving high levels of activity. *PvuII, MutH,* and *TevI* cleavage domains are useful alternatives to *FokI* and *FokI* variants for use with TALEs. *PvuII* functions as a highly specific cleavage domain when coupled to a TALE (*see* Yank et al. 2013. PLoS One. 8: e82539). *MutH* is capable of introducing strand-specific nicks in DNA (*see* Gabsalilow et al. 2013. Nucleic Acids Research. 41: e83). *TevI* introduces double-stranded breaks in DNA at targeted sites(*see* Beurdeley et al., 2013. Nature Communications. 4: 1762).

The relationship between amino acid sequence and DNA recognition of the TALE binding domain allows for designable proteins. Software programs such as DNA Works can be used to design TALE constructs. Other methods of designing TALE constructs are known to those of skill in the art. *See* Doyle et al., Nucleic Acids Research (2012) 40: W117-122; Cermak et al., Nucleic Acids Research (2011). 39:e82; and tale-nt.cac.cornell.edu/about.

In one aspect, a method and/or composition provided herein comprises one or more, two or more, three or more, four or more, or five or more TALENs. In another aspect, a TALEN provided herein is capable of generating a targeted DSB. In one aspect, vectors comprising polynucleotides encoding one or more, two or more, three or more, four or more, or five or more TALENs are provided to a cell by transformation methods known in the art (*e.g.,* without being limiting, viral transfection, particle bombardment, PEG-mediated protoplast transfection or *Agrobacterium-mediated* transformation).

In one aspect, a targeted editing technique described herein comprises the use of a RNA-guided nuclease. A CRISPR/Cas9 system or a CRISPR/Cpf1 system are alternatives to the *FokI*-based methods ZFN and TALEN. The CRISPR systems are based on RNA-guided engineered nucleases that use complementary base pairing to recognize DNA sequences at target sites.

In an aspect, a vector provided herein can comprise any combination of a nucleic acid sequence encoding a RNA-guided nuclease (non-limiting examples of RNA-guided nucleases include Cas1, Cas1B, Cas2, Cas3, Cas4, Cas5, Cas6, Cas7, Cas8, Cas9 (also known as Csn1 and Csx12), Cas10, Csy1, Csy2, Csy3, Csel, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, Cpfl, CasX, CasY, homologs thereof, or modified versions thereof); and, optionally, a guide RNA necessary for targeting the respective nucleases. As used herein, the term "guide RNA" or gRNA generally refers to an RNA molecule (or a group of RNA molecules collectively) that can bind to an RNA-guided endonuclease and aid in targeting the nuclease to a specific location within a target polynucleotide (e.g., a DNA).

While not being limited by any particular scientific theory, CRISPR/Cas nucleases are part of the adaptive immune system of bacteria and archaea, protecting them against invading nucleic acids such as viruses by cleaving target DNA in a sequence-dependent manner. The immunity is acquired by the integration of short fragments of the invading DNA, known as spacers, between ~20 nucleotide long CRISPR repeats at the proximal end of a CRISPR locus (a CRISPR array). A well described Cas protein is the Cas9 nuclease (also known as Csn1), which is part of the Class 2, type II CRISPR/Cas system in *Streptococcus pyogenes. See* Makarova et al. Nature Reviews Microbiology (2015) doi: 10.1038/nrmicro3569. Cas9 comprises an RuvC-like nuclease domain at its amino terminus and an HNH-like nuclease domain positioned in the middle of the protein. Cas9 proteins also contain a PAM-interacting (PI) domain, a recognition lobe (REC), and a BH domain. The Cpfl nuclease, another type II system, acts in a similar manner to Cas9, but Cpfl does not require a tracrRNA. *See* Cong et al. Science (2013) 339: 819-823; Zetsche et al., Cell (2015) doi: 10.1016/j.cell.2015.09.038; U. S. Patent Publication No. 2014/0068797; U. S. Patent Publication No. 2014/0273235; U. S. Patent Publication No. 2015/0067922; U. S. Patent No. 8,697,359; U. S. Patent No. 8,771,945; U. S. Patent No. 8,795,965; U. S. Patent No. 8,865,406; U. S. Patent No. 8,871,445; U. S. Patent No. 8,889,356; U. S. Patent No. 8,889,418; U. S. Patent No. 8,895,308; and U. S. Patent No. 8,906,616, each of which is herein incorporated by reference in its entirety.

When Cas9 or Cpfl cleaves targeted DNA, endogenous double stranded break (DSB) repair mechanisms are activated. DSBs can be repaired via non-homologous end joining, which can incorporate insertions or deletions (indels) into the targeted locus. If two DSBs flanking one target region are created, the breaks can be repaired by reversing the orientation of the targeted DNA. Alternatively, if a donor polynucleotide with homology to the target DNA sequence is provided, the DSB can be repaired via homology-directed repair. This repair mechanism allows for the precise integration of a donor polynucleotide into the targeted DNA sequence.

While not being limited by any particular scientific theory, in Class 2, type II CRISPR/Cas systems, CRISPR arrays, including spacers, are transcribed during encounters with recognized invasive DNA and are processed into small interfering CRISPR RNAs (crRNAs), which are approximately 40 nucleotides in length. The crRNAs hybridize with trans-activating crRNAs (tracrRNAs) to activate and guide the Cas9 nuclease to a target site. Nucleic acid molecules provided herein can combine a crRNA and a tracrRNA into one nucleic acid molecule in what is herein referred to as a "single-chain guide RNA (sgRNA)." A prerequisite for cleavage of the target site by Cas9 is the presence of a conserved protospacer-adjacent motif (PAM) downstream of the target DNA which usually has the sequence 5-NGG-3 but less frequently NAG. Specificity is provided by the so-called "seed sequence" approximately 12 bases upstream of the PAM, which must match between the RNA and target DNA. Cpfl acts in a similar manner to Cas9, but Cpfl does not require a tracrRNA. Therefore, in an aspect utilizing Cpfl a sgRNA can be replaced by a crRNA. The PAM motif of Cpfl is upstream of the target site. Additionally, for Cpfl orthologs LbCpf1 and AsCpf1, the PAM sequence is 5-TTTV-3 where V can be A, C, or G. In an aspect, when two or more sgRNAs are provided herein, the first sgRNA and the second sgRNA are complementary to different strands of a double-stranded DNA molecule. In another aspect, when two or more sgRNAs are provided herein, the first sgRNA and the second sgRNA are complementary to the same strand of a double-stranded DNA molecule. As used herein, a "protospacer adjacent motif"(PAM) refers to a 2-6 base pair DNA sequence immediately upstream or downstream of a target sequence of a CRISPR complex.In another aspect, a first and a second gRNA target different PAM sequences. In another aspect, a first and a second gRNA targetthe same PAM sequences.

In one aspect, a method and/or composition provided herein comprises one or more, two or more, three or more, four or more, or five or more Cas9 nucleases. In one aspect, a method and/or composition provided herein comprises one or more polynucleotides encoding one or more, two or more, three or more, four or more, or five or more Cas9 nucleases. In another aspect, a Cas9 nuclease provided herein is capable of generating a targeted DSB. In one aspect, a method and/or composition provided herein comprises one or more, two or more, three or more, four or more, or five or more Cpfl nucleases. In one aspect, a method and/or composition provided herein comprises one or more polynucleotides encoding one or more, two or more, three or more, four or more, or five or more Cpfl nucleases. In another aspect, a Cpf1 nuclease provided herein is capable of generating a targeted DSB.

When a Cas9 nuclease hybridizes to a target site via an sgRNA, Cas9 produces two blunt-end cuts in the double-stranded DNA. The "target strand" of the double-stranded DNA is complementary to the sgRNA, while the "non-target strand" comprises the PAM motif adjacent to, and on the 3' end of, the cut site on the non-target strand. Cas9 holds the target stand and the PAM motif, but the 3' cut end of the non-target strand is free and is referred to as the "3' flap." In one aspect, the 3' flap comprises at least 10, at least 15, at least 20, at least 25, at least 26, at least 27, at least 28, at least 29, at least 30, at least 31, at least 32, at least 33, at least 34, at least 35, at least 36, at least 37, at least 38, at least 39, or at least 40 nucleotides.

In one aspect, vectors comprising polynucleotides encoding a site-specific nuclease, and optionally one or more, two or more, three or more, or four or more sgRNAs are provided to a plant cell by transformation methods known in the art (e.g., without being limiting, particle bombardment, PEG-mediated protoplast transfection or *Agrobacterium-mediated* transformation). In one aspect, vectors comprising polynucleotides encoding a Cas9 nuclease, and optionally one or more, two or more, three or more, or four or more sgRNAs are provided to a plant cell by transformation methods known in the art (*e.g.*, without being limiting, particle bombardment, PEG-mediated protoplast transfection or *Agrobacterium-mediated* transformation).In another aspect, vectors comprising polynucleotides encoding a Cpfl and, optionally one or more, two or more, three or more, or four or more crRNAs are provided to a cell by transformation methods known in the art (*e.g.,* without being limiting, viral transfection, particle bombardment, PEG-mediated protoplast transfection or *Agrobacterium-mediated* transformation).

In one aspect, an RNA-guided nuclease provided herein is selected from the group consisting of Cas1, Cas1B, Cas2, Cas3, Cas4, Cas5, Cas6, Cas7, Cas8, Cas9 (also known as Csn1 and Csx12), Cas10, Csy1, Csy2, Csy3, Csel, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, Cpfl, CasX, CasY, homologs thereof, or modified versions thereof, an Argonaute (non-limiting examples of Argonaute proteins include *Thermus thermophilus* Argonaute (TtAgo), *Pyrococcus furiosus* Argonaute (PfAgo), *Natronobacterium gregoryi* Argonaute (NgAgo), homologs thereof, modified versions thereof), a DNA guide for an Argonaute protein, and any combination thereof. In another aspect, an RNA-guided nuclease provided herein is selected from the group consisting of Cas9 and Cpf1. an RNA-guided nuclease provided herein comprises Cas9. In one aspect, an RNA-guided nuclease provided herein is selected from the group consisting of Cas1, Cas1B, Cas2, Cas3, Cas4, Cas5, Cas6, Cas7, Cas8, Cas9 (also known as Csn1 and Csx12), Cas10, Csy1, Csy2, Csy3, Csel, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, Cpfl, CasX, CasY, homologs thereof, or modified versions thereof. In one aspect a site-specific nuclease is selected from the group consisting of Cas1, Cas1B, Cas2, Cas3, Cas4, Cas5, Cas6, Cas7, Cas8, Cas9, Cas10, Csy1, Csy2, Csy3, Csel, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, Cpfl, CasX, CasY, TtAgo, PfAgo, and NgAgo. In another aspect, an RNA-guided nuclease is selected from the group consisting of Cas1, Cas1B, Cas2, Cas3, Cas4, Cas5, Cas6, Cas7, Cas8, Cas9, Cas10, Csy1, Csy2, Csy3, Csel, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, Cpfl, CasX, CasY, TtAgo, PfAgo, and NgAgo.

Nucleases, such as Cas9, can also be engineered to form a catalytically deactivated from, such catalytically deactivated Cas9 (dCas9). dCas9 binds to DNA at a target site specified by a gRNA and creates a loop structure accessible for template-based editing (Figure 11, Panel 1). dCas9 can be further modified to form a fusion with a ssDNA binding domain for further facilitating template-based editing (Figure 11, Panel 2). The editing efficiency with this modified dCas9-ssDNA binding scheme is expected to be higher compared to a dCas9-alone approach, because a ssDNA template is bound to dCas9 complex and would be brought into proximity of the gRNA target. As used herein, a "deactivated Cas nuclease" (dCas) refers to an enzymatically inactive form of a Cas nuclease protein which can bind DNA, but cannot cleave DNA. In one aspect, a nuclease provided herein is a dCas. In another aspect, a site-specific nuclease provided herein is a dCas.

In one aspect, methods and compositions provided herein can be used to edit a locus in a eukaryotic cell. In one aspect, a eukaryotic cell provided herein is part of a multicellular eukaryotic organism. In another aspect, a eukaryotic cell provided herein is a unicellular organism. In another aspect, a eukaryotic cell provided herein is selected from the group consisting of an animal cell, a plant cell, a fungus cell, and a protozoan cell. In one aspect, an animal cell provided herein is selected from the group consisting of an insect cell, an arachnid cell, an arthropod cell, a crustacean cell, a rotifer cell, a cnidarian cell, a Platyhelminthes cell, a mollusk cell, a gastropod cell, a nematode cell, an annelid cell, a vertebrate cell, a mammal cell, an avian cell, a fish cell, a reptile cell, and an amphibian cell. In another aspect a plant cell provided herein is a monocot cell or a dicot cell. In still another aspect a plant cell provided herein is an algae cell. In yet another aspect, a plant cell provided herein is selected from the group consisting of a corn cell, a wheat cell, a sorghum cell, a canola cell, a soybean cell, an alfalfa cell, a cotton cell, and a rice cell. In still another aspect, a plant cell provided herein is selected from the group consisting of an Acacia cell, an alfalfa cell, an aneth cell, an apple cell, an apricot cell, an artichoke cell, an arugula cell, an asparagus cell, an avocado cell, a banana cell, a barley cell, a bean cell, a beet cell, a blackberry cell, a blueberry cell, a broccoli cell, a Brussels sprout cell, a cabbage cell, a canola cell, a cantaloupe cell, a carrot cell, a cassava cell, a cauliflower cell, a celery cell, a Chinese cabbage cell, a cherry cell, a cilantro cell, a citrus cell, a clementine cell, a coffee cell, a corn cell, a cotton cell, a cucumber cell, a Douglas fir cell, an eggplant cell, an endive cell, an escarole cell, an eucalyptus cell, a fennel cell, a fig cell, a forest tree cell, a gourd cell, a grape cell, a grapefruit cell, a honey dew cell, a jicama cell, kiwifruit cell, a lettuce cell, a leek cell, a lemon cell, a lime cell, a Loblolly pine cell, a mango cell, a maple tree cell, a melon cell, a mushroom cell, a nectarine cell, a nut cell, an oat cell, an okra cell, an onion cell, an orange cell, an ornamental plant cell, a papaya cell, a parsley cell, a pea cell, a peach cell, a peanut cell, a pear cell, a pepper cell, a persimmon cell, a pine cell, a pineapple cell, a plantain cell, a plum cell, a pomegranate cell, a poplar cell, a potato cell, a pumpkin cell, a quince cell, a radiata pine cell, a radicchio cell, a radish cell, a rapeseed cell, a raspberry cell, a rice cell, a rye cell, a sorghum cell, a Southern pine cell, a soybean cell, a spinach cell, a squash cell, a strawberry cell, a sugar beet cell, a sugarcane cell, a sunflower cell, a sweet corn cell, a sweet potato cell, a sweetgum cell, a tangerine cell, a tea cell, a tobacco cell, a tomato cell, a turf cell, a vine cell, watermelon cell, a wheat cell, a yam cell, and a zucchini cell. In another aspect, a plant cell provided herein is selected from the group consisting of a corn cell, a soybean cell, a canola cell, a cotton cell, a wheat cell, and a sugarcane cell.

In still another aspect an engineered plant provided herein is an algae. In yet another aspect, an engineered plant or seed provided herein is selected from the group consisting of a corn plant, a wheat plant, a sorghum plant, a canola plant, a soybean plant, an alfalfa plant, a cotton plant, and a rice plant. In still another aspect, an engineered plant or seed provided herein is selected from the group consisting of an Acacia plant, an alfalfa plant, an aneth plant, an apple plant, an apricot plant, an artichoke plant, an arugula plant, an asparagus plant, an avocado plant, a banana plant, a barley plant, a bean plant, a beet plant, a blackberry plant, a blueberry plant, a broccoli plant, a Brussels sprout plant, a cabbage plant, a canola plant, a cantaloupe plant, a carrot plant, a cassava plant, a cauliflower plant, a celery plant, a Chinese cabbage plant, a cherry plant, a cilantro plant, a citrus plant, a clementine plant, a coffee plant, a corn plant, a cotton plant, a cucumber plant, a Douglas fir plant, an eggplant plant, an endive plant, an escarole plant, an eucalyptus plant, a fennel plant, a fig plant, a forest tree plant, a gourd plant, a grape plant, a grapefruit plant, a honey dew plant, a jicama plant, kiwifruit plant, a lettuce plant, a leek plant, a lemon plant, a lime plant, a Loblolly pine plant, a mango plant, a maple tree plant, a melon plant, a mushroom plant, a nectarine plant, a nut plant, an oat plant, an okra plant, an onion plant, an orange plant, an ornamental plant, a papaya plant, a parsley plant, a pea plant, a peach plant, a peanut plant, a pear plant, a pepper plant, a persimmon plant, a pine plant, a pineapple plant, a plantain plant, a plum plant, a pomegranate plant, a poplar plant, a potato plant, a pumpkin plant, a quince plant, a radiata pine plant, a radicchio plant, a radish plant, a rapeseed plant, a raspberry plant, a rice plant, a rye plant, a sorghum plant, a Southern pine plant, a soybean plant, a spinach plant, a squash plant, a strawberry plant, a sugar beet plant, a sugarcane plant, a sunflower plant, a sweet corn plant, a sweet potato plant, a sweetgum plant, a tangerine plant, a tea plant, a tobacco plant, a tomato plant, a turf plant, a vine plant, watermelon plant, a wheat plant, a yam plant, and a zucchini plant. In another aspect, a plant provided herein is selected from the group consisting of a corn plant, a soybean plant, a canola plant, a cotton plant, a wheat plant, and a sugarcane plant.

In still another aspect a modified plant provided herein is an algae. In yet another aspect, a modified plant provided herein is selected from the group consisting of a corn plant, a wheat plant, a sorghum plant, a canola plant, a soybean plant, an alfalfa plant, a cotton plant, and a rice plant. In still another aspect, a modified plant provided herein is selected from the group consisting of an Acacia plant, an alfalfa plant, an aneth plant, an apple plant, an apricot plant, an artichoke plant, an arugula plant, an asparagus plant, an avocado plant, a banana plant, a barley plant, a bean plant, a beet plant, a blackberry plant, a blueberry plant, a broccoli plant, a Brussels sprout plant, a cabbage plant, a canola plant, a cantaloupe plant, a carrot plant, a cassava plant, a cauliflower plant, a celery plant, a Chinese cabbage plant, a cherry plant, a cilantro plant, a citrus plant, a clementine plant, a coffee plant, a corn plant, a cotton plant, a cucumber plant, a Douglas fir plant, an eggplant plant, an endive plant, an escarole plant, an eucalyptus plant, a fennel plant, a fig plant, a forest tree plant, a gourd plant, a grape plant, a grapefruit plant, a honey dew plant, a jicama plant, kiwifruit plant, a lettuce plant, a leek plant, a lemon plant, a lime plant, a Loblolly pine plant, a mango plant, a maple tree plant, a melon plant, a mushroom plant, a nectarine plant, a nut plant, an oat plant, an okra plant, an onion plant, an orange plant, an ornamental plant, a papaya plant, a parsley plant, a pea plant, a peach plant, a peanut plant, a pear plant, a pepper plant, a persimmon plant, a pine plant, a pineapple plant, a plantain plant, a plum plant, a pomegranate plant, a poplar plant, a potato plant, a pumpkin plant, a quince plant, a radiata pine plant, a radicchio plant, a radish plant, a rapeseed plant, a raspberry plant, a rice plant, a rye plant, a sorghum plant, a Southern pine plant, a soybean plant, a spinach plant, a squash plant, a strawberry plant, a sugar beet plant, a sugarcane plant, a sunflower plant, a sweet corn plant, a sweet potato plant, a sweetgum plant, a tangerine plant, a tea plant, a tobacco plant, a tomato plant, a turf plant, a vine plant, watermelon plant, a wheat plant, a yam plant, and a zucchini plant.

In yet another aspect, a modified seed provided herein is selected from the group consisting of a corn seed, a wheat seed, a sorghum seed, a canola seed, a soybean seed, an alfalfa seed, a cotton seed, and a rice seed. In still another aspect, a modified seed provided herein is selected from the group consisting of an Acacia seed, an alfalfa seed, an aneth seed, an apple seed, an apricot seed, an artichoke seed, an arugula seed, an asparagus seed, an avocado seed, a banana seed, a barley seed, a bean seed, a beet seed, a blackberry seed, a blueberry seed, a broccoli seed, a Brussels sprout seed, a cabbage seed, a canola seed, a cantaloupe seed, a carrot seed, a cassava seed, a cauliflower seed, a celery seed, a Chinese cabbage seed, a cherry seed, a cilantro seed, a citrus seed, a clementine seed, a coffee seed, a corn seed, a cotton seed, a cucumber seed, a Douglas fir seed, an eggplant seed, an endive seed, an escarole seed, an eucalyptus seed, a fennel seed, a fig seed, a forest tree seed, a gourd seed, a grape seed, a grapefruit seed, a honey dew seed, a jicama seed, kiwifruit seed, a lettuce seed, a leek seed, a lemon seed, a lime seed, a Loblolly pine seed, a mango seed, a maple tree seed, a melon seed, a mushroom seed, a nectarine seed, a nut seed, an oat seed, an okra seed, an onion seed, an orange seed, an ornamental plant seed, a papaya seed, a parsley seed, a pea seed, a peach seed, a peanut seed, a pear seed, a pepper seed, a persimmon seed, a pine seed, a pineapple seed, a plantain seed, a plum seed, a pomegranate seed, a poplar seed, a potato seed, a pumpkin seed, a quince seed, a radiata pine seed, a radicchio seed, a radish seed, a rapeseed seed, a raspberry seed, a rice seed, a rye seed, a sorghum seed, a Southern pine seed, a soybean seed, a spinach seed, a squash seed, a strawberry seed, a sugar beet seed, a sugarcane seed, a sunflower seed, a sweet corn seed, a sweet potato seed, a sweetgum seed, a tangerine seed, a tea seed, a tobacco seed, a tomato seed, a turf seed, a vine seed, watermelon seed, a wheat seed, a yam seed, and a zucchini seed.

In still another aspect a modified chromosome provided herein is an algae. In yet another aspect, a modified chromosome provided herein is selected from the group consisting of a corn chromosome, a wheat chromosome, a sorghum chromosome, a canola chromosome, a soybean chromosome, an alfalfa chromosome, a cotton chromosome, and a rice chromosome. In still another aspect, a modified chromosome provided herein is selected from the group consisting of an Acacia chromosome, an alfalfa chromosome, an aneth chromosome, an apple chromosome, an apricot chromosome, an artichoke chromosome, an arugula chromosome, an asparagus chromosome, an avocado chromosome, a banana chromosome, a barley chromosome, a bean chromosome, a beet chromosome, a blackberry chromosome, a blueberry chromosome, a broccoli chromosome, a Brussels sprout chromosome, a cabbage chromosome, a canola chromosome, a cantaloupe chromosome, a carrot chromosome, a cassava chromosome, a cauliflower chromosome, a celery chromosome, a Chinese cabbage chromosome, a cherry chromosome, a cilantro chromosome, a citrus chromosome, a clementine chromosome, a coffee chromosome, a corn chromosome, a cotton chromosome, a cucumber chromosome, a Douglas fir chromosome, an eggplant chromosome, an endive chromosome, an escarole chromosome, an eucalyptus chromosome, a fennel chromosome, a fig chromosome, a forest tree chromosome, a gourd chromosome, a grape chromosome, a grapefruit chromosome, a honey dew chromosome, a jicama chromosome, kiwifruit chromosome, a lettuce chromosome, a leek chromosome, a lemon chromosome, a lime chromosome, a Loblolly pine chromosome, a mango chromosome, a maple tree chromosome, a melon chromosome, a mushroom chromosome, a nectarine chromosome, a nut chromosome, an oat chromosome, an okra chromosome, an onion chromosome, an orange chromosome, an plant chromosome chromosome, a papaya chromosome, a parsley chromosome, a pea chromosome, a peach chromosome, a peanut chromosome, a pear chromosome, a pepper chromosome, a persimmon chromosome, a pine chromosome, a pineapple chromosome, a plantain chromosome, a plum chromosome, a pomegranate chromosome, a poplar chromosome, a potato chromosome, a pumpkin chromosome, a quince chromosome, a radiata pine chromosome, a radicchio chromosome, a radish chromosome, a rapeseed chromosome, a raspberry chromosome, a rice chromosome, a rye chromosome, a sorghum chromosome, a Southern pine chromosome, a soybean chromosome, a spinach chromosome, a squash chromosome, a strawberry chromosome, a sugar beet chromosome, a sugarcane chromosome, a sunflower chromosome, a sweet corn chromosome, a sweet potato chromosome, a sweetgum chromosome, a tangerine chromosome, a tea chromosome, a tobacco chromosome, a tomato chromosome, a turf chromosome, a vine chromosome, watermelon chromosome, a wheat chromosome, a yam chromosome, and a zucchini chromosome.

In one aspect, a cell provided herein is a modified cell. In another aspect, a plant provided herein is a modified plant. In yet another aspect, a plant cell provided herein is a modified plant cell. In still another aspect, a seed provided herein is a modified seed. In a further aspect, a chromosome provided herein is a modified chromosome.

According to one aspect, a modified plant, plant cell, cell, seed, or chromosome provided herein comprises at least one, at least two, at least three, at least four, at least five, at least six, at least seven, at least eight, at least nine, or at least ten deletions generated by a targeted editing technique. According to one aspect, a modified plant, plant cell, cell, seed, or chromosome provided herein comprises at least one, at least two, at least three, at least four, at least five, at least six, at least seven, at least eight, at least nine, or at least ten insertions generated by a targeted editing technique. According to one aspect, a modified plant, plant cell, cell, seed, or chromosome provided herein comprises at least one, at least two, at least three, at least four, at least five, at least six, at least seven, at least eight, at least nine, or at least ten inversions generated by a targeted editing technique. According to one aspect, a modified plant, plant cell, cell, seed, or chromosome provided herein comprises at least one, at least two, at least three, at least four, at least five, at least six, at least seven, at least eight, at least nine, or at least ten deletions generated by a targeted editing technique, at least one, at least two, at least three, at least four, at least five, at least six, at least seven, at least eight, at least nine, or at least ten insertions generated by a targeted editing technique, at least one, at least two, at least three, at least four, at least five, at least six, at least seven, at least eight, at least nine, or at least ten inversions generated by a targeted editing technique, or any combination thereof. In still another aspect, a modified plant, plant cell, cell, seed, or chromosome provided herein comprises at least one, at least two, at least three, at least four, at least five, at least six, at least seven, at least eight, at least nine, or at least ten dominant negative alleles generated by a targeted editing technique. In still another aspect, a modified plant, plant cell, cell, seed, or chromosome provided herein comprises at least one, at least two, at least three, at least four, at least five, at least six, at least seven, at least eight, at least nine, or at least ten dominant positive alleles generated by a targeted editing technique. In still another aspect, a modified plant, plant cell, cell, seed, or chromosome provided herein comprises at least one, at least two, at least three, at least four, at least five, at least six, at least seven, at least eight, at least nine, or at least ten dominant negative alleles generated by a targeted editing technique, at least one, at least two, at least three, at least four, at least five, at least six, at least seven, at least eight, at least nine, or at least ten dominant positive alleles generated by a targeted editing technique, or any combination thereof.

According to another aspect of the present application, a modified plant(s), plant cell(s), seed(s), chromosome(s) and plant part(s) are provided comprising a genome editing event in the genome of at least one plant cell thereof, the genome editing event comprising an insertion, a deletion, a substitution, or an inversion of a targeted locus.

According to one aspect, the present disclosure provides a modified plant cell produced by any one of the methods provided herein. In another aspect, the present disclosure provides a modified chromosome produced by any one of the methods provided herein. In still another aspect, the present disclosure provides a modified cell comprising a modified chromosome provided herein. In still a further aspect, this disclosure provides a modified plant or modified plant tissue regenerated from a modified cell provided herein. In still another aspect, the present disclosure provides a product comprising a modified chromosome provided herein. In an aspect, the present disclosure provides a product comprising a modified cell provided herein. As used herein, a "product" refers to any article or substance that is intended for human use, human consumption, animal use, or animal consumption, including any component, part, or accessory that comprises a modified cell or modified chromosome provided herein.

The methods and compositions provided herein are capable of editing any locus in a genome. Also provided herein are chromosomes edited by using the methods and compositions provided herein. In an aspect, a genome provided herein is a nuclear genome, a mitochondrial genome, or a plastid genome. In another aspect, a plastid genome provided herein comprises a chloroplast genome. In one aspect, a method provided herein generates a double-stranded break on a chromosome. In an aspect, a chromosome provided herein is a nuclear chromosome, a mitochondrial chromosome, or a chloroplast chromosome. In another aspect a chromosome provided herein is a supernumerary chromosome or an artificial chromosome. Supernumerary, or B chromosomes, are extra chromosomes found in addition to the normal diploid complement of chromosomes in a cell. Supernumerary chromosomes are dispensable and not required for normal development of a cell or organism. In an aspect, a supernumerary chromosome provided herein is a maize supernumerary chromosome or a rye supernumerary chromosome.

A method of targeted editing disclosed herein can involve transient transfection or stable transformation of a cell of interest (e.g., a plant cell). According to one aspect of the present application, methods are provided for transforming a cell, tissue or explant with a recombinant DNA molecule or construct comprising a transcribable DNA sequence or transgene operably linked to a promoter to produce a transgenic or genome edited cell. According to another aspect of the present application, methods are provided for transforming a plant cell, tissue or explant with a recombinant DNA molecule or construct comprising a transcribable DNA sequence or transgene operably linked to a plant-expressible promoter to produce a transgenic or genome edited plant or plant cell. As used herein, a "transgene" refers to a polynucleotide that has been transferred into a genome by any method known in the art.

Numerous methods for transforming chromosomes or plastids in a plant cell with a recombinant DNA molecule or construct are known in the art, which can be used according to methods of the present application to produce a transgenic plant cell and plant. Any suitable method or technique for transformation of a plant cell known in the art can be used according to present methods. Effective methods for transformation of plants include bacterially mediated transformation, such as *Agrobacterium-mediated* or *Rhizobium-*mediated transformation and microprojectile bombardment-mediated transformation. A variety of methods are known in the art for transforming explants with a transformation vector via bacterially mediated transformation or microprojectile bombardment and then subsequently culturing, etc., those explants to regenerate or develop transgenic plants. Other methods for plant transformation, such as microinjection, electroporation, vacuum infiltration, pressure, sonication, silicon carbide fiber agitation, PEG-mediated transformation, etc., are also known in the art. Transgenic plants produced by these transformation methods can be chimeric or non-chimeric for the transformation event depending on the methods and explants used.

Methods of transforming plant cells are well known by persons of ordinary skill in the art. For instance, specific instructions for transforming plant cells by microprojectile bombardment with particles coated with recombinant DNA are found in U.S. Patent Nos. 5,550,318; 5,538,880 6,160,208; 6,399,861; and 6,153,812 and *Agrobacterium-mediated* transformation is described in U.S. Patent Nos. 5,159,135; 5,824,877; 5,591,616: 6,384,301; 5,750,871; 5,463,174; and 5,188,958 , all of which are incorporated herein by reference. Additional methods for transforming plants can be found in, for example, Compendium of Transgenic Crop Plants (2009) Blackwell Publishing. Any appropriate method known to those skilled in the art can be used to transform a plant cell with any of the nucleic acid molecules provided herein.

Recipient cell or explant targets for transformation include, but are not limited to, a seed cell, a fruit cell, a leaf cell, a cotyledon cell, a hypocotyl cell, a meristem cell, an embryo cell, an endosperm cell, a root cell, a shoot cell, a stem cell, a pod cell, a flower cell, an inflorescence cell, a stalk cell, a pedicel cell, a style cell, a stigma cell, a receptacle cell, a petal cell, a sepal cell, a pollen cell, an anther cell, a filament cell, an ovary cell, an ovule cell, a pericarp cell, a phloem cell, a bud cell, or a vascular tissue cell. In another aspect, this disclosure provides a plant chloroplast. In a further aspect, this disclosure provides an epidermal cell, a stomata cell, a trichome cell, a root hair cell, a storage root cell, or a tuber cell. In another aspect, this disclosure provides a protoplast. In another aspect, this disclosure provides a plant callus cell. Any cell from which a fertile plant can be regenerated is contemplated as a useful recipient cell for practice of this disclosure. Callus can be initiated from various tissue sources, including, but not limited to, immature embryos or parts of embryos, seedling apical meristems, microspores, and the like. Those cells which are capable of proliferating as callus can serve as recipient cells for transformation. Practical transformation methods and materials for making transgenic plants of this disclosure (e.g., various media and recipient target cells, transformation of immature embryos, and subsequent regeneration of fertile transgenic plants) are disclosed, for example, in U. S. Patents 6,194,636 and 6,232,526 and U. S. Patent Application Publication 2004/0216189, all of which are incorporated herein by reference. Transformed explants, cells or tissues can be subjected to additional culturing steps, such as callus induction, selection, regeneration, etc., as known in the art. Transformed cells, tissues or explants containing a recombinant DNA insertion can be grown, developed or regenerated into transgenic plants in culture, plugs or soil according to methods known in the art. In one aspect, this disclosure provides plant cells that are not reproductive material and do not mediate the natural reproduction of the plant. In another aspect, this disclosure also provides plant cells that are reproductive material and mediate the natural reproduction of the plant. In another aspect, this disclosure provides plant cells that cannot maintain themselves via photosynthesis. In another aspect, this disclosure provides somatic plant cells. Somatic cells, contrary to germline cells, do not mediate plant reproduction. In one aspect, this disclosure provides a non-reproductive plant cell.

Modified plants can be further crossed to themselves or other plants to produce modified seeds and progeny. A modified plant can also be prepared by crossing a first plant comprising a recombinant DNA sequence insertion with a second plant lacking the insertion. For example, a recombinant DNA sequence can be introduced into a first plant line that is amenable to transformation, which can then be crossed with a second plant line to introgress the recombinant DNA sequence into the second plant line. A modified plant can also be prepared by crossing a modified plant with an unmodified plant. Progeny of these crosses can be further back crossed into the more desirable line multiple times, such as through 6 to 8 generations or back crosses, to produce a progeny plant with substantially the same genotype as the original parental line but for the introduction of the recombinant DNA construct or modified sequence.

A modified plant, cell, or explant provided herein can be of an elite variety or an elite line. An elite variety or an elite line refers to any variety that has resulted from breeding and selection for superior agronomic performance. A modified plant, cell, or explant provided herein can be a hybrid plant, cell, or explant. As used herein, a "hybrid" is created by crossing two plants from different varieties, lines, or species, such that the progeny comprises genetic material from each parent. Skilled artisans recognize that higher order hybrids can be generated as well. For example, a first hybrid can be made by crossing Variety C with Variety D to create a C x D hybrid, and a second hybrid can be made by crossing Variety E with Variety F to create an E x F hybrid. The first and second hybrids can be further crossed to create the higher order hybrid (C x D) x (E x F) comprising genetic information from all four parent varieties. A modified plant provided herein is fertile. A modified plant provided herein is a male or female sterile modified plant, which cannot reproduce without human intervention. In one aspect, a modified plant provided herein reproduces via asexual or vegetative reproduction. In still another aspect, a modified plant provided herein reproduces via sexual reproduction.

A recombinant DNA molecule or construct of the present application can comprise or be included within a DNA transformation vector for use in transformation of a target plant cell, tissue or explant. Such a transformation vector of the present application can generally comprise sequences or elements necessary or beneficial for effective transformation in addition to at least one selectable marker gene, at least one expression cassette and/or transcribable DNA sequence encoding one or more site-specific nucleases, and, optionally, one or more sgRNAs or crRNAs. For *Agrobacterium*-mediated transformation, the transformation vector can comprise an engineered transfer DNA (or T-DNA) segment or region having two border sequences, a left border (LB) and a right border (RB), flanking at least a transcribable DNA sequence or transgene, such that insertion of the T-DNA into the plant genome will create a transformation event for the transcribable DNA sequence, transgene or expression cassette. In other words, the transgene, a transcribable DNA sequence, transgene or expression cassette encoding the site-specific nuclease(s), and/or sgRNA(s) or crRNA(s) would be located between the left and right borders of the T-DNA, perhaps along with an additional transgene(s) or expression cassette(s), such as a plant selectable marker transgene and/or other gene(s) of agronomic interest that can confer a trait or phenotype of agronomic interest to a plant. According to an alternative aspect, the transcribable DNA sequence, transgene or expression cassette encoding at least one site-specific nuclease, any necessary sgRNAs or crRNAs, and the plant selectable marker transgene (or other gene of agronomic interest) can be present in separate T-DNA segments on the same or different recombinant DNA molecule(s), such as for co-transformation. A transformation vector or construct can further comprise prokaryotic maintenance elements, which for *Agrobacterium-*mediated transformation can be located in the vector backbone outside of the T-DNA region(s).

A plant selectable marker transgene in a transformation vector or construct of the present application can be used to assist in the selection of transformed cells or tissue due to the presence of a selection agent, such as an antibiotic or herbicide, where the plant selectable marker transgene provides tolerance or resistance to the selection agent. Thus, the selection agent can bias or favor the survival, development, growth, proliferation, etc., of transformed cells expressing the plant selectable marker gene, such as to increase the proportion of transformed cells or tissues in the R₀ plant. Commonly used plant selectable marker genes include, for example, those conferring tolerance or resistance to antibiotics, such as kanamycin and paromomycin (*nptII*), hygromycin B (*aph IV*), streptomycin or spectinomycin (*aadA*) and gentamycin (*aac3* and *aacC4*), or those conferring tolerance or resistance to herbicides such as glufosinate (*bar* or *pat*), dicamba (*DMO*) and glyphosate (*aroA* or *Cp4-EPSPS*). Plant screenable marker genes can also be used, which provide an ability to visually screen for transformants, such as luciferase or green fluorescent protein (GFP), or a gene expressing a beta glucuronidase or *uidA* gene (GUS) for which various chromogenic substrates are known. In one aspect, a vector or polynucleotide provided herein comprises at least one marker gene selected from the group consisting of *nptII*, *aphIV*, *aadA*, *aac3, aacC4, bar, pat, DMO, EPSPS, aroA,* GFP, and GUS.

According to an aspect of the present application, methods for transforming a plant cell, tissue or explant with a recombinant DNA molecule or construct can further include site-directed or targeted integration using site-specific nucleases. According to these methods, a portion of a recombinant DNA donor molecule (i.e., an insertion sequence) can be inserted or integrated at a desired site or locus within a genome. The insertion sequence of the donor template can comprise a transgene or construct, such as a designed element or a tissue-specific promoter. The donor molecule can also have one or two homology arms flanking the insertion sequence to promote the targeted insertion event through homologous recombination and/or homology-directed repair. Thus, a recombinant DNA molecule of the present application can further include a donor template for site-directed or targeted integration of a transgene or construct, such as a transgene or transcribable DNA sequence encoding a designed element or a tissue-specific promoter into a genome.

As used herein, a "portion" of a nucleic acid sequence or molecule refers to any number of nucleotides less than the full length of the nucleic sequence. For example, a portion of a 100 nucleotide nucleic acid sequence can be any number of nucleotides from 1 to 99 nucleotides. Alternatively, a "portion" of a nucleic sequence refers to anywhere from 0.01% to 99.99% of the full length of a given nucleic acid sequence.

Provided herein are methods of generating dominant alleles of gene regions using targeted editing techniques. Also provided herein are cells generated by such methods and compositions used in such methods. The instant description further provides modified plants regenerated from cells subjected to the methods provided herein. In one aspect, a dominant negative allele provided herein is capable of suppressing transcription of a locus or a gene in a heterozygous state. In another aspect, a dominant negative allele provided herein is capable of suppressing transcription of a locus or a gene in a homozygous state.

Dominant negative alleles of a gene region can reduce or eliminate the function of a gene region product in a heterozygous state. Without being limiting, a dominant negative allele can be generated by editing an allele of a gene region such that at least a portion of the polynucleotide encoding the gene region is inverted in orientation (e.g.. a portion of the gene is flipped to a 3' to 5' orientation while the rest of the gene remains in a 5' to 3' orientation). Expression of the edited allele of the gene region will comprise an antisense RNA segment that is complementary to the sense RNA expressed by the unedited gene region. Without being bound by any scientific theory, the complementary segment between the sense and antisense portions of the gene region RNAs can be processed by RNA silencing mechanisms native to the cell to reduce the expression of the edited and unedited gene region alleles in a dominant negative manner. In one aspect, an antisense RNA transcript provided herein is capable of suppressing a complementary sense RNA transcript. In another aspect, an antisense RNA transcript provided herein is suppresses a complementary sense RNA transcript.

In one aspect, this disclosure provides a method of generating a dominant negative allele of a gene in a cell comprising inverting a portion of the gene using a targeted editing technique, generating an antisense RNA transcript capable of triggering suppression of an unmodified allele. In one aspect, expression of the unmodified allele is reduced as compared to a control cell that does not comprise the antisense RNA transcript. In another aspect, a targeted editing technique provided here comprises the use of at least one site-specific nuclease. In an aspect, an antisense RNA transcript provided herein is a partial antisense RNA transcript. In another aspect, an antisense RNA transcript provided herein is a complete antisense RNA transcript. Without being limiting, a partial antisense RNA transcript can be generated by inverting only one region of a gene as opposed to inverting the entire gene. For instance, if an mRNA transcript is encoded by three exons, inverting only the second exon would enable the generation of a partial antisense RNA transcript. It will be appreciated that inverting any number of nucleotides of a gene region that is less than the length of the entire gene region can give rise to a partial antisense RNA transcript. As an example, if a gene region comprises 500 nucleotides, inverting a 200 nucleotide region will give rise to a partial antisense RNA transcript. If all 500 nucleotides were inverted a complete antisense RNA transcript would be generated. In an aspect, an antisense RNA transcript provided herein is capable of suppressing expression of a complementary nucleic acid sequence. In an aspect, an antisense RNA transcript provided herein is capable of suppressing expression of at least one, at least two, at least three, at least four, at least five, at least six, at least seven, at least eight, at least nine, or at least ten genes. In an aspect, an antisense RNA transcript provided herein is capable of suppressing expression of a first gene region. In an aspect, an antisense RNA transcript provided herein is capable of suppressing expression of a protein encoded by a complementary nucleic acid sequence. One of skill in the art would recognize that 100% complementarity is not required between an antisense RNA transcript and a second nucleic acid in order to induce suppressed expression of the second nucleic acid. For example, an antisense RNA transcript comprising at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, at least 99.5%, or 100% complementarity to a second nucleic acid sequence can be capable of suppressing expression of the second nucleic acid sequence.

In one aspect, an antisense RNA transcript transcribed by a dominant negative allele provided herein is capable of downregulating expression of itself. In another aspect, an antisense RNA transcript transcribed by a dominant negative allele provided herein is capable of downregulating expression of an unmodified allele of the same locus. In one aspect, expression of the unmodified allele is reduced as compared to a control cell that does not comprise the antisense RNA transcript.

In one aspect, this disclosure provides a method of generating a dominant negative allele of a gene in one or more cells comprising: a) inducing a first double-stranded break and a second double-stranded break flanking a targeted region of the gene: b) identifying one or more cells comprising an inversion of the targeted region of the gene, where the inversion results in the production of an antisense RNA transcript from the targeted region of the gene; and c) selecting one or more cells comprising the inversion of the targeted region of the gene.

In another aspect, this disclosure provides a method of reducing the expression of a protein in a cell comprising: a) inducing a first double-stranded break and a second double-stranded break flanking a targeted region of a chromosome; and b) identifying one or more cells comprising an inversion in the targeted region of the chromosome, where expression of the protein is reduced as compared to a control cell that does not comprise the inversion in the targeted region.

In a further aspect, this disclosure provides a method of generating an inversion in a targeted region of a gene comprising: a) providing to one or more cells at least one RNA-guided nuclease or one or more vectors encoding an RNA-guided nuclease, where the at least one RNA-guided nuclease is capable of binding to at least 18, at least 19, at least 20, at least 21, at least 22, at least 23, at least 24, at least 25, or at least 26 consecutive nucleotides of a first target site and a second target site flanking the targeted region of the gene, where the first target site and the second target site are linked, where the at least one RNA-guided nuclease creates double-stranded breaks in the gene at the first target site and the second target site; b) identifying one or more cells comprising an inversion in the targeted region of the gene, where the inversion results in the production of an antisense RNA transcript from the targeted region: and c) selecting one or more cells comprising the inversion in the targeted region of the gene.

In one aspect, a method or composition provided herein comprises at least one, at least two, at least three, at least four, at least five, at least six, at least seven, at least eight, at least nine, or at least ten site-specific nucleases. In another aspect, a method or composition provided herein comprises at least one, at least two, at least three, at least four, at least five, at least six, at least seven, at least eight, at least nine, or at least ten sgRNAs. In a further aspect, a method or composition provided herein comprises at least one, at least two, at least three, at least four, at least five, at least six, at least seven, at least eight, at least nine, or at least ten tgOligos. In another aspect, a method or composition provided herein comprises at least one, at least two, at least three, at least four, at least five, at least six, at least seven, at least eight, at least nine, or at least ten donor molecules. In another aspect, a method or composition provided herein comprises at least one, at least two, at least three, at least four, at least five, at least six, at least seven, at least eight, at least nine, or at least ten donor sequences.

In another aspect, a method or composition provided herein comprises at least one, at least two, at least three, at least four, at least six, at least seven, at least eight, at least nine, or at least ten vectors encoding at least one, at least two, at least three, at least four, at least five, at least six, at least seven, at least eight, at least nine, or at least ten site-specific nucleases. In another aspect, a method or composition provided herein comprises at least one, at least two, at least three, at least four, at least six, at least seven, at least eight, at least nine, or at least ten vectors encoding at least one, at least two, at least three, at least four, at least five, at least six, at least seven, at least eight, at least nine, or at least ten sgRNAs. In another aspect, a method or composition provided herein comprises at least one, at least two, at least three, at least four, at least six, at least seven, at least eight, at least nine, or at least ten vectors encoding at least one, at least two, at least three, at least four, at least five, at least six, at least seven, at least eight, at least nine, or at least ten tgOligos. In another aspect, a method or composition provided herein comprises at least one, at least two, at least three, at least four, at least six, at least seven, at least eight, at least nine, or at least ten vectors encoding at least one, at least two, at least three, at least four, at least five, at least six, at least seven, at least eight, at least nine, or at least ten donor molecules. In another aspect, a method or composition provided herein comprises at least one, at least two, at least three, at least four, at least six, at least seven, at least eight, at least nine, or at least ten vectors encoding at least one, at least two, at least three, at least four, at least five, at least six, at least seven, at least eight, at least nine, or at least ten donor sequences.

In one aspect, a vector provided herein encodes at least one, at least two, at least three, at least four, or at least five site-specific nucleases. In one aspect, a vector provided herein encodes at least one, at least two, at least three, at least four, or at least five RNA-guided nucleases. In another aspect, a vector provided herein encodes at least one, at least two, at least three, at least four, or at least five sgRNAs. In one aspect, a method or composition provided herein comprises one or more vectors comprising a first sgRNA and a second sgRNA. In aIn a further aspect, a vector provided herein encodes at least one, at least two, at least three, at least four, or at least five donor molecules.

In an aspect, a vector provided herein encodes at least one, at least two, at least three, at least four, or at least five site-specific nucleases and at least one, at least two, at least three, at least four, or at least five sgRNAs. In another aspect, a vector provided herein encodes at least one, at least two, at least three, at least four, or at least five RNA-guided nuclease and at least one, at least two, at least three, at least four, or at least five sgRNAs. In an aspect, a vector provided herein encodes at least one, at least two, at least three, at least four, or at least five sgRNAs and at least one, at least two, at least three, at least four, or at least five donor molecules.

In another aspect a vector provided herein encodes at least one RNA-guided nuclease, a first sgRNA, and a second sgRNA. In a further aspect, at least one RNA-guided nuclease, a first sgRNA, and a second sgRNA are encoded by two or more or three or more vectors. In another aspect, a vector provided herein encodes at least one RNA-guided nuclease, a sgRNA, and a donor molecule. In a further aspect, at least one RNA-guided nuclease, a sgRNA, and a donor molecule are encoded by two or more or three or more vectors.

In another aspect, a vector provided herein encodes at least one, at least two, at least three, at least four, or at least five site-specific nucleases and at least one, at least two, at least three, at least four, or at least five donor molecules. In one aspect, a vector provided herein encodes at least one, at least two, at least three, at least four, or at least five RNA-guided nucleases and at least one, at least two, at least three, at least four, or at least five donor molecules. In another aspect, a vector provided herein encodes at least one, at least two, at least three, at least four, or at least five site-specific nucleases, at least one, at least two, at least three, at least four, or at least five sgRNAs, and at least one, at least two, at least three, at least four, or at least five donor molecules. In another aspect, a vector provided herein encodes at least one, at least two, at least three, at least four, or at least five RNA-guided nucleases, at least one, at least two, at least three, at least four, or at least five sgRNAs, and at least one, at least two, at least three, at least four, or at least five donor molecules. In another aspect, a vector provided herein encodes at least one site-specific nuclease, at least one donor molecule, and at least one sgRNA. In another aspect, a vector provided herein encodes at least one RNA-guided nuclease, at least one donor molecule, and at least one sgRNA.

In one aspect, one or more site-specific nucleases, one or more sgRNAs, and one or more donor molecule provided herein are encoded by one vector. In one aspect, one or more site-specific nucleases, one or more sgRNAs, and one or more donor molecule provided herein are encoded by two or more or three or more vectors. In still another aspect, one or more sgRNAs, and one or more donor molecule provided herein are encoded by two or more, three or more, four or more, five or more, six or more, seven or more, eight or more, nine or more, or ten or more vectors. In one aspect, at least one RNA-guided nuclease, a first sgRNA, and a second sgRNA are encoded by one vector. In another aspect, at least one RNA-guided nuclease, a first sgRNA, and a second sgRNA are encoded by two or more or three or more vectors. In one aspect, at least one site-specific nuclease, a first sgRNA, and a second sgRNA are encoded by one vector. In another aspect, at least one site-specific nuclease, a first sgRNA, and a second sgRNA are encoded by two or more or three or more vectors. In one aspect, at least one RNA-guided nuclease, at least one sgRNA, and at least one donor molecule are encoded by one vector. In one aspect, at least one RNA-guided nuclease, at least one sgRNA, and at least one donor molecule are encoded by two or more or three or more vectors. In one aspect, at least one site-specific nuclease, at least one sgRNA, and at least one donor molecule are encoded by one vector. In one aspect, at least one site-specific nuclease, at least one sgRNA, and at least one donor molecule are encoded by two or more or three or more vectors.

In one aspect, one or more Cas9 nucleases, one or more sgRNAs, and one or more donor molecule provided herein are encoded by one vector. In one aspect, one or more Cas9 nucleases, one or more sgRNAs, and one or more donor molecule provided herein are encoded by two or more or three or more vectors. In one aspect, at least one Cas9 nuclease, a first sgRNA, and a second sgRNA are encoded by one vector. In another aspect, at least one Cas9 nuclease, a first sgRNA, and a second sgRNA are encoded by two or more or three or more vectors. In one aspect, at least one Cas9 nuclease, at least one sgRNA, and at least one donor molecule are encoded by one vector. In one aspect, at least one Cas9 nuclease, at least one sgRNA, and at least one donor molecule are encoded by two or more or three or more vectors.

In still another aspect, any vector described herein further encodes at least one, at least two, at least three, at least four, or at least five marker genes. In one aspect, a marker gene provided herein is selected from the group consisting of *nptII*, *aph IV*, *aadA*, *aac3, aacC4, bar, pat, DMO, EPSPS, aroA*, GFP. and GUS.

Targeted editing techniques can be used to convert a genomic locus into a locus that is capable of generating an RNAi-inducing hairpin when the edited locus is transcribed into RNA. In a cell that is heterozygous at the locus of interest (*e.g*., two polymorphic alleles are present), two or more nucleases are used to generate two double-stranded breaks (*e.g*., a first double-stranded break and a second double-stranded break) in the first allele, and one double-stranded break (*e.g*., a third double-stranded break) in the second allele. When the nucleases cut the alleles, the portion of the first allele that is flanked by the first and second double-stranded breaks is released from the genomic DNA. In one outcome, the released portion of the first allele is inverted and integrated into the third double-stranded break in the second allele, thereby creating an edited locus that is capable of generating an RNAi-inducing hairpin when the edited locus is transcribed.

This disclosure provides a method comprising: a) generating a first double-stranded break and a second double-stranded break in a first allele of a gene in a cell using a targeted editing technique; generating a third double-stranded break in a second allele of the gene in the cell using a targeted editing technique; and c) identifying a cell comprising an insertion of a region of the first allele in inverted orientation at the third double-stranded break site in the second allele, thereby generating a modified second allele. In one aspect, a modified second allele is a dominant negative allele. In another aspect, a modified second allele is a dominant positive allele. In an aspect, a first double-stranded break and a second double-stranded break are at the same nucleotide sequence or the same nucleotide position in the first allele and the second allele. In an aspect, a first double-stranded break and a second double-stranded break are at the same nucleotide sequence in the first allele and the second allele. In an aspect, a first double-stranded break and a second double-stranded break are at the same nucleotide position in the first allele and the second allele. In an aspect, the nucleotide sequence of a first allele is not identical to the nucleotide sequence of the second allele (*e.g.*, the cell is heterozygous for the locus). In an aspect, the nucleotide sequence at the third double-stranded break site in the second allele is not present in the first allele. In an aspect, a modified second allele provided herein transcribes an RNA capable of forming a hairpin loop secondary structure. In an aspect, a region of the first allele can comprise any number of nucleotides up to and including the full length of the first allele. In an aspect, a region of the first allele comprises at least 10, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 40, at least 50, at least 60, at least 70, at least 80, at least 90, at least 100, at least 150, at least 200, at least 250, at least 300, at least 400, at least 500, at least 600, at least 700, at least 800, at least 900, at least 1000, at least 1500, at least 2000, at least 3000, at least 4000, at least 5000, or at least 10,000 nucleotides. In another aspect, a region of the first allele comprises between 18 and 5000, between 18 and 4000, between 18 and 3000, between 18 and 2000, between 18 and 1000, between 18 and 500, between 18 and 400, between 18 and 300, between 18 and 200, between 18 and 100, between 18 and 50, between 18 and 30, between 50 and 500, between 50 and 1000, between 100 and 500, between 100 and 1000, or between 500 and 5000 nucleotides.

In one aspect, this disclosure provides a modified cell comprising at least one dominant negative allele of at least one gene generated by a targeted editing technique, where the allele generates an RNA transcript capable of forming a hairpin-loop secondary structure when the at least one dominant negative allele is transcribed.

This disclosure provides a method of generating a dominant negative allele of a gene in a cell comprising inserting an inverted copy of the gene, or a portion thereof, adjacent to a native copy of the gene using a targeted editing technique to generate an inverted repeat sequence capable of producing an antisense RNA transcript of the gene, or a portion thereof. In one aspect, the inverted repeat sequence is capable of forming a hairpin-loop secondary structure. In another aspect, the dominant negative allele generates at least one RNA transcript capable of forming a hairpin-loop secondary structure. In one aspect, the inverted copy of the gene and the native copy of the gene are separated by a spacer sequence. In an aspect, a spacer sequence comprises at least 1, at least 2, at least 3, at least 4, at least 5, at least 10, at least 25, at least 50, at least 75, at least 100, at least 150, at least 250, at least 500, or at least 1000 nucleotides. In yet another aspect, the dominant negative allele is operably linked to a promoter of the native copy of the gene.

In another aspect, this disclosure provides a modified cell comprising a dominant negative allele of at least one gene comprising an inverted copy of the gene adjacent to a native copy of the gene at the endogenous locus of the gene.

A dominant negative allele can also be generated by editing a genome to delete a region of DNA between a first gene region and a second, neighboring gene region, where the first gene region and the second gene region are present on the chromosome in opposite orientations (*e.g*., the first gene region is present in 5' to 3' orientation, while the second gene region is present on the same DNA strand of the chromosome in 3' to 5' orientation). Without being bound by any scientific theory, such a deletion allows the expression of an antisense RNA transcript of the first gene region by the promoter of the second gene region, while the native promoter of the first gene region expresses a sense RNA transcript. The sense and antisense RNA transcripts are complementary to each other and can be processed by RNA silencing mechanisms native to the cell to reduce the expression of the edited and unedited first gene region alleles in a dominant negative manner. Without being bound by any scientific theory, it is also contemplated that the antisense RNA molecule transcribed from the mutant or edited allele of the endogenous gene or locus may affect the expression level(s) of the gene through different mechanisms, such as nonsense mediated decay, non-stop decay, no-go decay, DNA or histone methylation or other epigenetic changes, inhibition or decreased efficiency of transcription and/or translation, ribosomal interference, interference with mRNA processing or splicing, and/or ubiquitin-mediated protein degradation via the proteasome. See, e.g., Nickless, A. et al., "Control of gene expression through the nonsense-mediated RNA decay pathway", Cell Biosci 7:26 (2017); Karamyshev, A. et al., "Lost in Translation: Ribosome-Associated mRNA and Protein Quality Controls", Frontiers in Genetics 9:431 (2018); Inada, T., "Quality controls induced by aberrant translation", Nucleic Acids Res 48:3 (2020); and Szadeczky-Kardoss, I. et al., "The nonstop decay and the RNA silencing systems operate cooperatively in plants", Nucleic Acids Res 46:9 (2018), the entire contents and disclosures of which are incorporated herein by reference. Each of these different mechanisms may act alternatively or in addition to RNA interference (RNAi), transcriptional gene silencing (PGS) and/or post transcriptional gene silencing (PTGS) mechanisms. See, e.g., Wilson, R.C. et al., "Molecular Mechanisms of RNA Interference", Annu Rev Biophysics 42:217-39 (2013); and Guo, Q. et al., "RNA Silencing in Plants: Mechanism, Technologies and Applications in Horticulture Crops", Current Genomics 17:476-489 (2016), the entire contents and disclosures of which is incorporated herein by reference. Some of the above mechanisms may reduce expression of the edited allele itself, while others may also reduce the expression of other copy/-ies or allele(s) of the endogenous locus/loci or gene(s). Such dominant or semi-dominant effect(s) on the gene(s) may operate through non-canonical suppression mechanisms that do not involve RNAi and/or formation of targeted small RNAs at a significant or detectable level.

In one aspect, this disclosure provides a method of generating a dominant negative allele of a gene in a cell comprising deleting a portion of a chromosome between a first gene region and a second gene region using a targeted editing technique, where an antisense RNA transcript of the first gene region is generated following the deletion of the portion of the chromosome. In another aspect, a targeted editing technique provided here comprises the use of at least one site-specific nuclease. In an aspect, an antisense RNA transcript provided herein is a partial antisense RNA transcript. In an aspect, a partial antisense RNA transcript is shorter than the corresponding sense RNA transcript. In an aspect, a partial antisense RNA transcript is at least 1, at least 2, at least 3, at least 4, at least 5, at least 6, at least 7, at least 8, at least 9, at least 10, at least 20, at least 30, at least 40, at least 50, at least 60, at least 70, at least 80, at least 90, at least 100, at least 150, at least 200, at least 250, at least 300, at least 400, at least 500, at least 600, at least 700, at least 800, at least 900, at least 1000, at least 1500, at least 2000, or at least 2500 nucleotides shorter than a corresponding sense RNA transcript. In another aspect, a partial antisense RNA transcript is at least 1%, at least 2%, at least 3%, at least 4%, at least 5%, at least 10%, at least 20%, at least 30%, at least 40%, at least 50%, at least 60%, at least 70%, at least 80%, at least 90%, or at least 95% shorter than a corresponding sense RNA transcript. In another aspect, an antisense RNA transcript provided herein is a complete antisense RNA transcript. In an aspect, a complete antisense RNA transcript is the same length as a corresponding sense RNA transcript. In an aspect, an antisense RNA transcript provided herein suppresses expression of a first gene region. In an aspect, an antisense RNA transcript provided herein is capable of suppressing expression of a first gene region.

In another aspect, this disclosure provides a method comprising: a) identifying a chromosomal region comprising a first gene region comprising a first promoter and a first coding region, and a second gene region comprising a second promoter and a second coding region, where the first coding region and the second coding region are separated by an intervening region, and where the first promoter and the second promoter are positioned in opposite orientations; b) inducing a first double-stranded break and a second double-stranded break flanking a targeted region; c) identifying one or more cells comprising a deletion of the targeted region of the chromosome; and d) selecting one or more cells comprising the deletion of the targeted region of the chromosome.

As used herein, an "intervening region" or "intervening sequence" refers to a polynucleotide sequence between a physically linked first polynucleotide sequence and second polynucleotide sequence. In one aspect, an intervening region or intervening sequence is between a first gene and a second gene. In an aspect, an intervening region or intervening sequence is between a first gene region and a second gene region. In one aspect, an intervening region or intervening sequence is between a first coding region and a second coding region. In another aspect, an intervening region or intervening sequence is between a first target site and a second target site. In one aspect, an intervening region or intervening sequence is between a first target gene and a second target gene. In one aspect, all or part of an intervening region or intervening sequence is inverted via a targeted editing technique. In another aspect, all or part of an intervening region or intervening sequence is deleted via a targeted editing technique. In one aspect, an intervening region or intervening sequence comprises at least 1, at least 2, at least 3, at least 4, at least 5, at least 10, at least 25, at least 50, at least 100, at least 150, at least 200, at least 250, at least 500, at least 1000, at least 1250, at least 1500, at least 1750, at least 2000, at least 2500, at least 3000, at least 4000, at least 5000, at least 6000, at least 7000, at least 8000, at least 9000, at least 10,000, at least 15,000, at least 20,000, at least 25,000, or at least 50,000 nucleotides. In an aspect, an intervening region or an intervening sequence comprises at least one, at least two, at least three, at least four, at least five, at least six, at least seven, at least eight, at least nine, or at least ten genes. In one aspect, an intervening region or intervening sequence is positioned on a chromosome. In one aspect, an intervening region or intervening sequence is positioned on a vector. In one aspect, an intervening region or intervening sequence comprises a DNA sequence. In one aspect, an intervening region or intervening sequence comprises an RNA sequence. In one aspect, an intervening region or intervening sequences comprises an endogenous nucleic acid sequence. In another aspect, an intervening region or intervening sequences comprises a transgenic nucleic acid sequence. In one aspect, an intervening region or intervening sequences comprises an endogenous nucleic acid sequence and a transgenic nucleic acid sequence.

In one aspect, a first gene region is selected from the group of a GA20 oxidase gene region, a GA3 oxidase gene region, a brachytic2 gene region, and a Y1 gene region. In another aspect, a first gene region is a GA20 oxidase gene region or a GA3 oxidase gene region. In a further aspect, a first gene region is a GA20 oxidase gene region. In an aspect, a first gene region is a GA3 oxidase gene region. In still another aspect, a first gene region is a brachytic2 gene region. In another aspect, a first gene region is a Y1 gene region.

GA oxidases in cereal plants consist of a family of related GA oxidase genes. For example, corn has a family of at least nine GA20 oxidase genes that includes GA20 oxidase_1, GA20 oxidase_2, GA20 oxidase_3, GA20 oxidase_4, GA20 oxidase_5, GA20 oxidase_6, GA20 oxidase_7, GA20 oxidase_8, and GA20 oxidase_9. The DNA and protein sequences by SEQ ID NOs for each of GA20 oxidase_3 and GA20 oxidase_5 are provided in Table 1.

**Table 1. DNA and protein sequences by sequence identifier for GA20 oxidase_3 and GA20 oxidase_5 genes in corn.**

| **GA20 oxidase Gene** | **Genomic DNA** | **cDNA** | **Coding Sequence (CDS)** | **Protein** |
|---|---|---|---|---|
| GA20 oxidase_3 | SEQ ID NO: 27 | SEQ ID NO: 28 | SEQ ID NO: 29 | SEQ ID NO: 30 |
| GA20 oxidase_5 | SEQ ID NO: 31 | SEQ ID NO: 32 | SEQ ID NO: 33 | SEQ ID NO: 34 |

A wild-type genomic DNA sequence of the GA20 oxidase_3 locus from a reference genome is provided in SEQ ID NO: 27, and A wild-type genomic DNA sequence of the GA20 oxidase_5 locus from a reference genome is provided in SEQ ID NO: 31.

For the corn GA20 oxidase_3 gene (also referred to as Zm.GA20ox3), SEQ ID NO: 27 provides 3000 nucleotides upstream (5') of the GA20 oxidase_3 5'-UTR; nucleotides 3001-3096 correspond to the 5'-UTR; nucleotides 3097-3665 correspond to the first exon: nucleotides 3666-3775 correspond to the first intron; nucleotides 3776-4097 correspond to the second exon; nucleotides 4098-5314 correspond to the second intron; nucleotides 5315-5584 correspond to the third exon; and nucleotides 5585-5800 correspond to the 3'-UTR. SEQ ID NO: 27 also provides 3000 nucleotides downstream (3') of the end of the 3'-UTR (nucleotides 5801-8800).

For the corn GA20 oxidase_5 gene (also referred to as Zm.GA20ox5), SEQ ID NO: 31 provides 3000 nucleotides upstream of the GA20 oxidase_5 start codon (nucleotides 1-3000); nucleotides 3001-3791 correspond to the first exon; nucleotides 3792-3906 correspond to the first intron; nucleotides 3907-4475 correspond to the second exon; nucleotides 4476-5197 correspond to the second intron; nucleotides 5198-5473 correspond to the third exon; and nucleotides 5474-5859 correspond to the 3'-UTR. SEQ ID NO: 31 also provides 3000 nucleotides downstream (3') of the end of the 3'-UTR (nucleotides 5860-8859).

In the corn genome, the Zm.GA20ox5 gene located next to the Zm.SAMT gene. These two genes are separated by an intergenic region of about 550 bp, with the Zm.SAMT gene positioned downstream and oriented in the opposite orientation relative to the Zm.GA20ox5 gene. A reference genomic sequence of the region encompassing the Zm.GA20ox5 and Zm.SAMT genes is provided in SEQ ID NOs. 35 and 36. SEQ ID NO. 35 represents the sequence of the sense strand of the Zm.GA20ox5 gene encompassing both Zm.GA20ox5 and Zm.SAMT genes (the "GA20ox5_SAMT genomic sequence" in Table 2). SEQ ID NO: 35 partially overlaps with SEQ ID NO: 31 and has a shorter Zm.GA20ox5 upstream sequence and a longer Zm.GA20ox5 downstream sequence compared to the SEQ ID NO: 31. SEQ ID NO. 36 represents the sequence of the sense strand of the Zm.SAMT gene (*i.e.*, the antisense strand of the Zm.GA20ox5 gene) encompassing both Zm.GA20ox5 and Zm.SAMT genes (the "SAMT_GA20ox5 genomic sequence" in Table 2). The elements or regions of the reference genomic Zm.GA20ox5 / Zm.SAMT sequence are annotated in Table 2 below by reference to the nucleotide coordinates of those elements or regions in SEQ ID NO. 35 or 36.

It was previously shown that suppression of GA20 oxidase gene(s) and/or targeting of a subset of one or more GA oxidase genes via transgenic suppression (*e.g*., an artificial microRNA-mediated suppression of both GA20 oxidase_3 and GA20 oxidase_5 genes) can be effective in achieving a short stature, semi-dwarf phenotype with increased resistance to lodging, but without reproductive off-types in the ear. *See* PCT Application No. PCT/US2017/047405 and US Application No. 15/679,699, both filed on August 17, 2017, and published as WO/2018/035354 and US20180051295, respectively. Furthermore, knocking out GA20 oxidase_3, GA20 oxidase_5, or both genes via genome editing also can cause reduced plant height and increased lodging resistance, and impacts GA hormonal levels. *See* PCT Application Nos. PCT/US2019/018128, PCT/US2019/018131, and PCT/US2019/018133, all filed on February 15, 2019.

In one aspect, a first gene region comprises a polynucleotide sequence having at least 80%, at least 85%, at least 90%, at least 91%, at least 92%, at least 93%, at least 94%, at least 95%, at least 96%, at least 97%, at least 98%, at least 99%, at least 99.5%, or 100% identity or complementarity to a sequence selected from the group consisting of SEQ ID NOs: (insert GA20 cDNA sequences).

In another aspect, a first gene region comprises a polynucleotide sequence having at least 80%, at least 85%, at least 90%, at least 91%, at least 92%, at least 93%, at least 94%, at least 95%, at least 96%, at least 97%, at least 98%, at least 99%, at least 99.5%, or 100% identity or complementarity to a sequence selected from the group consisting of SEQ ID NOs: (insert BR2 cDNA sequences).

In one aspect, a first gene region comprises a polynucleotide sequence having at least 80%, at least 85%, at least 90%, at least 91%, at least 92%, at least 93%, at least 94%, at least 95%, at least 96%, at least 97%, at least 98%, at least 99%, at least 99.5%, or 100% identity or complementarity to a sequence selected from the group consisting of SEQ ID NOs: (insert GA3 cDNA sequences).

In one aspect, a first gene region comprises a polynucleotide sequence having at least 80%, at least 85%, at least 90%, at least 91%, at least 92%, at least 93%, at least 94%, at least 95%, at least 96%, at least 97%, at least 98%, at least 99%, at least 99.5%, or 100% identity or complementarity to a sequence selected from the group consisting of SEQ ID NOs: (insert Y1 cDNA sequences).

In an aspect, a deletion provided herein comprises all or part of a second gene region. In another aspect, a deletion provided herein comprises all of a second gene region. In still another aspect, a deletion provided herein comprises part of a second gene region.

In still another aspect, this disclosure provides a method of reducing expression of a gene in a cell comprising: a) identifying a chromosomal region comprising a first gene region comprising a first promoter and a first coding region, and a second gene region comprising a second promoter and a second coding region, where the first coding region and the second coding region are separated by an intervening region, and where the first promoter and the second promoter are positioned in opposite orientations; b) inducing a first double-stranded break and a second double-stranded break flanking a targeted region using a targeted editing technique, where the targeted region comprises the second coding region and the intervening region; and c) identifying one or more cells comprising a deletion of the targeted region of the chromosome, where the second promoter generates at least one antisense RNA of the first coding region, and where expression of the first coding region is reduced as compared to a control cell that does not comprise the deletion of the targeted region. In one aspect, a deletion leads to a portion of a first coding region being transcribed in reverse orientation.

In a further aspect, this disclosure provides a method comprising: a) identifying a chromosomal region comprising a first gene region comprising a first promoter and a first coding region, and a second gene region comprising a second promoter and a second coding region, where the first coding region and the second coding region are separated by an intervening region, and where the first promoter and the second promoter are positioned in opposite orientations; b) providing to one or more cells at least one RNA-guided nuclease or one or more vectors encoding at least one RNA-guided nuclease, where the at least one RNA-guided nuclease is capable of binding to at least 18, at least 19, at least 20, at least 21, at least 22, at least 23, at least 24, at least 25, or at least 26 consecutive nucleotides of a first target site and a second target site flanking a targeted region of a chromosome, where the targeted region comprises the second coding region and the intervening region, where the RNA-guided nuclease creates double-stranded breaks in the chromosome at the first target site and the second target site; b) identifying one or more cells comprising a deletion of the targeted region: and c) selecting one or more cells comprising the deletion of the targeted region.

In one aspect, this disclosure provides a modified plant, or part thereof, comprising a non-transposon mediated genome deletion or inversion of a gene or a portion thereof, at the endogenous locus of the gene, where the deletion or inversion results in the production of an RNA transcript comprising a sequence complementary to a native transcript sequence of the gene, or portion thereof. In one aspect, this disclosure provides a modified plant cell comprising a non-transposon mediated genome deletion or inversion of a gene or a portion thereof, at the endogenous locus of the gene, where the deletion or inversion results in the production of an RNA transcript comprising a sequence complementary to a native transcript sequence of the gene, or portion thereof. In another aspect, this disclosure provides a modified plant or modified plant tissue comprising a modified plant cell comprising a non-transposon mediated genome deletion or inversion of a gene or a portion thereof, at the endogenous locus of the gene, where the deletion or inversion results in the production of an RNA transcript comprising a sequence complementary to a native transcript sequence of the gene, or portion thereof.

It is known in the art that transposons, or transposable elements, are DNA sequences that can change their position within a genome. Transposons can create insertions, deletions, or inversions in a genome. In an aspect, methods, compositions, and cells provided herein do not comprise the use of a transposon (*e.g*., "non-transposon mediated").

In one aspect, this disclosure provides a modified chromosome comprising a non-transposon mediated genome deletion or inversion of a gene or a portion thereof, at the endogenous locus of the gene, where the deletion or inversion results in the production of an RNA transcript comprising a sequence complementary to a native transcript sequence of the gene, or portion thereof. In one aspect, this disclosure provides a modified cell comprising a non-transposon mediated genome deletion or inversion of a gene or a portion thereof, at the endogenous locus of the gene, where the deletion or inversion results in the production of an RNA transcript comprising a sequence complementary to a native transcript sequence of the gene, or portion thereof. In another aspect, this disclosure provides a modified cell comprising a modified chromosome comprising a non-transposon mediated genome deletion or inversion of a gene or a portion thereof, at the endogenous locus of the gene, where the deletion or inversion results in the production of an RNA transcript comprising a sequence complementary to a native transcript sequence of the gene, or portion thereof.

In one aspect, this disclosure provides a product comprising a modified chromosome comprising a non-transposon mediated genome deletion or inversion of a gene or a portion thereof, at the endogenous locus of the gene, where the deletion or inversion results in the production of an RNA transcript comprising a sequence complementary to a native transcript sequence of the gene, or portion thereof. In one aspect, this disclosure provides a product comprising a modified plant, or part thereof, comprising a non-transposon mediated genome deletion or inversion of a gene or a portion thereof, at the endogenous locus of the gene, where the deletion or inversion results in the production of an RNA transcript comprising a sequence complementary to a native transcript sequence of the gene, or portion thereof. In one aspect, this disclosure provides a product comprising a modified plant cell comprising a non-transposon mediated genome deletion or inversion of a gene or a portion thereof, at the endogenous locus of the gene, where the deletion or inversion results in the production of an RNA transcript comprising a sequence complementary to a native transcript sequence of the gene, or portion thereof. In one aspect, this disclosure provides a product comprising a modified cell comprising a non-transposon mediated genome deletion or inversion of a gene or a portion thereof, at the endogenous locus of the gene, where the deletion or inversion results in the production of an RNA transcript comprising a sequence complementary to a native transcript sequence of the gene, or portion thereof. In an aspect, a product comprises silage, flour, cellulose, sugar, starch, fat, syrup, or protein derived from a plant, plant part, or plant cell.

In an aspect, this disclosure provides a modified cell comprising a) a non-transposon mediated genome deletion of at least one gene or a portion thereof, at the endogenous locus of the at least one gene, or b) a non-transposon mediated and non-T-DNA mediated insertion of a polynucleotide sequence into the at least one gene, where the deletion or insertion creates a dominant positive allele of the at least one gene. In one aspect, an insertion comprises a regulatory element. In another aspect, a regulatory element is selected from the group consisting of a promoter sequence, a transcriptional start site sequence, a transcriptional termination site sequence, an enhancer sequence, and a designed element.

In another aspect, the present disclosure provides a modified cell comprising a non-transposon mediated genome deletion or inversion of at least one gene or a portion thereof, at the endogenous locus of the at least one gene, where the deletion or inversion creates a dominant negative allele of the at least one gene. In still another aspect, this disclosure provides a modified cell comprising a non-transposon mediated genome deletion or inversion of a gene or a portion thereof, at the endogenous locus of the at least one gene, where the deletion or inversion results in the production of an RNA transcript comprising a sequence complementary to a native transcript sequence of the gene. In an aspect, this disclosure provides a modified cell comprising a targeted edit of at least one gene or a portion thereof, where the targeted edit generates an RNA transcript that is complementary to a native transcript sequence of the gene. In an aspect, an RNA transcript is a complete antisense transcript. In another aspect, an RNA transcript is a partial antisense transcript. In a further aspect, an RNA transcript is a partial sense transcript. In still another aspect, an RNA transcript is a complete sense transcript. In another aspect, an RNA transcript is a native transcript of a gene. In a further aspect, a native transcript of a gene is a partial or complete sense transcript.

A dominant allele of a gene region can also be created by inserting a designed element into the promoter of a gene region to induce constitutive expression of the gene region.

In one aspect, this disclosure provides a method of modifying gene expression comprising: a) inducing a double-stranded break using a targeted editing technique at a target site of the gene; b) inserting a donor sequence at the double-stranded break, where the donor sequence comprises a designed element capable of inducing increased or ectopic expression of the gene; and c) identifying at least one cell comprising the insertion of the donor sequence, where expression of the gene is increased in at least one tissue as compared to a control cell that does not comprise the insertion of the donor sequence.

In another aspect, this disclosure provides a method comprising a) providing to one or more cells at least one RNA-guided nuclease and at least one donor molecule or one or more vectors encoding at least one RNA-guided nuclease and at least one donor molecule, where the at least one RNA-guided nuclease is capable of binding to at least 18, at least 19, at least 20, at least 21, at least 22, at least 23, at least 24, at least 25, or at least 26 consecutive nucleotides of a target site of at least one gene, where the donor molecule comprises a designed element, where the RNA-guided nuclease creates a double-stranded break at the target site, and where the donor molecule is inserted at the double-stranded break; b) identifying one or more cells comprising the insertion of the donor molecule at the target site: and c) selecting one or more cells comprising the insertion of the donor molecule at the target site.

In an aspect, the target site is positioned downstream of a TATA box upstream of the gene. In an aspect, the target site is positioned upstream of a TATA box upstream of the gene. In an aspect, a target site is positioned upstream of a TATA box that is operably linked to at least one gene. In another aspect, a target site is positioned downstream of a TATA box that is operably linked to at least one gene. In an aspect, a target site is positioned within 10, within 20, within 30, within 40, within 50, within 60, within 70, within 80, within 90, within 100, within 200, within 300, within 400, within 500, within 600, within 700, within 800, within 900, within 1000, within 1500, within 2000, within 2500, or within 5000 nucleotides of a TATA box that is operably linked to at least one gene. In still another aspect, a target site is positioned between 10 and 5000, between 10 and 2500, between 10 and 1500, between 10 and 1000, between 10 and 750, between 10 and 500, between 10 and 250, between 10 and 100, between 20 and 100, between 20 and 250, between 20 and 500, or between 50 and 500 nucleotides of a TATA box that is operably linked to at least one gene. In still another aspect the target site is positioned within 10, within 20, within 30, within 40, within 50, within 60, within 70, within 80, within 90, within 100, within 200, within 300, within 400, within 500, within 600, within 700, within 800, within 900, within 1000, within 1500, within 2000, within 2500, or within 5000 nucleotides of a promoter of a gene. In still another aspect, the target site is positioned upstream of an initiator element upstream of the gene. In still another aspect, the target site is positioned downstream of an initiator element upstream of the gene. In still another aspect the target site is positioned within 10, within 20, within 30, within 40, within 50, within 60, within 70, within 80, within 90, within 100, within 200, within 300, within 400, within 500, within 600, within 700, within 800, within 900, within 1000, within 1500, within 2000, within 2500, or within 5000 nucleotides of an initiator element of a gene.

In one aspect, a "TATA box" comprises a core DNA sequence of 5'-TATAAA-3' or a variant thereof, and is frequently associated with the promoters of eukaryotic genes. Typically, but not always, a TATA box is positioned approximately 25 to 35 nucleotides upstream of a transcription start site of a gene. TATA boxes often serve as a binding site for a transcription factor to enable expression of an operably linked gene, or a histone to block expression of an operably linked gene. In one aspect, a TATA box is an initiator element. An initiator element is a core promoter that facilitates the binding of a transcription factor to promote expression of an operably linked gene. In one aspect, an initiator sequence provided herein comprises the sequence of 5'-[C/T][C/T]AN[A/T][C/T][C/T]-3'.

A dominant negative allele can also be created by editing a genome to comprise a tissue-specific or a tissue-preferred promoter of a gene region, such that the tissue-specific or tissue-preferred promoter is in opposite orientation of a targeted gene. For example, placing an tissue-specific promoter downstream of the 3'-UTR of a gene region in reverse orientation would allow the tissue-specific promoter to generate a complete antisense gene region RNA transcript. Without being bound to any theory, the antisense gene region RNA transcript expressed by the antisense tissue-specific promoter is able to suppress the expression of the gene region in a tissue-specific manner.

In one aspect, this disclosure provides a method of reducing the expression of a gene in at least one cell comprising a) inducing a double-stranded break using a targeted editing technique at a target site of the gene: b) inserting a donor sequence at the double-stranded break, where the donor sequence comprises a tissue-specific or tissue-preferred promoter, and where the donor sequence is inserted into the target site such that the tissue-specific or tissue-preferred promoter is in reverse orientation as compared to the gene; and c) identifying at least one cell comprising the insertion of the donor sequence in reverse orientation, where expression of the gene is reduced as compared to a control cell that does not comprise the insertion of the donor sequence. In one aspect, a method provided herein further comprises using a targeted editing technique to remove the native promoter of the gene. As used herein, a "native promoter" refers to a promoter that generates a sense mRNA transcript of an operably linked gene.

In another aspect, this disclosure provides a method comprising a) providing to one or more cells at least one RNA-guided nuclease and at least one donor molecule or one or more vectors encoding at least one RNA-guided nuclease and at least one donor molecule, where the at least one RNA-guided nuclease is capable of binding to at least 18, at least 19, at least 20, at least 21, at least 22, at least 23, at least 24, at least 25, or at least 26 consecutive nucleotides of a target site of at least one gene, where the donor molecule comprises a sequence encoding a tissue-specific or tissue-preferred promoter, where the RNA-guided nuclease creates a double-stranded break at the target site, and where the donor molecule is inserted at the double-stranded break; b) identifying one or more cells comprising the insertion of the donor molecule at the target site such that the tissue-specific or tissue-preferred promoter is in reverse orientation as compared to the gene; and c) selecting one or more cells comprising the insertion of the donor molecule at the target site.

In an aspect, a target site is positioned downstream of the 3'-UTR of a gene. In another aspect, a target site is positioned within the 3'-UTR of a gene. In another aspect, a target site is positioned within an intron of a gene. In a further aspect, a target site is positioned within an exon of a gene. In an aspect, a target site is positioned with a 5'-UTR of a gene. In another aspect, a target site is positioned upstream of a 5'-UTR of a gene. In a still further aspect, a target site is positioned within a promoter of a gene.

In one aspect, the donor molecule comprises a polynucleotide that encodes a promoter. In one aspect, the donor molecule comprises a polynucleotide that encodes a promoter that is selected from the group consisting of a tissue-specific promoter, a tissue-preferred promoter, a constitutive promoter, and an inducible promoter. In another aspect, the donor molecule provided herein comprises a polynucleotide that encodes a tissue-specific or tissue-preferred promoter. In still another aspect, the donor molecule provided herein comprises a polynucleotide that encodes a constitutive promoter. In another aspect, the donor molecule provided herein comprises a polynucleotide that encodes an inducible promoter.

In an aspect, a tissue-specific or tissue-preferred promoter is selected from the group consisting of a leaf-specific promoter, a leaf-preferred promoter, a stem-specific promoter, a stem-preferred promoter, a vascular-specific promoter, a vascular-preferred promoter, a root-specific promoter, a root-preferred promoter, an inflorescence-specific promoter, an inflorescence-preferred promoter, a pollen-specific promoter, a pollen-preferred promoter, an anther-specific promoter, an anther-preferred promoter, an ovule-specific promoter, an ovule-preferred promoter, a seed-specific promoter, a seed-preferred promoter, an embryo-specific promoter, an embryo-preferred promoter, an endosperm-specific promoter, an endosperm-preferred promoter, a pericarp-specific promoter, a pericarp-preferred promoter, an aleurone-specific promoter, an aleurone-preferred promoter, a meristem-specific promoter, a meristem-preferred promoter, a fruit-specific promoter, a fruit-preferred promoter, a pod-specific promoter, a pod-preferred promoter, an epidermis-specific promoter, an epidermis-preferred promoter, a mitochondrial-specific promoter, a mitochondrial-preferred promoter, a chloroplast-specific promoter, and a chloroplast-preferred promoter. In another aspect, a tissue-specific or tissue-preferred promoter provided herein is an RTBV promoter.. In an aspect, a tissue-specific or tissue-preferred promoter provided herein expresses an antisense mRNA transcript of a gene. In an aspect, a tissue-specific or tissue-preferred promoter provided herein is expresses an antisense mRNA transcript of a gene.

Targeted editing techniques can be used to insert a donor molecule into a target site in a genomic locus. If a donor molecule comprising a non-coding RNA target site is inserted into a 5'-UTR, an exon, an intron, or a 3'-UTR of a gene of interest, RNA transcription or protein translation of the gene of interest can be suppressed by a complementary non-coding RNA. When a gene of interest is a target of a non-coding RNA (*e.g.*, a miRNA or an siRNA), a cleaved mRNA from the gene of interest can generate secondary siRNAs, which can further suppress the transcription or translation of the gene of interest. Such secondary suppression can act in a dominant manner, as the secondary siRNAs are complementary to alleles with and without the insertion of the non-coding RNA target site.

In one aspect, an engineered or artificial miRNA is created to target a native gene region. In another aspect, a gene region is edited to be complementary to a native miRNA. Engineered miRNAs are useful for targeted gene suppression with increased specificity. See, e.g., Parizotto et al., Genes Dev. 18:2237-2242 (2004), and U.S. Patent Application Publication Nos. 2004/0053411, 2004/0268441, 2005/0144669, and 2005/0037988, the contents and disclosures of which are incorporated herein by reference. miRNAs are non-protein coding RNAs. When a miRNA precursor molecule is cleaved, a mature miRNA is formed that is typically from about 19 to about 25 nucleotides in length (commonly from about 20 to about 24 nucleotides in length in plants), such as 19, 20, 21, 22, 23, 24, or 25 nucleotides in length, and has a sequence corresponding to the gene targeted for suppression and/or its complement. The mature miRNA hybridizes to target mRNA transcripts and guides the binding of a complex of proteins to the target transcripts, which can function to inhibit translation and/or result in degradation of the transcript, thus negatively regulating or suppressing expression of the targeted gene. miRNA precursors are also useful in plants for directing in-phase production of siRNAs, trans-acting siRNAs (ta-siRNAs), in a process that requires a RNA-dependent RNA polymerase to cause suppression of a target gene. See, e.g., Allen et al., Cell 121:207-221 (2005), Vaucheret Science STKE, 2005:pe43 (2005), and Yoshikawa et al. Genes Dev., 19:2164-2175 (2005), the contents and disclosures of which are incorporated herein by reference.

Plant miRNAs regulate their target genes by recognizing and binding to a near-perfectly complementary sequence (miRNA recognition site) in the target transcript, followed by cleavage of the transcript by RNase III enzymes such as ARGONAUTE1. In plants, certain mismatches between a given miRNA recognition site and the corresponding mature miRNA are not tolerated, particularly mismatched nucleotides at positions 10 and 11 of the mature miRNA. Positions within the mature miRNA are given in the 5' to 3' direction. Perfect complementarity between a given miRNA recognition site and the corresponding mature miRNA is usually required at positions 10 and 11 of the mature miRNA. See, for example, Franco-Zorrilla et al. (2007) Nature Genetics, 39:1033-1037; and Axtell et al. (2006) Cell, 127:565-577.

Many microRNA genes (MIR genes) have been identified and made publicly available in a database ("miRBase", available on line at microrna.sanger.ac.uk/sequences; also see Griffiths-Jones et al. (2003) Nucleic Acids Res., 31:439-441). MIR genes have been reported to occur in intergenic regions, both isolated and in clusters in the genome, but can also be located entirely or partially within introns of other genes (both protein-coding and non-protein-coding). For a recent review of miRNA biogenesis, see Kim (2005) Nature Rev. Mol. Cell. Biol., 6:376-385. Transcription of MIR genes can be, at least in some cases, under promotional control of a MIR gene's own promoter. The primary transcript, termed a "pri-miRNA", can be quite large (several kilobases) and can be polycistronic, containing one or more pre-miRNAs (fold-back structures containing a stem-loop arrangement that is processed to the mature miRNA) as well as the usual 5' "cap" and polyadenylated tail of an mRNA. See, for example, FIG. 1 in Kim (2005) Nature Rev. Mol. Cell. Biol., 6:376-385.

Transgenic expression of miRNAs (whether a naturally occurring sequence or an artificial sequence) can be employed to regulate expression of the miRNA's target gene or genes. Recognition sites of miRNAs have been validated in all regions of a mRNA, including the 5' untranslated region, coding region, and 3' untranslated region, indicating that the position of the miRNA target site relative to the coding sequence may not necessarily affect suppression (see, e.g., Jones-Rhoades and Bartel (2004). Mol. Cell, 14:787-799, Rhoades et al. (2002) Cell, 110:513-520, Allen et al. (2004) Nat. Genet., 36:1282-1290, Sunkar and Zhu (2004) Plant Cell, 16:2001-2019). Because miRNAs are important regulatory elements in eukaryotes, transgenic suppression of miRNAs is useful for manipulating biological pathways and responses. Promoters of MIR genes can have very specific expression patterns (e.g., cell-specific, tissue-specific, temporally specific, or inducible), and thus are useful in recombinant constructs to induce such specific transcription of a DNA sequence to which they are operably linked. Various utilities of miRNAs, their precursors, their recognition sites, and their promoters are described in detail in U.S. Patent Application Publication 2006/0200878 A1, incorporated by reference herein. Non-limiting examples of these utilities include: (1) the expression of a native miRNA or miRNA precursor sequence to suppress a target gene; (2) the expression of an artificial miRNA or miRNA precursor sequence to suppress a target gene; (3) expression of a transgene with a miRNA recognition site, where the transgene is suppressed when the mature miRNA is expressed: (4) expression of a transgene driven by a miRNA promoter.

Designing an artificial miRNA sequence can be as simple as substituting sequence that is complementary to the intended target for nucleotides in the miRNA stem region of the miRNA precursor, as demonstrated by Zeng et al. (2002) Mol. Cell, 9:1327-1333. One non-limiting example of a general method for determining nucleotide changes in the native miRNA sequence to produce the engineered miRNA precursor includes the following steps: (a) Selecting a unique target sequence of at least 18 nucleotides specific to the target gene, e.g., by using sequence alignment tools such as BLAST (see, for example, Altschul et al. (1990) J. Mol. Biol., 215:403-410: Altschul et al. (1997) Nucleic Acids Res., 25:3389-3402), for example, of both tobacco cDNA and genomic DNA databases, to identify target transcript orthologues and any potential matches to unrelated genes, thereby avoiding unintentional silencing of non-target sequences; (b) Analyzing the target gene for undesirable sequences (e.g., matches to sequences from non-target species), and score each potential 19-mer segment for GC content, Reynolds score (see Reynolds et al. (2004) Nature Biotechnol., 22:326-330), and functional asymmetry characterized by a negative difference in free energy (".DELTA..DELTA.G" or "ΔΔG") (see Khvorova et al. (2003) Cell, 115:209-216). Preferably 19-mers are selected that have all or most of the following characteristics: (1) a Reynolds score>4, (2) a GC content between about 40% to about 60%, (3) a negative ΔΔG, (4) a terminal adenosine, (5) lack of a consecutive run of 4 or more of the same nucleotide: (6) a location near the 3' terminus of the target gene: (7) minimal differences from the miRNA precursor transcript. Positions at every third nucleotide in an siRNA have been reported to be especially important in influencing RNAi efficacy and an algorithm, "siExplorer" is publicly available at ma.chem.t.u-tokyo.ac.jp/siexplorer.htm (see Katoh and Suzuki (2007) Nucleic Acids Res., 10.1093/nar/gkl1120); (c) Determining the reverse complement of the selected 19-mers to use in making a modified mature miRNA. The additional nucleotide at position 20 is preferably matched to the selected target sequence, and the nucleotide at position 21 is preferably chosen to either be unpaired to prevent spreading of silencing on the target transcript or paired to the target sequence to promote spreading of silencing on the target transcript; and (d) transforming the artificial miRNA into a plant.

The siRNA pathway involves the non-phased cleavage of a longer double-stranded RNA intermediate (an RNA duplex) into small interfering RNAs (siRNAs). The size or length of siRNAs ranges from about 19 to about 25 nucleotides or base pairs, but common classes of siRNAs include those containing 21 base pairs or 24 base pairs. Thus, a transcribable DNA sequence or suppression element of the present application can encode a RNA molecule that is at least about 19 to about 25 nucleotides in length, such as 19, 20, 21, 22, 23, 24, or 25 nucleotides in length.

In the ta-siRNA pathway, miRNAs serve to guide in-phase processing of siRNA primary transcripts in a process that requires an RNA-dependent RNA polymerase for production of a double-stranded RNA precursor: ta-siRNAs are defined by lack of secondary structure, a miRNA target site that initiates production of double-stranded RNA, requirements of DCL4 and an RNA-dependent RNA polymerase (RDR6), and production of multiple perfectly phased ~21-nt small RNAs with perfectly matched duplexes with 2-nucleotide 3' overhangs (see Allen et al. (2005) Cell, 121:207-221). The size or length of ta-siRNAs ranges from about 20 to about 22 nucleotides or base pairs, but are mostly commonly 21 base pairs. Thus, a donor molecule or vector of the present application can encode a RNA molecule that is at least about 20 to about 22 nucleotides in length, such as 20, 21, or 22 nucleotides in length. Donor molecules and vectors provided herein can also comprise a ta-siRNA scaffold. For methods of constructing suitable ta-siRNA scaffolds, *see* U.S. Patent No. 9,309,512, which is incorporated herein by reference in its entirety.

This disclosure provides a method of generating a dominant negative allele of a gene comprising using a targeted editing technique to introduce at least one non-coding RNA target site into the gene. In an aspect, the dominant negative allele of the gene is downregulated as compared to an allele of the gene that does not comprise the at least one non-coding RNA target site. In another aspect, secondary siRNAs complementary to the gene are generated. In another aspect, the at least one non-coding RNA target site is a miRNA target site or a siRNA target site. In a further aspect, the at least one non-coding RNA target site is introduced into a region of the gene selected from the group consisting of a 5'-UTR, an exon, an intron, and a 3'-UTR. In another aspect, the at least one non-coding RNA target site is introduced into an exon of the gene. In another aspect, the at least one non-coding RNA target site is introduced into an intron of the gene. In another aspect, the at least one non-coding RNA target site is introduced into a 5'-UTR of the gene. In still another aspect, the at least one non-coding RNA target site is introduced into a 3'-UTR of the gene.

In another aspect, this disclosure provides a modified cell comprising a non-transgenic dominant negative allele of a gene, the dominant negative allele comprising a heterologous non-coding RNA target site in the endogenous locus of the gene.

A dominant allele can also be created by editing an allele of a protein-coding gene region such that a truncated protein is generated, where the edited, truncated protein interferes with the activity of the wild-type protein and produces a dominant effect. In one aspect, the introduction of a targeted edit to a protein-coding gene to generate a truncated protein creates a dominant positive allele. In one aspect, the introduction of a targeted edit to a protein-coding gene to generate a truncated protein creates a dominant negative allele. In an aspect, a truncated protein provided herein interferes with protein-protein binding, DNA-protein binding, or RNA-protein binding. In one aspect, a truncated protein provided herein is a microprotein. As used herein, a microprotein refers to a ~100-200 amino acid long protein that only encodes a protein-protein interaction or binding domain (*see*, for example, Seo et al., Trends in Plant Sciences, 2011, 10:541-549). Microproteins often evolve from functional genes that underwent mutations to eliminate functional protein domains. In one aspect, a microprotein is at least 50, at least 75, at least 100, at least 125, at least 150, at least 175, at least 200, or at least 225 amino acids in length. In one aspect a microprotein suppresses activity of at least one, at least two, at least three, at least four, at least five, at least six, at least seven, at least eight, at least nine, or at least ten protein in a cell. In another aspect, a microprotein enhances activity of at least one, at least two, at least three, at least four, at least five, at least six, at least seven, at least eight, at least nine, or at least ten proteins in a cell. Without being limiting, a microprotein can compete with a second protein for a binding site in a third protein. In one aspect, a microprotein blocks the binding of a second protein to a third protein a suppression of activity can occur. Alternatively, in another aspect, the binding of the microprotein to the third protein, in place of the second protein, enhances the activity of the third protein.

In one aspect, plant comprising a dominant negative allele encoding a microprotein comprises an improvement in a trait selected from the group consisting of flowering time, meristem size, insect resistance, herbicide tolerance, and shade avoidance. In one aspect, plant comprising a dominant positive allele encoding a microprotein comprises an improvement in a trait selected from the group consisting of flowering time, meristem size, insect resistance, herbicide tolerance, and shade avoidance.

In one aspect, a truncated protein provided herein is selected from the group consisting of a truncated CLAVATA protein, a truncated CORYNE protein, a truncated BAM receptor, a truncated RECEPTOR-LIKE PROTEIN KINASE2 (RPK2) protein, and a truncated G PROTEIN BETA-SUBUNIT1 (AGB1) protein. In another aspect, a CLAVATA protein provided herein is a CLAVATA1 protein, a CLAVATA2 protein, or a CLAVATA3 protein.

In one aspect, this disclosure provides a method of generating a dominant negative allele of a gene comprising a) inducing a double-stranded break in a genome of at least one cell using a targeted editing technique at a target site of the gene, where the double-stranded break is repaired by non-homologous end-joining; and b) identifying at least one cell comprising an insertion or deletion at the target site, where the insertion or deletion at the target site results in the generation of a dominant negative allele of the gene.

In still another aspect, this disclosure provides a modified cell comprising at least one insertion or deletion at the endogenous locus of the at least one gene generated by a targeted editing technique, where the insertion or deletion results in expression of a truncated protein.

In another aspect, this disclosure provides a method comprising a) providing to one or more cells at least one RNA-guided nuclease or one or more vectors encoding at least one RNA-guided nuclease, where the at least one RNA-guided nuclease is capable of binding to at least 18, at least 19, at least 20, at least 21, at least 22, at least 23, at least 24, at least 25, or at least 26 consecutive nucleotides of a target site of at least one gene, where the RNA-guided nuclease creates a double-stranded break at the target site; b) identifying at least one cell comprising an insertion or deletion at the target site, where the insertion or deletion at the target site results in the generation of a dominant negative allele of the at least one gene: and, c) selecting one or more cells comprising the dominant negative allele of the at least one gene.

This disclosure also provides a method of generating a dominant allele of a gene comprising using a targeted editing technique to introduce a nonsense mutation in the gene to create a truncated protein. In an aspect, the truncated protein is a microprotein. In an aspect, the targeted editing technique comprises a deletion of at least 1, at least 2, at least 3. at least 4, at least 5, at least 6, at least 7, at least 8, at least 9, at least 10, at least 25, at least 50, at least 75, at least 100, at least 150, at least 200, at least 250, at least 500, at least 1000, at least 2500, or at least 5000 nucleotides. In an aspect, the targeted editing technique comprises an insertion of at least 1, at least 2, at least 3, at least 4, at least 5, at least 6, at least 7, at least 8, at least 9, at least 10, at least 25, at least 50, at least 75, at least 100, at least 150, at least 200, at least 250, at least 500, at least 1000, at least 2500, or at least 5000 nucleotides. In an aspect, the targeted editing technique comprises an inversion of at least 1, at least 2, at least 3, at least 4, at least 5, at least 6, at least 7, at least 8, at least 9, at least 10, at least 25, at least 50, at least 75, at least 100, at least 150, at least 200, at least 250, at least 500, at least 1000, at least 2500, or at least 5000 nucleotides.

In one aspect, the introduction of a targeted edit to a protein-coding gene to generate a truncated protein creates a dominant positive allele. In one aspect, this disclosure provides a method of generating a dominant positive allele of a gene comprising a) inducing a double-stranded break in a genome of at least one cell using a targeted editing technique at a target site of the gene, where the double-stranded break is repaired by non-homologous end-joining: and b) identifying at least one cell comprising an insertion or deletion at the target site, where the insertion or deletion at the target site results in the generation of a dominant positive allele of the gene.

In another aspect, this disclosure provides a method comprising a) providing to one or more cells one or more vectors, where the one or more vectors comprises at least one polynucleotide encoding at least one RNA-guided nuclease, where the at least one RNA-guided nuclease is capable of binding to at least 18, at least 19, at least 20, at least 21, at least 22, at least 23, at least 24, at least 25, or at least 26 consecutive nucleotides of a target site of at least one gene, where the RNA-guided nuclease creates a double-stranded break at the target site, and where the double-stranded break is repaired by non-homologous end-joining; b) identifying at least one cell comprising an insertion or deletion at the target site, where the insertion or deletion at the target site results in the generation of a dominant positive allele of the at least one gene; and, c) selecting one or more cells comprising the dominant positive allele of the at least one gene.

In one aspect, an insertion or deletion provided herein abrogates an intron/exon splice site. An intron/exon splice site refers to the boundary between an intron and an exon in a gene. In eukaryotes, introns are typically, but not always, processed out of an RNA transcript by a spliceosome to produce an mRNA transcript that comprises only exonic sequence. If an intron/exon splice site is perturbed the spliceosome can fail to properly remove the intron sequence leading to a protein with one or more nonsense mutations to generate a premature stop codon. In an aspect, a nonsense mutation generates a truncated protein. In an aspect, a truncated protein comprises at least 1, at least 2, at least 3, at least 4, at least 5, at least 6, at least 7, at least 8, at least 9, at least 10, at least 15, at least 20, at least 25, at least 30, at least 35, at least 40, at least 45, at least 50, at least 60, at least 70, at least 80, at least 90, at least 100, at least 125, at least 150, at least 175, at least 200, at least 225, at least 250, at least 275, at least 300, at least 350, at least 400, at least 450, or at least 500 fewer amino acids than an endogenous protein encoded by a gene lacking a nonsense mutation.

In one aspect, a nonsense mutation is a mutation that results in a premature stop codon in a transcribed mRNA. In another aspect, an insertion or deletion provided herein is located in an exon. In another aspect, an insertion or deletion provided herein is located in an intron. In another aspect, an insertion or deletion provided herein is located in a 5'-UTR or a 3'-UTR. In an aspect, an insertion or deletion provided herein is located in a structure selected from the group consisting of an intron/exon splice site, an exon, an intron, a 5'-UTR, and a 3'-UTR. In still another aspect, a dominant negative allele provided herein comprises one or more, two or more, three or more, four or more, or five or more insertions and/or deletions. In still another aspect, a dominant positive allele provided herein comprises one or more, two or more, three or more, four or more, or five or more insertions and/or deletions.

In another aspect, a nonsense mutation provided herein is located in an exon. In an aspect, an insertion or deletion provided herein is located in a structure selected from the group consisting of an intron/exon splice site and an exon. An insertion or deletion provided herein can generate a protein with one or more, two or more, three or more, four or more, five or more, six or more, seven or more, eight or more, nine or more, or ten or more nonsense mutations.

In an aspect, a dominant negative allele provided herein comprises a polynucleotide comprising a premature stop codon as compared to a polynucleotide of a control allele. A premature stop codon is a stop codon positioned upstream of the normal stop codon of a gene. A premature stop codon generates a truncated protein. A stop codon is a nucleotide triplet in an mRNA that signals the termination of protein translation from the mRNA. In one aspect, a dominant negative allele provided herein comprises a polynucleotide encoding a truncated protein. In one aspect, a truncated protein provided herein is at least 1, at least 2, at least 3, at least 4, at least 5, at least 6, at least 7, at least 8, at least 9, at least 10, at least 15, at least 20, at least 25, at least 30, at least 40, at least 50, at least 60, at least 70, at least 80, at least 90, at least 100, at least 125, at least 150, at least 175, at least 200, at least 225, at least 250, at least 275, at least 300, at least 400, or at least 500 amino acids shorter than a full-length protein. In an aspect, a truncated protein provided herein is generated by the insertion or deletion of at least 1, at least 2, at least 3, at least 4, at least 5, at least 6, at least 7, at least 8, at least 9, at least 10, at least 15, at least 20, at least 25, at least 30, at least 40, at least 50, at least 60, at least 70, at least 80, at least 90, at least 100, at least 125, at least 150, at least 175, at least 200, at least 250, at least 300, at least 400, at least 500, at least 1000, at least 1500, or at least 2000 nucleotides.

In an aspect, a dominant positive allele provided herein comprises a polynucleotide comprising a premature stop codon as compared to a polynucleotide of a control allele. A premature stop codon is a stop codon positioned upstream of the normal stop codon of a gene. A premature stop codon generates a truncated protein. A stop codon is a nucleotide triplet in an mRNA that signals the termination of protein translation from the mRNA. In one aspect, a dominant positive allele provided herein comprises a polynucleotide encoding a truncated protein. In one aspect, a truncated protein provided herein is at least 1, at least 2, at least 3, at least 4, at least 5, at least 6, at least 7, at least 8, at least 9, at least 10, at least 15, at least 20, at least 25, at least 30, at least 40, at least 50, at least 60, at least 70, at least 80, at least 90, at least 100, at least 125, at least 150, at least 175, at least 200, at least 225, at least 250, at least 275, at least 300, at least 400, or at least 500 amino acids shorter than a full-length protein. In an aspect, a truncated protein provided herein is generated by the insertion or deletion of at least 1, at least 2, at least 3, at least 4, at least 5, at least 6, at least 7, at least 8, at least 9, at least 10, at least 15, at least 20, at least 25, at least 30, at least 40, at least 50, at least 60, at least 70, at least 80, at least 90, at least 100, at least 125, at least 150, at least 175, at least 200, at least 250, at least 300, at least 400, at least 500, at least 1000, at least 1500, or at least 2000 nucleotides.

This disclosure provides a method of generating a dominant negative allele of a gene in a cell comprising deleting a portion of a gene using a targeted editing technique, where a microprotein is generated following the deletion of the portion of the gene. In one aspect, a truncated protein is a microprotein. In another aspect, a dominant negative allele provided herein encodes a microprotein. In a further aspect, a dominant positive allele provided herein encodes a microprotein. As used herein, a "microprotein" refers to short, single-domain proteins that possess the ability to interfere with larger multi-domain proteins. In one aspect, a microprotein provided herein interferes with at least one, at least two, at least three, at least four, at least five, at least six, at least seven, at least eight, at least nine, or at least ten other proteins. In one aspect, a microprotein provided herein is capable of preventing a second protein from binding to a nucleic acid molecule. In another aspect, a microprotein provided herein is capable of preventing a second protein from binding to a third protein. The third protein can be identical or non-identical to the second protein. In another aspect, a microprotein provided herein is capable of binding to at least two, at least three, at least four, at least five, at least six, at least seven, at least eight, at least nine, or at least ten other proteins. In one aspect, a microprotein provided herein can form heterodimers with at least two, at least three, at least four, at least five, at least six, at least seven, at least eight, at least nine, or at least ten other proteins. In another aspect, a microprotein provided herein is capable of forming a homodimer. As used herein, a "homodimer" refers to hybridization or binding of two identical molecules (*e.g*., protein A and protein A), while a "heterodimer" refers to the hybridization or binding of two different macromolecules (*e.g*., protein A and protein B; protein A and DNA; protein A and RNA).

Members of the pentatricopeptide repeat (PPR) gene family are common in plant genomes. Many PPR proteins are capable of binding RNA molecules in a sequence-specific manner. PPR proteins comprise 2-30 PPR motifs, each of which aligns to a single nucleotide in an RNA molecule. Within the PPR motifs, the amino acids present at two or three specific positions confer nucleotide specificity. For example, without being limiting, a PPR motif binds an adenine nucleotide when a threonine is in position 6 and an asparagine is at the 1' position: a PPR motif binds a guanine nucleotide when a threonine is in position 6 and an aspartic acid is at the 1' position; a PPR motif binds a uracil (or thymine) nucleotide when an asparagine is at position 6 and an aspartic acid is at the 1' position; and a PPR motif binds a cytosine nucleotide when an asparagine is at position 6 and an asparagine or a serine is at the 1' position.

Without being limiting, an engineered PPR protein can be generated by at least two building strategies. In the first strategy, a PPR protein is constructed by treating each PPR motif as a separate block, such that a PPR protein is constructed by putting multiple desired motifs in order. The resulting engineered PPR protein is then capable of binding a target RNA molecule. However, such a strategy may not always work because each PPR motif comprises an internal scaffolding between the 1' and 6 positions, and that intra-motif scaffold is not shared across different PPR proteins. The second strategy makes use of the preexisting intra-motif scaffolding. In the second strategy, site-directed mutagenesis of the 1' and 6 positions is used to edit an existing PPR protein such that it will be specific to a new target RNA molecule.

As used herein, an "engineered PPR protein," "engineered PPR motif," refers to a synthetically created PPR protein or PPR motif that does not exist in nature and is capable of binding an RNA sequence in a site-specific manner.

This disclosure provides a method comprising: a) providing to a cell an engineered PPR protein or a vector encoding the engineered PPR protein operably linked to a promoter, where the engineered PPR protein is capable of binding to an RNA transcript of a target gene; b) selecting one or more cells from step (a) expressing the engineered PPR protein; and c) identifying one or more cells selected in step (b) comprising altered expression of the target gene. In one aspect, the engineered PPR protein is capable of binding to at least one non-coding RNA target site of the RNA transcript. In one aspect, the engineered PPR protein binds to at least one non-coding RNA target site of the RNA transcript. In one aspect, the altered expression is increased expression. In another aspect, the altered expression is reduced expression. In an aspect, the promoter is the native promoter of the target gene. In another aspect, the promoter is selected from the group consisting of a constitutive promoter, a tissue-specific promoter, a tissue-preferred promoter, and an inducible promoter.

In one aspect, an engineered PPR protein provided herein binds to a non-coding RNA target site of a target RNA molecule and blocks the non-coding RNA from cleaving the target RNA or inhibiting translation of the target RNA. In another aspect, an engineered PPR protein provided herein directs the degradation of a target RNA molecule. In one aspect, an engineered PPR protein provided herein comprises at least one RNA nuclease domain. In another aspect, an RNA nuclease domain provided herein is a NYN nuclease domain or a small MutS-related (SMR) domain.

In an aspect, an engineered PPR protein or an engineered PPR motif provided herein binds to at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, or at least 35 nucleotides of an RNA molecule. In another aspect, an engineered PPR protein or an engineered PPR motif provided herein binds to between 5 and 35, between 5 and 30, between 5 and 25, between 5 and 20, between 5 and 15, between 5 and 14, between 5 and 13, between 5 and 12, between 5 and 11, between 5 and 10, between 10 and 35, between 10 and 30, between 10 and 25, between 10 and 20, between 10 and 15, between 10 and 14, between 10 and 13, between 10 and 12, or between 15 and 30 nucleotides of an RNA molecule.

In an aspect, an engineered PPR protein provided herein is capable of acting as a dominant negative allele. In an aspect, an engineered PPR protein provided herein is capable of acting as a dominant positive allele.

In one aspect, an engineered PPR protein is targeted to a mitochondria or a chloroplast. In another aspect, an engineered PPR protein is targeted to a nucleus. In still another aspect, an engineered PPR protein is targeted to a cytoplasm of a cell. Without being limited by any theory, a protein can be targeted to a specific cellular structure by adding or editing a transit peptide on the N-terminus of the protein.

In one aspect, a genome editing system provided here comprises a tgOligo as a tether molecule. In another aspect, a tether molecule is a cross-linker coupled to a nuclease or a DNA-targeting guide molecule. In a further aspect, a tether molecule is a dimerization domain coupled to a nuclease.

In one aspect, a tether molecule is capable of tethering two or more DNA binding machineries bound to two genomic loci. In another aspect, a tether molecule is capable of tethering two or more DNA binding machineries bound to two genomic loci located in in a single chromosome flanking a target genomic region. In another aspect, a tether molecule is capable of tethering two or more DNA binding machineries bound to two genomic loci are on separate chromosomes.

In one aspect, this disclosure provides a method of generating a dominant negative allele of at least one gene in at least one cell comprising: a) introducing to the at least one cell a genome editing system comprising: i) a site-specific nuclease, or a molecule encoding a site-specific nuclease, ii) a sgRNA, or a molecule encoding a sgRNA, and iii) at least a first tether-guided oligo (tgOligo) and a second tgOligo, or one or more molecules encoding a first and a second tgOligo, operably linked to at least one promoter: b) generating a first double-stranded break and a second double-stranded break in the at least one gene, where the first tgOligo and the second tgOligo hybridize to the 3' free ends of opposing strands at the first double-stranded break and the second double-stranded break, where at least 1, at least 2, at least 3, at least 4, at least 5, at least 10, at least 25, at least 50, at least 100, at least 250, at least 500, at least 750, at least 1000, at least 2500, or at least 5000 nucleotides of the at least one gene are deleted, thereby generating a dominant negative allele of the gene that encodes a truncated protein: and c) identifying and selecting at least one cell comprising the truncated protein.

In one aspect, this disclosure provides a method of generating a dominant negative allele of at least one gene in at least one cell comprising: a) introducing to the at least one cell a genome editing system comprising: i) a site-specific nuclease, or a molecule encoding a site-specific nuclease, ii) a sgRNA, or a molecule encoding a sgRNA, and iii) at least a first tether-guided oligo (tgOligo) and a second tgOligo, or one or more molecules encoding a first and a second tgOligo, operably linked to at least one promoter; b) generating a first double-stranded break and a second double-stranded break in the at least one gene, where the first tgOligo and the second tgOligo hybridize to the 3' free ends of opposing strands at the first double-stranded break and the second double-stranded break, where between land 5000, between 5 and 5000, between 10 and 5000, between 25 and 2500, between 25 and 1000, between 25 and 750, between 25 and 500, between 25 and 100, between 50 and 5000, between 50 and 1000, between 50 and 500, between 100 and 1000, or between 1000 and 5000 nucleotides of the at least one gene are deleted, thereby generating a dominant negative allele of the gene that encodes a truncated protein: and c) identifying and selecting at least one cell comprising the truncated protein.

In another aspect, this disclosure provides a method of generating a dominant negative allele of at least one gene in at least one cell comprising: a) introducing to the at least one cell one or more vectors encoding: i) at least one site-specific nuclease, ii) at least one sgRNA, and iii) at least a first tgOligo and a second tgOligo operably linked to at least one promoter; b) generating a first double-stranded break and a second double-stranded break in the gene, where the first tgOligo and the second tgOligo hybridize to the 3' free ends of opposing strands at the first double-stranded break and the second double-stranded break, where the region of the at least one gene between the first double-stranded break and the second double-stranded break is inverted in orientation, thereby generating a dominant negative allele of the at least one gene that encodes an antisense RNA transcript of the gene; and c) identifying and selecting at least one cell comprising the antisense RNA transcript of the at least one gene.

As used herein, a "tether guide oligo" (tgOligo) refers to an oligonucleotide comprising a sequence segment capable of hybridizing with the 3' free end of the non-target strand of a double-stranded DNA molecule recognized and cleaved by a CRISPR gRNA-Cas complex (this 3' free end is also referred to as 3' free flap). A tgOligo corresponds to a gRNA when that tgOligo recognizes and hybridizes the 3' free end of the non-target strand of that gRNA's target site. A tgOligo can be a DNA molecule, a RNA molecule, or a mix of nucleotides. A hybrid tgOligo is a tgOligo that can recognize and hybridize with two non-target 3' free ends created by two separate CRISPR gRNA-Cas complexes.

As used herein, a "tether guide RNA" (tgRNA) refers to a RNA molecule comprising both a guide RNA (gRNA) sequence and a tether RNA sequence, where the tether RNA sequence is capable of hybridizing with a desired genomic site (which site is called "tether site").

In one aspect, a method provided herein comprises the use of one or more, two or more, three or more, four or more, five or more, six or more, seven or more, eight or more, nine or more, or ten or more tgOligos. In one aspect, a tgOligo is a DNA molecule. In another aspect, a tgOligo is an RNA molecule. In a still further aspect, a tgOligo is a mixture of DNA and RNA molecules. In one aspect, a tgOligo is single-stranded. In another asked, a tgOligo is double-stranded. In one aspect, at least one or at least two tgOligos are used concurrently with at least one, at least two, at least three, at least four, at least five, at least six, at least seven, at least eight, at least nine, or at least ten site-specific nucleases. In another aspect, at least one tgOligo is not used concurrently with a site-specific nuclease. In an aspect, at least one or at least two tgOligos are tethered to at least one or at least two Cas9 proteins. In one aspect, a first tgOligo is tethered to a first Cas9 protein and a second tgOligo is tethered to a second Cas9 protein. In another aspect, at least one or at least two tgOligos are tethered to at least one or at least two deactivated Cas9 proteins.

In still another aspect, a tgOligo provided herein comprises at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 65, at least 70, at least 75, at least 80, at least 85, at least 90, at least 95, at least 100, at least 125, at least 150, at least 175, at least 200, at least 250, at least 500, at least 1000, at least 2500, at least 5000, at least 10,000, or at least 25,000 nucleotides. In a further aspect, a tgOligo provided herein comprises between 5 and 25,000 nucleotides, between 5 and 10,000 nucleotides, between 5 and 5000 nucleotides, between 20 and 10,000 nucleotides, between 20 and 5000 nucleotides, between 20 and 1000 nucleotides, between 20 and 500 nucleotides, between 20 and 250 nucleotides, between 50 and 2500 nucleotides, between 50 and 1000 nucleotides, between 50 and 500 nucleotides, between 50 and 250 nucleotides, between 100 and 2500 nucleotides, between 100 and 1000 nucleotides, between 100 and 500 nucleotides, or between 1000 and 10,000 nucleotides.

In one aspect, a first tgOligo and a second tgOligo are at least 80%, at least 85%, at least 90%, at least 91%, at least 92%, at least 93%, at least 94%, at least 95%, at least 96%, at least 97%, at least 98%, at least 99%, at least 99.5%, or 100% complementary to each other. In one aspect, a first tgOligo and a second tgOligo are at least 80%, at least 85%, at least 90%, at least 91%, at least 92%, at least 93%, at least 94%, at least 95%, at least 96%, at least 97%, at least 98%, at least 99%, at least 99.5%, or 100% complementary to each other for at least 10, at least 11, at least 12, at least 13, at least 14, at least 15, at least 20, at least 25, at least 30, at least 35, at least 40, at least 45, at least 50, at least 55, at least 60, at least 65, at least 70, at least 75, at least 80, at least 85, at least 90, at least 95, at least 100, at least 125, at least 150, at least 175, at least 200, at least 250, at least 500, at least 1000, at least 2500, or at least 5000 nucleotides. In one aspect, a first tgOligo comprises a sense strand and a second tgOligo comprises an antisense strand.

In an aspect, a tgOligo is provided to a cell. In another aspect, a tgOligo is encoded by a vector. In another aspect, a site-specific nuclease and a tgOligo are encoded by one vector. In yet another aspect, a site-specific nuclease and a tgOligo are encoded by two or more vectors.

Methods provided herein are suitable for generating dominant alleles of protein-coding genes and non-coding RNAs. Without being limiting, examples of target genes of envisioned by the present disclosure in a plant genome would include genes for disease, insect, or pest tolerance; herbicide tolerance; genes for quality improvements such as yield, nutritional enhancements, environmental or stress tolerances; or any desirable changes in plant physiology, growth, development, morphology or plant product(s) including starch production (U.S. Pat. Nos. 6,538,181; 6,538,179: 6,538,178; 5,750,876; 6,476,295); modified oils production (U.S. Pat. Nos. 6,444,876; 6,426,447; 6,380,462); high oil production (U.S. Pat. Nos. 6,495,739; 5,608,149; 6,483,008; 6,476,295); modified fatty acid content (U.S. Pat. Nos. 6,828,475; 6,822,141; 6,770,465; 6,706,950; 6,660,849; 6,596,538; 6,589,767; 6,537,750; 6,489,461; 6,459,018); high protein production (U.S. Pat. No. 6,380,466); fruit ripening (U.S. Pat. No. 5,512,466); enhanced animal and human nutrition (U.S. Pat. Nos. 6,723,837: 6,653,530; 6.5412.59; 5,985,605; 6,171,640); or biopolymers (U.S. Pat. Nos. RE37,543: 6,228,623; 5,958,745 and U.S. Patent Publication No. US20030028917). Also environmental stress resistance (U.S. Pat. No. 6,072,103); pharmaceutical peptides and secretable peptides (U.S. Pat. Nos. 6,812,379; 6,774,283; 6,140,075; 6,080,560); improved processing traits (U.S. Pat. No. 6,476,295); improved digestibility (U.S. Pat. No. 6,531,648); low raffinose (U.S. Pat. No. 6,166,292); industrial enzyme production (U.S. Pat. No. 5,543,576); improved flavor (U.S. Pat. No. 6,011,199); nitrogen fixation (U.S. Pat. No. 5,229,114); hybrid seed production (U.S. Pat. No. 5,689,041); fiber production (U.S. Pat. Nos. 6,576,818; 6,271,443; 5,981,834; 5,869,720); and biofuel production (U.S. Pat. No. 5,998,700).

In one aspect, a gene edited by the methods provided herein is selected from the group consisting of a Y1 gene, a brachytic2 gene, a GA3 oxidase gene, and a GA20 oxidase gene. In another aspect, a gene edited by the methods provided herein encodes a non-coding RNA. In an aspect, a non-coding RNA edited by the methods provided herein is selected from the group consisting of a microRNA, a small interfering RNA, a transfer RNA, a ribosomal RNA, a *trans-*acting small interfering RNA, a naturally occurring antisense small interfering RNA, a heterochromatic small interfering RNA, and precursors thereof. In still another aspect, a gene edited by the methods provided herein encodes a miRNA. In a further aspect, a gene edited by the methods provided herein encodes a precursor miRNA (pre-miRNA).

In one aspect, a GA20 oxidase gene provided herein is encoded by an mRNA that encodes a protein having at least 80%, at least 85%, at least 90%, at least 91%, at least 92%, at least 93%, at least 94%, at least 95%, at least 96%, at least 97%, at least 98%, at least 99%, at least 99.5%, or 100% identical to a sequence selected from the group consisting of SEQ ID NOs: (insert protein sequences for GA20). In another aspect, a brachytic2 gene provided herein is encoded by an mRNA that encodes a protein having at least 80%, at least 85%, at least 90%, at least 91%, at least 92%, at least 93%, at least 94%, at least 95%, at least 96%, at least 97%, at least 98%, at least 99%, at least 99.5%, or 100% identical to a sequence selected from the group consisting of SEQ ID NOs: (insert protein sequences for BR2).

In one aspect, an unmodified allele provided herein comprises a polynucleotide sequence having at least 80%, at least 85%, at least 90%, at least 91%, at least 92%, at least 93%, at least 94%, at least 95%, at least 96%, at least 97%, at least 98%, at least 99%, at least 99.5%, or 100% identity or complementarity to a sequence selected from the group consisting of SEQ ID NOs: (list GA and BR2 sequences).

In another aspect, a non-coding RNA edited by the methods provided herein is selected from the group consisting of a microRNA, a small interfering RNA, a transfer RNA. a ribosomal RNA, a *trans*-acting small interfering RNA, a naturally occurring antisense small interfering RNA, a heterochromatic small interfering RNA, and precursors thereof.

### EXAMPLES

### Example 1. Generating a dominant allele of GA20 oxidase via targeted genomic inversion

Two functional guide RNAs (gRNAs) for a CRISPR/RNA-guided nuclease system are created to target flanking regions (a left target site and a right target site) of a GA20 oxidase_5 gene in the corn genome. *See* Figure 1, Panel A. Each of the two target sites are unique within the corn genome. The plant hormone gibberellin plays an important role in a number of plant developmental processes including germination, cell elongation, flowering, embryogenesis and seed development. Certain biosynthetic enzymes (e.g., GA20 oxidase and GA3 oxidase) and catabolic enzymes (e.g., GA2 oxidase) in the GA pathway are critical to affecting GA levels in plant tissues.

A transfer DNA (T-DNA) vector suitable for use in *Agrobacterium* transformation is used. The T-DNA construct comprises several expression cassettes between a left border (LB) sequence and a right border (RB) sequence. The first expression cassette comprises a promoter that is operable in a plant cell operably linked to a polynucleotide encoding an RNA guided nuclease. A second expression cassette comprises a promoter that is operable in a plant cell operably linked to the CP4-EPSPS marker gene. The construct also comprises expression cassettes comprising promoters operable in a plant cell operably linked to polynucleotides encoding the two gRNAs described above.

Immature corn embryos are co-cultured with *Agrobacterium* containing the T-DNA vector for three days. The polynucleotides between the LB and RB sequences are integrated into the nuclear genome of the immature corn embryos. Upon expression of the integrated polynucleotides, the gRNAs guide the nuclease to each of the two target sites in the GA20 oxidase_5 gene, where the nuclease creates a double-stranded break at each target site.

In the majority of events, the region between the target sites is deleted and non-homologous end-joining repair mechanisms joins the flanking regions. Less frequently, some events create insertion/deletion mutations at the left target site, the right target site, or both. In still other events the entire targeted region is inverted in what is referred to as a "complete inversion." *See* Figure 1, Panel B. Suitable methods known in the art (*e.g.*, PCR, DNA hybridization (Southern) blots, sequencing) are used to identify transformation events comprising a complete inversion. Transformed embryos comprising a targeted complete inversion in the 5' end of the GA20 oxidase_5 gene are selected and used to regenerate modified plants using techniques standard in the field.

Without being bound to any scientific theory, the presence of a complete inversion in one allele of the GA20 oxidase_5 creates a population of antisense mRNAs under the control of the native GA20 oxidase_5 promoter. The inverted region of the edited GA20 oxidase_5 allele mRNA and the corresponding region in the unedited GA20 oxidase_5 allele mRNA are complementary to each other and are capable of forming a dsRNA. Therefore, even though the modified corn plant can be heterozygous for an edited GA20 oxidase_5 allele, the edited allele can reduce the expression of the GA20 oxidase_5 gene in the modified corn plant.

RNA is extracted from modified corn plants identified as comprising a complete inversion of the targeted region of the GA20 oxidase_5 gene. RNA is also extracted from control corn plants that lack a complete inversion. Suitable methods known in the art (*e.g.*, quantitative reverse-transcriptase PCR, reverse-transcriptase PCR, RNA sequencing) are used to confirm that down-regulation of GA20 oxidase_5 occurs in modified corn plants comprising a complete inversion of the targeted region.

### Example 2. Generating a dominant allele of BR2 via targeted genomic inversion

Two functional guide RNAs (gRNAs) for an RNA guided nuclease system are created to target flanking regions (a left target site and a right target site) of a BRACHYTIC2 (BR2) gene in the corn genome. Each of the two target sites are unique within the corn genome.

A transfer DNA (T-DNA) vector suitable for use in *Agrobacterium tumefaciens* transformation is used. The T-DNA construct comprises several expression cassettes between a left border (LB) sequence and a right border (RB) sequence. The first expression cassette comprises a promoter that is operable in a plant cell operably linked to a polynucleotide encoding an RNA guided nuclease. A second expression cassette comprises a promoter that is operable in a plant cell operably linked to the CP4-EPSPS marker gene. The construct also comprises expression cassettes comprising promoters operable in a plant cell operably linked to polynucleotides encoding the two gRNAs described above.

Immature corn embryos are co-cultured with *Agrobacterium tumefaciens* containing the T-DNA vector for three days. The polynucleotides between the LB and RB sequences are integrated into the nuclear genome of the immature corn embryos. Upon expression of the integrated polynucleotides, the gRNAs guide the CRISPR endonuclease to each of the two target sites in the BR2 gene, where the CRISPR endonuclease creates a double-stranded break at each target site.

In the majority of events, the region between the target sites is deleted and non-homologous end-joining repair mechanisms joins the flanking regions. Less frequently, some events create insertion/deletion mutations at the left target site, the right target site, or both. In still other events the entire targeted region is inverted in what is referred to as a "complete inversion." Suitable methods known in the art (*e.g*., PCR, DNA hybridization (Southern) blots, sequencing) are used to identify transformation events comprising a complete inversion. Transformed embryos comprising a targeted complete inversion in the 5' end of the BR2 gene are selected and used to regenerate plants using techniques standard in the field.

Without being bound to any scientific theory, the presence of a complete inversion in one allele of the BR2 gene creates a population of anti-sense mRNAs under the control of the native BR2 promoter. The inverted region of the edited BR2 allele mRNA and the corresponding region in the unedited BR2 allele mRNA are complementary to each other and are capable of forming a dsRNA. Therefore, even though the corn plant may be heterozygous for an edited BR2 allele, the edited allele can reduce the expression both alleles of the BR2 gene in the corn plant, and thus results in a brachytic phenotype.

### Example 3. Generating a dominant allele of GA20 oxidase via a targeted genomic deletion to render a GA20 oxidase gene under the control of two reverse oriented promoters

A gene encoding GA20 oxidase_5 (also called GA20ox5) is located on corn chromosome 8. It is adjacent to the corn gene GRMZM2G049269, which encodes an S-adenosyl-L-methionine-dependent methyltransferase superfamily protein ("SAMT" hereinafter). The SAMT is a member of a large, redundant gene family, and no phenotypes associated with mutations to this gene have been reported in corn or *Arabidopsis.* The SAMT gene is positioned in the opposite orientation as compared to the GA20 oxidase_5 gene (*i.e.*, the SAMT gene is oriented to read 5' to 3', while the GA20 oxidase_5 gene is oriented to read 3' to 5' on the same DNA strand). *See* Figure 2, Panel A.

Two functional guide RNAs (gRNAs) for an RNA guidednuclease system are created to target the genomic DNA region between the GA20 oxidase_5 gene and the SAMT gene. The first gRNA targets an area near the transcriptional start site of the SAMT gene, and the second gRNA targets a region near the transcriptional stop site of the GA20 oxidase_5 gene. Each of the two target sites are unique within the corn genome. A transfer DNA (T-DNA) vector suitable for use in *Agrobacterium* transformation is used. The T-DNA construct comprises several expression cassettes between a left border (LB) sequence and a right border (RB) sequence. The first expression cassette comprises a promoter that is operable in a plant cell operably linked to a polynucleotide encoding an RNA guided nuclease. A second expression cassette comprises a promoter that is operable in a plant cell operably linked to the CP4-EPSPS marker gene. The construct also comprises expression cassettes comprising promoters operable in a plant cell operably linked to polynucleotides encoding the two gRNAs described above.

Immature corn embryos are co-cultured with *Agrobacterium* containing the T-DNA vector for three days. The polynucleotides between the LB and RB sequences are integrated into the nuclear genome of the immature corn embryos. Upon expression of the integrated polynucleotides, the gRNAs guide the nuclease to each of the two target sites in the genomic DNA region between the GA20 oxidase_5 gene and the SAMT gene, where the nuclease creates a double-stranded break at each target site.

In the majority of events, the region between the target sites is deleted and non-homologous end-joining repair mechanisms joins the flanking regions. Suitable methods known in the art (*e.g.*, PCR, DNA hybridization (Southern) blots, sequencing) are used to identify transformation events comprising a complete deletion. Transformed embryos comprising a targeted deletion between the GA20 oxidase_5 gene and SAMT gene are selected and used to regenerate modified plants using techniques standard in the field.

Without being bound to any scientific theory, by removing the genomic DNA between the GA20 oxidase_5 gene and the SAMT promoter, the native SAMT promoter can generate an antisense mRNA transcript of the GA20 oxidase_5 gene, while the native GA20 oxidase_5 promoter generates a sense mRNA transcript of the GA20 oxidase_5 gene. The complementary sense and antisense mRNA transcripts of the GA20 oxidase_5 gene are capable of forming a dsRNA that can be processed by RNA silencing mechanisms native to the corn cell. *See* Figure 2, Panel B. The processed dsRNA can then suppresses the expression of both GA20 oxidase_5 alleles. Additionally, because the mRNA encoding GA20 oxidase_3 is so similar to the sense GA20 oxidase_5 transcript, it is expected that the deletion between the SAMT promoter and the GA20 oxidase_5 gene will also down-regulate the expression of GA20 oxidase_3. It is also envisioned that suppression or silencing of the GA20 oxidase_5 gene may occur through other mechanisms as provided herein (e.g., nonsense-mediated decay), alternatively or in addition to any RNAi or PTGS forms of suppression.

RNA is extracted from modified corn plants identified as comprising a targeted deletion between the SAMT promoter and the GA20 oxidase_5 gene. RNA is also extracted from control corn plants that lack the deletion. Suitable methods known in the art (*e.g.*, quantitative reverse-transcriptase PCR, reverse-transcriptase PCR, RNA sequencing) are used to confirm that down-regulation of GA20 oxidase_5 and/or GA20 oxidase_3 occurs in corn modified plants comprising a deletion of the targeted region.

### Example 4. Generating a dominant allele of BR2 via a targeted genomic deletion to render a BR2 gene under the control of two reverse oriented promoters

A gene encoding BR2 is located on corn chromosome 1. It is adjacent to the corn gene GRMZM2G491632 which is expressed in the opposite orientation to BR2. Two functional guide RNAs (gRNAs) for an RNA guided nuclease system are created to target the genomic DNA region between the BR2 gene and the GRMZM2G491632 gene. The first gRNA targets an area near the end of the BR2 gene exon 1, and the second gRNA targets a region near the beginning of the coding sequence of the GRMZM2G491632 gene exon 1.

A transfer DNA (T-DNA) vector suitable for use in *Agrobacterium* transformation is used. The T-DNA construct comprises several expression cassettes between a left border (LB) sequence and a right border (RB) sequence. The first expression cassette comprises a promoter that is operable in a plant cell operably linked to a polynucleotide encoding an RNA guided nuclease. A second expression cassette comprises a promoter that is operable in a plant cell operably linked to the CP4-EPSPS marker gene. The construct also comprises expression cassettes comprising promoters operable in a plant cell operably linked to polynucleotides encoding the two gRNAs described above

Immature corn embryos are co-cultured with *Agrobacterium* containing the T-DNA vector for three days. The polynucleotides between the LB and RB sequences are integrated into the nuclear genome of the immature corn embryos. Upon expression of the integrated polynucleotides, the gRNAs guide the endonuclease to each of the two target sites in the genomic DNA region between the BR2 gene and the GRMZM2G491632 gene, where the endonuclease creates a double-stranded break at each target site.

In the majority of events, the region between the target sites is deleted and non-homologous end-joining repair mechanisms joins the flanking regions. Suitable methods known in the art (*e.g.*, PCR, DNA hybridization (Southern) blots, sequencing) are used to identify transformation events comprising a deletion. Transformed embryos comprising a targeted deletion between the BR2 gene and the GRMZM2G491632 gene are selected and used to regenerate plants using techniques standard in the field.

Without being bound to any scientific theory, by removing the genomic DNA between the BR2 gene and the GRMZM2G491632 promoter, the native GRMZM2G491632 promoter can generate an anti-sense mRNA transcript of the BR2 gene while the native BR2 promoter generates a sense mRNA transcript of the BR2 gene. The complementary sense and anti-sense mRNA transcripts of the BR2 gene can form a dsRNA that can be processed by RNAi machinery native to the corn cell. The processed dsRNA could then suppress the expression of both BR2 alleles, resulting in a brachytic phenotype.

### Example 5. Insertion of designed elements into native promoters using genome editing techniques to create a dominant allele.

A gene comprising a root-specific promoter is identified in the *Arabidopsis thaliana* genome. *See* Figure 3, Panel A. This promoter is used to drive the expression of GUS in the roots of the plant. *See* Figure 3, Panel B. A functional gRNA is designed to target a region upstream of a TATA box of the root-specific promoter (the "target site"). A transfer DNA (T-DNA) vector suitable for use in *Agrobacterium* transformation is used to introduce the gRNA to *Arabidopsis.* The T-DNA construct comprises several expression cassettes between a left border (LB) sequence and a right border (RB) sequence. The first expression cassette comprises a promoter that is operable in a plant cell operably linked to a polynucleotide encoding an RNA guided nuclease. A second expression cassette comprises a promoter that is operable in a plant cell operably linked to the CP4-EPSPS marker gene. The construct also comprises an expression cassette comprising a promoter operable in a plant cell operably linked to a polynucleotide encoding the gRNA described above. A second T-DNA construct comprises a polynucleotide encoding a donor molecule comprising a designed element to be inserted at the target site, between a LB sequence and a RB sequence. In one embodiment, the donor molecule comprises the designed element flanked by homologous regions that are homologous to sequences present on either side of the target site. The designed element enables the formerly root-specific gene to be expressed constitutively in all tissues of the plant when it is inserted into the promoter region of a gene. In another embodiment, the donor molecule comprises the designed sequence flanked by target sites that are targeted by the gRNA in T-DNA vector 1.

The floral dip method is used to transform Arabidopsis using the vector described above. See Clough and Bent. 1998, Plant J, 16: 735-743, which is incorporated herein in its entirety. Upon expression of the integrated polynucleotides, the gRNA guides the nuclease to the target site and creates a double-stranded break at the target site. For donor molecues comprising designed sequence flanked by Homology arms, homologous repair mechanisms native to the *Arabidopsis* cells insert the designed element at the site of the double-stranded break. For donor molecules comprising the designed sequence flanked by gRNA target sites, the gRNAs guide the nuclease to create double standed breaks within the second T-DNA thereby releasing the designed sequence, which can then integrate within the genomic target site via NHEJ (Non Homologous End Joining) repair mechanisms. In a subset of the insertion events, the promoter inserts in the desired orientation. Without being bound by any particular theory, the presence of the designed element upstream of the TATA box induces constitutive expression of the gene throughout the modified plant, thereby creating a dominant allele of the gene.

Suitable methods known in the art (*e.g.*, PCR, DNA hybridization (Southern) blots, sequencing) are used to identify transformation events comprising a targeted insertion of the designed element in the desired orientation. Transformed *Arabidopsis* plants comprising the designed element at the target site are selected and further examined.

Plants identified as comprising the designed element upstream of the TATA box (*see* Figure 3, Panel D) are examined for the expression of GUS using techniques standard in the art. Plants comprising the designed element exhibit GUS expression throughout the plant. *See* Figure 3, Panel C. Additionally, RNA is extracted from various tissues (*e.g.*, roots, stem, leaf, inflorescence) of the modified *Arabidopsis* plants identified as comprising the designed element. RNA is also extracted from control *Arabidopsis* plants that lack the designed element. Suitable methods known in the art (*e.g*., quantitative reverse-transcriptase PCR, reverse-transcriptase PCR, RNA sequencing) are used to confirm that GUS is more broadly expressed and/or more strongly expressed in the modified *Arabidopsis* plants.

### Example 6. Insertion of tissue-specific suppression elements using genome editing techniques to create a dominant allele.

An *Arabidopsis thaliana* plant comprising a GUS transgene under the control of a promoter that is functional in leaf, vascular, and root tissue is created using standard techniques in the art. *See* Figure 4 for a general overview of the concept provided in this Example; and Figure 5, Panels A and B. A functional gRNA is designed to target a region downstream of the GUS gene (the "target site"). A transfer DNA (T-DNA) vector suitable for use in *Agrobacterium* transformation is used to introduce the gRNA to *Arabidopsis.* The T-DNA construct comprises several expression cassettes between a left border (LB) sequence and a right border (RB) sequence. The first expression cassette comprises a promoter that is operable in a plant cell operably linked to a polynucleotide encoding an RNA guided nuclease. A second expression cassette comprises a promoter that is operable in a plant cell operably linked to the CP4-EPSPS marker gene. The construct also comprises an expression cassette comprising a promoter operable in a plant cell operably linked to a polynucleotide encoding the gRNA described above. A second T-DNA construct comprises a donor molecule comprising a leaf-specific promoter such as the COOLAIR promoter (see Chen and Penfield, Science, 2018, 360: 6392) between a LB sequence and a RB sequence. In one embodiment, the donor molecule comprises the promoter in an antisense orientation flanked by homologous regions that are homologous to sequences present on either side of the target site. The antisense oriented leaf-specific promoter enables the expression of a GUS antisense mRNA in tissues where the promoter is expressed (*e.g*., leaf tissue). Without being bound by any particular theory, the antisense RNA transcript of the GUS gene leads to the silencing of GUS in leaf tissue, but not root tissue. In another embodiment, the donor molecule comprises the promoter sequence flanked by target sites that are targeted by the gRNA in T-DNA vector 1.

The floral dip method is used to transform *Arabidopsis* using the vector described above. *See* Clough and Bent, 1998, Plant J, 16: 735-743, which is incorporated herein in its entirety. Upon expression of the polynucleotides, the gRNA guides the nuclease to the target site and creates a double-stranded break at the target site. For donor molecues comprising the antisense oriented promoter flanked by homology arms, homologous repair mechanisms native to the *Arabidopsis* cells insert the leaf-specific promoter at the site of the double-stranded break downstream of the GUS gene, thereby placing the promoter in an anti-sense orientation to the GUS gene. For donor molecues comprising the promoter flanked by gRNA target sites, the gRNAs guide the nuclease to create double standed breaks within the second T-DNA thereby releasing the promoter sequence that can then integrate within the genomic target site via NHEJ (Non Homologous End Joining) repair mechanisms. In a subset of the insertion events, the promoter inserts in an anti-sense orientation. Without being bound by a particular theory, the presence of the antisense leaf-specific promoter induces reduced expression of GUS throughout leaf tissue of the plant, thereby creating a dominant allele of the gene.

Suitable methods known in the art (*e.g.*, PCR, DNA hybridization (Southern) blots, sequencing) are used to identify transformation events comprising a targeted insertion of the promoter in the desired orientation. Transformed *Arabidopsis* plants comprising the leaf-specific promoter at the target site are selected and further examined.

Plants identified as comprising the leaf-specific promoter downstream of the GUS gene in antisense orientation to the GUS gene (*see* Figure 5, Panel D) are examined for the expression of GUS using techniques standard in the art. Plants comprising the leaf-specific promoter in antisense orientation to the GUS gene exhibit GUS expression only in root tissues. *See* Figure 5, Panel C. Additionally, RNA is extracted from various tissues (*e.g.*, roots, stem, leaf, inflorescence) of the modified *Arabidopsis* plants identified as comprising the leaf-specific promoter. RNA is also extracted from control *Arabidopsis* plants that lack the leaf-specific promoter in antisense orientation to the GUS gene. Suitable methods known in the art (*e.g*., quantitative reverse-transcriptase PCR, reverse-transcriptase PCR, RNA sequencing) are used to confirm that expression of GUS is reduced in leaf tissues.

### Example 7. Insertion of tissue-specific suppression elements using genome editing techniques to create a dominant GA20 oxidase_5 allele.

A functional gRNA is designed to target a region downstream of the 3'-UTR of the GA20 oxidase_5 gene (the "target site"). A transfer DNA (T-DNA) vector suitable for use in *Agrobacterium* transformation is used to introduce the gRNA to a corn cell. The T-DNA construct comprises several expression cassettes between a left border (LB) sequence and a right border (RB) sequence. The first expression cassette comprises a promoter that is operable in a plant cell operably linked to a polynucleotide encoding an RNA guided nuclease. A second expression cassette comprises a promoter that is operable in a plant cell operably linked to the CP4-EPSPS marker gene. The construct also comprises an expression cassette comprising a promoter operable in a plant cell operably linked to a polynucleotide encoding the gRNA described above. A second T-DNA construct comprises a donor molecule comprising an RTBV promoter between a LB sequence and a RB sequence. In one embodiment, the donor molecule comprises the RTBV promoter in an antisense orientation, flanked by homologous regions that are homologous to sequences present on either side of the target site. In another embodiment, the donor molecule comprises the promoter sequence flanked by target sites that are targeted by the gRNA in T-DNA vector 1.The antisense oriented RTBV promoter enables the expression of a GA20 oxidase_5 antisense mRNA in tissues where RTBV is expressed (*e*.*g*., stem and vascular tissue). Without being bound by any particular theory, the antisense RNA transcript of the GA20 oxidase_5 gene leads to the silencing of both GA20 oxidase_5 and GA20 oxidase_3 in stem and vascular tissue.

Immature corn embryos are co-cultured with *Agrobacterium* containing the T-DNA vectors for three days. Upon expression of the polynucleotides, the gRNA guides the nuclease to the target site and creates a double-stranded break at the target site. For donor molecues comprising the antisense oriented RTBV promoter flanked by Homology arms, homologous repair mechanisms native to the corn cell insert the the antisense RTBV promoter at the target site downstream of the 3' end of the GA20 oxidase_5 gene. For donor molecules comprising the promoter flanked by gRNA target sites, the gRNAs guide the nuclease to create double standed breaks within the second T-DNA there by releasing the promoter sequence that can then integrate within the genomic target site via NHEJ (Non Homologous End Joining) repair mechanisms. In a subset of the insertion events, the promoter inserts in an anti-sense orientation.

Suitable methods known in the art (*e.g.*, PCR, DNA hybridization (Southern) blots, sequencing) are used to identify transformation events comprising a targeted insertion of the antisense RTBV promoter. Transformed embryos comprising the antisense RTBV promoter at the target site are selected and used to regenerate modified plants using techniques standard in the field.

RNA is extracted from various tissues (*e.g*., roots, stem, leaf, inflorescence) of the modified corn plants identified as comprising the antisense RTBV promoter. RNA is also extracted from control corn plants that lack the antisense RTBV promoter at the target site. Suitable methods known in the art (*e.g*., quantitative reverse-transcriptase PCR, reverse-transcriptase PCR, RNA sequencing) are used to confirm that expression of GA20 oxidase_5 and/or GA20 oxidase_3 are reduced in stem and vascular tissue in the modified corn plants comprising the antisense RTBV promoter as compared to control corn plants.

### Example 8. Engineering a truncated protein using genome editing techniques to create a dominant allele.

**A) Engineering GA20 oxidase truncated proteins:** Targeted editing of genes that result in truncated proteins or non-sense mutations to the proteins can create dominant alleles. *See* Figure 6. The corn GA20 Oxidase_5 gene and GA20 Oxidase_3 gene are highly similar in sequence and structure. Both genes comprise three exons. A gRNA is designed to introduce an edit in an exon of both genes.

A transfer DNA (T-DNA) vector suitable for use in *Agrobacterium* transformation is used to introduce the gRNA to a corn cell. The T-DNA construct comprises several expression cassettes between a left border (LB) sequence and a right border (RB) sequence. The first expression cassette comprises a promoter that is operable in a plant cell operably linked to a polynucleotide encoding an RNA guided nuclease. A second expression cassette comprises a promoter that is operable in a plant cell operably linked to the CP4-EPSPS marker gene. The construct also comprises an expression cassette comprising a promoter operable in a plant cell operably linked to a polynucleotide encoding the gRNA described above.

Immature corn embryos are co-cultured with *Agrobacterium* containing the T-DNA vectors for three days. The polynucleotides between the LB and RB sequences are integrated into the nuclear genome of the immature corn embryos. Upon expression of the integrated polynucleotides, the gRNA guides the nuclease to the target site and creates a double-stranded break at the target site. Without being bound by a particular theory, the non-homologous end-joining repair mechanisms native to the cell frequently repair such breaks imperfectly, which can lead to the insertion or deletion of one or more nucleotides. These insertions or deletions to an exon can produce premature stop codons (which would generate a truncated protein), or non-sense mutations. A premature stop codon has the capability of generating a dominant allele of GA20 Oxidase_5 and GA20 Oxidase_3.

Suitable methods known in the art (*e.g.*, PCR, DNA hybridization (Southern) blots, sequencing) are used to identify transformation events comprising a targeted insertion or deletion in the GA20 Oxidase_5 and/or GA20 Oxidase_3 genes. Transformed embryos comprising an insertion or deletion capable of introducing a premature stop codon causing a truncated protein or a non-sense mutation at the target site are selected and used to regenerate modified plants using techniques standard in the field.

Protein is extracted from the modified corn plants identified as comprising the identified insertion/deletion. Suitable methods known in the art (*e.g*., Western blot; HPLC; LC/MS; ELISA; immunoprecipitation) are used to confirm that a truncated protein or a non-sense mutation has been introduced to GA20 Oxidase_5 and/or GA20 Oxidase_3 genes. Additional experiments to confirm a reduction in gibberellin acid in stem and/or vascular tissue are performed as described in Bensen et al., Plant Physiol. 1990, 94: 77-84, which is incorporated by reference herein in its entirety.

**B)Engineering Brachytic 2 (Br2) truncated protein:** A gRNA is designed to introduce an edit within an exon of the Brachytic 2 gene from corn.

A transfer DNA (T-DNA) vector suitable for use in *Agrobacterium* transformation is used to introduce the gRNA to a corn cell. The T-DNA construct comprises several expression cassettes between a left border (LB) sequence and a right border (RB) sequence. The first expression cassette comprises a promoter that is operable in a plant cell operably linked to a polynucleotide encoding an RNA guided nuclease. A second expression cassette comprises a promoter that is operable in a plant cell operably linked to the CP4-EPSPS marker gene. The construct also comprises an expression cassette comprising a promoter operable in a plant cell operably linked to a polynucleotide encoding the gRNA described above.

Immature corn embryos are co-cultured with *Agrobacterium* containing the T-DNA vectors for three days. The polynucleotides between the LB and RB sequences are integrated into the nuclear genome of the immature corn embryos. Upon expression of the integrated polynucleotides, the gRNA guides the nuclease to the target site where the nuclease creates a double-stranded break at the target site. Without being bound by a particular theory, the non-homologous end-joining repair mechanisms native to the cell frequently repair such breaks imperfectly, which can lead to the insertion or deletion of one or more nucleotides. These insertions or deletions to an exon can produce premature stop codons (which would generate a truncated protein), or non-sense mutations. A premature stop codon has the capability of generating a dominant allele of Brachytic 2 (Br2).

Suitable methods known in the art (*e.g.*, PCR, DNA hybridization (Southern) blots, sequencing) are used to identify transformation events comprising a targeted insertion or deletion in the Br2 gene. Transformed embryos comprising an insertion or deletion capable of introducing a premature stop codon (causing a truncated protein) or a non-sense mutation at the target site are selected and used to regenerate modified plants using techniques standard in the field.

Protein is extracted from the modified corn plants identified as comprising the identified insertion/deletion. Suitable methods known in the art (*e.g*., Western blot; HPLC; LC/MS; ELISA; immunoprecipitation) are used to confirm that a Br2 truncated protein is generated.

### Example 9. Generating an inverted repeat within a target gene

Targeted editing techniques can be used to convert a genomic locus into a locus that is capable of generating an RNAi-inducing hairpin when the edited locus is transcribed into RNA. *See* Figure 7. In a cell that is heterozygous at the locus of interest (*e.g.*, two polymorphic alleles are present), one or more nucleases are used to generate two double-stranded breaks (*e.g.*, a first double-stranded break and a second double-stranded break) in the first allele, and one double-stranded break (*e.g.*, a third double-stranded break) in the second allele. When the nucleases cut the first and second alleles, the portion of the first allele that is flanked by the first and second double-stranded breaks is released from the genomic DNA. In one outcome, the released portion of the first allele is inverted in orientation and integrated into the third double-stranded break in the second allele, thereby creating an edited locus that is capable of generating an RNAi-inducing hairpin when the edited locus is transcribed.

A first and a second functional guide RNA (gRNA) for a RNA guided nuclease system are created. The first and second gRNAs are complementary to a first target site and a second target site, respectively, flanking a portion of a first allele of a GA20 oxidase_5 gene in the corn genome. The first gRNA is also complementary to a second allele of the GA20 oxidase_5 gene at a third target site (which is homologous to the first target site), but the second gRNA is not complementary to the second allele due to a polymorphism between the first and second GA20 oxidase_5 alleles at the second target site.

A transfer DNA (T-DNA) vector suitable for use in *Agrobacterium* transformation is constructed. The T-DNA construct comprises several expression cassettes between a left border (LB) sequence and a right border (RB) sequence. The first expression cassette comprises a promoter that is operable in a plant cell operably linked to a polynucleotide encoding an RNA guided nuclease. A second expression cassette comprises a promoter that is operable in a plant cell operably linked to the CP4-EPSPS marker gene. The construct also comprises an expression cassette comprising a promoter operable in a plant cell operably linked to a polynucleotide encoding the first and second gRNAs described above.

Immature corn embryos are co-cultured with *Agrobacterium* containing the T-DNA vector for three days. Upon expression of the polynucleotides, the gRNAs guide the nuclease to each of the three target sites in the GA20 oxidase_5 alleles, where the nuclease creates a double-stranded break at each target site.

In the majority of events, the region between the first and second target sites in the first GA20 oxidase_5 allele is deleted and non-homologous end-joining repair mechanisms joins the flanking regions. Less frequently, some events create insertion/deletion mutations at the first target site, the second target site, or both. In still other events the entire targeted region of the first allele integrates into the double-stranded break at the third target site in an inverted orientation. *See* Figure 7, Panel D. Suitable methods known in the art (*e.g.,* PCR, DNA hybridization (Southern) blots, sequencing) are used to identify transformation events comprising an inversion in the second GA20 oxidase_5 allele. Transformed embryos comprising an inversion in the second GA20 oxidase_5 allele are selected and used to regenerate modified plants using techniques standard in the field.

Without being bound to any scientific theory, the presence of an inversion in one allele of the GA20 oxidase_5 creates a population of RNA transcripts capable of forming a hairpin structure. Such a hairpin induces RNAi machinery in the cell, which leads to down-regulation of GA20 oxidase_5 RNA transcripts in a dominant manner (*e.g*., edited and unedited alleles are both down-regulated).

RNA is extracted from modified corn plants identified as comprising an inversion in the second allele capable of generating a hairpin RNA transcript. RNA is also extracted from control corn plants that lack edited GA20 oxidase_5 alleles. Suitable methods known in the art (*e.g*., quantitative reverse-transcriptase PCR, reverse-transcriptase PCR, RNA sequencing) are used to confirm that down-regulation of GA20 oxidase_5 occurs in modified corn plants comprising an inversion in the second allele capable of generating a hairpin RNA transcript. Additionally, due to the sequence similarities between GA20 oxidase_5 and GA20 oxidase_3, the inversion in the second allele of GA20 oxidase_5 also causes down-regulation of GA20 oxidase_3 RNA transcripts.

### Example 10. Inserting a miRNA target site into a desired genomic locus

Targeted editing techniques can be used to insert a donor molecule into a target site in a genomic locus. *See* Figure 8. If a donor molecule comprising a non-coding RNA target site is inserted into a 5'-UTR, an exon, an intron, or a 3'-UTR of a gene of interest, RNA transcription or protein translation of the gene of interest can be suppressed by a complementary non-coding RNA. When a gene of interest is a target of a non-coding RNA (*e.g.*, a miRNA or an siRNA), a cleaved mRNA from the gene of interest can generate secondary siRNAs, which can further suppress the transcription or translation of the gene of interest. Such secondary suppression can act in a dominant manner, as the secondary siRNAs are complementary to alleles with and without the insertion of the non-coding RNA target site.

A functional guide RNA (gRNA) is created that is complementary to a target site in the 3'-UTR of a GA20 oxidase_5 gene. A transfer DNA (T-DNA) vector suitable for use in *Agrobacterium* transformation is constructed. The T-DNA construct comprises a promoter that is operable in a plant cell operably linked to polynucleotides encoding a) an RNA guided/nuclease; b) CP4-EPSPS marker gene; c) the gRNA described above; and d) a donor molecule, between a left border (LB) sequence and a right border (RB) sequence. The donor molecule comprises a 21 nucleotide sequence homologous to miR166, as well as a first and a second homologous region that are homologous to the 3'-UTR of the GA20 oxidase_5 gene on either side of the target site.

Immature corn embryos are co-cultured with *Agrobacterium* containing the T-DNA vector for three days. Upon expression of the polynucleotides, the gRNA guides the nuclease to the target site in the GA20 oxidase_5 3'-UTR, where the nuclease creates a double-stranded break. Homologous recombination repair mechanisms then insert the donor molecule into the target site, thereby incorporating a miR166 target site into the 3'-UTR of the GA20 oxidase_5 gene.

Suitable methods known in the art (*e.g*., PCR, DNA hybridization (Southern) blots, sequencing) are used to identify transformation events comprising the insertion of the miR166 target site into the 3'-UTR of the GA20 oxidase_5 gene. Transformed embryos comprising the insertion of the miR166 target site into the 3'-UTR of the GA20 oxidase_5 gene are selected and used to regenerate modified plants using techniques standard in the field.

Without being bound to any scientific theory, the presence of a miRNA binding site in GA20 oxidase_5 creates a population of secondary siRNA transcripts capable of suppressing GA20 oxidase_5 RNA transcription in a dominant negative manner.

RNA is extracted from modified corn plants identified as comprising a miR166 target site insertion in the 3'-UTR of a GA20 oxidase_5 gene. RNA is also extracted from control corn plants that lack an edited GA20 oxidase_5 gene. Suitable methods known in the art (e.g., quantitative reverse-transcriptase PCR, reverse-transcriptase PCR, RNA sequencing) are used to confirm that down-regulation of GA20 oxidase_5 occurs in modified corn plants. Additionally, due to the sequence similarities between GA20 oxidase_5 and GA20 oxidase_3, the miRNA target site in the GA20 oxidase_5 gene is also capable of causing down-regulation of GA20 oxidase_3 RNA transcripts.

### Example 11. Generating dominant negative alleles by creating truncated proteins

Targeted editing of genes that result in truncated proteins (*e.g.*, non-sense mutations) can create dominant negative alleles. In one embodiment, the targeted gene encodes a protein that has protein: protein interaction domains. *See* Figure 6. Examples of dominant truncated proteins are known in various plant species. For example, dominant mutant phenotypes caused by truncated proteins are known for *FAZ1* in rice and *AGAMOUS* and *SOC1* in *Arabidopsis thaliana.* The peptide CLAVATA3 (CLV3) is processed to a signaling peptide (CLE), which is bound by the receptor-like kinases CLV1 and CORYNE (CRN) and the receptor-like protein CLV2 to regulate the expression of *WUSCHEL* (*WUS*) in meristems. Corn plants comprising mutant alleles of *CLV2* often exhibit ears with increased number of kernel rows. Without being limited by a particular theory, when *CLV2* is mutated, *WUS* expression can increase, which leads to an increased meristem size, which in turn gives rise to an increased number of kernel rows on the ear. CLV2 comprises an extracellular domain and a transmembrane domain, which forms a complex with CRN. A truncated CLV2 protein could function in a dominant manner to increase meristem size in corn.

A gRNA is designed to introduce a stop codon in the extracellular domain of CLV2 in com. A transfer DNA (T-DNA) vector suitable for use in *Agrobacterium* transformation is used to introduce the gRNA to a corn cell. The T-DNA construct comprises a promoter that is operable in a plant cell operably linked to polynucleotides encoding a) a RNA guidednuclease; b) CP4-EPSPS marker gene; and c) the gRNA described above, between a left border (LB) sequence and a right border (RB) sequence.

Immature corn embryos are co-cultured with *Agrobacterium* containing the T-DNA vectors for three days. The polynucleotides between the LB and RB sequences are integrated into the nuclear genome of the immature corn embryos. Upon expression of the integrated polynucleotides, the gRNA guides the nuclease to the target site and creates a double-stranded break at the target site. Without being bound by any theory, the non-homologous end-joining repair mechanisms native to the cell frequently repair such breaks imperfectly, which can lead to the insertion or deletion of one or more nucleotides. These insertions or deletions to an exon can produce premature stop codons (which would generate a truncated protein), or non-sense mutations. Such a mutation can generate a dominant negative allele of *CLV2.*

Suitable methods known in the art (*e.g.,* PCR, DNA hybridization (Southern) blots, sequencing) are used to identify transformation events comprising a targeted insertion or deletion in the *CLV2* gene. Transformed embryos comprising an insertion or deletion capable of causing a truncated protein at the target site are selected and used to regenerate modified plants using techniques standard in the field.

Protein is extracted from the modified corn plants identified as comprising the identified insertion/deletion. Suitable methods known in the art (*e.g.*, Western blot: HPLC: LC/MS: ELISA; immunoprecipitation) are used to confirm that a non-sense mutation has been introduced to the *CLV2* gene. Additional phenotypic screening of ear kernel rows is done to an increase in meristem size. Light microscopy of sectioned meristems from modified and control plants is also done to quantify the increase in meristem size.

### Example 12. Preventing cleavage of a target gene using an engineered PPR protein

Members of the pentatricopeptide repeat (PPR) gene family are common in plant genomes. Many PPR proteins are capable of binding RNA molecules in a sequence-specific manner. PPR proteins comprise 2-30 PPR motifs, each of which aligns to a single nucleotide in an RNA molecule. Within the PPR motifs, the amino acids present at two or three specific positions confer nucleotide specificity. For example, without being limiting, a PPR motif binds an adenine nucleotide when a threonine is in position 6 and an asparagine is at the 1' position: a PPR motif binds a guanine nucleotide when a threonine is in position 6 and an aspartic acid is at the 1' position; a PPR motif binds a uracil (or thymine) nucleotide when an asparagine is at position 6 and an aspartic acid is at the 1' position; and a PPR motif binds a cytosine nucleotide when an asparagine is at position 6 and an asparagine or a serine is at the 1' position.

Without being limiting, an engineered PPR protein can be generated by at least two building strategies. In the first strategy, a PPR protein is constructed by treating each PPR motif as a separate block, such that a PPR protein is constructed by putting multiple desired motifs in order. The resulting engineered PPR protein is then capable of binding a target RNA molecule. However, such a strategy may not always work because each PPR motif comprises an internal scaffolding between the 1' and 6 positions, and that intra-motif scaffold is not shared across different PPR proteins. The second strategy makes use of the preexisting intra-motif scaffolding. In the second strategy, site-directed mutagenesis of the 1' and 6 positions is used to edit an existing PPR protein such that it will be specific to a new target RNA molecule.

An engineered PPR protein comprising PPR motifs is engineered such that the PPR protein can specifically bind to nucleotides of the miRNA target site of a suitable gene and the PPR protein is targeted to the cytoplasm. A nucleic acid sequence encoding the engineered PPR protein is inserted into a transfer DNA (T-DNA) vector suitable for use in *Agrobacterium* transformation. The nucleic acid sequence encoding the engineered PPR protein is operably linked to the promoter of the target gene or a constitutive promoter to ensure overlapping expression of the engineered PPR protein and the target gene mRNA. The nucleic acid molecule encoding the engineered PPR protein and operably linked promoter is positioned between a left border (LB) sequence and a right border (RB) sequence within the T-DNA construct.

Immature corn embryos are co-cultured with *Agrobacterium* containing the T-DNA vector for three days. The polynucleotides between the LB and RB sequences are integrated into the nuclear genome of the immature corn embryos. Upon expression of the integrated polynucleotides, the engineered PPR protein is expressed and binds to complementary target mRNAs.

Suitable methods known in the art (*e.g.,* PCR, DNA hybridization (Southern) blots, sequencing) are used to identify transformation events comprising the insertion of the engineered PPR protein encoding nucleic acid molecule. Transformed embryos comprising the insertion of the engineered PPR protein encoding nucleic acid molecule are selected and used to regenerate modified plants using techniques standard in the field.

Without being bound to any scientific theory, the engineered PPR protein binds to the target gene mRNA and prevents the microRNA from suppressing the target gene.

RNA and protein is extracted from modified corn plants identified as comprising the engineered PPR protein. RNA and protein is also extracted from control corn plants that lack the engineered PPR protein. Suitable methods known in the art (*e.g.*, quantitative reverse-transcriptase PCR, reverse-transcriptase PCR, RNA sequencing, Western blot, HPLC, LC/MS, ELISA, immunoprecipitation) are used to confirm that expression of THE TARGET GENE is increased in modified corn plants as compared to unmodified control corn plants.

### Example 13. Cleaving a target gene transcript using an engineered PPR protein coupled to a nuclease

An engineered PPR protein comprising an NYN nuclease domain and PPR motifs is engineered as described in Example 12 such that the PPR protein can specifically bind to certain nucleotides of a target gene and the PPR protein is targeted to the nucleus. A nucleic acid sequence encoding the engineered PPR protein is inserted into a transfer DNA (T-DNA) vector suitable for use in *Agrobacterium* transformation. The nucleic acid sequence encoding the engineered PPR protein is operably linked to the promoter of the target gene or a constitutive promoter to ensure overlapping expression of the engineered PPR protein and the target gene mRNA. The nucleic acid molecule encoding the engineered PPR protein and operably linked promoter is positioned between a left border (LB) sequence and a right border (RB) sequence within the T-DNA construct.

Immature corn embryos are co-cultured with *Agrobacterium* containing the T-DNA vector for three days. The polynucleotides between the LB and RB sequences are integrated into the nuclear genome of the immature corn embryos. Upon expression of the integrated polynucleotides, the engineered PPR protein is expressed and binds to complementary target mRNAs.

Suitable methods known in the art (*e.g.,* PCR, DNA hybridization (Southern) blots, sequencing) are used to identify transformation events comprising the insertion of the engineered PPR protein encoding nucleic acid molecule. Transformed embryos comprising the insertion of the engineered PPR protein encoding nucleic acid molecule are selected and used to regenerate modified plants using techniques standard in the field.

Without being bound to any scientific theory, the engineered PPR protein binds to the target gene mRNA, and the NYN nuclease domain cleaves the target gene mRNA. Therefore, expression of the target gene decreases.

RNA and protein are extracted from modified corn plants identified as comprising the engineered PPR protein. RNA and protein are also extracted from control corn plants that lack the engineered PPR protein. Suitable methods known in the art (*e.g.*, quantitative reverse-transcriptase PCR, reverse-transcriptase PCR, RNA sequencing, Western blot, HPLC, LC/MS, ELISA, immunoprecipitation) are used to confirm that expression of the target gene is decreased in modified corn plants as compared to unmodified control corn plants.

### Example 14. Blocking translation of a target gene transcript using an engineered PPR protein

An engineered PPR protein comprising PPR motifs is engineered such that the PPR protein can specifically bind to specific nucleotides of an mRNA encoded by the target gene[GENE X], and the PPR protein is targeted to the cytoplasm. A nucleic acid sequence encoding the engineered PPR protein is inserted into a transfer DNA (T-DNA) vector suitable for use in *Agrobacterium* transformation. The nucleic acid sequence encoding the engineered PPR protein is operably linked to the promoter of the target gene or a constitutive promoter to ensure overlapping expression of the engineered PPR protein and the target gene mRNA. The nucleic acid molecule encoding the engineered PPR protein and operably linked promoter is positioned between a left border (LB) sequence and a right border (RB) sequence within the T-DNA construct.

Immature corn embryos are co-cultured with *Agrobacterium* containing the T-DNA vector for three days. The polynucleotides between the LB and RB sequences are integrated into the nuclear genome of the immature corn embryos. Upon expression of the integrated polynucleotides, the engineered PPR protein is expressed and binds to complementary target mRNAs.

Suitable methods known in the art (*e.g*., PCR, DNA hybridization (Southern) blots, sequencing) are used to identify transformation events comprising the insertion of the engineered PPR protein encoding nucleic acid molecule. Transformed embryos comprising the insertion of the engineered PPR protein encoding nucleic acid molecule are selected and used to regenerate modified plants using techniques standard in the field.

Without being bound to any scientific theory, the engineered PPR protein binds to the target gene mRNA and prevents translation of the mRNA into protein. Therefore, protein translation of the target gene decreases.

Protein is extracted from modified corn plants identified as comprising the engineered PPR protein. Protein is also extracted from control corn plants that lack the engineered PPR protein. Suitable methods known in the art (*e.g*., Western blot, HPLC, LC/MS, ELISA, immunoprecipitation) are used to confirm that protein translation of the target gene is decreased in modified corn plants.

### Example 15. Design of a tether guide oligo (tgOligo).

A Cas9/sgRNA complex binds to a dsDNA molecule comprising target and non-target strands (Figure 9). Cas9-PAM interaction occurs on the non-target strand: gRNA-DNA annealing occurs on the target strand. RuvC (His840) and HNH (Asp10) nuclease domains cut the non-target and target strands, respectively (triangles). The blunt ends at the Cas9 cut site are held in place by Cas9 at the 5' end of the non-target strand (PAM location), and at both cut ends (3' and 5') of the target strand. The 3' cut end of the non-target strand is free and 'flaps' around. The 3' free 'flap' end of the non-target strand can be up to 35 nucleotides which can be sufficient for specific complementarity binding. A tgOligo (e.g., a ssDNA molecule commentary to the 3' free 'flap' end) is designed and can serve as a template for integration of desired nucleotide modifications. A tgOligo can be DNA, RNA, or a mix of nucleotides depending on the need and design of the edits. In the case of nucleases (e.g., Cpf1) that provide overhangs from a double-stranded break (DSB) cut, the overhangs can act in place of, or in conjunction with, tgOligos.

### Example 16. Engineering of Cas9-like nuclease.

Nucleases, such as Cas9, can be repurposed for structural and functional genomics in plants. Various dimerization domains can be conjugated to Cas9 to achieve dimerization (Figure 10). For example, inducible dimerization domains from Clonetech's homodimerization or heterodimerization iDimerize system can be used to achieve Cas9 dimerization.. Additional dimerization systems can also be used such as those described in Andersen et al., Scientific Reports 6, Article number: 27766 (2016); and Miyamoto, et al., Nature Chemical Biology 8(5):465-70(2012).

Nucleases, such as Cas9, can also be engineered to form a catalytically deactivated from, such catalytically deactivated Cas9 (dCas9). dCas9 binds to DNA at a target site specified by a gRNA and creates a loop structure accessible for template-based editing (Figure 1). dCas9 can be further modified to form a fusion with a ssDNA binding domain from Affymetrix or NEB for further facilitating template-based editing (Figure 1). The editing efficiency with this modified dCas9-ssDNA binding scheme is expected to be higher compared to a dCas9-alone approach, because a ssDNA template is bound to dCas9 complex and would be brought into proximity of the gRNA target.

### Example 17. Introduction of multiple tgOligo and gRNA molecules.

Multiple approaches can be used to incorporate tgOligos with editing components (*e.g*., nuclease, gRNA). Essentially, tgOligos can be incorporated in any manner available to deliver nucleases and gRNAs (transfection, transformation, etc.). The optimal approach depends on the editing component delivery system and the target organism to be edited. For example, in mammalian systems where RNPs (ribonucleoproteins - complexes of nuclease and gRNA) can be transfected across the cell membrane, tgOligos can be simultaneously transfected. Alternatively, a single transcription unit (STU) can be used to incorporate the nuclease (Cas9) and gRNAs in the same transgene construct. Similarly, tgOligos can be incorporated in a similar design (Figure 12). Multiple constructs could also be used, such as one for the nuclease, one for the gRNAs, and one for tgOligos - or any combination thereof from inclusive in constructs to combining constructs and transfection delivery. For tgOligos included in constructs (such as Figure 12), these would be RNA-based tgOligos. To utilize DNA-based or mixed nucleotide (DNA+RNA) tgOligos, a transfection or other delivery mechanism would likely be needed. Also if any tgOligo designs result in the tgOligo containing the same gRNA+PAM recognition site as the original gRNA target site, the tgOligo sequence can be modified to eliminate the PAM.

### Example 18. Genome editing based on a two-gRNA approach.

Two nuclease/gRNA complexes flanking a target genomic region are designed for achieving INDELs or complete inversion of the flanked genomic region. (Figure 1). With two nuclease/gRNA complexes, the flanked genomic region is most often deleted and NHEJ repair combining the two cut sites back together. There is also occurrence of INDEL (insertion/deletion) mutations at either nuclease+gRNA flanking site. It is also possible to recover with lower frequency complete inversions of the flanked genomic region.

### Example 19. Enhancement of the two-gRNA approach.

The two-gRNA approach from Example 18 is modified to improve genome editing efficiency. Using dimerization domains (see Figure 10), tgOligos (see Figure 9), or combination thereof can enhance recovery of complete knockout (deletion) of the genomic region flanked by the two gRNA target sites (Figure 13). Panel 1 of Figure 13 shows a dimerization-enhanced knock out (KO) event. Panel 2 of Figure 13 shows a tgOligo-enhanced KO event. Panel 3 of Figure 13 shows an enhanced KO event via a combination of dimerization and tgOligos. Panel 4 of Figure 13 shows a tgOligo-enhanced inversion event. Without being bound to any theory, tgOligos can facilitate recovery of inversion events by using complementarity of the tether portions to the opposite end of the flanked segment. The tgOligos can vary in length for the complementation to the 3' flap of the non-target strand as well as the template tether extension beyond the flap complementation.

Paired dimerization domains coupled with nuclease (eg:Cas9) or dead nuclease (eg:dCas9) (either alone or in conjunction with tgOligos) can also be used to facilitate inversion of flanked sequence target. Panel 5 of Figure 13 shows a dimerization-enhanced inversion event. Panel 6 of Figure 13 shows an inversion event assisted by a combination of nuclease(eg:Cas9) dimerization/deactivation and tgOligos.

### Example 20. Genome editing of corn BR2 gene by a tgOligo-assisted genomic inversion approach.

A tgOligo-assisted inversion approach (as illustrated in Panel 4 of Figure 13) is used to edit the corn BR2 gene to generate a dominant knockout (KO) mutant allele. The rationale for a genome inversion-based dominant KO mutation approach is depicted in Figure 14. In essence, two gRNAs are used. A first gRNA (shown on the left) targets the end of the first exon of BR2; a second gRNA (shown on the right) recognizes the start codon region of the adjacent GRMZM2G491632 gene. Inversion of the genomic segment flanked by these two gRNAs can lead to a BR2 antisense partial transcript. This BR2 antisense transcript is produced via the GRMZM2G491632 promoter activity. Adjusting the relative position of the two gRNAs can achieve a BR2 antisense complete transcript (e.g., moving the first gRNA on the left to target the start codon region of the BR2 gene) or a BR2 antisense transcript under the control of the native BR2 promoter (e.g., moving the second gRNA on the right to target the stop codon region of the BR2 gene).

Reference sequences are listed in SEQ ID NO: 1 for BR2 (NCBI accession AY366085) and SEQ ID NO: 2 for GRMZM2G491632 (from MaizeGDB). GRMZM2G491632 is a gene annotated immediately adjacent to BR2; and these two genes are in reverse orientation of each other. SEQ ID NO: 3 is the gRNA to the sense strand at the proximal end of BR2. SEQ ID NO: 4 is the gRNA to the antisense strand at the proximal end of GRMZM2G491632.

A first RNA tgOligo corresponding to the BR2 gRNA (SEQ ID NO: 3) is designed to complement the sense strand flank gRNA target site, generally about 20 nt long. Optionally, a 20 nt segment upstream of the target site is added. An example of a BR2 RNA tgOligo comprises a DNA-complementary section as set forth in SEQ ID NO: 5 (serving as a DSB 3' flap complement region), which is complementary to SEQ ID NO: 3 with 10 nt included from upstream. Next, a sequence having at least 20 nt starting with the first base of the PAM of the antisense strand gRNA (SEQ ID NO: 4) is selected to give rise to a 50 nt sequence including the PAM (SEQ ID NO: 6, serving as a tether region). Subsequently, the 3' flap complement (SEQ ID NO: 5) is reversed and attached to the end of the tether (SEQ ID NO: 6) to form a complete tgOligo which complements both the sense gRNA and template from antisense gRNA segment for inversion (SEQ ID NO: 7).

A second RNA tgOligo corresponding to the GRMZM2G491632 gRNA (SEQ ID NO: 4) is designed as follows: a) from the reference sequence (SEQ ID NO: 2) reverse complement the antisense strand flank gRNA target site; b) select at least 20 nt starting with the first base of the PAM of the sense strand gRNA (SEQ ID NO: 3) and reverse complement. This example is 50 nt including the PAM (SEQ ID NO: 9); c) attach the 3' flap complement (SEQ ID NO: 8) to the end of the tether (SEQ ID NO: 9) to complete the tgOligo design complementing the sense gRNA and template from antisense gRNA segment for inversion (SEQ ID NO: 10).

A combination of two gRNAs and the first and second tgOligos are used to edit the corn BR2 locus to achieve a genomic inversion. The resulting inversion of BR2 and GRMZM2G491632 is expected to form a sequence with high similarity (95%+) to SEQ ID NO: 11.

### Example 21. Enhancement of template-based genome editing or site directed integration (SDI).

Nuclease dimerization or deactivation, tgOligos, or their combination can be used to enhance targeting of template-based editing or site directed integration (SDI) at a single location or multiple locations. Various representative schemes are depicted in Figure 15. In these schemes, a template molecule (regardless of its homology to a target site in the genome) is brought into proximity of the target site by nuclease (Cas9) complexes with dimerization domains (Panels 1 to 3 of Figure 15), tgOligos (not illustrated alone), or a combination thereof (Panel 4 of Figure 15). dCas9 can be used on the template (Panel 1 of Figure 15) or active Cas9 can help facilitate integration of the template (Panels 2 to 4 of Figure 15).

### Example 22. Genome editing of corn Y1 gene to generate dominant alleles.

The enhanced genome editing schemes depicted in Figure 15 are tested in creating a dominant allele for a traditionally recessive trait. The following is a summary of the molecular designs for the corn Y1 gene. (SEQ ID NO: 12).

For Y1, the first exon from SEQ ID NO: 12 is shown in SEQ ID NO: 13. To make an antisense template, SEQ ID NO: 13 is reverse complemented into SEQ ID NO: 14 which is used as a template sequence for editing (corresponding to the template sequences between the dCas9 complexes and Cas9 complexes depicted in Figure 15's Panels 1 and 2). The sense strand gRNA for Y1 (SEQ ID NO: 12) is in the 5-UTR (SEQ ID NO: 15). The antisense strand gRNA for Y1 (SEQ ID NO: 12) is in the 3-UTR (SEQ ID NO: 16). The region between these two gRNAs corresponds to the to-be-replaced genomic sequences between the Cas9 complexes depicted in Figure 15's Panels 1, 2, and 4.

To provide a template for integration (as depicted in Figure 15's Panels 1 and 2), SEQ ID NO: 14 is added between the gRNA target sites targeted by (SEQ ID NOs: 15 and 16). The resulting SEQ ID NO: 17 comprises the sense strand gRNA site with 10 nt upstream, SEQ ID NO: 14, and the antisense strand gRNA site with 10 nt downstream.

This template molecule (SEQ ID NO: 17) is then paired with gRNAs (SEQ ID NOs: 15 and 16) and used in editing following the schemes depicted in Figure 15's Panels 1 and 2. To utilize tgOligos to further help facilitate integration of the template (SEQ ID NO: 17) (see Panel 4 of Figure 15), two tgOligos are incorporated (SEQ ID NOs: 18 and 19).

### Example 23. Genome editing of corn BR2 gene to generate dominant alleles.

The enhanced genome editing schemes depicted in Figure 15 are also tested in creating a dominant allele for corn BR2 gene (SEQ ID NO: 1). The following is a summary of the molecular designs for BR2.

New gRNAs are designed to be able to replace the BR2 gene with an antisense template similar to the Y1 concept described in Example 20. A sense strand gRNA is provided in SEQ ID NO: 20 and an antisense strand gRNA is provided in SEQ ID NO: 21. The region between these two gRNAs corresponds to the to-be-replaced genomic sequences between the Cas9 complexes depicted in Figure 15's Panels 1, 2, and 4.

The first 250 nt coding sequence of the BR2 gene (SEQ ID NO: 22) is made into an antisense template. SEQ ID NO: 22 is reverse-complemented to create a BR2 Exon 1 antisense sequence template (SEQ ID NO: 23).

To provide a template for integration (as depicted in Figure 15's Panels 1 and 2), SEQ ID NO: 23 is added between the gRNA target sites targeted by gRNAs SEQ ID NOs: 20 and 21. SEQ ID NO: 24 comprises the sense strand gRNA site with 3 nt upstream, SEQ ID NO: 23, and the antisense strand gRNA site with 10 nt downstream.

This template molecule (SEQ ID NO: 23) is then paired with gRNAs (SEQ ID NOs: 20 and 21) and used in editing following the schemes depicted in Figure 15's Panels 1 and 2. To utilize tgOligos to further help facilitate integration of the template (SEQ ID NO: 24) (see Panel 4 of Figure 15), two tgOligos are incorporated (SEQ ID NOs: 25 and 26).

The examples shown above for editing Y1 and BR2 corn genes can be followed to design neighboring template edits or integrations as illustrated in Panel 3 of Figure 15. It should also be obvious that while the examples provided for Y1 and BR2 use antisense templates of the first exon of these genes, the template integrations could be more subtle, such as changing nucleotides to alter amino acids in the native proteins, or more complex such as integrating a non-native sequence or gene. This is further illustrated in Figure 16.

A potential advantage to creating antisense templates in the native genomic region of Y1 and BR2 as described above is that the native promoter and gene expression elements are used to regulate the antisense transcript to appropriately achieve gene silencing of a native allele in a heterozygous organism (e.g., in a dominant manner).

### Example 24. Genome editing-based dominant mutant allele via stacking of an inverted Y1 gene head-to-tail.

The tgOligo and nuclease dimerization concepts described in the above examples can also be used to stack an inverted gene head-to-tail next to the native copy. This would result in an antisense transcript to silence the gene expression, and therefore create a dominant mutant allele for a normally recessive trait (*e.g.,* the corn Y1 gene, the corn BR2 gene, the corn GA20 oxidase gene (see Figure 17).

### Example 25. Genome editing to trigger microprotein interference and dominant suppression.

Microproteins are short, single-domain proteins that possess the ability to interfere with larger multi-domain proteins. *See* Figure 6 and Figure 18. Targets of microproteins are often transcriptional regulators that bind to DNA as active homodimers. Microproteins interfere with their targets by forming non-functional heterodimeric complexes that cannot bind to DNA.

Expression of an *Arabidopsis thaliana* microprotein, ATHB17, in maize exhibits a dominant phenotype (*e.g.,* increased ear weight at silking). *See* Rice et al., 2014, PLoS ONE, 9(4):e94238, which is incorporated in its entirety herein. Without being bound to any theory, an ATHB17 microprotein forms non-functional heterodimers with maize transcriptional repressor proteins. The heterodimers are non-functional thus increase the transcription of maize genes that would be normally be repressed in the absence of the ATHB17 microprotein.

The maize homolog of *ATHB17, Zmhdz18,* is selected as a target for genome editing to create a maize microprotein. *Zmhdz18* is an HD-Zip II member comprising a homeodomain immediately adjacent to a leucine zipper domain, a hypothetical N-terminal repression domain, and a redox sensing motif. Without being bound by a particular theory, a Zmhdz18 microprotein will form non-functional heterodimers with transcriptional repressor proteins to increase expression of typically repressed genes and exhibit a dominant phenotype.

A first and a second functional guide RNA (gRNA) for a CRISPR/ nuclease system are created. The first and second gRNAs are complementary to a first target site and a second target site, respectively, flanking a portion of the *Zmhdz18* gene in the corn genome. The flanked portion encodes the amino terminus of the normal Zmhdz18 protein.

A transfer DNA (T-DNA) vector suitable for use in *Agrobacterium* transformation is constructed. The T-DNA construct comprises comprises several expression cassettes between a left border (LB) sequence and a right border (RB) sequence. The first expression cassette comprises a promoter that is operable in a plant cell operably linked to a polynucleotide encoding an RNA guided nuclease. A second expression cassette comprises a promoter that is operable in a plant cell operably linked to the CP4-EPSPS marker gene. The construct also comprises expression cassettes comprising a promoter operable in a plant cell operably linked to polynucleotides encoding the two gRNAs described above.

Immature corn embryos are co-cultured with *Agrobacterium* containing the T-DNA vector for three days. The polynucleotides between the LB and RB sequences are integrated into the nuclear genome of the immature corn embryos. Upon expression of the integrated polynucleotides, the gRNAs guide the nuclease to the target sites in the *Zmhdz18* gene, where the nuclease creates a double-stranded break at each target site.

In the majority of events, the region between the first and second target sites in the *Zmhdz18* gene is deleted and non-homologous end-joining repair mechanisms joins the flanking regions. Suitable methods known in the art (*e.g*., PCR, DNA hybridization (Southern) blots, sequencing) are used to identify transformation events comprising a deletion between the first and second target sites in the *Zmhdz18* gene. Transformed embryos comprising the deletion are selected and used to regenerate modified plants using techniques standard in the field.

Without being bound to any scientific theory, the deletion creates a dominant *Zmhdz18* allele that encodes a Zmhdz18 microprotein. This microprotein can form homodimers and heterodimers with other proteins to interfere with the transcription of maize genes that results in a dominant phenotype.

RNA and protein are extracted from modified corn plants identified as comprising a deletion in a *Zmhdz18* allele capable of encoding a microprotein. RNA and protein are also extracted from control corn plants that lack a deletion in *Zmhdz18.* Suitable methods known in the art (*e.g*., quantitative reverse-transcriptase PCR, reverse-transcriptase PCR, RNA sequencing) are used to confirm that expression of maize genes in pathways downstream of *Zmhdz18* are altered. Additional suitable methods (*e.g.,* Western blot, HPLC, LC/MS, ELISA, immunoprecipitation) are used to confirm that the deletion in *Zmhdz18* generates a microprotein.

Having described the present disclosure in detail, it will be apparent that modifications, variations, and equivalent aspects are possible without departing from the spirit and scope of the present disclosure as described herein and in the appended claims. Furthermore, it should be appreciated that all examples in the present disclosure are provided as non-limiting examples. All references cited herein are incorporated by reference in their entireties.

### Example 26: Generating a dominant positive allele of MIR1 via targeted genomic deletion to render MIR1 gene under the control of an upstream promoter.

The GRMZM2G150276 gene (SEQ ID NO: 99) encoding the MIR1 protein (SEQ ID NO: 100) is located on corn chromosome 6. MIR1 is predicted to be involved in insect resistance and encodes a cysteine protease that accumulates in the whorls in response to larval feeding in maize genotypes resistant to some lepidopteran pests (*see* Pechan et al., Plant Cell., 2000 (7) :1031-40). The MIR1 gene is adjacent to the gene GRMZM2G150302 and expressed in the same orientation. See Figure 19, Panel A. The GRMZM2G150302 gene is a member of a large, redundant gene family of xylan synthases. The GRMZM2G150302 promoter (SEQ ID NO: 101) is hypothesized to have a broader/expanded expression profile when compared to the native MIR1 gene expression, which is restricted to the whorls.

Two functional guide RNAs (gRNAs) for an RNA guided nuclease system are created to target the genomic DNA region between the MIR1 gene and the GRMZM2G150302 gene. The first gRNA targets an area near the transcriptional start site of the MIR1 gene and the second gRNA targets a region near the transcriptional start site of the GRMZM2G150302 gene. Each of the two target sites are unique within the corn genome. A transfer DNA (T-DNA) vector suitable for use in Agrobacterium transformation is used. The T-DNA construct comprises several expression cassettes between a left border (LB) sequence and a right border (RB) sequence. The first expression cassette comprises a promoter that is operable in a plant cell operably linked to a polynucleotide encoding an RNA guided nuclease. A second expression cassette comprises a promoter that is operable in a plant cell operably linked to the CP4-EPSPS marker gene. The construct also comprises expression cassettes comprising promoters operable in a plant cell operably linked to polynucleotides encoding the two gRNAs described above.

Immature corn embryos are co-cultured with Agrobacterium containing the T-DNA vector for three days. The polynucleotides between the LB and RB sequences are integrated into the nuclear genome of the immature corn embryos. Upon expression of the integrated polynucleotides, the gRNAs guide the nuclease to each of the two target sites in the genomic DNA region between the MIR1 gene and the GRMZM2G150302 gene, where the nuclease creates a double-stranded break at each target site.

In the majority of events, the region between the target sites is deleted and non-homologous end-joining repair mechanisms joins the flanking regions. Suitable methods known in the art *(e.g.,* PCR, DNA hybridization (Southern) blots, sequencing) are used to identify transformation events comprising a complete deletion. Transformed embryos comprising a targeted deletion between the MIR1 gene and GRMZM2G150302 gene are selected and used to regenerate modified plants using techniques standard in the field.

Without being bound by a particular theory, by removing the genomic DNA between the MIR1 gene and the GRMZM2G150302 gene, the native GRMZM2G150302 promoter (SEQ ID NO: 101) can drive the transcription of the MIR1 gene (SEQ ID NO: 99) thereby broadening/expanding the expression profile of the MIR1 gene. See Fig 19, panel B.

RNA is extracted from various tissue types (*e.g*., roots, stem, leaf, inflorescence) from modified corn plants identified as comprising a targeted deletion between the MIR1 gene and the GRMZM2G150302 gene. RNA is also extracted from control corn plants that lack the deletion. Suitable methods known in the art (*e.g*., quantitative reverse-transcriptase PCR, reverse-transcriptase PCR, RNA sequencing) are used to confirm that the MIR1 gene is more broadly expressed and/or more strongly expressed in corn modified plants comprising a deletion of the targeted region.

### Example 27. Constructs for creation of dominant negative deletion mutant alleles

The endogenous Zm.GA20ox5 gene is separated from an endogenous Zm.SAMT gene in the maize genome by an intergenic region of about 550 bp, or by 1170 bp if measured between stop codons, with the Zm.SAMT gene positioned downstream and oriented in the opposite orientation relative to the Zm.GA20ox5 gene. The sequence of the genomic locus or region encompassing the Zm.GA20ox5 and Zm.SAMT genes is provided in SEQ ID NOs. 35 and 36. SEQ ID NO. 35 represents a sequence of the GA20ox5-SAMT genomic locus corresponding to the sense strand of the Zm.GA20ox5 gene and encompassing both Zm.GA20ox5 and Zm.SAMT genes (the "GA20ox5_SAMT genomic sequence" in Table 2). SEQ ID NO. 36 represents a sequence of the GA20ox5-SAMT genomic locus corresponding to the sense strand of the Zm.SAMT gene (the antisense strand of the Zm.GA20ox5 gene) and encompassing both Zm.GA20ox5 and Zm.SAMT genes (the "SAMT_GA20ox5 genomic sequence" in Table 2). The elements or regions of the genomic sequences encompassing both Zm.GA20ox5 and Zm.SAMT genes are annotated in Table 2 below by reference to the nucleotide coordinates of those elements or regions in each of SEQ ID NOs. 35 and 36. As proposed herein, if a genomic region between the neighboring Zm.GA20ox5 and Zm.SAMT genes (including possibly all or part of those genes) were deleted, then the endogenous Zm.SAMT gene promoter may drive expression of an antisense RNA transcript through all or part of the Zm.GA20ox5 gene that can hybridize to a separate RNA transcript expressed from one or both of the copies or alleles of the Zm.GA20ox5 and/or Zm.GA20ox3 gene(s). Since the Zm.GA20ox3 and Zm.GA20ox5 genes share a high level of nucleotide sequence similarity in their respective exon coding regions, the antisense RNA transcript expressed from the oppositely oriented Zm.SAMT gene promoter may hybridize to transcripts of both GA20 oxidase genes and cause the suppression or silencing of one or both of the Zm.GA20ox3 and/or Zm.GA20ox5 gene(s). Thus, a mutant allele having a deletion between the Zm.GA20ox5 and Zm.SAMT genes may behave as a dominant or semi-dominant negative mutation or allele by causing suppression or silencing of one or both (wild-type and/or mutant) copies or alleles of the endogenous Zm.GA20ox5 gene, in addition to possible further suppression or silencing of one or both copies or alleles of the endogenous Zm.GA20ox3 gene.

**Table 2. Annotation of genomic sequence elements of Zm. GA20ox5 and Zm. SAMT genomic region**

| **Gene Name or Region** | **Element / Feature** | **Location in the GA20ox5_SAMT genomic sequence (SEQ ID NO: 35)** | **Location in the SAMT_GA20ox5 genomic sequence (SEQ ID NO: 36)** |
|---|---|---|---|
| GA20ox5 | Promoter and 5' UTR | 1..398 | 8670..9067 |
| GA20ox5 | Exon 1 | 399..1189 | 7879..8669 |
| GA20ox5 | Intron 1 | 1190..1304 | 7764..7878 |
| GA20ox5 | Exon 2 | 1305..1629 | 7439..7763 |
| GA20ox5 | Intron 2 | 1630..2595 | 6473..7438 |
| GA20ox5 | Exon 3 | 2596..2871 | 6197..6472 |
| GA20ox5 | 3' UTR | 2872..3180 | 5888..6196 |
| ***Intergenic Region*** | | **3181..3736** | **5332..5887** |
| SAMT | 3' UTR | 3737..4141 | 4927..5331 |
| SAMT | Exon 8 | 4042..4258 | 4810..5026 |
| SAMT | Intron 8 | 4259..4512 | 4556..4809 |
| SAMT | Exon 7 | 4513..4707 | 4361..4555 |
| SAMT | Intron 7 | 4708..4989 | 4079..4360 |
| SAMT | Exon 6 | 4990..5262 | 3806..4078 |
| SAMT | Intron 6 | 5263..5348 | 3720..3805 |
| SAMT | Exon 5 | 5349..5523 | 3545..3719 |
| SAMT | Intron 5 | 5524..6037 | 3031..3544 |
| SAMT | Exon 4 | 6038..6148 | 2920..3030 |
| SAMT | Intron 4 | 6129..6239 | 2829..2939 |
| SAMT | Exon 3 | 6240..6510 | 2558..2828 |
| SAMT | Intron 3 | 6511..6894 | 2174..2557 |
| SAMT | Exon 2 | 6895..7044 | 2024..2173 |
| SAMT | Intron 2 | 7045..7139 | 1929..2023 |
| SAMT | Exon 1 | 7140..8126 | 942..1928 |
| SAMT | 5' UTR 2 | 8127..8268 | 800..941 |
| SAMT | Intron 1 | 8269..8771 | 297..799 |
| SAMT | 5' UTR 1 | 8772..8942 | 126..296 |
| SAMT | Promoter | 8943..9067 | 1..125 |

FIG. 20 illustrates the concept for creating an antisense RNA molecule that targets the Zm.GA20ox5 gene by deleting a genomic region between the Zm.GA20ox5 and its neighboring Zm.SAMT gene oriented in the opposite direction, through genome editing. The deletion can be generated using two or more guide RNAs that create double stranded breaks in the genome at the two ends of the intended deletion. The antisense RNA molecule generated from the oppositely oriented Zm.SAMT gene promoter can then hybridize to a sense Zm.GA20ox5 RNA transcript and trigger suppression or silencing of one or both copies or alleles (wild-type or mutant) of the endogenous Zm.GA20ox5 gene. FIG. 20 provides an embodiment where small RNAs may be generated through RNA interference. However, it is envisioned that suppression or silencing of the Zm.GA20ox5 gene may occur through other mechanisms as provided herein, alternatively or in addition to any RNAi or PTGS forms of suppression. Given that the Zm.GA20ox3 and Zm.GA20ox5 genes share a high level of nucleotide sequence similarity in their respective coding regions, the antisense RNA transcript may also hybridize to RNA transcripts of the Zm.GA20ox3 gene and cause the suppression or silencing of one or both of the Zm.GA20ox3 and/or Zm.GA20ox5 gene(s). Thus, a deletion between the Zm.GA20ox5 and Zm.SAMT genes may act as a dominant or semi-dominant negative mutation or allele for one or both of the Zm.GA20ox3 and/or Zm.GA20ox5 gene(s).

In the illustrative example provided in FIG. 20, a pair of guide RNAs are used including one guide RNA having a targeting or spacer sequence designed to target a site in the Zm.GA20ox5 gene, and another guide RNA having a targeting or spacer sequence designed to target a site in the Zm.SAMT gene. The size of the deletion and the location of the two breakpoints at the ends of the deletions may be determined by selecting which guide RNAs are used with a RNA-guided endonuclease to create the genome breaks. By creating a double strand break at both target sites, a deletion of the intervening region can be generated that will condense the genomic locus and bring the oppositely oriented Zm.SAMT gene promoter into closer proximity to the GA20ox5 gene, such that the Zm.SAMT gene promoter can drive the expression of an antisense RNA transcript that reads through at least a portion of the GA20ox5 gene. Even though a 3' portion of the GA20ox5 gene may be deleted, the remaining 5' portion of the GA20ox5 gene can be sufficient for an antisense RNA transcript or molecule to be generated under the control of the Zm.SAMT gene promoter that causes suppression or silencing of the Zm.GA20ox3 and/or GA20ox5 gene(s). Thus, the presence of a single copy or allele of the deletion mutant may act in a dominant or semi-dominant negative manner to cause a corn plant to have a short stature, lodging resistant phenotype.

Deletions in the Zm.GA20ox5 / Zm.SAMT genomic region were generated using three different plasmid vector constructs for transformation. Each vector construct comprises a functional cassette for the expression of Cpfl (or Cas12a), and further comprises one or two functional cassettes for the expression of guide RNAs, in addition to a selectable marker gene and plasmid maintenance elements. For the Vector-1 and Vector-2 constructs, the Cpfl (or Cas12a) expression cassette comprises a maize ubiquitin promoter (SEQ ID NO: 37) operably linked to a sequence encoding a wild-type Lachnospiraceae bacterium Cpfl RNA-guided endonuclease enzyme (SEQ ID NO: 38) fused to two nuclear localization signals (SEQ ID NOs: 40 and 41). The wild-type Cpfl expression cassette further contains a synthetic sequence (atggcg) which provides a start codon. For the Vector-3 construct, the Cpfl (or Cas12a) expression cassette comprises a maize ubiquitin promoter (SEQ ID NO: 37) operably linked to a sequence encoding a *Lachnospiraceae bacterium* G532R/K595R mutant Cpfl RNA-guided endonuclease enzyme (SEQ ID NO: 39) fused to two nuclear localization signals (SEQ ID NOs: 42 and 43). *See, e.g.,* Gao, L. et al., Nature Biotechnol. 35(8): 789-792 (2017), the entire contents and disclosure of which are incorporated herein by reference.

Table 3 below provides the target site, spacer and targeting/spacer sequence for each guide RNA encoded by the guide RNA cassette(s) in each vector construct. Each guide RNA unit within the guide RNA cassettes comprises a guide RNA scaffold sequence compatible with the LbCpf1 enzyme along with the unique spacer or targeting sequence complementary to its intended target site. For the Vector-2 construct, the guide RNA expression cassette comprises a maize RNA polymerase III (Pol3) promoter (SEQ ID NO: 44) operably linked to a sequence encoding two guide RNAs having targeting/spacer sequences encoded by the SP1b and SP1f DNA sequences in Table 3 below, with one guide RNA (SP1b) targeting a site in the first exon of the Zm.SAMT gene, and the other guide RNA (SP1f) targeting a site in the first intron of the Zm.GA20ox5 gene (see also FIG. 21 (top panel) showing the placement of the two guide RNA target sites for SP1b and SP1f (SAMT_408 and GA20ox5_6531) relative to the genomic region encompassing the endogenous Zm.GA20ox5 and Zm.SAMT genes).

The Vector-1 construct has two guide RNA expression cassettes. One guide RNA expression cassette of the Vector-1 construct comprises a maize Pol3 promoter (SEQ ID NO: 44) operably linked to a sequence encoding two guide RNAs having targeting/spacer sequences encoded by the SP2f1 and SP2f2 DNA sequences in Table 3 below, with one guide RNA (SP2f1) targeting a site in the first exon of the Zm.GA20ox5 gene, and the other guide RNA (SP2f2) targeting a site in the second exon of the Zm.GA20ox5 gene. The other guide RNA expression cassette of the Vector-1 construct comprises a synthetic promoter operably linked to a sequence encoding two guide RNAs having targeting/spacer sequences encoded by the SP2b1 and SP2b2 DNA sequences in Table 3 below, with each guide RNA (SP2b1 and SP2b2) targeting different sites in the first exon of the Zm.SAMT gene. For the Vector-1 construct, see also the middle panel of FIG. 21 showing the placement of the four guide RNA target sites for SP2f1, SP2f1, SP2b1 and SP2b2 (GA20ox5_7090, GA20ox5_1654, SAMT_304 and SAMT_161) relative to the genomic region encompassing the endogenous Zm.GA20ox5 and Zm. SAMT genes.

The Vector-3 construct has two guide RNA expression cassettes. One guide RNA expression cassette of the Vector-3 construct comprises a maize Pol3 promoter (SEQ ID NO: 74) operably linked to a sequence encoding two guide RNAs having targeting/spacer sequences encoded by the SP3f1 and SP3f2 DNA sequences in Table 3 below, with each guide RNA (SP3f1 and SP3f2) targeting different sites in the second intron of the Zm.GA20ox5 gene. The other guide RNA expression cassette of the Vector-1 construct comprises a synthetic promoter operably linked to a sequence encoding two guide RNAs having targeting/spacer sequences encoded by the SP3b1 and SP3b2 DNA sequences in Table 3 below, with one guide RNA (SP3b1) targeting a site in the first exon of the Zm.SAMT gene, and another guide RNA (SP3b2) targeting a site in the 5' UTR of the Zm.SAMT gene. For the Vector-3 construct, see also the lower panel of FIG. 21 showing the placement of the four guide RNA target sites for SP3f1, SP3f1, SP3b1 and SP3b2 (GA20ox5_1695_TYC, GA20ox5_1732_TYC, SAMT_8110_TYC and SAMT_8165_TYC) relative to the genomic region encompassing the endogenous Zm.GA20ox5 and Zm.SAMT genes.

**Table 3. Transgenic constructs and their respective target sites and guide RNA spacers**

| **Vector Construct** | **guide RNA Spacer ID** | **Target Site** | **Spacer Sequence** | **SEQ ID NO** |
|---|---|---|---|---|
| **Vector-2** | SP1b | SAMT_408 | AGGACACCGACAACAATGATGCC | 45 |
| | SP1f | GA20ox5_6531 | GGTCCACTAGGATTCGGGAAATA | 46 |
| | | | | |
| **Vector-1** | SP2f1 | GA20ox5_7090 | GAGCCAATGGGGTAAGTAAGGTA | 47 |
| | SP2f2 | GA20ox5_1654 | GTTACCATGAAGGTGTCGCCGAT | 48 |
| | SP2b1 | SAMT_304 | GTCCAATAAGAAGCCGGTGGTGA | 49 |
| | SP2b2 | SAMT_161 | CACCTCGGCCAAATGCCATCAGT | 50 |
| | | | | |
| **Vector-3** | SP3f2 | GA20ox5_1695_TYC | GTTGAGCTCTCTCTGTGCTGTTA | 51 |
| | SP3f1 | GA20ox5_1732_TYC | CTAGGATTCGGGAAATAACAGCA | 52 |
| | SP3b1 | SAMT_8110_TYC | CCTCGGCCAAATGCCATCAGTGC | 53 |
| | SP3b2 | SAMT_8165_TYC | CGTGGTTTATCTCCACCAACAAC | 54 |

### Example 28. Characterization of Deletion Mutant Alleles of GA20Ox5 gene

An inbred corn plant line was transformed via *Agrobacterium-*mediated transformation with a transformation vector having one of the constructs as described above in Example 27. The transformed plant tissue was grown to mature R0 plants. R0 plants having one or more unique genome edit(s) were selfed to produce R1 plants. To characterize the edits and recover plants with a deletion between the GA20Ox5 and SAMT genes, a PCR-based assay was performed using a pair of PCR primers flanking the intended deletion region. The same pair of primers (SEQ ID NOs: 55 and 56) were used for all three vectors in Table 3. If a deletion is present between the GA20Ox5 and SAMT genes, the PCR assay would result in an amplicon that could be sequenced. However, due to the large size of the intended deletion, the PCR assay would not produce a PCR product in the absence of a larger deletion. For each PCR assay, a 15 µL PCR reaction volume was used containing the Phusion PCR master mix from Thermo Fisher Scientific, 3 µL of genomic DNA template, and two PCR primers. After PCR amplification, a 3 µL PCR mixture was added to 21 µL of Tris-EDTA buffer and then analyzed on a ZAG instrument for the presence or absence of PCR products that indicate a GA20Ox5-SAMT deletion. The PCR products were sequenced to determine the junction sequence generated in each deletion around the GA20ox5-SAMT genomic locus (see Table 4).

R0 plants with a deletion between the GA20ox5 and SAMT genes were selected and selfed to produce R1 plants. The R1 plants were subject to a quantitative PCR assay to determine the zygosity of the GA20ox5-SAMT genomic locus (see Table 5). Each R1 plant was sequenced to determine all of the deletion edits around the GA20Ox5-SAMT genomic locus. Due to multiple gRNAs with a given construct, multiple deletions may occur on the same chromosome of a R0 plant and thus be present in a R1 plant, which may be homozygous or heterozygous for a mutant allele comprising the genomic deletion(s) (see Table 5). In Table 5, "homo" means homozygous for the mutant allele, and "hetero" means heterozygous for the mutant allele.

**Table 4. Individual deletion junction sequences for edits made using the vectors in Table 3.**

| **SEQ ID NO.** | **Deletion Junction Number** | **Junction Sequence (with deletion size shown in the parentheses)** | **Junction Sequence Description** |
|---|---|---|---|
| 57 | 1001 | GCGGCCGTCCATCTTTCCACCTCGGCCAAA- (-8)- GTGCCTGGCGAACATGTACCAGAGCACCAG | 8 nt deletion at SAMT_161 |
| 58 | 1002 | GGCCGTCCATCTTTCCACCTCGGCCAAATG- (-3)- TCAGTGCCTGGCGAACATGTACCAGAGCAC | 3 nt deletion at SAMT_161 |
| 59 | 1003 | GGCCGTCCATCTTTCCACCTCGGCCAAATG- (-6)- GTGCCTGGCGAACATGTACCAGAGCACCAG | 6 nt deletion at SAMT_161 |
| 60 | 1004 | GAGTGGCGCCCCGTCCGGCCCGTCCCGGGC- (-6357)- TTCTTATTGGACGAAATCTCCAGCGGGAAG | 6357 nt deletion between GA20ox5_1654 and SAMT_304 |
| 61 | 1005 | CCGGCCCGTCCCGGGCGCCATGGTCATCAA- (-6518)- GTGCCTGGCGAACATGTACCAGAGCACCAG | 6518 nt deletion between GA20ox5_1654 and SAMT_161 |
| 62 | 1006 | GTCCGGCCCGTCCCGGGCGCCATGGTCATC- (-6342)- GGCTTCTTATTGGACGAAATCTCCAGCGGG | 6342 nt deletion between GA20ox5_1654 and SAMT_304 |
| 63 | 1007 | GTCCGGCCCGTCCCGGGCGCCATGGTCATC- (-6348)- TTATTGGACGAAATCTCCAGCGGGAAGACA | 6348 nt deletion between GA20ox5_1654 and SAMT_304 |
| 64 | 1008 | CGTCCGGCCCGTCCCGGGCGCCATGGTCAT- (-6344)- GCTTCTTATTGGACGAAATCTCCAGCGGGA | 6344 nt deletion between GA20ox5_1654 and SAMT_304 |
| 65 | 1009 | CTGTGTGTATATTCAGTTGAGCTCTCTCTG-(- 6478)- CACGGCTGGACCAACAGCCCCCCCAAAATC | 6478 nt deletion between GA20ox5_1695 and SAMT_8165 |
| 66 | 1010 | CTTGGCCGCTCTTGTCCTGTGTGTATATTC-(- 6160)- GGTGTCCTCAAATTTCTCGGACCCTTCACC | 6160 nt deletion between GA20ox5_6531 and SAMT_408 |
| 67 | 1011 | TGTATATTCAGTTGAGCTCTCTCTGTGCTG-(- 6133)- GTTGTCGGTGTCCTCAAATTTCTCGGACCC | 6133 nt deletion between GA20ox5_6531 and SAMT_408 |
| 68 | 1012 | TATATTCAGTTGAGCTCTCTGTGCTGTT-(- 6130)- TGTTGTCGGTGTCCTCAAATTTCTCGGACC | 6130 nt deletion between GA20ox5_6531 and SAMT_408 |
| 69 | 1013 | ATATTCAGTTGAGCTCTCTCTGTGCTGTTA-(- 6130)- GTTGTCGGTGTCCTCAAATTTCTCGGACCC | 6130 nt deletion between GA20ox5_6531 and SAMT_408 |
| 70 | 1014 | ATTCAGTTGAGCTCTCTCTGTGCTGTTATT-(- 6131)- GTCGGTGTCCTCAAATTTCTCGGACCCTTC | 6131 nt deletion between GA20ox5_6531 and SAMT_408 |
| 71 | 1015 | CTCGGCCAGGATTTCGAGCCAATGGGGTAA- (-6759)- CTTCTTATTGGACGAAATCTCCAGCGGGAA | 6759 nt deletion between GA20ox5_7090 and SAMT_304 |
| 72 | 1016 | CGGCCAGGATTTCGAGCCAATGGGGTAAGT- (-6753)- CCGGCTTCTTATTGGACGAAATCTCCAGCG | 6753 nt deletion between GA20ox5_7090 and SAMT_304 |
| 73 | 1017 | TCGGCCAGGATTTCGAGCCAATGGGGTAAG- (-12)- AAGGAGCGCCGGTTTACATTTACCGCACGT | 12 nt deletion at GA20ox5_7090 |
| 74 | 1018 | TCGGCCAGGATTTCGAGCCAATGGGGTAAG- (-4)- GTAGTAAGAAGGAGCGCCGGTTTACATTTA | 4 nt deletion at GA20ox5_7090 |
| 75 | 1019 | GGACTACTTCGTCGGCACCCTCGGCCAGGA- (-39)- GCCGGTTTACATTTACCGCACGTCGGCGTG | 39 nt deletion at GA20ox5_7090 |

**Table 5. Deletion edits and genotype of R0 ad R1 plants.**

| **RO Edit ID** | **R1 Plant ID** | **Editing Deletion Type** | **R1 zygosity call for deletion mutant** | **Deletion Junction Number(s) (Table 4)** |
|---|---|---|---|---|
| E221089 | P43596818 | 6759 nt deletion between GA20ox5_7090 and SAMT_304; 6 nt deletion at T161 | Homozygous | 1015; 1003 |
| E221089 | P43596820 | 6759 nt deletion between GA20ox5_7090 and SAMT_304; 6 nt deletion at T161 | Homozygous | 1015; 1003 |
| E221089 | P43596823 | 6759 nt deletion between GA20ox5_7090 and SAMT_304; 6 nt deletion at T161 | Homozygous | 1015; 1003 |
| E221089 | P43596801 | 6759 nt deletion between GA20ox5_7090 and SAMT_304; 6 nt deletion at T161 | Homozygous | 1015; 1003 |
| E221089 | P43596831 | 6759 nt deletion between GA20ox5_7090 and SAMT_304; 6 nt deletion at T161 | Homozygous | 1015; 1003 |
| E220938 | P43596469 | 6753 nt deletion between GA20ox5_7090 and SAMT_304; 8 nt deletion at T161 | Homozygous | 1016; 1001 |
| E220938 | P43596438 | 6753 nt deletion between GA20ox5_7090 and SAMT_304; 8 nt deletion at T161 | Homozygous | 1016; 1001 |
| E220938 | P43596489 | 6753 nt deletion between GA20ox5_7090 and SAMT_304; 8 nt deletion at T161 | Homozygous | 1016; 1001 |
| E220242 | P95046375 | 6344 nt deletion between GA20ox5_1654 and SAMT_304; 12 nt deletion at GA20ox5_7090; 3 nt deletion at SAMT_161 | Homozygous | 1008; 1017; 1002 |
| E220242 | P95046377 | 6344 nt deletion between GA20ox5_1654 and SAMT_304; 12 nt deletion at GA20ox5_7090; 3 nt deletion at SAMT_161 | Homozygous | 1008; 1017; 1002 |
| E220242 | P95046392 | 6344 nt deletion between GA20ox5_1654 and SAMT_304; 12 nt deletion at GA20ox5_7090; 3 nt deletion at SAMT_161 | Homozygous | 1008; 1017; 1002 |
| E220242 | P95046378 | 6344 nt deletion between GA20ox5_1654 and SAMT_304; 12 nt deletion at GA20ox5_7090; 3 nt deletion at SAMT_161 | Homozygous | 1008; 1017; 1002 |
| E220242 | P95046370 | 6344 nt deletion between GA20ox5_1654 and SAMT_304; 12 nt deletion at GA20ox5_7090; 3 nt deletion at SAMT_161 | Homozygous | 1008; 1017; 1002 |
| E220242 | P95046369 | 6344 nt deletion between GA20ox5_1654 and SAMT_304; 12 nt deletion at GA20ox5_7090; 3 nt deletion at SAMT_161 | Homozygous | 1008; 1017; 1002 |
| E220242 | P95046368 | 6344 nt deletion between GA20ox5_1654 and SAMT_304; 12 nt deletion at GA20ox5_7090; 3 nt deletion at SAMT_161 | Homozygous | 1008; 1017; 1002 |
| E220242 | P95046395 | 6344 nt deletion between GA20ox5_1654 and SAMT_304; 12 nt deletion at GA20ox5_7090; 3 nt deletion at SAMT_161 | Homozygous | 1008; 1017; 1002 |
| E220242 | P95046396 | 6344 nt deletion between GA20ox5_1654 and SAMT_304; 12 nt deletion at GA20ox5_7090; 3 nt deletion at SAMT_161 | Homozygous | 1008; 1017; 1002 |
| E220698 | P43596662 | 6518 nt deletion between GA20ox5_1654 and SAMT_161; 4 nt deletion at T7090 | Homozygous | 1005; 1018 |
| E220698 | P43596671 | 6518 nt deletion between GA20ox5_1654 and SAMT_161; 4 nt deletion at T7090 | Heterozygous | 1005; 1018 |
| E220698 | P43596694 | 6518 nt deletion between GA20ox5_1654 and SAMT_161; 4 nt deletion at T7090 | Homozygous | 1005; 1018 |
| E220698 | P43596679 | 6518 nt deletion between GA20ox5_1654 and SAMT_161; 4 nt deletion at T7090 | Homozygous | 1005; 1018 |
| E220698 | P43596701 | 6518 nt deletion between GA20ox5_1654 and SAMT_161; 4 nt deletion at T7090 | Heterozygous | 1005; 1018 |
| E220698 | P43596654 | 6518 nt deletion between GA20ox5_1654 and SAMT_161; 4 nt deletion at T7090 | Homozygous | 1005; 1018 |
| E220698 | P43596690 | 6518 nt deletion between GA20ox5_1654 and SAMT_161; 4 nt deletion at T7090 | Heterozygous | 1005; 1018 |
| E220698 | P43596703 | 6518 nt deletion between GA20ox5_1654 and SAMT_161; 4 nt deletion at T7090 | Homozygous | 1005; 1018 |
| E220698 | P43596711 | 6518 nt deletion between GA20ox5_1654 and SAMT_161; 4 nt deletion at T7090 | Homozygous | 1005; 1018 |
| E220055 | P95046321 | 6348 nt deletion between GA20ox5_1654 and SAMT_304; 39 nt deletion at T7090 | Homozygous | 1007; 1019 |
| E220055 | P95046342 | 6348 nt deletion between GA20ox5_1654 and SAMT_304; 39 nt deletion at T7090 | Homozygous | 1007; 1019 |
| E220055 | P95046314 | 6348 nt deletion between GA20ox5_1654 and SAMT_304; 39 nt deletion at T7090 | Homozygous | 1007; 1019 |
| E220055 | P95046297 | 6348 nt deletion between GA20ox5_1654 and SAMT_304; 39 nt deletion at T7090 | Homozygous | 1007; 1019 |
| E220228 | P43596770 | 6357 nt deletion between GA20ox5_1654 and SAMT_304 | Homozygous | 1004 |
| E220141 | P43596991 | 6342 nt deletion between GA20ox5_1654 and SAMT_304 | Homozygous | 1006 |
| E220141 | P43597019 | 6342 nt deletion between GA20ox5_1654 and SAMT_304 | Homozygous | 1006 |
| E220141 | P43596954 | 6342 nt deletion between GA20ox5_1654 and SAMT_304 | Homozygous | 1006 |
| E220141 | P43596970 | 6342 nt deletion between GA20ox5_1654 and SAMT_304 | Homozygous | 1006 |
| E220141 | P43596980 | 6342 nt deletion between GA20ox5_1654 and SAMT_304 | Homozygous | 1006 |
| E187994 | P43597077 | 6160 nt deletion between GA20ox5_6531 and SAMT_408 | Homozygous | 1010 |
| E187994 | P43597052 | 6160 nt deletion between GA20ox5_6531 and SAMT_408 | Heterozygous | 1010 |
| E187994 | P43597049 | 6160 nt deletion between GA20ox5_6531 and SAMT_408 | Heterozygous | 1010 |
| E187994 | P43597037 | 6160 nt deletion between GA20ox5_6531 and SAMT_408 | Homozygous | 1010 |
| E188579 | P43596586 | 6133 nt deletion between GA20ox5_6531 and SAMT_408 | Heterozygous | 1011 |
| E188579 | P43596582 | 6133 nt deletion between GA20ox5_6531 and SAMT_408 | Heterozygous | 1011 |
| E188579 | P43596603 | 6133 nt deletion between GA20ox5_6531 and SAMT_408 | Heterozygous | 1011 |
| E188579 | P43596594 | 6133 nt deletion between GA20ox5_6531 and SAMT_408 | Homozygous | 1011 |
| E188790 | P09617231 | 6130 nt deletion between GA20ox5_6531 and SAMT_408 | Homozygous | 1012 |
| E188790 | P09617182 | 6130 nt deletion between GA20ox5_6531 and SAMT_408 | Heterozygous | 1012 |
| E188790 | P09617144 | 6130 nt deletion between GA20ox5_6531 and SAMT_408 | Heterozygous | 1012 |
| E188790 | P09617191 | 6130 nt deletion between GA20ox5_6531 and SAMT_408 | Heterozygous | 1012 |
| E188790 | P09617225 | 6130 nt deletion between GA20ox5_6531 and SAMT_408 | Homozygous | 1012 |
| E188790 | P09617216 | 6130 nt deletion between GA20ox5_6531 and SAMT_408 | Homozygous | 1012 |
| E188790 | P09617192 | 6130 nt deletion between GA20ox5_6531 and SAMT_408 | Homozygous | 1012 |
| E188790 | P09617208 | 6130 nt deletion between GA20ox5_6531 and SAMT_408 | Homozygous | 1012 |
| E188569 | P43596926 | 6130 nt deletion between GA20ox5_6531 and SAMT_408 | Homozygous | 1013 |
| E188569 | P43596908 | 6130 nt deletion between GA20ox5_6531 and SAMT_408 | Homozygous | 1013 |
| E188569 | P43596931 | 6130 nt deletion between GA20ox5_6531 and SAMT_408 | Homozygous | 1013 |
| E188569 | P43596895 | 6130 nt deletion between GA20ox5_6531 and SAMT_408 | Heterozygous | 1013 |
| E188569 | P43596896 | 6130 nt deletion between GA20ox5_6531 and SAMT_408 | Heterozygous | 1013 |
| E188569 | P43596911 | 6130 nt deletion between GA20ox5_6531 and SAMT_408 | Heterozygous | 1013 |
| E189115 | P43596944 | 6131 nt deletion between GA20ox5_6531 and SAMT_408 | Homozygous | 1014 |
| E180294 | P43596566 | 6478 nt deletion between GA20ox5_1695 and SAMT_8165 | Homozygous | 1009 |
| E180294 | P43596550 | 6478 nt deletion between GA20ox5_1695 and SAMT_8165 | Homozygous | 1009 |
| E180294 | P43596542 | 6478 nt deletion between GA20ox5_1695 and SAMT_8165 | Homozygous | 1009 |
| E180294 | P43596530 | 6478 nt deletion between GA20ox5_1695 and SAMT_8165 | Homozygous | 1009 |
| E180294 | P43596524 | 6478 nt deletion between GA20ox5_1695 and SAMT_8165 | Homozygous | 1009 |
| E180294 | P43596534 | 6478 nt deletion between GA20ox5_1695 and SAMT_8165 | Homozygous | 1009 |
| E180294 | P43596558 | 6478 nt deletion between GA20ox5_1695 and SAMT_8165 | Homozygous | 1009 |
| E180294 | P43596538 | 6478 nt deletion between GA20ox5_1695 and SAMT_8165 | Homozygous | 1009 |

### Example 29. Reduced plant height of corn plants with edited allele.

R1 corn plants homozygous or heterozygous for an edited allele of the GA20 oxidase 5 gene (as identified in Example 28) were grown to maturity to measure their plant heights along with wild type control plants. R1 seeds were planted in soil and grown to maturity in the greenhouse under day/night temperatures of 85°/70° and 16/8 hours of photoperiod using standard nutrient and light conditions for corn plant growth and development. Plant heights (PHT) of R1 plants were measured at R2 growth stage from the soil level to the base of the uppermost fully expanded leaf.

Table 6 provides the plant heights of individual R1 plants homozygous for deletion edits between the GA20ox5 and SAMT genes made using the Vector-2 or Vector-1 construct described in Example 27, along with wild type (WT) control plants. Average plant heights for WT and each homozygous deletion edit are also provided in Table 6 (see also FIG. 22 showing the average plant heights with error bars). These plant heights demonstrate that plants homozygous for an edited GA20 oxidase 5 allele comprising a deletion between the GA20ox5 and SAMT genes have significantly reduced plant heights averaging between 57.3 inches and 70.1 inches for plants having the edited alleles, versus an average plant height of 78.5 inches for the WT control.

Table 7 provides the plant heights of individual R1 plants homozygous or heterozygous for deletion edits between the GA20ox5 and SAMT genes made using the Vector-2 construct described in Example 27, along with wild type (WT) control plants (see also FIG. 23 showing average plant heights with error bars). The data in Table 7 overlaps with Table 6 since R1 plants homozygous for the deletion edits made using the Vector-2 construct and the wild type control plants are the same as in Table 6. These plant heights demonstrate that plants heterozygous or homozygous for the edited GA20 oxidase 5 alleles comprising a deletion between the GA20ox5 and SAMT genes and made using the Vector-2 construct have significantly reduced plant heights averaging between 57.3 inches and 64 inches for plants homozygous these edited alleles, and between 60.5 inches and 67 inches for plants heterozygous for these edited alleles, relative to an average plant height of 78.5 inches for the WT control plants. The reductions in plant height are similar between plants homozygous and heterozygous for the deletion edit alleles, but plant heights overall for plants comprising the deletion edit alleles regardless of zygosity are significantly lower than those of wild type control plants.

The plant height data described in this example demonstrate that deletion of the region between GA20ox5 and SAMT genes leads to reduced plant heights as compared to wild type control plants, for plants homozygous or heterozygous for the edited deletion alleles, suggesting that these deletion alleles of the GA20 oxidase 5 gene act in a dominant or semi-dominant manner to produce a reduced plant height phenotype (semi-dwarf or short stature corn plants), especially since edited loss-of-function alleles of the GA20 oxidase 3 or GA20 oxidase 5 genes alone without an antisense or inversion sequence have been shown to not produce short stature corn plants. See, e.g., Published PCT Application Nos. WO/2019/161149, WO/2019/161147 and WO/2019/161144, the entire contents and disclosures of which are incorporated herein by reference. Further plant height measurements will be made in subsequent generations to confirm the shorter plant height phenotype.

**Table 6. Plant Heights of homozygous R1 plants using Vector-2 and Vector-1.**

| **Editing Construct ID** | **Edit ID** | **R1 Plant ID** | **Plant height (inches)** |
|---|---|---|---|
| Vector-2 | E187994 | P43597037 | 65 |
| Vector-2 | E187994 | P43597077 | 63 |
| | **E187994 Average** | | 64.0 |
| Vector-2 | E188569 | P43596931 | 65 |
| Vector-2 | E188569 | P43596908 | 55.75 |
| Vector-2 | E188569 | P43596926 | 51 |
| | **E188569 Average** | | 57.3 |
| Vector-2 | E188579 | P43596594 | 61 |
| Vector-2 | E188790 | P09617225 | 70 |
| Vector-2 | E188790 | P09617231 | 60.75 |
| Vector-2 | E188790 | P09617208 | 60.25 |
| Vector-2 | E188790 | P09617216 | 59.5 |
| Vector-2 | E188790 | P09617192 | 55 |
| | **E188790 Average** | | 61.1 |
| Vector-2 | E189115 | P43596944 | 58.5 |
| Vector-1 | E220055 | P95046314 | 69.5 |
| Vector-1 | E220055 | P95046342 | 69 |
| Vector-1 | E220055 | P95046321 | 68 |
| Vector-1 | E220055 | P95046297 | 66.25 |
| | **E220055 Average** | | 68.2 |
| Vector-1 | E220141 | P43596991 | 72 |
| Vector-1 | E220141 | P43596954 | 72 |
| Vector-1 | E220141 | P43596970 | 71.5 |
| Vector-1 | E220141 | P43596980 | 68 |
| Vector-1 | E220141 | P43597019 | 67 |
| | **E220141 Average** | | 70.1 |
| Vector-1 | E220228 | P43596770 | 52 |
| Vector-1 | E220242 | P95046370 | 74 |
| Vector-1 | E220242 | P95046369 | 71.5 |
| Vector-1 | E220242 | P95046392 | 69.5 |
| Vector-1 | E220242 | P95046395 | 69 |
| Vector-1 | E220242 | P95046378 | 69 |
| Vector-1 | E220242 | P95046368 | 66 |
| Vector-1 | E220242 | P95046396 | 65 |
| Vector-1 | E220242 | P95046377 | 64.75 |
| Vector-1 | E220242 | P95046399 | 64 |
| Vector-1 | E220242 | P95046375 | 63.5 |
| | **E220242 Average** | | 67.6 |
| Vector-1 | E220698 | P43596694 | 70 |
| Vector-1 | E220698 | P43596662 | 68 |
| | **E220698 Average** | | 69.0 |
| Vector-1 | E220938 | P43596438 | 68.5 |
| Vector-1 | E220938 | P43596469 | 60.5 |
| Vector-1 | E220938 | P43596489 | 58 |
| | **E220938 Average** | | 62.3 |
| Vector-1 | E221089 | P43596831 | 69 |
| Vector-1 | E221089 | P43596820 | 67 |
| Vector-1 | E221089 | P43596823 | 65 |
| | **E221089 Average** | | 67 |
| Wild type | WT1 | | 80 |
| Wild type | WT2 | | 79.5 |
| Wild type | WT3 | | 79 |
| Wild type | WT4 | | 79 |
| Wild type | WT5 | | 75 |
| | **Wild type Average** | | 78.5 |

**Table 7. Plant Heights of homozygous and heterozygous R1 plants using Vector-2.**

| **Event ID** | **R1 Plant ID** | **R1 zygosity for deletion mutant** | **Plant height (inches)** |
|---|---|---|---|
| E187994 | P43597052 | Heterozygous | 60.5 |
| E187994 | P43597049 | Heterozygous | 73.5 |
| | **E187994 Heterozygous Average** | | 67.0 |
| E187994 | P43597037 | Homozygous | 65 |
| E187994 | P43597077 | Homozygous | 63 |
| | **E187994 Homozygous Average** | | 64.0 |
| E188569 | P43596895 | Heterozygous | 63.5 |
| E188569 | P43596911 | Heterozygous | 59.5 |
| E188569 | P43596896 | Heterozygous | 69 |
| | **E188569 Heterozygous** | | 64.0 |
| | **Average** | | |
| E188569 | P43596908 | Homozygous | 55.75 |
| E188569 | P43596926 | Homozygous | 51 |
| E188569 | P43596931 | Homozygous | 65 |
| | **E188569 Homozygous Average** | | 57.3 |
| E188579 | P43596582 | Heterozygous | 62 |
| E188579 | P43596603 | Heterozygous | 59 |
| E188579 | P43596586 | Heterozygous | 65 |
| | **E188579 Heterozygous Average** | | 62.0 |
| E188579 | P43596594 | Homozygous | 61 |
| E188790 | P09617182 | Heterozygous | 65.75 |
| E188790 | P09617238 | Heterozygous | 65 |
| E188790 | P09617144 | Heterozygous | 61 |
| E188790 | P09617191 | Heterozygous | 50.25 |
| | **E188790 Heterozygous Average** | | 60.5 |
| E188790 | P09617192 | Homozygous | 55 |
| E188790 | P09617208 | Homozygous | 60.25 |
| E188790 | P09617225 | Homozygous | 70 |
| E188790 | P09617231 | Homozygous | 60.75 |
| E188790 | P09617216 | Homozygous | 59.5 |
| | **E188790 Homozygous Average** | | 61.1 |
| Wild type | WT1 | | 80 |
| Wild type | WT2 | | 79.5 |
| Wild type | WT3 | | 79 |
| Wild type | WT4 | | 79 |
| Wild type | WT5 | | 75 |
| | **Wild type Average** | | 78.5 |

### Example 30. Collection of samples from R2 plants for molecular assays.

For the E220141 and E221089 deletion edits from the Vector-1 construct, R1 plants homozygous for those deletion edits (P43596991 and P43596831, respectively) were selfed to produce homozygous inbred R2 plants. The R2 inbred plants containing one of the E220141 and E221089 edits, and wild type control plants of the same inbred line, were grown under standard conditions in the greenhouse and sampled at V2 growth stage for the molecular assays described below. The plants were cut just above the soil level and the entire above-ground portion of the plants were placed in 50ml conical tubes and immediately frozen in liquid nitrogen. Each sample contained one or two sibling plants of the same genotype. The number of samples for each assay and genotype are provided in Table 8. The frozen samples were milled and used for the small RNA and GA hormone assays described in Examples 31 and 32 below.

**Table 8. Description of samples for small RNA and GA hormones assays.**

| **Editing Construct ID** | **Edit ID (R2 Inbreds)** | **Number of samples for small RNA assay** | **Number of samples for GA hormone assay** |
|---|---|---|---|
| Inbred Wild type | | 2 | |
| Vector-1 | E220141 | 2 | 7 |
| Vector-1 | E221089 | 1 | 10 |

### Example 31. Detection of small RNAs in plants having an edited deletion allele.

To generate small RNA libraries for sequencing, Illumina's TruSeq small RNA Library Preparation Kit was used according to the manufacturer's protocol (Document # 15004197v02) with a modification at the library purification step. Samples of each genotype for this small RNA assay experiment are identified in Example 30 above. After amplification of cDNA, individual libraries were gel purified using a 6% Novex TBE PAGE Gel for size separation. The gel was stained with 1xSYBR Gold for 20 minutes. The final library product was sequenced on Illumina's NextSeq platform with a minimum depth of 3 million reads per sample. After sequencing, reads were processed through the following steps: the sequencing adapters were trimmed: reads matching housekeeping noncoding RNAs were removed and libraries normalized to reads per million. Between 1 and 9 samples per genotype were assayed.

The mutated GA20 oxidase 5 (GA20ox5) gene containing the E220141 and E221089 deletion edits were predicted to produce antisense RNA transcripts spanning all or part of the coding sequence of the GA20ox5 gene under the control of the downstream native SAMT promoter in the reverse orientation that could hybridize to mRNA transcripts expressed from the wild type and/or mutant GA20 oxidase 5 alleles and/or the GA20 oxidase 3 gene or allele(s). Since antisense RNA sequences can trigger RNA interference (RNAi) and suppression of genes encoding identical or homologous RNA sequences, plants containing the deletion edits were assayed for the presence of small RNAs. Processing of the double stranded RNA would be expected to produce small RNAs of about 21, 22 or 24 nucleotides in length corresponding to the coding sequence of the GA20ox5 gene. In this experiment, the edited R2 plants, as well as wild type control plants, did not show a noticeable accumulation of small RNAs corresponding to the GA20ox5 gene in the 21, 22 or 24-nucleotide small RNA range, which was measured to be 0 or 1 read per million total sequencing reads (data not shown). These data indicate that the edited plants either do not produce small RNAs at the V2 growth stage sampled in this example or act through a different dominant negative mechanism. However, the pattern of expression of antisense RNA transcripts complementary to all or part of the coding sequence of the GA20 oxidase 5 gene is also dependent on the SAMT gene promoter, which may not drive expression (or expression at a sufficiently high level) at the V2 growth stage to produce a measurable effect on the levels of small RNAs. Without being bound by theory, it is possible that expression of antisense transcripts from an edited deletion allele of the endogenous GA20ox5 gene may be more robust at later stages of development and thus have a greater or more measurable effect on the level of small RNAs and RNAi suppression at those later stages.

Future experiments will also seek to determine whether the levels of GA20ox3 and/or GA20ox5 mRNA transcripts are reduced in plants homozygous or heterozygous for an edited GA20ox5 allele having a deletion between the GA20ox5 and SAMT genes, relative to controls.

### Example 32. Detection of GA hormones in plants having an edited deletion allele

Reduced expression of GA20 oxidase genes can alter the levels of GA hormones in corn plants, which can in turn affect plant height with lower levels of active GAs potentially reducing plant height. The levels of bioactive GA hormones and their precursors were measured in plants containing the edited GA20ox5 alleles. GA20 oxidase is active in the GA biosynthetic pathway and catalyzes the sequential oxidation of metabolic intermediates GA12 and GA53 into GA9 and GA20, respectively (the "early 13-hydroxylation pathway" and "non 13-hydroxylation pathway"). The primary bioactive forms of GA include GA1, GA3 and GA4, which are further downstream (3') of GA20 oxidase activity and the GA9 and GA20 intermediates in the biosynthetic pathway. A reduction or suppression of the expression level and/or enzymatic function of GA20 oxidase genes, as may be expected with the GA20ox5 deletion edits, may result in reduction of downstream metabolites (GA20 and GA9) and accumulation of upstream precursors (GA53 and GA12).

For this experiment, samples were collected as provided in Example 30 above. Freshly frozen plant sample tissues were extracted and cleaned using Waters solid phase extraction MAX cartridge plate. GA hormones and 2 internal standards were analyzed using UPLC coupled with an ABSciex 5500 Mass Spectrometry with MRM method. The final GA hormone values were calculated based on the calibration curve with ABSciex software Multi-Quan. Each GA hormone calibration curve was in good linear fit, the R2 linear regression >0.99. The 8 technical controls per 96-well plate for each hormone were also included and evaluated in analytical process for meeting the standard criterion. GA levels were measured in terms of pmol/gram of sample tissue.

As shown in FIG. 24, the levels of GA12 were increased in inbred plants homozygous for the edited E221089 allele but were statistically neutral or unchanged in inbred plants homozygous for the edited E220141 allele, relative to wild type control plants. As further shown in FIG. 24, the levels of GA9 were decreased in inbred plants homozygous for the edited E220141 allele but neutral in inbred plants homozygous for the edited E221089 allele, relative to wild type control plants.

As shown in FIG. 25, the levels of GA20 were decreased in inbred plants homozygous for either of the edited alleles (E221089 or E220141), relative to wild type control plants. As further shown in FIG. 25, the levels of GA53 were increased in inbred plants homozygous for either of the edited alleles (E221089 or E220141), relative to wild type control plants.

FIG. 26 provides the results for levels of active GAs (GA1, GA3 and GA4) measured in samples collected at V2 growth stage of the edited inbred plants relative to wild type controls. As shown in FIG. 26, the levels of these active GAs were generally not statistically changed in the inbred plants homozygous for the edited alleles (E221089 or E220141), except for an increase in GA4 in inbred plants homozygous for either of the edited alleles (E221089 or E220141).

These data support the theory that an antisense transcript may be expressed from the edited GA20 oxidase 5 gene, allele or locus having a deletion between the neighboring GA20 oxidase 5 and SAMT genes, that may reduce the expression level(s) of the GA20 oxidase 5 and/or GA20 oxidase 3 gene(s) and thus affect the levels of GA hormones in plants containing the edited alleles. The data in this experiment show increased accumulation of the GA12 and GA53 precursors upstream (5') of GA20 oxidase activity and decreased levels of GA9 and GA20 products of GA20 oxidase activity in plants containing the edited GA20 oxidase 5 allele. although the levels of GA12 and GA9 were unchanged in the edited E220141 and E221089 inbred plants, respectively.

Although the levels of bioactive GAs were not shown to be reduced in this example, this may be due to the early V2 growth stage when the plant tissue samples were collected for this experiment. Indeed, the pattern of expression of an antisense RNA transcript complementary to all or part of the coding sequence of the GA20 oxidase 5 gene is dependent on the SAMT gene promoter, which may not drive expression (or expression at a sufficiently high level) at the early V2 growth stage to produce a measurable effect on the levels of active GAs. Without being bound by theory, it is possible that expression of antisense transcripts from the edited deletion alleles of the endogenous GA20ox5 gene under the control of the endogenous SAMT gene promoter may be more robust at later stages of development and thus have a greater or more measurable effect on the level(s) of active GAs at those later stages. The active GAs are also further downstream and not a direct product of GA20 oxidase enzyme activity. Future experiments will determine if lower active GA levels are observed at later stages of development in plants heterozygous or homozygous for an edited GA20 oxidase 5 locus comprising a deletion between the GA20ox5 and SAMT genes. which is supported by the altered levels of GA precursors observed in this example at the early V2 growth stage.

### Example 33. Creating dominant alleles by genome editing to produce a hairpin-containing transcript

FIG. 27 provides illustrative examples for the production, through targeted gene editing, of a genomic modification of the Zm.GA20ox3 locus to encode a RNA transcript with an inverted sequence that can hybridize to a corresponding sequence of the RNA transcript to produce a stem-loop structure, to cause the suppression of one or both of the copies or alleles at the endogenous Zm.GA20ox3 and Zm.GA20ox5 loci. The Zm.GA20ox3 and Zm.GA20ox5 genes share a high level of nucleotide sequence similarity of their respective exon regions. Thus, a fragment of the Zm.GA20ox5 gene, sharing sufficient sequence identity or homology with a corresponding sequence of the Zm.GA20ox3 gene, inserted into a preselected site in the Zm.GA20ox3 gene in the reverse orientation near the corresponding Zm.GA20ox3 sequence, can hybridize to the corresponding sequence to form a stem-loop structure and trigger suppression of the Zm.GA20ox3 and Zm.GA20ox5 genes. The insertion site is determined by the design of the guide RNA directing a double-stranded genomic DNA cleavage. Without being bound to any particular theory, the edited Zm.GA20ox3 gene produces a transcript able to form a stem-loop structure which can induce suppression or gene silencing of the wild-type or other alleles of the Zm.GA20ox3 and Zm.GA20ox5 genes. In this example, the inserted Zm.GA20ox5 fragment can be excised from either copy or allele of the endogenous Zm.GA20ox5 gene. This type of editing is called trans-fragment targeting (TFT) editing since the fragment originates from the endogenous Zm.GA20ox5 gene. The boundaries of the excision fragment are defined by a pair of properly designed guide RNAs. According to this example, the inserted Zm.GA20ox5 fragment can also be excised from a donor template which is a double-stranded DNA molecule comprising the desired Zm.GA20ox5 fragment and flanked by target sites for guide RNA(s). The flanking gRNA target sites can be identical, utilizing the same gRNA, or different and according to some embodiments oriented to limit the amount of gRNA target site included in the DNA insertion fragment post cleavage. These target sites can match to the endogenous genome, be artificially-derived, or match to a non-endogenous genome, relative to the endogenous genome of the plant to be edited. According to some embodiments, the target sites flanking the insertion fragment of the donor template to be excised for insertion into the endogenous locus may be heterologous with respect to the native or endogenous genome, such that any off-target DNA cleavage within the endogenous genome can be minimized or eliminated. This method is referred to as "template assist" when performed in combination with guide RNAs targeted for excising a fragment from the endogenous Zm.GA20ox5 locus. Conversely, a Zm.GA20ox3 fragment may be excised via TFT from either copy or allele of the Zm.GA20ox3 gene and/or from a donor template (*e.g*., template assist method) and inserted into a pre-selected site of the endogenous Zm.GA20ox5 gene to produce an inverted-repeat sequence which can be transcribed into a RNA transcript comprising a stem-loop structure that triggers RNA-mediated suppression or silencing of the wild-type or other alleles of the Zm.GA20ox3 and/or Zm.GA20ox5 genes.

With either of these approaches, however, it is possible for other types of edits or mutations to be formed, such as deletion(s) and/or inversion(s) depending on which DNA cut(s) or break(s) are created at the gRNA or editing target site(s) and the fragment(s) inserted into or between those cut site(s). An inserted DNA fragment may originate from either copy or allele of the Zm.GA20ox3 or Zm.GA20ox5 gene, or from a DNA template molecule. Therefore, a deletion can be generated from cutting one or more target sites, and/or an inversion sequence can be generated by a DNA fragment being inserted in an opposite, reverse or antisense orientation relative to the coding sequence of the edited Zm.GA20 oxidase gene. The inversion may be present in the edited gene with or without a corresponding sequence that when expressed could hybridize to form a RNA hairpin or stem-loop structure with the encoded inversion sequence of the mRNA transcribed from the edited gene. The presence of an antisense inversion sequence without the corresponding sequence and resulting hairpin or stem-loop structure may be sufficient to trigger suppression of one or both of the Zm.GA20ox3 and Zm.GA20ox5 genes through canonical or non-canonical RNA mechanisms.

A plant transformation construct (Vector-4) was designed to create a double stranded break (DSB) in the Zm.GA20ox3 gene to allow for insertion of an antisense DNA fragment of the Zm.GA20ox5 gene either from the endogenous Zm.GA20ox5 locus or an exogenously provided donor template. In this example, the construct generally contains 4 functional regions or cassettes relevant to gene editing and creation of the insertion (*e.g*., inversion) in the edited gene: expression of a Cpfl or Cas12a variant protein, expression of three guide RNAs for the Zm.GA20ox3 gene locus, expression of an additional three guide RNAs for the Zm.GA20ox5 gene locus, and a donor template region comprising a Zm.GA20ox5 gene fragment for the template assist method of inserting the Zm.GA20ox5 gene fragment (approximately 400 nucleotides in length) from the donor template. Each guide RNA unit contains a common scaffold compatible with the Cpfl mutant, and a unique spacer/targeting sequence complementary to its intended target site.

The Cpfl expression cassette comprises a maize ubiquitin promoter (SEQ ID NO: 37) operably linked to a sequence encoding a *Lachnospiraceae bacterium* G532R/K595R mutant Cpfl RNA-guided endonuclease enzyme (SEQ ID NO: 39) fused to two nuclear localization signals (SEQ ID NOs: 42 and 43). *See, e.g.,* Gao, L. et al., Nature Biotechnol. 35(8): 789-792 (2017), the entire contents and disclosure of which are incorporated herein by reference.

One expression cassette comprises a sequence encoding three guide RNAs (two guide RNAs having targeting/spacer sequences encoded by the SP1 and SP2 DNA sequences in Table 9 below (see also FIG. 27) that target two closely spaced-apart sites in exon 1 of the Zm.GA20ox3 gene; and another guide RNA having a targeting/spacer sequence encoded by the SP3 DNA sequence in Table 9 below (see also FIG. 27) targeting a site in exon 1 of the Zm.GA20ox5 gene), operably linked to a maize RNA polymerase III (Pol3) promoter (SEQ ID NO: 44). Spacer sequences SP1 and SP2 target two alternative breakage sites in exon-1 of Zm.GA20ox3 that are spaced apart by about 68 nucleotides, and either breakage site is able to receive a reverse complement insertion fragment, or the insertion fragment could replace the sequence between SP1 and SP2. It is also possible that the insertion fragment could integrate at both SP1 and SP2 target sites.

Another expression cassette comprises a sequence encoding an additional three guide RNAs (two guide RNAs having targeting/spacer sequences encoded by the SP4 and SP5 DNA sequences in Table 9 (see also FIG. 27) that target two closely spaced-apart target sites in exon 1 of the Zm.GA20ox5 gene; and another guide RNA having targeting/spacer sequences encoded by the SP6 DNA sequence in Table 9 (see also FIG. 27) targeting two identical engineered sites flanking the Zm.GA20ox5 gene fragment in the donor template region), operably linked to a synthetic promoter. Spacer sequences SP3 or SP4 target two alternative breakage sites in exon-1 of the endogenous Zm.GA20ox5 gene that are spaced apart by about 83 nucleotides, whereas the spacer sequence SP5 targets another breakage point in exon-1 of the endogenous Zm.GA20ox5 gene spaced apart from the cleavage site for the SP4 spacer by a greater distance of about 577 nucleotides. The targeting/spacer sequence SP6 is derived from a soybean PDS gene that has been separately demonstrated to work in combination with Cpfl to direct cleavage of a target site in an endogenous PDS gene of a soybean plant.

Another nearly identical plant transformation construct (Vector-5) was designed to create a double stranded break (DSB) in the Zm.GA20ox3 gene to allow for insertion of an antisense DNA fragment of the Zm.GA20ox5 gene, but this second construct did not encode the guide RNA having the targeting/spacer sequence encoded by the SP6 DNA sequence for the template assist method, such that the fragment would originate from an endogenous copy of the Zm.GA20ox5 gene.

With the constructs described in this example, guide RNAs with spacers SP3 and SP4 may work in combination with a guide RNA with spacer SP5 would produce a fragment between about 500 and 700 bp from exon-1 of the endogenous Zm.GA20ox5 gene that could be inserted into a site within exon-1 of the endogenous Zm.GA20ox3 gene in the reverse complementary orientation, such that the RNA molecule transcribed from the endogenous Zm.GA20ox3 gene forms a stem-loop structure in the RNA transcript that can trigger suppression or silencing of the other copy/copies or allele(s) of the endogenous Zm.GA20ox3 and/or Zm.GA20ox5 gene(s). The resulting fragment could be referred to as a SP3-SP5 fragment (SEQ ID NO: 76) or a SP4-SP5 fragment (SEQ ID NO: 77), depending on whether spacer SP3 or SP4 was involved. In addition, a donor template containing a Zm.GA20ox5 gene fragment flanked by two SP6 spacer sequences can produce a Zm.GA20ox5 gene fragment (referred to as a SP6-SP6 fragment (e.g., SEQ ID NO: 88), if used in combination with the first transformation construct described above containing the SP6 sequence, for insertion into a site within the endogenous Zm.GA20ox3 gene in the reverse complementary orientation.

The DNA sequences encoding the guide RNA spacers and their intended target sites are listed in Table 9.

**TABLE 9. Example guide RNAs used for editing the Zm.GA20ox3 locus.**

| **Guide RNA Spacer** | **Target Site** | **Spacer Sequence** | **SEQ ID** |
|---|---|---|---|
| SP1 | GA20ox3_2587 | CTGGAAGGAGACCCTGTCCTTCG | 78 |
| SP2 | GA20ox3_2655 | CCGGCACCCTCGGCCAAGATTTC | 79 |
| SP3 | GA20ox5_428 | CTCCCTGCCTTCGTCTTTGTCGT | 80 |
| SP4 | GA20ox5_511 | CTGCATACTTGCAGCTCGCACAT | 81 |
| SPS | GA20ox5_1088 | CTGGAAGGAGACCCTGTCGTTCG | 82 |
| SP6 | Template Gm.PDS site | GTAAGAAGCTCTTCACCGTTCCA | 83 |

### Example 34. Confirmation of inversion edits in plants using the constructs in Example 33

An inbred corn plant line was transformed via *Agrobacterium-mediated* transformation with one of the transformation vectors described above in Example 33. The transformed plant tissues were grown to produce mature R0 plants. R0 plants having one or more unique genome edit(s) were self-crossed to produce R1 plants. To determine the edits and insertions in the endogenous Zm.GA20ox3 gene of the R0 and R1 plants, one or two PCR assay approaches were performed, with primers designed to identify the size or junctions of the intended insertions. One approach used a PCR primer pair including one primer (SEQ ID NO: 84) hybridizing to a sequence in the inserted Zm.GA20ox5 gene fragment and another primer (SEQ ID NO: 85) hybridizing to a sequence in the endogenous GA20ox3 gene, where the primers are oriented such that a PCR product is generated when the Zm.GA20ox5 gene fragment is inserted in the antisense orientation in the endogenous GA20ox3 gene (i.e., the PCR product was only generated across the 3' end of the inserted fragment when oriented in the inverted antisense direction). If a PCR fragment was generated, then a Zm.GA20ox5 gene fragment was inserted at the target site in the antisense orientation. In addition, whether the inserted Zm.GA20ox5 fragment originated from the endogenous Zm.GA20ox5 locus or the donor template region could also be determined and distinguished by PCR product size and/or sequencing the PCR products. This first PCR approach was used to determine which type of inverted insertion occurred in the endogenous Zm.GA20ox3 gene (see Tables 10 and 11).

According to a second PCR approach, a PCR primer pair including one primer (SEQ ID NO: 86) hybridizing to a sequence upstream (on the 5' side) of the two guide RNA target sites (SP1 and SP2) in the Zm.GA20ox3 gene and the other primer (SEQ ID NO: 87) hybridizing to a sequence downstream of the two SP1 and SP2 guide RNA target sites, such that a PCR product is generated spanning the possible insertion sites in the Zm.GA20ox3 gene. Thus, the presence and size of the PCR fragment using this approach would show whether an insertion occurred at the target sites, but independent of orientation. The PCR product can also be sequenced to determine the type and orientation of the insertion. According to this second approach, the size/sequence of the PCR product could also be used to determine whether the inserted GA20ox5 fragment originated from the endogenous GA20ox5 locus or the donor template region, and the zygosity of the plant could be determined by whether the wild-type PCR fragment size/sequence was present.

Individual R1 plants produced by selfing R0 plants having one or more of the edits were assayed for the type of insertion and the zygosity of the insertion mutant or allele (see Tables 10 and 11). As used herein, "homo" means homozygous for the mutant allele, and "hetero" means heterozygous for the mutant allele. Tables 10 and 11 further provide the genomic DNA sequence of the coding sequence or region of the edited GA20 oxidase 3 gene from the start to stop codon and the sequence of the inversion or antisense sequence within such coding sequence or region of the edited GA20 oxidase 3 gene (each by SEQ ID NO). To avoid repetition, the inversion type and coding and inversion sequences are only provided in the first row of Tables 10 and 11 for each Edit ID. Edit IDs E270933 and E271059 in Table 10 were generated with the Vector-4 construct, and Edit IDs E376333 and E376314 in Table 11 (and Edit ID E376274) were created using the Vector-5 construct. Additional edits were generated with these constructs, but either were not recovered in R1 plants/seeds, had other T-DNA insertions and/or did not produce small RNAs, and were therefore discarded and not advanced for further testing. Edit ID E376274 created with the Vector-5 construct comprising an inverted GA20ox5 SP4-SP5 fragment inserted into the GA20ox3 SP1 target site was not recovered in R1 plants/seeds and was therefore not advanced. Edit ID E376274 for the GA20 oxidase 3 gene has a genomic coding sequence of SEQ ID NO: 97 and an inversion sequence of SEQ ID NO: 98.

Tables 10 and 11 also provide information about possible simple/small or larger edit(s) or deletion(s) that may be present in the GA20 oxidase 5 gene. Simple or small deletion(s) may also be present in the endogenous GA20 oxidase 5 (GA20ox5) gene at one or more of the individual SP3, SP4 and SP5 target sites, and large deletion(s) may be present in the endogenous GA20ox5 gene spanning between the SP3/SP5 or SP4/SP5 target sites. For the Vector-5 construct, Table 11 provides pooled information and numbers for the R1 plants grouped by Edit ID. As can be seen in Tables 10 and 11, R0 and R1 plants in many cases did contain one or more edits or deletions in the GA20ox5 locus, although some R1 plants (designated as "unknown" in the tables) were not determined to contain an edit or deletion in the GA20ox5 gene. In other cases, the zygosity of an edited GA20ox5 allele was not determined and is therefore designated as "homozygous or heterozygous". However, the edited GA20ox5 alleles present in R0 and R1 plants were removed and segregated away from the edited GA20ox3 alleles in subsequent generations.

**TABLE 10. Editing Inversion and Zygosity in R0 and R1 plants for Vector-4**

| **Edit ID** | **R1 Plant ID** | **Editing inversion type** | **R1 zygosity call for GA20ox3 mutant** | **GA20ox5 Edit** | **Genomic Coding Sequence (SEQ ID NO)** | **Inversion Sequence (SEQ ID NO)** |
|---|---|---|---|---|---|---|
| E270933 | P757870 | GA20ox5 SP4-SP5 fragment into GA20ox3 SP1 target site; 6 nucleotide deletion at SP2 | hetero | unknown | 89 | 90 |
| E270933 | P757885 | | hetero | unknown | | |
| E270933 | P758012 | | hetero | unknown | | |
| E270933 | P758049 | | hetero | unknown | | |
| E270933 | P758040 | | hetero | Heterozygous large deletion | | |
| E270933 | P758007 | | hetero | unknown | | |
| E270933 | P757888 | | hetero | Heterozygous large deletion | | |
| E270933 | P757932 | | hetero | Heterozygous large deletion | | |
| E270933 | P757965 | | hetero | unknown | | |
| E270933 | P758046 | | hetero | unknown | | |
| E270933 | P757857 | | hetero | unknown | | |
| E270933 | P757881 | | hetero | unknown | | |
| E270933 | P757925 | | hetero | unknown | | |
| E270933 | P757982 | | hetero | unknown | | |
| E270933 | P757985 | | hetero | Heterozygous large deletion | | |
| E270933 | P758051 | | hetero | unknown | | |
| E270933 | P757886 | | hetero | unknown | | |
| E270933 | P757853 | | hetero | unknown | | |
| E270933 | P757904 | | hetero | unknown | | |
| E270933 | P757956 | | hetero | unknown | | |
| E270933 | P757970 | | hetero | unknown | | |
| E270933 | P757987 | | hetero | unknown | | |
| E270933 | P757962 | | hetero | unknown | | |
| E270933 | P757949 | | hetero | unknown | | |
| E270933 | P758001 | | hetero | unknown | | |
| E270933 | P758004 | | hetero | unknown | | |
| E271059 | P758336 | donor template fragment into GA20ox3 SP1 target site; 9 nucleotide deletion at SP2 | hetero | Homozygous or heterozygous small deletion | 91 | 92 |
| E271059 | P758342 | | hetero | Homozygous or heterozygous small deletion | | |
| E271059 | P758343 | | hetero | Biallelic for small deletions; T-DNA insert | | |
| E271059 | P758349 | | hetero | Homozygous or heterozygous small deletion | | |
| E271059 | P758352 | | hetero | Homozygous small deletion | | |
| E271059 | P758354 | | hetero | Homozygous or heterozygous small deletion | | |
| E271059 | P758355 | | hetero | Homozygous small deletion | | |
| E271059 | P758330 | | hetero | Homozygous or heterozygous small deletion | | |

**TABLE 11. Editing Inversion and Zygosity in R0 and R1 plants for Vector-5**

| **Edit ID** | **Number of R1 Plants** | **Editing inversion type** | **R1 zygosity call for GA20ox3 mutant** | **GA20ox5 Deletion** | **Genomic Coding Sequence (SEQ ID NO)** | **Inversion Sequence (SEQ ID NO)** |
|---|---|---|---|---|---|---|
| E376333 | 8 | GA20ox5 SP4-SP5 fragment to GA20ox3 SP2; 7 nucleotide deletion at SP1 | hetero | 4 with large GA20ox5 deletion; 1 with small GA20ox5 deletion; and 3 with both. | 93 | 94 |
| E376333 | 17 | | homo | 2 with large GA20ox5 deletion; 1 with small GA20ox5 deletion; 6 with both; and 8 unknown. | | |
| E376333 | 10 | | homo or hetero | unknown | | |
| E376314 | 8 | GA20ox5 SP4-SP5 fragment to GA20ox3 SP2; 6 nucleotide at SP1 | hetero | unknown | 95 | 96 |
| E376314 | 1 | | homo | unknown | | |
| E376314 | 1 | | homo or hetero | unknown | | |

### Example 35. Reduced plant height of corn plants with edited allele

R1 corn plants heterozygous for an edited allele of the GA20 oxidase 3 gene with the corresponding inversion identified in Example 34 were grown to maturity to measure their plant heights along with wild type control plants. R1 seeds were planted in soil and grown to maturity in the greenhouse under day/night temperatures of 85°F / 70°F (29.4°C / 21.1°C) and a photoperiod of 16 hours light/8 hours dark using standard nutrient and light conditions for corn plant growth and development. Plant heights (PHT) of these R1 plants were measured at R2 growth stage from the soil level to the base of the uppermost fully expanded leaf. Table 12 provides the plant heights of individual R1 plants heterozygous for one of two hairpin inversion edits, along with wild type control plants. Average plant heights for WT and each edit are also provided (see also FIG. 28 showing the average plant heights with error bars).

These plant heights demonstrate that plants heterozygous for an edited GA20 oxidase 3 allele comprising an inversion sequence have reduced plant heights averaging 54.0 inches or 57.3 inches for the two edited alleles, versus an average plant height of 64.2 inches for the WT control.

The plant height data shown in this example demonstrate that plants heterozygous for an edited allele of the GA20 oxidase 3 gene comprising an antisense inversion sequence have significantly reduced plant heights in comparison to wild type control plants, suggesting that these edited hairpin inversion alleles of the GA20 oxidase 3 gene act in a dominant or semi-dominant manner to produce a reduced plant height phenotype (i.e., semi-dwarf or short stature corn plants), especially since edited loss-of-function alleles of the GA20 oxidase 3 or GA20 oxidase 5 genes alone without an antisense or inversion sequence have been shown to not produce short stature corn plants. See, e.g., Published PCT Application Nos. WO/2019/161149, WO/2019/161147 and WO/2019/161144, the entire contents and disclosures of which are incorporated herein by reference. However, many of these R1 plants may also be homozygous or heterozygous for edited GA20ox5 allele(s) (see Table 10). The presence and zygosity of edited GA20ox5 alleles is unknown for many of the R1 plants, but R1 Plant IDs P758040, P757888, P757932 and P757985 for the E270933 Edit ID were heterozygous for a large deletion in the GA20ox5 gene, R1 Plant ID P758352 was homozygous for a small deletion in the GA20ox5 gene, R1 Plant ID P758343 contained small deletion(s) and a T-DNA insert in the GA20ox5 gene, and P758336 was homozygous or heterozygous for a small deletion in the GA20ox5 gene. Therefore, it is possible that additional mutation(s) in the GA20ox5 gene could also have an effect on R1 plant height. Further plant height measurements will be made in subsequent generations having the edited GA20ox5 alleles removed to confirm the shorter plant height phenotype.

**Table 12. Plant Heights of R1 plants heterozygous for edited inversion alleles of Zm.GA20ox3**

| **Edit ID** | **R1 Plant ID** | **Plant height (inches)** |
|---|---|---|
| E270933 | P757870 | 56.25 |
| E270933 | P757885 | 49.75 |
| E270933 | P758012 | 55 |
| E270933 | P758049 | 48.5 |
| E270933 | P758040 | 54.5 |
| E270933 | P758007 | 62 |
| E270933 | P757888 | 58 |
| E270933 | P757932 | 58 |
| E270933 | P757965 | 52.75 |
| E270933 | P758046 | 56.5 |
| E270933 | P757857 | 61.5 |
| E270933 | P757881 | 63.5 |
| E270933 | P757925 | 53.25 |
| E270933 | P757982 | 56.75 |
| E270933 | P757985 | 50.25 |
| E270933 | P758051 | 50.5 |
| | **Edit ID E270933 Average** | 55.4 |
| E271059 | P758352 | 54 |
| E271059 | P758343 | 54.5 |
| E271059 | P758336 | 63.5 |
| | **Edit ID E271059 Average** | 57.3 |
| Wild type | WT 1 | 63.5 |
| Wild type | WT 2 | 63.75 |
| Wild type | WT 3 | 68.5 |
| Wild type | WT 4 | 61 |
| | **Wild type Average** | 64.2 |

### Example 36. Collection of samples from R2 or R3 plants for molecular assays.

For the E270933 inversion edit from the Vector-4 construct, a R1 plant heterozygous for the E270933 edit (P757982) was selfed (self-pollinated) to obtain selected homozygous R2 plants, which were themselves either (i) self-pollinated to produce homozygous inbred R3 plants or (ii) crossed to another elite parental line to produce heterozygous hybrid R3 plants. For the E376333 inversion edit from the Vector-5 construct, a R1 plant homozygous for the E376333 edit (P127584) containing a large deletion in the GA20ox5 gene was either (i) self-pollinated to produce homozygous inbred R2 plants or (ii) crossed to another elite parental line to produce heterozygous hybrid R2 plants. Edited GA20ox5 alleles present in R1 plants were removed in R2 and R3 plants by segregation and selection. The R3 plants containing the E270933 edit, the R2 plants containing the E376333 edit, and wild type control plants of the same parental lines, were grown under standard conditions in the greenhouse and sampled at V2 growth stage for the molecular assays described below. The plants were cut just above the soil level and the entire above-ground portion of the plants were placed in 50 mL conical tubes and immediately frozen in liquid nitrogen. Each sample contained one or two sibling plants of the same genotype. The number of samples for each assay and genotype are provided in Table 13. The frozen samples were milled and used for the small RNA and GA hormone assays described in Examples 37 and 38 below.

**Table 13. Description of samples for small RNA and GA hormones assays.**

| **Editing Construct ID** | **Edit ID** | **Parental elite line(s)** | **Number of samples for small RNA assay** | **Number of samples for GA hormone assay** |
|---|---|---|---|---|
| Vector-4 | E270933 | Hybrid | 8 | 10 |
| Vector-5 | E376333 | Hybrid | 5 | 5 |
| Vector-4 | E270933 | Inbred | 9 | 10 |
| Vector-5 | E376333 | Inbred | 9 | 10 |
| Hybrid Wild type | | | 1 | |
| Inbred Wild type | | | 2 | |

### Example 37. Detection of small RNAs in plants having an edited inversion allele.

To generate small RNA libraries for sequencing, Illumina's TruSeq small RNA Library Preparation Kit was used according to the manufacturer's protocol (Document # 15004197v02) with a modification at the library purification step. Samples of each genotype for this small RNA assay experiment are identified in Example 36 above. After amplification of cDNA, individual libraries were gel purified using a 6% Novex TBE PAGE gel for size separation. The gel was stained with 1x SYBR Gold for 20 minutes. The final library product was sequenced on Illumina's NextSeq platform with a minimum depth of 3 million reads per sample. After sequencing, reads were processed through the following steps: the sequencing adapters were trimmed: reads matching housekeeping noncoding RNAs were removed; and libraries were normalized to reads per million. Between 1 and 9 samples per genotype were assayed.

mRNAs expressed from the edited GA20 oxidase 3 genes containing the E270933 and E376333 inversion edits were predicted to produce a hairpin or stem-loop RNA structure comprising the inversion sequence and the native sequence in the GA20 oxidase 3 gene that is complementary and could hybridize to the inversion sequence. Since double stranded RNA hairpins or stem-loop structures can trigger RNA interference (RNAi) and suppression of genes encoding identical or homologous RNA sequences, plants containing the inversion edits were assayed for the presence of small RNAs. RNAi would be expected to produce small RNAs of about 21 nucleotides in length (21-mers) from the stem of the stem-loop structure consisting in this example of the GA20ox5 inversion sequence and the GA20ox3 native sequence.

As shown in FIG. 29, small 21-mer RNAs corresponding to the regions in the stem of the edited stem-loop comprising the inversion sequence from GA20ox5 and a corresponding sequence of the edited GA20ox3 gene were detected in samples from plants containing the edited GA20ox3 alleles indicating the presence of small RNAs in tissues from these edited plants, which were not present in wild type control plants. The abundance of these small RNAs was measured as the number of reads per million total sequencing reads. Small RNAs were present in inbred plants homozygous for the edited allele and hybrid plants heterozygous for the edited allele, with these small RNAs ranging between 19 and 84 reads per million. The abundance of small RNAs appeared consistent with the number of copies of the edited GA20ox3 allele given that heterozygous hybrid plants produced fewer small RNAs than the homozygous inbred plants.

The presence in these samples of small RNAs corresponding to the edited complementary stem region of the edited GA20ox3 gene is consistent with the edited GA20ox3 inversion allele triggering RNAi suppression of the GA20ox3 gene and possibly also the GA20ox5 gene. Additional experiments will determine whether the levels of GA20ox3 and/or GA20ox5 mRNA transcripts are reduced in plants homozygous or heterozygous for edited GA20ox3 or GA20ox5 alleles containing an inversion sequence, relative to controls.

### Example 38. Detection of GA hormones in plants having an edited inversion allele.

Reduced expression of GA20 oxidase genes can alter the levels of GA hormones in com plants, which can in turn affect plant height with lower levels of active GAs potentially reducing plant height. The levels of bioactive GA hormones and their precursors were measured in plants containing the edited GA20ox3 alleles. GA20 oxidase is active in the GA biosynthetic pathway and catalyzes the sequential oxidation of metabolic intermediates GA12 and GA53 into GA9 and GA20, respectively (the "early 13-hydroxylation pathway" and "non 13-hydroxylation pathway"). The primary bioactive forms of GA include GA1, GA3 and GA4, which are further downstream of GA20 oxidase activity and the GA9 and GA20 intermediates in the biosynthetic pathway. A reduction or suppression of the expression level and/or enzymatic function of GA20 oxidase genes, as may be expected with the GA20ox3 inversion edits, may result in reduction of downstream metabolites (GA20 and GA9) and accumulation of upstream precursors (GA53 and GA12).

For this experiment, samples were collected as provided in Example 36 above. Freshly frozen plant sample tissues were extracted and cleaned using Waters solid phase extraction MAX cartridge plate. GA hormones and two internal standards were analyzed using UPLC coupled with an ABSciex 5500 Mass Spectrometry with MRM method. The final GA hormone values were calculated based on the calibration curve with ABSciex software Multi-Quan. Each GA hormone calibration curve was in good linear fit, the R2 linear regression was >0.99. The eight technical controls per 96-well plate for each hormone were also included and evaluated in analytical process for meeting the standard criterion. GA levels were measured in terms of pmol/gram of sample tissue.

As shown in FIG. 30, the levels of GA12 were increased in inbred plants homozygous for the edited allele (E270933 and E376333) but were statistically neutral or unchanged in hybrid plants heterozygous for the edited E270933 and E376333 alleles, relative to wild type control plants. As further shown in FIG. 30, the levels of GA9 were increased in inbred plants homozygous for one of the edited alleles (E270933) but neutral in inbred plants homozygous for the other edited allele (E376333), and the levels of GA9 were neutral (E270933) or decreased (E376333) in hybrid plants heterozygous for the edited allele, each relative to wild type control plants.

As shown in FIG. 31, the levels of GA20 were decreased in inbred plants homozygous for one of the edited alleles (E376333) but were increased in inbred plants homozygous for the other edited allele (E270933), and the levels of GA20 were neutral (E270933) or decreased (E376333) in hybrid plants heterozygous for these edited alleles, each relative to wild type control plants. As further shown in FIG. 31, the levels of GA53 were increased in inbred plants homozygous for each edited allele (E270933 and E376333) and in hybrid plants heterozygous for each edited allele (E270933 and E376333), relative to wild type control plants.

FIG. 32 provides the results for levels of active GAs (GA1, GA3 and GA4) measured in samples collected at V2 growth stage of the edited inbred and hybrid plants relative to wild type controls. As shown in FIG. 32, the levels of these active GAs were generally not statistically changed in the inbred and hybrid plants containing each of the edited alleles (E270933 and E376333), except for a small increase in GA4 in inbred plants homozygous for one of the edited alleles (E270933).

These data support the theory that the edited GA20 oxidase 3 gene containing an inversion sequence and encoding a transcript that may form a RNA stem-loop structure is able to affect the levels of GA hormones in inbred and hybrid plants containing the edited alleles. While the data in this experiment are mixed, there is support for increased accumulation of the GA12 and GA53 precursors upstream of GA20 oxidase activity and decreased levels of GA9 and GA20 products of GA20 oxidase activity in plants containing the edited GA20 oxidase 3 allele, although some samples had increased levels of the downstream GA9 and GA20 products. Greater support for decreased GA20 oxidase expression and/or activity with the edited GA20 oxidase 3 alleles is provided in this example by the accumulated levels of upstream GA12 and GA53 precursors. GA12 was neutral to increased in samples from plants with the edited GA20 oxidase 3 allele, and GA53 was increased across all samples from plants having the edited GA20 oxidase 3 allele.

Although the levels of bioactive GAs were not shown to be reduced in this example, this may be due to the early V2 growth stage when the plant tissue samples were collected. The pattern of expression from the endogenous GA20 oxidase 3 locus of transcripts containing the inversion, antisense or stem-loop sequence is also dependent on the endogenous GA20 oxidase 3 gene promoter, which may not drive expression (or expression at a sufficiently high level) at the V2 growth stage to produce a measurable effect on the levels of GA hormones. Without being bound by theory, it is possible that expression of an inversion/hairpin-containing transcript from an edited allele of an endogenous GA20ox3 or GA20ox5 gene under the control of the respective GA20ox3 or GA20ox5 endogenous promoter may be greater at later stages of development and thus have a greater effect on the level(s) of GA hormones at those later stages. The active GAs are also further downstream and not a direct product of GA20 oxidase activity. Future experiments will determine if lower active GA levels are observed at later stages of development in plants heterozygous or homozygous for an edited GA20 oxidase 3 or GA20 oxidase 5 allele. This is supported by the altered levels of GA precursors observed in this example at the early V2 growth stage.

Having described the present disclosure in detail, it will be apparent that modifications, variations, and equivalent aspects are possible without departing from the spirit and scope of the present disclosure as described herein and in the appended claims. Furthermore, it should be appreciated that all examples in the present disclosure are provided as non-limiting examples.
The following represent further embodiments of the present invention
Embodiment 1. A method of generating a dominant negative allele of a gene in a cell comprising inverting a portion of the gene using a targeted editing technique, generating an antisense RNA transcript capable of triggering suppression of an unmodified allele of the gene.
Embodiment 2. A method of generating a dominant negative allele of a gene in a cell comprising deleting a portion of a chromosome between a first gene region and a second gene region using a targeted editing technique, wherein an antisense RNA transcript of the first gene region is generated following the deletion of the portion of the chromosome.
Embodiment 3. A method of generating a dominant negative allele of a gene in one or more cells comprising:
   a) inducing a first double-stranded break and a second double-stranded break flanking a targeted region of the gene;
   b) identifying one or more cells comprising an inversion of the targeted region of the gene, wherein the inversion results in the production of an antisense RNA transcript from the targeted region; and
   c) selecting one or more cells comprising the inversion of the targeted region of the gene.
Embodiment 4. A method of reducing the expression of a protien in a cell comprising:
   a) inducing a first double-stranded break and a second double-stranded break at locations flanking a targeted region of a chromosome; and
   b) identifying one or more cells comprising an inversion in the targeted region of the chromosome, wherein expression of the protein is reduced as compared to a control cell that does not comprise the inversion in the targeted region.
Embodiment 5. A method comprising:
   a) identifying a chromosomal region comprising a first gene region comprising a first promoter and a first coding region, and a second gene region comprising a second promoter and a second coding region, wherein the first coding region and the second coding region are separated by an intervening region, and wherein the first promoter and the second promoter are positioned in opposite orientations;
   b) inducing a first double-stranded break and a second double-stranded break flanking a targeted region;
   c) identifying one or more cells comprising a deletion of the targeted region of the chromosome; and
   d) selecting one or more cells comprising the deletion of the targeted region of the chromosome.
Embodiment 6. A method of reducing the expression of a gene in at least one cell comprising:
   a) inducing a double-stranded break using a targeted editing technique at a target site of the gene;
   b) inserting a donor sequence at the double-stranded break, wherein the donor sequence comprises a tissue-specific or tissue-preferred promoter, and wherein the donor sequence is inserted into the target site such that the tissue-specific or tissue-preferred promoter is in reverse orientation as compared to the gene; and
   c) identifying at least one cell comprising the insertion of the donor sequence in reverse orientation, wherein expression of the gene is reduced as compared to a control cell that does not comprise the insertion of the donor sequence.
Embodiment 7. A method of modifying gene expression comprising:
   a) inducing a double-stranded break using a targeted editing technique at a target site:
   b) inserting a donor sequence at the double-stranded break, wherein the donor sequence comprises a designed element capable of inducing increased or ectopic expression of the gene; and
   c) identifying at least one cell comprising the donor sequence, wherein expression of the target gene is increased in at least one tissue as compared to a control cell that does not comprise the donor sequence.
Embodiment 8. A method of reducing expression of a gene in a cell comprising:
   a) identifying a chromosomal region comprising a first gene region comprising a first promoter and a first coding region, and a second gene region comprising a second promoter and a second coding region, wherein the first coding region and the second coding region are separated by an intervening region, and wherein the first promoter and the second promoter are positioned in opposite orientations;
   b) inducing a first double-stranded break and a second double-stranded break flanking a targeted region using a targeted editing technique, wherein the targeted region comprises the second coding region and the intervening region; and
   c) identifying one or more cells comprising a deletion of the targeted region, wherein the second promoter generates at least one antisense RNA of the first coding region, and wherein expression of the first coding region is reduced as compared to a control cell that does not comprise the deletion of the targeted region.
Embodiment 9. A method of generating an inversion in a targeted region of a gene comprising:
   a) providing to one or more cells at least one RNA-guided nuclease or one or more vectors encoding at least one RNA-guided nuclease,
      wherein the at least one RNA-guided nuclease is capable of binding to at least 18, at least 19, at least 20, at least 21, at least 22, at least 23, at least 24, at least 25, or at least 26 consecutive nucleotides of a first target site and a second target site flanking the targeted region of the gene,
      wherein the first target site and the second target site are linked,
      wherein the at least one RNA-guided nuclease creates double-stranded breaks in the gene at the first target site and the second target site;
   b) identifying one or more cells comprising an inversion in the targeted region of the gene, wherein the inversion results in the production of an antisense RNA transcript from the targeted region; and
   c) selecting one or more cells comprising the inversion in the targeted region of the gene.
Embodiment 10. A method comprising:
   a) identifying a chromosomal region comprising a first gene region comprising a first promoter and a first coding region, and a second gene region comprising a second promoter and a second coding region, wherein the first coding region and the second coding region are separated by an intervening region, and wherein the first promoter and the second promoter are positioned in opposite orientations;
   b) providing to one or more cells at least one RNA-guided nuclease or one or more vectors encoding at least one RNA-guided nuclease,
      wherein the at least one RNA-guided nuclease is capable of binding to at least 18, at least 19, at least 20, at least 21, at least 22, at least 23, at least 24, at least 25, or at least 26 consecutive nucleotides of a first target site and a second target site flanking a targeted region of a chromosome, wherein the targeted region comprises the second encoding region and the intervening region,
      wherein the RNA-guided nuclease creates double-stranded breaks in the chromosome at the first target site and the second target site;
   c) identifying one or more cells comprising a deletion of the targeted region; and
   d) selecting one or more cells comprising the deletion of the targeted region.
Embodiment 11. A method comprising:
   a) providing to one or more cells at least one RNA-guided nuclease and at least one donor molecule, or one or more vectors encoding at least one RNA-guided nuclease and at least one donor molecule,
      wherein the at least one RNA-guided nuclease is capable of binding to at least 18, at least 19, at least 20, at least 21, at least 22, at least 23, at least 24, at least 25, or at least 26 consecutive nucleotides of a target site of at least one gene, wherein the donor molecule comprises a designed element,
      wherein the RNA-guided nuclease creates a double-stranded break at the target site,
      and wherein the designed element is inserted at the double-stranded break;
   b) identifying one or more cells comprising an insertion of the designed element at the target site; and
   c) selecting one or more cells comprising the insertion of the designed element at the target site.
Embodiment 12. A method comprising:
   a) providing to one or more cells at least one RNA-guided nuclease and at least one donor molecule, or one or more vectors encoding at least one RNA-guided nuclease and at least one donor molecule,
      wherein the at least one RNA-guided nuclease is capable of binding to at least 18, at least 19, at least 20, at least 21, at least 22, at least 23, at least 24, at least 25, or at least 26 consecutive nucleotides of a target site of at least one gene, wherein the donor molecule comprises a sequence encoding a tissue-specific or tissue-preferred promoter,
      wherein the RNA-guided nuclease creates a double-stranded break at the target site, and
      wherein the sequence encoding the tissue-specific or tissue-preferred promoter is inserted at the double-stranded break;
   b) identifying one or more cells comprising the insertion of the sequence encoding the tissue-specific or tissue-preferred promoter at the target site such that the sequence encoding the tissue-specific or tissue-preferred promoter is in reverse orientation as compared to the gene; and
   c) selecting one or more cells comprising the insertion of the sequence encoding the tissue-specific or tissue-preferred promoter at the target site.
Embodiment 13. A method comprising:
   a) providing to one or more cells one or more RNA-guided nucleases or one or more vectors encoding one or more RNA nucleases, wherein the one or more RNA-guided nucleases are capable of binding to at least 18, at least 19, at least 20, at least 21, at least 22, at least 23, at least 24, at least 25, or at least 26 consecutive nucleotides of a target site, wherein the one or more RNA-guided nucleases create a double-stranded break at the target site:
   b) identifying at least one cell comprising an insertion or a deletion at the target site, wherein the insertion or deletion at the target site results in the generation of a dominant negative allele of the at least one gene; and
   c) selecting one or more cells comprising the dominant negative allele of the at least one gene.
Embodiment 14. A method comprising:
   a) generating a first double-stranded break (DSB) and a second DSB in a first allele of a gene in a cell using a targeted editing technique;
   b) generating a third DSB in a second allele of the gene in the cell using a targeted editing technique: and
   c) identifying a cell comprising an insertion of a region of the first allele in inverted orientation at the third DSB site in the second allele, thereby generating a modified second allele.
Embodiment 15. A method of generating a dominant negative allele of a gene comprising using a targeted editing technique to introduce at least one non-coding RNA target site into the gene.
Embodiment 16. A method of generating a dominant allele of a gene comprising using a targeted editing technique to introduce a nonsense mutation in the gene to create a truncated protein.
Embodiment 17. A method comprising:
   a) providing to a cell an engineered pentatricopeptide repeat (PPR) protein or a vector encoding the engineered PPR protein operably linked to a promoter, wherein the engineered PPR protein is capable of binding to an RNA transcript of a target gene;
   b) selecting one or more cells from step (a) expressing the engineered PPR protein; and
   c) identifying one or more cells selected in step (b) comprising altered expression of the target gene.
Embodiment 18. A method of generating a dominant negative allele of a gene in a cell comprising inserting an inverted copy of the gene, or a portion thereof, adjacent to a native copy of the gene using a targeted editing technique to generate an inverted repeat sequence capable of producing an antisense RNA transcript of the gene, or a portion thereof.
Embodiment 19. A method of generating a dominant negative allele of a gene in a cell comprising deleting a portion of a gene using a targeted editing technique, wherein a microprotein is generated following the deletion of the portion of the gene.
Embodiment 20. A method of generating a dominant negative allele of at least one gene in at least one cell comprising:
   a) introducing to the at least one cell a genome editing system comprising:
      i) a site-specific nuclease, or a molecule encoding a site-specific nuclease,
      ii) a single guide RNA (sgRNA), or a molecule encoding a sgRNA and
      iii) at least a first tether guided oligo (tgOligo) and a second tgOligo, or one or more molecules encoding a first and a second tgOligo,
      operably linked to at least one promoter;
   b) generating a first double-stranded break (DSB) and a second DSB in the at least one gene, wherein the first tgOligo and the second tgOligo hybridize to the 3' free ends of opposing strands at the first DSB and second DSB, wherein at least 1, at least 2, at least 3, at least 4, at least 5, at least 10, at least 25, at least 50, at least 100, at least 250, at least 500, at least 750, at least 1000, at least 2500, or at least 5000 nucleotides of the at least one gene are deleted, thereby generating a dominant negative allele of the gene that encodes a truncated protein: and
   c) identifying and selecting at least one cell comprising the truncated protein.
Embodiment 21. A modified plant cell comprising a non-transposon mediated genome deletion or inversion of a gene, or a portion thereof, at the endogenous locus of the gene, wherein the deletion or inversion results in the production of an RNA transcript comprising a sequence complementary to a native transcript sequence of the gene, or portion thereof.
Embodiment 22. A modified chromosome comprising a non-transposon mediated deletion or inversion of a gene or, a portion thereof, at the endogenous locus of the gene, wherein the deletion or inversion results in the production of an RNA transcript comprising a sequence complementary to a native transcript sequence of the gene, or portion thereof.
Embodiment 23. A modified cell comprising the modified chromosome of Embodiment 22.
Embodiment 24. A modified plant or modified plant tissue regenerated from the modified plant cell of Embodiment 21.
Embodiment 25. A product comprising the modified chromosome of Embodiment 22.
Embodiment 26. A product comprising the modified cell of Embodiment 23.
Embodiment 27. A modified plant, or part thereof, comprising a non-transposon mediated genome deletion or inversion of a gene, or a portion thereof, at the endogenous locus of the gene, wherein the deletion or inversion results in the production of an RNA transcript comprising a sequence complementary to a native transcript sequence of the gene, or portion thereof.
Embodiment 28. A product comprising the modified plant, or part thereof, of Embodiment 27.
Embodiment 29. A modified cell comprising a) a non-transposon mediated genome deletion of at least one gene, or a portion thereof, at the endogenous locus of the at least one gene, or b) a non-transposon mediated and non-T-DNA mediated insertion of a polynucleotide sequence into the at least one gene, wherein the deletion or insertion creates a dominant positive allele of the at least one gene.
Embodiment 30. A modified cell comprising a non-transposon mediated genome deletion or inversion of a gene, or a portion thereof, at the endogenous locus of the at least one gene, wherein the deletion or inversion results in the production of an RNA transcript comprising a sequence complementary to a native transcript sequence of the gene.
Embodiment 31. A modified cell comprising a targeted edit of at least one gene or a portion thereof, wherein the targeted edit generates an RNA transcript that is complementary to a native transcript sequence of the gene.
Embodiment 32. A modified cell comprising at least one dominant negative allele of at least one gene generated by a targeted editing technique, wherein the allele generates an RNA transcript capable of forming a hairpin loop secondary structure when the at least one dominant negative allele is transcribed.
Embodiment 33. A modified cell comprising a non-transgenic dominant negative allele of a gene, said dominant negative allele comprising a heterologous non-coding RNA target site in the endogenous locus of the gene.
Embodiment 34. A modified cell comprising at least one insertion or deletion at the endogenous locus of the at least one gene generated by a targeted editing technique, wherein the insertion or deletion results in expression of a truncated protein.
Embodiment 35. A modified cell comprising a dominant negative allele of at least one gene comprising an inverted copy of the gene adjacent to a native copy of the gene at the endogenous locus of the gene.

## Claims

1. A method of generating a dominant negative allele of a gene in a cell or of reducing the expression of a protein in a cell comprising inserting or inverting a portion of the gene using a targeted editing technique, generating an antisense RNA transcript capable of triggering suppression of an unmodified allele of the gene.

2. The method of generating a dominant negative allele of a gene according to claim 1 comprising:
a) inducing a first double-stranded break and a second double-stranded break flanking a targeted region of the gene;
b) identifying one or more cells comprising an inversion of the targeted region of the gene, wherein the inversion results in the production of an antisense RNA transcript from the targeted region; and
c) selecting one or more cells comprising the inversion of the targeted region of the gene.

3. The method of reducing the expression of a protein in a cell according to claim 1 comprising:
a) inducing a first double-stranded break and a second double-stranded break at locations flanking a targeted region of a chromosome; and
b) identifying one or more cells comprising an inversion in the targeted region of the chromosome, wherein expression of the protein is reduced as compared to a control cell that does not comprise the inversion in the targeted region.

4. The method of claim 1, wherein generating an inversion in a targeted region of a gene comprises:
a) providing to one or more cells at least one RNA-guided nuclease or one or more vectors encoding at least one RNA-guided nuclease,
wherein the at least one RNA-guided nuclease is capable of binding to at least 18, at least 19, at least 20, at least 21, at least 22, at least 23, at least 24, at least 25, or at least 26 consecutive nucleotides of a first target site and a second target site flanking the targeted region of the gene,
wherein the first target site and the second target site are linked,
wherein the at least one RNA-guided nuclease creates double-stranded breaks in the gene at the first target site and the second target site;
b) identifying one or more cells comprising an inversion in the targeted region of the gene, wherein the inversion results in the production of an antisense RNA transcript from the targeted region; and
c) selecting one or more cells comprising the inversion in the targeted region of the gene.

5. The method according to claim 1, comprising:
a) providing to one or more cells one or more RNA-guided nucleases or one or more vectors encoding one or more RNA nucleases, wherein the one or more RNA-guided nucleases are capable of binding to at least 18, at least 19, at least 20, at least 21, at least 22, at least 23, at least 24, at least 25, or at least 26 consecutive nucleotides of a target site, wherein the one or more RNA-guided nucleases create a double-stranded break at the target site;
b) identifying at least one cell comprising an insertion at the target site, wherein the insertion or deletion at the target site results in the generation of a dominant negative allele of the at least one gene; and
c) selecting one or more cells comprising the dominant negative allele of the at least one gene.

6. The method according to claim 1, wherein the unmodified allele encodes a protein having at least 95% sequence identity to a sequence selected from the group consisting of SEQ ID NO: 30 and SEQ ID NO:34.

7. The method according to claim 1, wherein the unmodified allele comprises a polynucleotide sequence having at least 95% identity or complementarity to a sequence selected from the group consisting of SEQ ID NOs: 1, 12, 27, 28, 29, 31, 32 and 33.

8. A method of generating a dominant allele of a gene comprising using a targeted editing technique to introduce a nonsense mutation in the gene to create a truncated protein.

9. A modified plant cell, or modified chromosome or modified plant, or part thereof comprising a non-transposon mediated genome insertion or inversion of a gene, or a portion thereof, at the endogenous locus of the gene, wherein the insertion or inversion results in the production of an RNA transcript comprising a sequence complementary to a native transcript sequence of the gene, or portion thereof.

10. The modified cell according to claim 9, comprising a) a non-transposon mediated genome inversion of at least one gene, or a portion thereof, at the endogenous locus of the at least one gene, or b) a non-transposon mediated and non-T-DNA mediated insertion of a polynucleotide sequence into the at least one gene, wherein the inversion or insertion creates a dominant positive allele of the at least one gene.

11. The modified cell according to claim 9, comprising a non-transposon mediated genome inversion of a gene, or a portion thereof, at the endogenous locus of the at least one gene, wherein the inversion results in the production of an RNA transcript comprising a sequence complementary to a native transcript sequence of the gene.

12. The modified cell according to claim 9, comprising a targeted edit of at least one gene or a portion thereof, wherein the targeted edit generates an RNA transcript that is complementary to a native transcript sequence of the gene.

13. The modified cell according to claim 9, comprising at least one dominant negative allele of at least one gene generated by a targeted editing technique, wherein the allele generates an RNA transcript capable of forming a hairpin loop secondary structure when the at least one dominant negative allele is transcribed.

14. The modified cell according to claim 9, comprising at least one insertion or deletion at the endogenous locus of the at least one gene generated by a targeted editing technique, wherein the insertion or deletion results in expression of a truncated protein.
